# EUROPEAN PATENT SPECIFICATION

(11) **EP 4 558 492 B1**
(45) Date of publication and mention of the grant of the patent: **16.09.2026**
(21) Application number: 23761334.4
(22) Date of filing: 20.07.2023
(51) Int. Cl.: C07D 401/04, C07D 403/14, C07D 405/14, C07D 413/14, C07D 417/14, C07D 471/04, C07D 487/04, C07D 498/04, A61P 25/00, A61P 25/14, A61P 25/16, A61P 25/28, A61P 29/00, A61P 35/00, A61P 35/02, A61P 37/00, A61K 31/444, A61K 31/437, A61K 31/4439, A61K 31/4375

(54) **3-(6-PYRIDIN-3-YL)-2-[4-(4-METHYL-4H-1,2,4-TRIAZOL-3-YL)PIPERIDIN-1-YL]BENZONITRILE DERIVATIVES AND SIMILAR COMPOUNDS AS QPCTL AND QPCT INHIBITORS FOR THE TREATMENT OF CANCER**
3-(6-PYRIDIN-3-YL)-2-[4-(4-METHYL-4H-1,2,4-TRIAZOL-3-YL)PIPERIDIN-1-YL]BENZONITRIL-DERIVATE UND ÄHNLICHE VERBINDUNGEN ALS QPCTL UND QPCT INHIBITOREN ZUR BEHANDLUNG VON KREBS
DÉRIVÉS DE 3-(6-PYRIDIN-3-YL)-2-[4-(4-MÉTHYL-4H-1,2,4-TRIAZOL-3-YL)PIPÉRIDIN-1-YL]BENZONITRILE ET COMPOSÉS SIMILAIRES EN TANT QU'INHIBITEURS DE QPCTL ET QPCT POUR LE TRAITEMENT DU CANCER

(30) Priority: 22.07.2022 US 202263391630 P
(43) Date of publication of application: 28.05.2025
(73) Proprietor: 858 Therapeutics, Inc., San Diego, CA 92121 (US)
(72) Inventor: VEAL, James M., San Diego, California 92121 (US); STAFFORD, Jeffrey A., San Diego, California 92121 (US); KARANEWSKY, Donald S., San Diego, California 92121 (US); HERATH, Shyama, San Diego, California 92121 (US)
(74) Representative: Secerna LLP
(86) International application number: PCT/US2023/070646
(87) International publication number: WO 2024/020517

(56) References cited:
- WO-A1-2022/086920

## Description

### BACKGROUND

Glutaminyl cyclases belong to the family of metal-dependent aminoacyltransferases and catalyze the intramolecular cyclization of peptide or protein N-terminal glutamine or glutamate amino acid residues to pyroglutamate (pE). Glutaminyl cyclases utilize a zinc-dependent catalytic mechanism to form the pyroglutamate residue and liberate ammonia. The pyroglutamate modification is often important for biological activity as it may protect the protein from degradation by proteolytic enzymes or modulate the interactions of the protein with other proteins. (W. Busby et al., J Biol Chem. 262 (18), 8532 (1987); W. Fischer and J Spiess, Pro Natl Acad Sci U.S.A. 84, 3628 (1987); A. Stephan et al., FEBS J. 276, 6522 (2009).

Two human glutaminyl cyclase isoforms have been identified. The first isoform, QPCT (also known as QC, sQC), is secreted. The second isoform QPCTL (also known as isoQC) contains an N-terminal sequence that leads to its retention within the Golgi complex. QPCT and QPCTL share high sequence similarity and structure in the region of their active sites. However, they differ in their cellular distribution and thus convert different substrates leading to distinct physiological roles. QPCT is abundant in the hypothalamus, medulla, and hippocampus, and the majority of the glutaminyl cyclase activity in the brain is mediated by QPCT. QPCTL is more broadly expressed and acts on substrates, such as cytokines, in peripheral cells. (H. Cynis et al., J Mol Bio. 379 (5), 966 (2008); S. Schilling et al., J Biol Chem. 286 (16), 14199 (2011).

Both QPCT and QPCTL are strongly implicated in disease pathology. For example, QPCT modifies amyloid-beta (Aβ) peptides to yield pE-Aβ. The pE-modification alters the biophysical characteristics of Aβ peptides by increasing their aggregation behavior. pE-Aβ has been shown to be one of the major constituents of Aβ deposits in patients with Alzheimer's disease (AD) and has been reported to trigger neurotoxic events in the pathogenesis of AD (K. Liu et al., Acta Neuropathol. 112 (2), 163 (2006); J. Nussbaum et al., Nature. 485, 7400 (2012) 651). QPCTL catalyzes the formation of pE on the integrin-associated transmembrane protein, CD47, which enhances its interaction with the regulatory membrane glycoprotein, SIRPα. CD47 expression is increased on tumor cells and the CD47-SIRPα interaction provides cancer cells with a phagocytosis checkpoint that enables their escape from immune surveillance (M. Logtenberg et al., Nat Med. 25, 612 (2019); Z. Wu et al., Cell Res. 29, 502 (2019)). QPCTL also catalyzes pE formation on chemotactic cytokines such as CCL2 and related family members, which protect them from proteolytic degradation. CCL2 regulates migration and infiltration of monocytes with a pivotal role in inflammatory conditions. CCL2 also enables recruitment of monocytes to the tumor microenvironment where they become tumor associated macrophages (TAMs) that support the growth and survival of the associated tumor cells (H. Cynis et al., EMBO Mol Med. 3, 545 (2011); R. Barreira da Silva et al., Nat Immun. 23, 568 (2022)).

WO 2022/086920 A1 discloses glutaminyl-peptide cyclotransferase like (QPCTL) inhibitors for the treatment of cancer.

Therefore, inhibiting either QPCT or QPCTL provides a therapeutic approach for treating disorders such as neurological diseases, cancer, and inflammation. Thus, there is a need for compounds that can modulate or inhibit QPCT and QPCTL.

### SUMMARY

The present disclosure provides in various embodiments a compound of formula (II) or a pharmaceutically acceptable salt and/or solvate thereof: 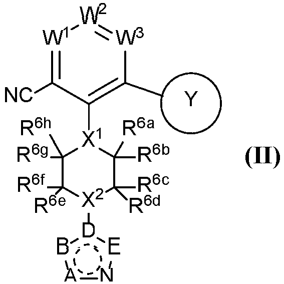
W¹ is N or CR¹, W² is N or CR², and W³ is N or CR³; wherein no more than one of W¹, W², and W³ is N;
X¹ and X² are independently selected from CR⁴ and N.

In some embodiments, Ring Y 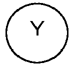 is of formula (a):

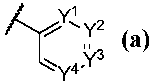

In formula (a), Y¹, Y², Y³, and Y⁴ are independently selected from CR⁵ and N wherein Y¹, Y², Y³, and Y⁴ are not simultaneously N.

In other embodiments, Ring Y 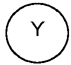 is of formula (b):

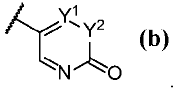

In formula (b), Y¹ is CR⁵ and Y² is NR^{5'}.

In still other embodiments, Ring Y 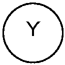 is of formula (c):

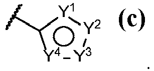

In formula (c), Y¹, Y², Y³, and Y⁴ are independently selected from CR⁵ and N.

In some embodiments, either Y¹ and Y², or Y² and Y³, or Y³ and Y⁴ represent a fused ring selected from a C₅-C₈-cycloalkyl, a C₆-C₁₀-aryl, a 5- to 10-membered heteroaryl (wherein 1-4 heteroaryl members are independently selected from N, O, and S), and a 5- to 8-membered heterocycloalkyl (wherein 1-4 ring members are independently selected from N, B, O, and S), wherein the ring is optionally substituted with 1 to 3 substituents independently selected from the group consisting of C₁-C₆-alkyl, C₃-C₈-cycloalkyl, 3- to 6-membered heterocycloalkyl (wherein 1-4 ring members are independently selected from N, O, and S), C₂-C₆-alkenyl, C₂-C₆-alkynyl, halo, C₁-C₆-haloalkyl, C₂-C₆-haloalkenyl, C₂-C₆-haloalkynyl, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2).

A, B, and E are independently selected from C, N, O, and S, and D is C or N.

The symbol 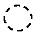 represents the presence of double bonds such that the ring A-B-D-EN is aromatic and is optionally substituted with one or two substituents independently selected from C₁-C₃-alkyl, C₃-C₅-cycloalkyl, OH, OMe, NH₂, N(H)Me, NMe₂. Further, no more than two of A, B, D, and E are simultaneously N, O, or S.

R¹, R², and R³ are independently selected from the group consisting of hydrogen, halo, cyano, nitro, -R^{b}-OR^{a}, -R^{b}-O-R^{c}-O-R^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})_{2,} -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₁-C₆-haloalkyl, C₂-C₆-alkenyl, C₂-C₆-alkynyl, C₃-C₈-cycloalkyl, C₆-C₁₀-aryl, -(C₁-C₆-alkyl)(C₆-C₁₀-aryl), 5- to 10-membered heteroaryl (wherein 1-4 heteroaryl members are independently selected from N, O, and S) optionally fused to 3- to 6-membered heterocycloalkyl (wherein 1-4 ring members are independently selected from N, O, and S), and 3- to 6-membered heterocycloalkyl (wherein 1-4 ring members are independently selected from N, O, and S.

In some embodiments, R¹ and R², or R² and R³, together with the carbon atoms to which they are bound, form a fused C₅-C₈-cycloalkyl, C₆-C₁₀-aryl, 5- to 10-membered heteroaryl (wherein 1-4 heteroaryl members are independently selected from N, O, and S), and 5- to 8-membered heterocycloalkyl (wherein 1-4 ring members are independently selected from N, O, and S).

Any heteroaryl or heterocycloalkyl in R¹, R², and R³ is optionally and independently substituted with 1 to 3 substituents selected from the group consisting of C₁-C₆-alkyl, halo, hydroxy, C₃-C₈-cycloalkyl, heterocycloalkyl (wherein 1-4 ring members are independently selected from N, O, and S), and -R^{b}-N(R^{a})₂.

R⁴ in each instance is independently H, OH, halo, C₁-C₆-alkyl, or C₁-C₆-alkoxy.

R⁵ in each instance is independently selected from the group consisting of hydrogen, halo, cyano, nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -B(OR^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-OS(O)ₜF (where t is 1 or 2), -R^{b}-S(O)ₜN(R^{a})₂ (where t is 1 or 2), C₁-C₆-alkyl, C₂-C₆-alkynyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S).

R^{5'} is selected from the group consisting of hydrogen, -R^{c}-R^{a}, -R^{c}-OR^{a}, -R^{b}-OC(O)-R^{a}, -R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S).

Any heteroaryl or heterocycloalkyl in R⁵ and R^{5'} is optionally and independently substituted with 1 to 3 substituents selected from the group consisting of C₁-C₆-alkyl, halo, hydroxy, C₃-C₈-cycloalkyl, and -R^{b}-N(R^{a})₂.

R^{6a}, R^{6b}, R^{6c}, R^{6d}, R^{6e}, R^{6f}, R^{6g}, and R^{6h} are independently selected from the group consisting of H, halo, NO₂, OH, CN, -R^{b}-N(R^{a})₂, -R^{b}-OH, C₁-C₆-alkyl, and C₁-C₆-alkoxy.

In some embodiments optionally in combination with any other embodiment described herein, R^{6a} and R^{6b}, or R^{6c} and R^{6d}, or R^{6e} and R^{6f}, or R^{6g} and R^{6h} independently represent oxo, thioxo, imino, or oximo.

In still further embodiments optionally in combination with any other embodiment described herein, R^{6a} and R^{6b}, or R^{6c} and R^{6d}, or R^{6e} and R^{6f}, or R^{6g} and R^{6h}, together with the carbon atoms to which they are bound, independently combine to form a fused ring selected from a C₃-C₆-cycloalkyl and C₃-C₆-heterocycloalkyl (wherein 1-4 ring members are independently selected from N, O, and S).

In additional embodiments optionally in combination with any other embodiment described herein, one of R^{6c} and R^{6d} together with one of R^{6e} and R^{6f} represent a bond between the ring carbon members to which they are bound.

R^{a} in each instance is independently selected from hydrogen, C₁-C₆-alkyl, C₃-C₈-cycloalkyl, -(C₁-C₆-alkyl)(C₃-C₈-cycloalkyl), C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S).

R^{b} in each instance is independently selected from a direct bond, a straight or branched C₂-C₆-alkylene, and C₂-C₆-alkenylene chain.

Any heteroaryl or heterocycloalkyl in R^{a} and R^{b} is optionally and independently substituted with 1 to 3 substituents selected from the group consisting of C₁-C₆-alkyl, halo, hydroxy,

R^{c} in each instance is independently selected from a straight or branched C₂-C₆-alkylene and C₂-C₆-alkenylene chain.

In another embodiment, the present disclosure provides a pharmaceutical composition comprising a compound or pharmaceutically acceptable salt and/or solvate thereof as described herein and a pharmaceutically acceptable carrier.

The present disclosure also provides, in additional embodiments, cormpounds for use in a method of treating a disease in a patient suffering therefrom, wherein the disease is associated with expression of glutaminyl-peptide cyclotransferase protein (QPCT) or glutaminyl-peptide cyclotransferase-like protein (QPCTL). The method comprises administering to the patient a compound or pharmaceutically acceptable salt and/or solvate thereof as described herein.

In still another embodiment, the present disclosure provides cormpounds for use in method of inhibiting a glutaminyl-peptide cyclotransferase (QPCT) or glutaminyl-peptide cyclotransferase-like (QPCTL) enzyme. The method comprises contacting the enzyme with a compound or pharmaceutically acceptable salt and/or solvate thereof as described herein.

In embodiments, the present disclosure provides a compound or pharmaceutically acceptable salt and/or solvate thereof as described herein for use in the treatment of a cancer, neurodegenerative disease, inflammatory disease, or autoimmune disease.

The present disclosure also provides a use of a compound or pharmaceutically acceptable salt and/or solvate thereof as described herein in the manufacture of a medicament for the treatment of a cancer, neurodegenerative disease, inflammatory disease, or autoimmune disease.

### BRIEF DESCRIPTION OF THE DRAWINGS

FIG. 1. X-ray crystal structure of reference compound SEN177 bound to QPCTL.
FIG. 2. X-ray crystal structure of Example 1 bound to QPCTL.
FIG. 3. Overlay of X-ray crystal structures of QPCTL with SEN177 and Example 1, respectively.

### DETAILED DESCRIPTION

The present disclosure provides compounds of formula (I) and formula (II) that are potent inhibitors of the QPCTL and QPCT enzymes. The compounds are useful in the treatment of diseases and conditions that are associated with the expression of QPCTL or QPCT, including various cancers and neurodegenerative diseases.

### Definitions

As used herein and in the appended claims, the singular forms "a," "and," and "the" include plural referents unless the context clearly dictates otherwise. Thus, for example, reference to "an agent" includes a plurality of such agents, and reference to "the cell" includes reference to one or more cells (or to a plurality of cells) and equivalents thereof known to those skilled in the art, and so forth. When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range, in some instances, will vary between 1% and 15% of the stated number or numerical range. The term "comprising" (and related terms such as "comprise" or "comprises" or "having" or "including") is not intended to exclude that in other certain embodiments, for example, an embodiment of any composition of matter, composition, method, or process, or the like, described herein, "consist of" or "consist essentially of" the described features.

As used in the specification and appended claims, unless specified to the contrary, the following terms have the meaning indicated below.

"Amino" refers to the -NH₂ moiety.

"Cyano" refers to the -CN moiety.

"Nitro" refers to the -NO₂ moiety.

"Oxa" refers to the -O- moiety.

"Oxo" refers to the =O moiety.

"Thioxo" refers to the =S moiety.

"Imino" refers to the =N-H moiety.

"Oximo" refers to the =N-OH moiety.

"Hydrazino" refers to the =N-NH₂ moiety.

"Alkyl" refers to a straight or branched hydrocarbon chain radical consisting solely of carbon and hydrogen atoms, containing no unsaturation, having from one to fifteen carbon atoms (e.g., C₁-C₁₅ alkyl). In certain embodiments, an alkyl comprises one to thirteen carbon atoms (e.g., C₁-C₁₃ alkyl). In certain embodiments, an alkyl comprises one to eight carbon atoms (e.g., C₁-C₈ alkyl). In other embodiments, an alkyl comprises one to five carbon atoms (e.g., C₁-C₅ alkyl). In other embodiments, an alkyl comprises one to four carbon atoms (e.g., C₁-C₄ alkyl). In other embodiments, an alkyl comprises one to three carbon atoms (e.g., C₁-C₃ alkyl). In other embodiments, an alkyl comprises one to two carbon atoms (e.g., C₁-C₂ alkyl). In other embodiments, an alkyl comprises one carbon atom (e.g., C₁ alkyl). In other embodiments, an alkyl comprises five to fifteen carbon atoms (e.g., C₅-C₁₅ alkyl). In other embodiments, an alkyl comprises five to eight carbon atoms (e.g., C₅-C₈ alkyl). In other embodiments, an alkyl comprises two to five carbon atoms (e.g., C₂-C₅ alkyl). In other embodiments, an alkyl comprises three to five carbon atoms (e.g., C₃-C₅ alkyl). In other embodiments, the alkyl group is selected from methyl, ethyl, 1-propyl (n-propyl), 1-methylethyl (iso-propyl), 1-butyl (n-butyl), 1-methylpropyl (sec-butyl), 2-methylpropyl (isobutyl), 1,1-dimethylethyl (tert-butyl), 1-pentyl (n-pentyl). The alkyl is attached to the rest of the molecule by a single bond. An alkyl group may be optionally substituted by one or more of the following substituents: halo, cyano, nitro, oxo, thioxo, imino, oximo, trimethylsilanyl, -OR^{a}, -SR^{a}, -OC(O)-R^{a}, -N(R^{a})2, - C(O)R^{a}, -C(O)OR^{a}, -C(O)N(R^{a})₂, -N(R^{a})C(O)OR^{a}, -OC(O)-N(R^{a})₂, -N(R^{a})C(O)R^{a}, - N(R^{a})S(O)ₜR^{a} (where t is 1 or 2), -S(O)ₜOR^{a} (where t is 1 or 2), -S(O)ₜR^{a} (where t is 1 or 2) and -S(O)ₜN(R^{a})2 (where t is 1 or 2) where each R^{a} is independently hydrogen, alkyl (optionally substituted with halogen, hydroxy, methoxy, or trifluoromethyl), fluoroalkyl, carbocyclyl (optionally substituted with halogen, hydroxy, methoxy, or trifluoromethyl), carbocyclylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl). In certain embodiments, an optionally substituted alkyl is a haloalkyl. In other embodiments, an optionally substituted alkyl is a fluoroalkyl. In other embodiments, an optionally substituted alkyl is a -CF₃ group.

"Alkoxy" refers to a radical bonded through an oxygen atom of the formula -O-alkyl, where alkyl is an alkyl chain as defined above.

"Alkenyl" refers to a straight or branched hydrocarbon chain radical group consisting solely of carbon and hydrogen atoms, containing at least one carbon-carbon double bond, and having from two to twelve carbon atoms. In certain embodiments, an alkenyl comprises two to eight carbon atoms. In other embodiments, an alkenyl comprises two to four carbon atoms. The alkenyl is attached to the rest of the molecule by a single bond, for example, ethenyl (i.e., vinyl), prop-1-enyl (i.e., allyl), but-1-enyl, pent-1-enyl, penta-1,4-dienyl, and the like. An alkenyl group may be optionally substituted by one or more of the following substituents: halo, cyano, nitro, oxo, thioxo, imino, oximo, trimethylsilanyl, -OR^{a}, -SR^{a}, -OC(O)-R^{a}, -N(R^{a})2, -C(O)R^{a}, -C(O)OR^{a}, - C(O)N(R^{a})₂, -N(R^{a})C(O)OR^{a}, -OC(O)-N(R^{a})₂, -N(R^{a})C(O)R^{a}, -N(R^{a})S(O)ₜR^{a} (where t is 1 or 2), -S(O)ₜOR^{a} (where t is 1 or 2), -S(O)ₜR^{a} (where t is 1 or 2) and -S(O)ₜN(R^{a})₂ (where t is 1 or 2) where each R^{a} is independently hydrogen, alkyl (optionally substituted with halogen, hydroxy, methoxy, or trifluoromethyl), fluoroalkyl, carbocyclyl (optionally substituted with halogen, hydroxy, methoxy, or trifluoromethyl), carbocyclylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl).

"Alkynyl" refers to a straight or branched hydrocarbon chain radical group consisting solely of carbon and hydrogen atoms, containing at least one carbon-carbon triple bond, having from two to twelve carbon atoms. In certain embodiments, an alkynyl comprises two to eight carbon atoms. In other embodiments, an alkynyl comprises two to six carbon atoms. In other embodiments, an alkynyl comprises two to four carbon atoms. The alkynyl is attached to the rest of the molecule by a single bond, for example, ethynyl, propynyl, butynyl, pentynyl, hexynyl, and the like. An alkynyl group may be optionally substituted by one or more of the following substituents: halo, cyano, nitro, oxo, thioxo, imino, oximo, trimethylsilanyl, -OR^{a}, -SR^{a}, -OC(O)-R^{a}, -N(R^{a})₂, - C(O)R^{a}, -C(O)OR^{a}, -C(O)N(R^{a})₂, -N(R^{a})C(O)OR^{a}, -OC(O)-N(R^{a})₂, -N(R^{a})C(O)R^{a}, - N(R^{a})S(O)ₜR^{a} (where t is 1 or 2), -S(O)ₜOR^{a} (where t is 1 or 2), -S(O)ₜR^{a} (where t is 1 or 2) and -S(O)ₜN(R^{a})₂ (where t is 1 or 2) where each R^{a} is independently hydrogen, alkyl (optionally substituted with halogen, hydroxy, methoxy, or trifluoromethyl), fluoroalkyl, carbocyclyl (optionally substituted with halogen, hydroxy, methoxy, or trifluoromethyl), carbocyclylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl).

"Alkylene" or "alkylene chain" refers to a straight or branched divalent hydrocarbon chain linking the rest of the molecule to a radical group, consisting solely of carbon and hydrogen, containing no unsaturation, and having from one to twelve carbon atoms, for example, methylene, ethylene, propylene, n-butylene, and the like. The alkylene chain is attached to the rest of the molecule through a single bond and to the radical group through a single bond. The points of attachment of the alkylene chain to the rest of the molecule and to the radical group are through one carbon in the alkylene chain or through any two carbons within the chain. In certain embodiments, an alkylene comprises one to eight carbon atoms (e.g., C₁-C₈ alkylene). In other embodiments, an alkylene comprises one to five carbon atoms (e.g., C₁-C₅ alkylene). In other embodiments, an alkylene comprises one to four carbon atoms (e.g., C₁-C₄ alkylene). In other embodiments, an alkylene comprises one to three carbon atoms (e.g., C₁-C₃ alkylene). In other embodiments, an alkylene comprises one to two carbon atoms (e.g., C₁-C₂ alkylene). In other embodiments, an alkylene comprises one carbon atom (e.g., C₁ alkylene). In other embodiments, an alkylene comprises five to eight carbon atoms (e.g., C₅-C₈ alkylene). In other embodiments, an alkylene comprises two to five carbon atoms (e.g., C₂-C₅ alkylene). In other embodiments, an alkylene comprises three to five carbon atoms (e.g., C₃-C₅ alkylene). An alkylene group may be optionally substituted by one or more of the following substituents: halo, cyano, nitro, oxo, thioxo, imino, oximo, trimethylsilanyl, -OR^{a}, -SR^{a}, - OC(O)-R^{a}, -N(R^{a})₂, -C(O)R^{a}, -C(O)OR^{a}, -C(O)N(R^{a})₂, -N(R^{a})C(O)OR^{a}, -OC(O)-N(R^{a})₂, - N(R^{a})C(O)R^{a}, -N(R^{a})S(O)ₜR^{a} (where t is 1 or 2), -S(O)ₜOR^{a} (where t is 1 or 2), -S(O)ₜR^{a} (where t is 1 or 2) and -S(O)ₜN(R^{a})₂ (where t is 1 or 2) where each R^{a} is independently hydrogen, alkyl (optionally substituted with halogen, hydroxy, methoxy, or trifluoromethyl), fluoroalkyl, carbocyclyl (optionally substituted with halogen, hydroxy, methoxy, or trifluoromethyl), carbocyclylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl).

"Alkenylene" or "alkenylene chain" refers to a straight or branched divalent hydrocarbon chain linking the rest of the molecule to a radical group, consisting solely of carbon and hydrogen, containing at least one carbon-carbon double bond, and having from two to twelve carbon atoms. The alkenylene chain is attached to the rest of the molecule through a single bond and to the radical group through a single bond. In certain embodiments, an alkenylene comprises two to eight carbon atoms (e.g., C₂-C₈ alkenylene). In other embodiments, an alkenylene comprises two to five carbon atoms (e.g., C₂-C₅ alkenylene). In other embodiments, an alkenylene comprises two to four carbon atoms (e.g., C₂-C₄ alkenylene). In other embodiments, an alkenylene comprises two to three carbon atoms (e.g., C₂-C₃ alkenylene). In other embodiments, an alkenylene comprises two carbon atoms (e.g., C₂ alkenylene). In other embodiments, an alkenylene comprises five to eight carbon atoms (e.g., C₅-C₈ alkenylene). In other embodiments, an alkenylene comprises three to five carbon atoms (e.g., C₃-C₅ alkenylene). An alkenylene group may be optionally substituted by one or more of the following substituents: halo, cyano, nitro, oxo, thioxo, imino, oximo, trimethylsilanyl, -OR^{a}, -SR^{a}, - OC(O)-R^{a}, -N(R^{a})₂, -C(O)R^{a}, -C(O)OR^{a}, -C(O)N(R^{a})₂, -N(R^{a})C(O)OR^{a}, -OC(O)-N(R^{a})₂, - N(R^{a})C(O)R^{a}, -N(R^{a})S(O)ₜR^{a} (where t is 1 or 2), -S(O)ₜOR^{a} (where t is 1 or 2), -S(O)ₜR^{a} (where t is 1 or 2) and -S(O)ₜN(R^{a})₂ (where t is 1 or 2) where each R^{a} is independently hydrogen, alkyl (optionally substituted with halogen, hydroxy, methoxy, or trifluoromethyl), fluoroalkyl, carbocyclyl (optionally substituted with halogen, hydroxy, methoxy, or trifluoromethyl), carbocyclylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl).

"Alkynylene" or "alkynylene chain" refers to a straight or branched divalent hydrocarbon chain linking the rest of the molecule to a radical group, consisting solely of carbon and hydrogen, containing at least one carbon-carbon triple bond, and having from two to twelve carbon atoms. The alkynylene chain is attached to the rest of the molecule through a single bond and to the radical group through a single bond. In certain embodiments, an alkynylene comprises two to eight carbon atoms (e.g., C₂-C₈ alkynylene). In other embodiments, an alkynylene comprises two to five carbon atoms (e.g., C₂-C₅ alkynylene). In other embodiments, an alkynylene comprises two to four carbon atoms (e.g., C₂-C₄ alkynylene). In other embodiments, an alkynylene comprises two to three carbon atoms (e.g., C₂-C₃ alkynylene). In other embodiments, an alkynylene comprises two carbon atoms (e.g., C₂ alkynylene). In other embodiments, an alkynylene comprises five to eight carbon atoms (e.g., C₅-C₈ alkynylene). In other embodiments, an alkynylene comprises three to five carbon atoms (e.g., C₃-C₅ alkynylene). An alkynylene group may be optionally substituted by one or more of the following substituents: halo, cyano, nitro, oxo, thioxo, imino, oximo, trimethylsilanyl, -OR^{a}, -SR^{a}, -OC(O)-R^{a}, -N(R^{a})₂, - C(O)R^{a}, -C(O)OR^{a}, -C(O)N(R^{a})₂, -N(R^{a})C(O)OR^{a}, -OC(O)-N(R^{a})₂, -N(R^{a})C(O)R^{a}, - N(R^{a})S(O)ₜR^{a} (where t is 1 or 2), -S(O)ₜOR^{a} (where t is 1 or 2), -S(O)ₜR^{a} (where t is 1 or 2) and -S(O)ₜN(R^{a})₂ (where t is 1 or 2) where each R^{a} is independently hydrogen, alkyl (optionally substituted with halogen, hydroxy, methoxy, or trifluoromethyl), fluoroalkyl, carbocyclyl (optionally substituted with halogen, hydroxy, methoxy, or trifluoromethyl), carbocyclylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl).

"Aryl" refers to a radical derived from an aromatic monocyclic or multicyclic hydrocarbon ring system by removing a hydrogen atom from a ring carbon atom. The aromatic monocyclic or multicyclic hydrocarbon ring system contains only hydrogen and carbon from five to eighteen carbon atoms, where at least one of the rings in the ring system is fully unsaturated, i.e., it contains a cyclic, delocalized (4n+2) π-electron system in accordance with the Hückel theory. For example, aryl is a C₆-C₁₀ ring system. The ring system from which aryl groups are derived include, but are not limited to, groups such as benzene, fluorene, indane, indene, tetralin and naphthalene. Unless stated otherwise specifically in the specification, the term "aryl" or the prefix "ar-" (such as in "aralkyl") may include aryl radicals optionally substituted by one or more substituents independently selected from optionally substituted alkyl, optionally substituted alkenyl, optionally substituted alkynyl, halo, cyano, nitro, -R^{b}-OR^{a}, -R^{b}-OC(O)-R^{a}, -R^{b}-OC(O)-OR^{a}, - R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), - R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl (optionally substituted with halogen, hydroxy, methoxy, or trifluoromethyl), fluoroalkyl, cycloalkyl (optionally substituted with halogen, hydroxy, methoxy, or trifluoromethyl), cycloalkylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl), each R^{b} is independently a direct bond or a straight or branched alkylene or alkenylene chain, and R^{c} is a straight or branched alkylene or alkenylene chain, and where each of the R^{a}, R^{b}, or R^{c} substituents is unsubstituted unless otherwise indicated.

"Aralkyl" refers to a radical of the formula -R^{c}-aryl where R^{c} is an alkylene chain as defined above, for example, methylene, ethylene, and the like. The alkylene chain part of the aralkyl radical may be optionally substituted as described above for an alkylene chain. The aryl part of the aralkyl radical is optionally substituted as described above for an aryl group.

"Aralkenyl" refers to a radical of the formula -R^{d}-aryl where R^{d} is an alkenylene chain as defined above. The aryl part of the aralkenyl radical may be optionally substituted as described above for an aryl group. The alkenylene chain part of the aralkenyl radical is optionally substituted as defined above for an alkenylene group.

"Aralkynyl" refers to a radical of the formula -R^{e}-aryl, where R^{e} is an alkynylene chain as defined above. The aryl part of the aralkynyl radical may be optionally substituted as described above for an aryl group. The alkynylene chain part of the aralkynyl radical is optionally substituted as defined above for an alkynylene chain.

"Aralkoxy" refers to a radical bonded through an oxygen atom of the formula - O-R^{c}-aryl where R^{c} is an alkylene chain as defined above, for example, methylene, ethylene, and the like. The alkylene chain part of the aralkyl radical may be optionally substituted as described above for an alkylene chain. The aryl part of the aralkyl radical is optionally substituted as described above for an aryl group.

"Carbocyclyl" refers to a stable non-aromatic monocyclic or polycyclic hydrocarbon radical consisting solely of carbon and hydrogen atoms, which includes fused or bridged ring systems, having from three to fifteen carbon atoms. In certain embodiments, a carbocyclyl comprises three to ten carbon atoms. In other embodiments, a carbocyclyl comprises five to seven carbon atoms. The carbocyclyl is attached to the rest of the molecule by a single bond. Carbocyclyl is saturated (i.e., containing single C-C bonds only) or unsaturated (i.e., containing one or more double bonds or triple bonds). A fully saturated carbocyclyl radical is also referred to as "cycloalkyl." Examples of monocyclic cycloalkyls include, e.g., cyclopropyl, cyclobutyl, cyclopentyl, cyclohexyl, cycloheptyl, and cyclooctyl. An unsaturated carbocyclyl is also referred to as "cycloalkenyl." Examples of monocyclic cycloalkenyls include, e.g., cyclopentenyl, cyclohexenyl, cycloheptenyl, and cyclooctenyl. Polycyclic carbocyclyl radicals include, for example, adamantyl, norbornyl (i.e., bicyclo[2.2.1]heptanyl), norbornenyl, decalinyl, 7,7-dimethyl-bicyclo[2.2.1]heptanyl, and the like. A carbocyclyl group may be optionally substituted by one or more substituents independently selected from optionally substituted alkyl, optionally substituted alkenyl, optionally substituted alkynyl, halo, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, - R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl (optionally substituted with halogen, hydroxy, methoxy, or trifluoromethyl), fluoroalkyl, cycloalkyl (optionally substituted with halogen, hydroxy, methoxy, or trifluoromethyl), cycloalkylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl), each R^{b} is independently a direct bond or a straight or branched alkylene or alkenylene chain, and R^{c} is a straight or branched alkylene or alkenylene chain, and where each of the R^{a}, R^{b}, and R^{c} substituents is unsubstituted unless otherwise indicated.

"Carbocyclylalkyl" refers to a radical of the formula -R^{c}-carbocyclyl where R^{c} is an alkylene chain as defined above. The alkynylene chain and the carbocyclyl radical may be optionally substituted as defined above.

"Carbocyclylalkoxy" refers to a radical bonded through an oxygen atom of the formula -O-R^{c}-carbocyclyl where R^{c} is an alkylene chain as defined above. The alkylene chain and the carbocyclyl radical may be optionally substituted as defined above.

"Halo" or "halogenn" refers to bromo, chloro, fluoro or iodo substituents.

"Fluoroalkyl" refers to an alkyl radical, as defined above, that is substituted by one or more fluoro radicals, as defined above, for example, trifluoromethyl, difluoromethyl, fluoromethyl, 2,2,2-trifluoroethyl, 1-fluoromethyl-2-fluoroethyl, and the like. In some embodiments, the alkyl part of the fluoroalkyl radical may be optionally substituted as defined above for an alkyl group.

"Heterocyclyl" or, equally, "heterocycloalkyl," refers to a stable 3- to 18-membered non-aromatic ring radical that comprises two to twelve carbon atoms and from one to six heteroatoms selected from nitrogen, oxygen, and sulfur. In embodiments, heterocycloalkyl is a 3- to 6-membered ring (wherein 1-4 ring members are independently selected from N, O, and S), wherein the ring is optionally substituted with 1 to 3 substituents. Unless stated otherwise specifically in the specification, the heterocyclyl radical is a monocyclic, bicyclic, tricyclic, or tetracyclic ring system, which optionally includes fused or bridged ring systems. The heteroatoms in the heterocyclyl radical are optionally oxidized. One or more nitrogen atoms, if present, are optionally quaternized. The heterocyclyl radical is partially or fully saturated. The heterocyclyl is attached to the rest of the molecule through any atom of the ring(s). Examples of such heterocyclyl radicals include, but are not limited to, dioxolanyl, thienyl, [1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. A heterocyclyl group may be optionally substituted by one or more substituents selected from optionally substituted alkyl, optionally substituted alkenyl, optionally substituted alkynyl, halo, fluoroalkyl, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl (optionally substituted with halogen, hydroxy, methoxy, or trifluoromethyl), fluoroalkyl, cycloalkyl (optionally substituted with halogen, hydroxy, methoxy, or trifluoromethyl), cycloalkylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl), each R^{b} is independently a direct bond or a straight or branched alkylene or alkenylene chain, and R^{c} is a straight or branched alkylene or alkenylene chain, and where each of the R^{a}, R^{b}, and R^{c} substituents is unsubstituted unless otherwise indicated.

"Heteroaryl" refers to a radical derived from a 3- to 18-membered aromatic ring radical that comprises two to seventeen carbon atoms and from one to six heteroatoms selected from nitrogen, oxygen, and sulfur. Heteroaryl includes a 5- to 10-membered ring wherein 1-4 heteroaryl members are independently selected from N, O, and S. As used herein, the heteroaryl radical is a monocyclic, bicyclic, tricyclic, or tetracyclic ring system, wherein at least one of the rings in the ring system is fully unsaturated, i.e., it contains a cyclic, delocalized (4n+2) π-electron system in accordance with the Hückel theory. Heteroaryl includes fused or bridged ring systems. The heteroatom(s) in the heteroaryl radical is optionally oxidized. One or more nitrogen atoms, if present, are optionally quaternized. The heteroaryl is attached to the rest of the molecule through any atom of the ring(s). Examples of heteroaryls include, but are not limited to, azepinyl, acridinyl, benzimidazolyl, benzindolyl, 1,3-benzodioxolyl, benzofuranyl, benzooxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothiophenyl, furanyl, furanonyl, furo[3,2-c]pyridinyl, 5,6,7,8,9,10-hexahydrocycloocta[d]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, indazolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, isoquinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pyridinyl, and thiophenyl (i.e. thienyl). A heteroaryl group may be optionally substituted by one or more substituents selected from optionally substituted alkyl, optionally substituted cycloalkylalkyl, optionally substituted heterocyclylalkyl, optionally substituted alkenyl, optionally substituted alkynyl, halo, optionally substituted fluoroalkyl, optionally substituted haloalkenyl, optionally substituted haloalkynyl, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, - R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl (optionally substituted with halogen, hydroxy, methoxy, or trifluoromethyl), fluoroalkyl, cycloalkyl (optionally substituted with halogen, hydroxy, methoxy, or trifluoromethyl), cycloalkylalkyl (optionally substituted with halogen, hydroxy, methoxy, or trifluoromethyl), aryl (optionally substituted with halogen, hydroxy, methoxy, or trifluoromethyl), aralkyl (optionally substituted with halogen, hydroxy, methoxy, or trifluoromethyl), heterocyclyl (optionally substituted with halogen, hydroxy, methoxy, or trifluoromethyl), heterocyclylalkyl (optionally substituted with halogen, hydroxy, methoxy, or trifluoromethyl), heteroaryl (optionally substituted with halogen, hydroxy, methoxy, or trifluoromethyl), or heteroarylalkyl (optionally substituted with halogen, hydroxy, methoxy, or trifluoromethyl), each R^{b} is independently a direct bond or a straight or branched alkylene or alkenylene chain, and R^{c} is a straight or branched alkylene or alkenylene chain, and where each of the R^{a}, R^{b}, and R^{c} substituents is unsubstituted unless otherwise indicated.

"Heteroarylalkoxy" refers to a radical of the formula -R^{c}-heteroaryl, where R^{c} is an alkylene chain as defined above. If the heteroaryl is a nitrogen-containing heteroaryl, the heteroaryl is optionally attached to the alkyl radical at the nitrogen atom. The alkylene chain of the heteroarylalkyl radical is optionally substituted as defined above for an alkylene chain. The heteroaryl part of the heteroarylalkyl radical is optionally substituted as defined above for a heteroaryl group. "Heteroarylalkoxy" alternatively refers to a radical bonded through an oxygen atom of the formula -O-R^{c}-heteroaryl, where R^{c} is an alkylene chain as defined above. If the heteroaryl is a nitrogen-containing heteroaryl, the heteroaryl is optionally attached to the alkyl radical at the nitrogen atom. The alkylene chain of the heteroarylalkoxy radical may be optionally substituted as defined above for an alkylene chain. The heteroaryl part of the heteroarylalkoxy radical is optionally substituted as defined above for a heteroaryl group.

The compounds disclosed herein, in some embodiments, contain one or more asymmetric centers and thus give rise to enantiomers, diastereomers, and other stereoisomeric forms that are defined, in terms of absolute stereochemistry, as (R)- or (S)-. Unless stated otherwise, it is intended that all stereoisomeric forms of the compounds disclosed herein are contemplated by this disclosure. When the compounds described herein contain alkene double bonds, and unless specified otherwise, it is intended that this disclosure includes both E and Z geometric isomers (e.g., cis or trans.) Likewise, all possible isomers, as well as their racemic and optically pure forms, and all tautomeric forms are also intended to be included. The term "geometric isomer" refers to E or Z geometric isomers (e.g., cis or trans) of an alkene double bond. The term "positional isomer" refers to structural isomers around a central ring, such as ortho-, meta-, and para- isomers around a benzene ring.

As used herein, "carboxylic acid bioisostere" refers to a functional group or moiety that exhibits similar physical, biological and/or chemical properties as a carboxylic acid moiety. Examples of carboxylic acid bioisosteres include, but are not limited to, 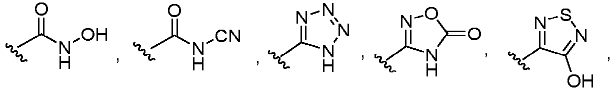 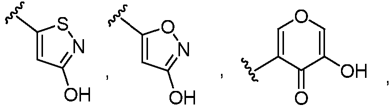 and the like.

A "tautomer" refers to a molecule wherein a proton shift from one atom of a molecule to another atom of the same molecule is possible. The compounds presented herein, in certain embodiments, exist as tautomers. In circumstances where tautomerization is possible, a chemical equilibrium of the tautomers will exist. The exact ratio of the tautomers depends on several factors, including physical state, temperature, solvent, and pH. Some examples of tautomeric equilibrium include:

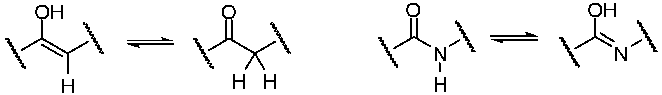

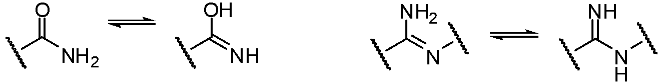

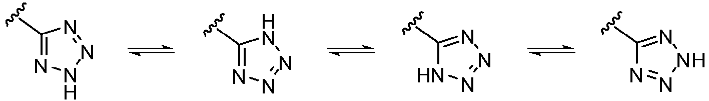

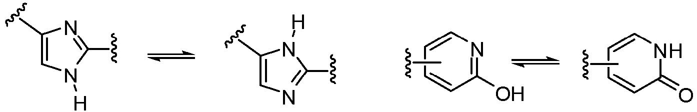

The compounds disclosed herein, in some embodiments, are used in different enriched isotopic forms, e.g., enriched in the content of ²H, ³H, ¹¹C, ¹³C and/or ¹⁴C. In one embodiment, the compound is deuterated in at least one position. Such deuterated forms can be made by the procedure described in U.S. Patent Nos. 5,846,514 and 6,334,997. As described in U.S. Patent Nos. 5,846,514 and 6,334,997, deuteration can improve the metabolic stability and or efficacy of a compound, and thereby increase the duration of therapeutic action of the compound.

Unless otherwise stated, structures depicted herein are intended to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon are within the scope of the present disclosure.

The compounds of the present disclosure optionally contain unnatural proportions of atomic isotopes at one or more atoms that constitute such compounds. For example, the compounds may be labeled with isotopes, such as for example, deuterium (2H), tritium (3H), iodine-125 (125I) or carbon-14 (14C). Isotopic substitution with ²H, ¹¹C, ¹³C, ¹⁴C, ¹⁵C, ¹²N, ¹³N, ¹⁵N, ¹⁶N, ¹⁶O, ¹⁷O, ¹⁴F, ¹⁵F, ¹⁶F, ¹⁷F, ¹⁸F, ³³S, ³⁴S, ³⁵S, ³⁶S, ³⁵Cl, ³⁷Cl, ⁷⁹Br, ⁸¹Br, ¹²⁵I are all contemplated. In some embodiments, isotopic substitution with 18F is contemplated. All isotopic variations of the compounds of the present disclosure, whether radioactive or not, are encompassed within the scope of the present disclosure.

In some embodiments, the compounds disclosed herein have some or all of the ¹H atoms replaced with ²H atoms. The methods of synthesis for deuterium-containing compounds are known in the art and include, by way of non-limiting example only, the following synthetic methods.

Deuterium substituted compounds are synthesized using various methods such as described in: Dean, Dennis C.; Editor. Recent Advances in the Synthesis and Applications of Radiolabeled Compounds for Drug Discovery and Development. [Curr., Pharm. Des., 2000; 6(10)] 2000, 110 pp; George W.; Varma, Rajender S. The Synthesis of Radiolabeled Compounds via Organometallic Intermediates, Tetrahedron, 1989, 45(21), 6601-21; and Evans, E. Anthony. Synthesis of radiolabeled compounds, J. Radioanal. Chem., 1981, 64(1-2), 9-32.

Deuterated starting materials are readily available and are subjected to the synthetic methods described herein to provide for the synthesis of deuterium-containing compounds. Large numbers of deuterium-containing reagents and building blocks are available commercially from chemical vendors, such as Aldrich Chemical Co.

Deuterium-transfer reagents suitable for use in nucleophilic substitution reactions, such as iodomethane-d3 (CD₃I), are readily available and may be employed to transfer a deuterium-substituted carbon atom under nucleophilic substitution reaction conditions to the reaction substrate. The use of CD₃I is illustrated, by way of example only, in the reaction schemes below.

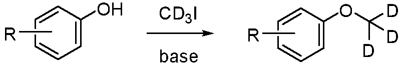

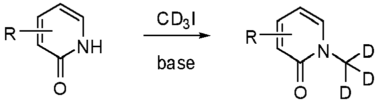

Deuterium-transfer reagents, such as lithium aluminum deuteride (LiAlD₄), are employed to transfer deuterium under reducing conditions to the reaction substrate. The use of LiAlD₄ is illustrated, by way of example only, in the reaction schemes below.

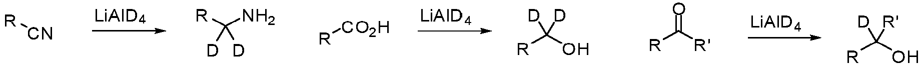

Deuterium gas and palladium catalyst are employed to reduce unsaturated carbon-carbon linkages and to perform a reductive substitution of aryl carbon-halogen bonds as illustrated, by way of example only, in the reaction schemes below.

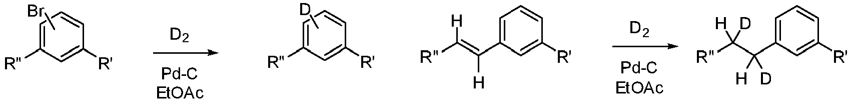

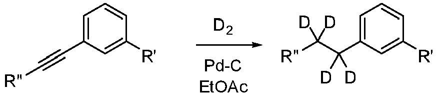

In one embodiment, the compounds disclosed herein contain one deuterium atom. In another embodiment, the compounds disclosed herein contain two deuterium atoms. In another embodiment, the compounds disclosed herein contain three deuterium atoms. In another embodiment, the compounds disclosed herein contain four deuterium atoms. In another embodiment, the compounds disclosed herein contain five deuterium atoms. In another embodiment, the compounds disclosed herein contain six deuterium atoms. In another embodiment, the compounds disclosed herein contain more than six deuterium atoms. In another embodiment, the compound disclosed herein is fully substituted with deuterium atoms and contains no non-exchangeable ¹H hydrogen atoms. In one embodiment, the level of deuterium incorporation is determined by synthetic methods in which a deuterated synthetic building block is used as a starting material.

With regard to the compounds provided herein, when a particular atom's position is designated as having deuterium or "D," it is understood that the abundance of deuterium at that position is substantially greater than the natural abundance of deuterium, which is about 0.015%. A position designated as having deuterium typically has a minimum isotopic enrichment factor of, in particular embodiments, at least 1000 (15% deuterium incorporation), at least 2000 (30% deuterium incorporation), at least 3000 (45% deuterium incorporation), at least 3500 (52.5% deuterium incorporation), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) at each designated deuterium atom. The isotopic enrichment and isotopic enrichment factor of the compounds provided herein can be determined using conventional analytical methods known to one of ordinary skill in the art, including mass spectrometry and nuclear magnetic resonance spectroscopy.

The present disclosure describes compounds interchangeably by chemical name and chemical structure. Insofar as any discrepancy might exist between the given chemical name and chemical structure for a compound, the chemical structure controls.

"Pharmaceutically acceptable salt" includes both acid and base addition salts. A pharmaceutically acceptable salt of any one of compounds described herein is intended to encompass any and all pharmaceutically suitable salt forms. Examples of pharmaceutically acceptable salts of the compounds described herein are pharmaceutically acceptable acid addition salts and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and. aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (see, for example, Berge S.M. et al., "Pharmaceutical Salts," Journal of Pharmaceutical Science, 66:1-19 (1997)). Acid addition salts of basic compounds are, in some embodiments, prepared by contacting the free base forms with a sufficient amount of the desired acid to produce the salt according to methods and techniques with which a skilled artisan is familiar.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. Pharmaceutically acceptable base addition salts are, in some embodiments, formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, for example, isopropylamine, trimethylamine, diethylamine, triethylamine, tripropylamine, ethanolamine, diethanolamine, 2-dimethylaminoethanol, 2-diethylaminoethanol, dicyclohexylamine, lysine, arginine, histidine, caffeine, procaine, N,N-dibenzylethylenediamine, chloroprocaine, hydrabamine, choline, betaine, ethylenediamine, ethylenedianiline, N-methylglucamine, glucosamine, methylglucamine, theobromine, purines, piperazine, piperidine, N-ethylpiperidine, polyamine resins and the like. See Berge et al., supra.

"Pharmaceutically acceptable solvate" refers to a composition of matter that is the solvent addition form. In some embodiments, solvates contain either stoichiometric or non-stoichiometric amounts of a solvent, and are formed during the process of making with pharmaceutically acceptable solvents such as water, ethanol, and the like. Hydrates are formed when the solvent is water, or alcoholates are formed when the solvent is alcohol. Solvates of compounds described herein are conveniently prepared or formed during the processes described herein. The compounds provided herein exist in either unsolvated or solvated forms.

As used herein, and unless otherwise specified to the contrary, the term "compound" is inclusive in that it encompasses a compound or a pharmaceutically acceptable salt, stereoisomer, isotopologue, and/or tautomer thereof. Thus, for instance, a compound of Formula (I) or Formula (II) includes a pharmaceutically acceptable salt of a tautomer of the compound. Similarly, a compound of Formula (I) or Formula (II) includes a pharmaceutically acceptable salt of an isotopologue of the compound.

The term "subject" or "patient" encompasses mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. In one aspect, the mammal is a human.

As used herein, "treatment" or "treating," or "palliating" or "ameliorating" are used interchangeably. These terms refer to an approach for obtaining beneficial or desired results including but not limited to therapeutic benefit and/or a prophylactic benefit. By "therapeutic benefit" is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient is still afflicted with the underlying disorder. For prophylactic benefit, the compositions are, in some embodiments, administered to a patient at risk of developing a particular disease, or to a patient reporting one or more of the physiological symptoms of a disease, even though a diagnosis of this disease has not been made.

The term "effective amount" refers to an amount of a compound as described herein or other active ingredient sufficient to provide a therapeutic or prophylactic benefit in the treatment or prevention of a disease or to delay or minimize symptoms associated with a disease. Further, a therapeutically effective amount with respect to a compound as described herein means that amount of therapeutic agent alone, or in combination with other therapies, that provides a therapeutic benefit in the treatment or prevention of a disease. Used in connection with a compound as described herein, the term can encompass an amount that improves overall therapy, reduces or avoids symptoms or causes of disease, or enhances the therapeutic efficacy of or is synergistic with another therapeutic agent.

### COMPOUNDS

The present disclosure provides in various embodiments a compound of formula (II) or a pharmaceutically acceptable salt and/or solvate thereof:

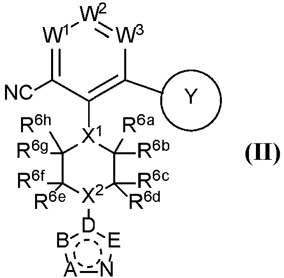

W¹ is N or CR¹, W² is N or CR², and W³ is N or CR³; wherein no more than one of W¹, W², and W³ is N.

X¹ and X² are independently selected from CR⁴ and N.

In some embodiments, Ring Y 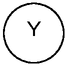 is of formula (a):

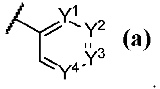

In formula (a), Y¹, Y², Y³, and Y⁴ are independently selected from CR⁵ and N wherein Y¹, Y², Y³, and Y⁴ are not simultaneously N.

In other embodiments, Ring Y 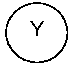 is of formula (b):

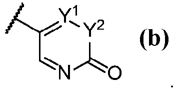

In formula (b), Y¹ is CR⁵ and Y² is NR^{5'}.

In still other embodiments, Ring Y 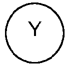 is of formula (c):

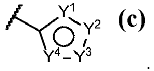

In formula (c), Y¹, Y², Y³, and Y⁴ are independently selected from CR⁵ and N.

In some embodiments, either Y¹ and Y², or Y² and Y³, or Y³ and Y⁴ represent a fused ring selected from a C₅-C₈-cycloalkyl, a C₆-C₁₀-aryl, a 5- to 10-membered heteroaryl (wherein 1-4 heteroaryl members are independently selected from N, O, and S), and a 5- to 8-membered heterocycloalkyl (wherein 1-4 ring members are independently selected from N, B O, and S), wherein the ring is optionally substituted with 1 to 3 substituents independently selected from the group consisting of C₁-C₆-alkyl, C₃-C₈-cycloalkyl, 3- to 6-membered heterocycloalkyl (wherein 1-4 ring members are independently selected from N, O, and S), C₂-C₆-alkenyl, C₂-C₆-alkynyl, halo, C₁-C₆-haloalkyl, C₂-C₆-haloalkenyl, C₂-C₆-haloalkynyl, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2).

A, B, and E are independently selected from C, N, O, and S, and D is C or N.

The symbol 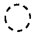 represents the presence of double bonds such that the ring A-B-D-E-N is aromatic and is optionally substituted with one or two substituents independently selected from C₁-C₃-alkyl, C₃-C₅-cycloalkyl, OH, OMe, NH₂, N(H)Me, NMe₂. Further, no more than two of A, B, D, and E are simultaneously N, O, or S.

R¹, R², and R³ are independently selected from the group consisting of hydrogen, halo, cyano, nitro, -R^{b}-OR^{a}, -R^{b}-O-R^{c}-O-R^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})_{2,} -R^{b}-N(R^{a})C(O)OR^{a}, -R^{b}-N(R^{a})C(O)R^{a}, - R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₁-C₆-haloalkyl, C₂-C₆-alkenyl, C₂-C₆-alkynyl, C₃-C₈-cycloalkyl, C₆-C₁₀-aryl, -(C₁-C₆-alkyl)(C₆-C₁₀-aryl), 5- to 10-membered heteroaryl (wherein 1-4 heteroaryl members are independently selected from N, O, and S) optionally fused to 3- to 6-membered heterocycloalkyl (wherein 1-4 ring members are independently selected from N, O, and S), and 3- to 6-membered heterocycloalkyl (wherein 1-4 ring members are independently selected from N, O, and S.

In some embodiments, R¹ and R², or R² and R³, together with the carbon atoms to which they are bound, form a fused C₅-C₈-cycloalkyl, C₆-C₁₀-aryl, 5- to 10-membered heteroaryl (wherein 1-4 heteroaryl members are independently selected from N, O, and S), and 5- to 8-membered heterocycloalkyl (wherein 1-4 ring members are independently selected from N, O, and S).

Any heteroaryl or heterocycloalkyl in R¹, R², and R³ is optionally and independently substituted with 1 to 3 substituents selected from the group consisting of C₁-C₆-alkyl, halo, hydroxy, C₃-C₈-cycloalkyl, heterocycloalkyl (wherein 1-4 ring members are independently selected from N, O, and S), and -R^{b}-N(R^{a})₂.

R⁴ in each instance is independently H, OH, halo, C₁-C₆-alkyl, or C₁-C₆-alkoxy.

R⁵ in each instance is independently selected from the group consisting of hydrogen, halo, cyano, nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -B(OR^{a})₂, -R^{b}-N(R^{a})-R^{c}-N(R^{a})_{2,} -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-OS(O)ₜF (where t is 1 or 2), -R^{b}-S(O)ₜN(R^{a})₂ (where t is 1 or 2), C₁-C₆-alkyl, C₂-C₆-alkynyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S).

R^{5'} is selected from the group consisting of hydrogen, -R^{c}-R^{a}, -R^{c}-OR^{a}, -R^{b}-OC(O)-R^{a}, -R^{b}-OC(O)-OR^{a}, -R^{b}-OC(O)-N(R^{a} )₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})_{2,} -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S).

Any heteroaryl or heterocycloalkyl in R⁵ and R^{5'} is optionally and independently substituted with 1 to 3 substituents selected from the group consisting of C₁-C₆-alkyl, halo, hydroxy, C₃-C₈-cycloalkyl, and -R^{b}-N(R^{a})₂.

R^{6a}, R^{6b}, R^{6c}, R^{6d}, R^{6e}, R^{6f}, R^{6g}, and R^{6h} are independently selected from the group consisting of H, halo, NO₂, OH, CN, -R^{d}-N(R^{a})₂, -R^{b}-OH, C₁-C₆-alkyl, and C₁-C₆-alkoxy;

In some embodiments optionally in combination with any other embodiment described herein, R^{6a} and R^{6b}, or R^{6c} and R^{6d}, or R^{6e} and R^{6f}, or R^{6g} and R^{6h} independently represent oxo, thioxo, imino, or oximo.

In still further embodiments optionally in combination with any other embodiment described herein, R^{6a} and R^{6b}, or R^{6c} and R^{6d}, or R^{6e} and R^{6f}, or R^{6g} and R^{6h}, together with the carbon atoms to which they are bound, independently combine to form a fused ring selected from a C₃-C₆-cycloalkyl and C₃-C₆-heterocycloalkyl (wherein 1-4 ring members are independently selected from N, O, and S).

In additional embodiments optionally in combination with any other embodiment described herein, one of R^{6c} and R^{6d} together with one of R^{6e} and R^{6f} represent a bond between the ring carbon members to which they are bound.

R^{a} in each instance is independently selected from hydrogen, C₁-C₆-alkyl, C₃-C₈-cycloalkyl, -(C₁-C₆-alkyl)(C₃-C₈-cycloalkyl), C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S).

R^{b} in each instance is independently selected from a direct bond, a straight or branched C₂-C₆-alkylene, and C₂-C₆-alkenylene chain.

Any heteroaryl or heterocycloalkyl in R^{a} and R^{b} is optionally and independently substituted with 1 to 3 substituents selected from the group consisting of C₁-C₆-alkyl, halo, hydroxy,

R^{c} in each instance is independently selected from a straight or branched C₂-C₆-alkylene and C₂-C₆-alkenylene chain.

In some embodiments, W¹ is N, W² is CR², and W³ is CR³. In other embodiments, W¹ is CR¹, W² is CR², and W³ is N. In still further embodiments, W¹ is CR¹, W² is CR², and W³ is CR³.

The present disclosure also provides in various embodiments a compound of formula (I) or a pharmaceutically acceptable salt and/or solvate thereof:

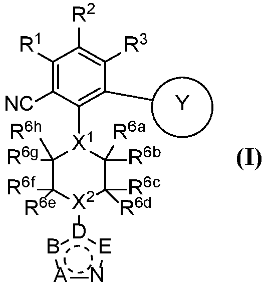

X¹ and X² are independently selected from CR⁴ and N.

In some embodiments, Ring Y 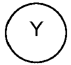 is of formula (a):

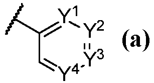

In formula (a), Y¹, Y², Y³, and Y⁴ are independently selected from CR⁵ and N wherein Y¹, Y², Y³, and Y⁴ are not simultaneously N.

In other embodiments, the Ring Y 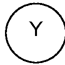 is of formula (b):

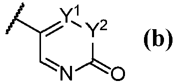

In formula (b), Y¹ is CR⁵ and Y² is NR^{5'}.

In some embodiments, either Y¹ and Y², or Y² and Y³, or Y³ and Y⁴ represent a fused ring selected from a C₅-C₈-cycloalkyl, a C₆-C₁₀-aryl, a 5- to 10-membered heteroaryl (wherein 1-4 heteroaryl members are independently selected from N, O, and S), and a 5- to 8-membered heterocycloalkyl (wherein 1-4 ring members are independently selected from N, O, and S), wherein the ring is optionally substituted with 1 to 3 substituents independently selected from the group consisting of C₁-C₆-alkyl, C₃-C₈-cycloalkyl, 3- to 6-membered heterocycloalkyl (wherein 1-4 ring members are independently selected from N, O, and S), C₂-C₆-alkenyl, C₂-C₆-alkynyl, halo, C₁-C₆-haloalkyl, C₂-C₆-haloalkenyl, C₂-C₆-haloalkynyl, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2),

A, B, and E are independently selected from C, N, O, and S, and D is C or N. 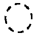 represents the presence of double bonds such that the ring A-B-D-E-N is aromatic and is optionally substituted with one or two substituents independently selected from C₁-C₃-alkyl, C₃-C₅-cycloalkyl, OH, OMe, NH₂, N(H)Me, NMe₂. No more than two of A, B, D, and E are simultaneously N, O, or S.

R¹, R², and R³ are independently selected from the group consisting of hydrogen, halo, cyano, nitro, -R^{b}-OR^{a}, -R^{b}-O-R^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, - R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, -(C₁-C₆-alkyl)(C₆-C₁₀-aryl), 5- to 10-membered heteroaryl or (wherein 1-4 heteroaryl members are independently selected from N, O, and S), and 3- to 6-membered heterocycloalkyl (wherein 1-4 ring members are independently selected from N, O, and S).

In some embodiments, R¹ and R², or R² and R³, together with the carbon atoms to which they are bound, form a fused C₅-C₈-cycloalkyl, C₆-C₁₀-aryl, 5- to 10-membered heteroaryl (wherein 1-4 heteroaryl members are independently selected from N, O, and S), and 5- to 8-membered heterocycloalkyl (wherein 1-4 ring members are independently selected from N, O, and S).

R⁴ in each instance is independently H, OH, halo, C₁-C₆-alkyl, or C₁-C₆-alkoxy.

R⁵ in each instance is independently selected from the group consisting of hydrogen, halo, cyano, nitro, -R^{b}-OR^{a}, -R^{b}-O-R^{c}-OR^{a}, -R^{b}-OC(O)-R^{a}, -R^{b}-OC(O)-OR^{a} , -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, - R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, 5- to 10-membered heteroaryl or (wherein 1-4 heteroaryl members are independently selected from N, O, and S), and 3- to 6-membered heterocycloalkyl (wherein 1-4 ring members are independently selected from N, O, and S).

R^{5'} is selected from the group consisting of hydrogen, -R^{c}-R^{a}, -R^{c}-OR^{a}, -R^{b}-OC(O)-R^{a}, -R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S).

R^{6a}, R^{6b}, R^{6c}, R^{6d}, R^{6e}, R^{6f}, R^{6g}, and R^{6h} are independently selected from the group consisting of H, halo, NO₂, OH, CN, -R^{b}-N(R^{a})₂, -R^{b}-OH, C₁-C₆-alkyl, and C₁-C₆-alkoxy.

In some embodiments, R^{6a} and R^{6b}, or R^{6c} and R^{6d}, or R^{6e} and R^{6f}, or R^{6g} and R^{6h} independently represent oxo, thioxo, imino, or oximo.

In other embodiments, R^{6a} and R^{6b}, or R^{6c} and R^{6d}, or R^{6e} and R^{6f}, or R^{6g} and R^{6h}, together with the carbon atoms to which they are bound, independently combine to form a fused ring selected from a C₃-C₆-cycloalkyl and C₃-C₆-heterocycloalkyl (wherein 1-4 ring members are independently selected from N, O, and S).

R^{a} in each instance is independently selected from hydrogen, C₁-C₆-alkyl, C₃-C₈-cycloalkyl, -(C₁-C₆-alkyl)(C₃-C₈-cycloalkyl), C₆-C₁₀-aryl, -(C₁-C₆-alkyl)(C₆-C₁₀-aryl), 5-to 10-membered heteroaryl or (wherein 1-4 heteroaryl members are independently selected from N, O, and S), and 3- to 6-membered heterocycloalkyl or -(C₁-C₆-alkyl)(3- to 6-membered heterocycloalkyl (wherein 1-4 ring members are independently selected from N, O, and S).

R^{b} in each instance is independently selected from a direct bond, a straight or branched C₂-C₆-alkylene, and C₂-C₆-alkenylene chain.

R^{c} in each instance is independently selected from a straight or branched C₂-C₆-alkylene and C₂-C₆-alkenylene chain.

In some embodiments, the ring member D is C. In additional embodiments, A, B, and E are independently selected from C and N. For example, at least one of A, B, and E is N, or in some embodiments two of A, B, and E is N. Specific examples of the A-B-D-EN ring is an optionally substituted ring selected from the group consisting of:

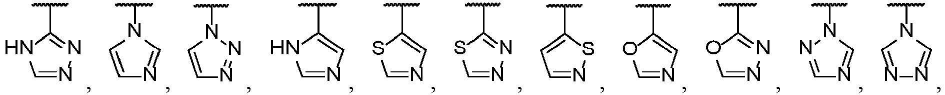

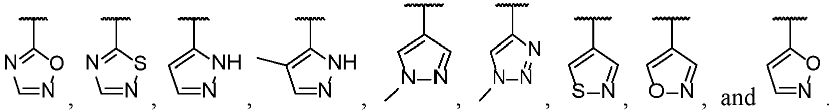

In some embodiments, the optionally substituted A-B-D-E-N ring is one selected from the group consisting of:

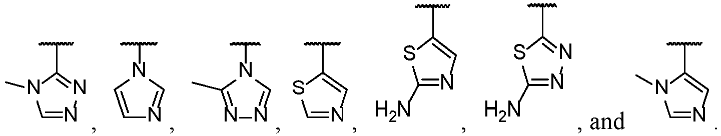

In a specific embodiment, the optionally substituted A-B-D-E-N ring is

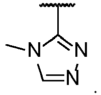

In some embodiments of the present disclosure, Ring Y is of formula (a). Examples of Ring Y include those in which one of Y¹, Y², Y³, and Y⁴ is N and each of the remaining three is CR⁵. Thus, in embodiments, each of Y¹, Y², Y³ is CR⁵ and Y⁴ is N. Illustrative embodiments of Ring Y are wherein each of Y¹ and Y² is CH and Y³ is CF.

In other embodiments, two of Y¹, Y², Y³, and Y⁴ are N and each of the remaining two is CR⁵. For example, each of Y¹ and Y² is CR⁵ and each of Y³ and Y⁴ is N.

In some embodiments, wherein Ring Y is of formula (a) or formula (b), either Y¹ and Y² or Y³ and Y⁴ represent an optionally substituted fused ring. For example, the fused ring in various embodiments is an optionally substituted fused 5- to 6-membered heteroaryl (wherein 1-4 heteroaryl members are independently selected from N, O, and S) or 5- to 6-membered heterocycloalkyl (wherein 1-4 ring members are independently selected from N, O, and S).

In various embodiments, one or each of X¹ and X² is N. For example, X¹ is N and X² is CR⁴, or X¹ is CR⁴ and X² is N, or each of X¹ and X² is CR⁴, or each of X¹ and X² is N. Optionally in combination with these embodiments, R⁴ is H.

In additional embodiments, R¹ is selected from the group consisting of H, halo, C₁-C₆-alkoxy, C₆-C₁₀-aryl, C₃-C₈-cycloalkyl,3- to 6-membered heterocycloalkyl (wherein 1-4 ring members are independently selected from N, O, and S), and 5- to 6-membered heteroaryl (wherein 1-4 heteroaryl members are independently selected from N, O, and S).

In still further embodiments, each of R² and R³ is independently H, halo, cyano, CH₃ or CF₃.

Other embodiments provide for formula (I) compounds wherein R^{6a}, R^{6b}, R^{6c}, R^{6d}, R^{6e}, R^{6f}, R^{6g}, and R^{6h} are independently selected from the group consisting of H, halo, C₁-C₆-alkyl, and C₁-C₆-alkoxy. Examples include compounds wherein each of R^{6a}, R^{6b}, R^{6c}, R^{6d}, R^{6e}, R^{6f}, R^{6g}, and R^{6h} is H.

The present disclosure also provides, in embodiments, a compound of formula (I) or (II) that is a compound of formula (IA):

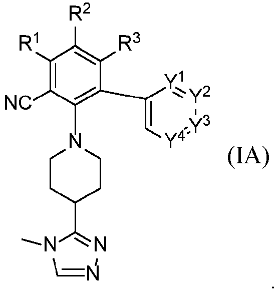

Optionally in combination with any embodiment described herein, R¹ is selected from the group consisting of H, halo, C₁-C₆-alkoxy, C₆-C₁₀-aryl, C₃-C₆-cycloalkyl, 5- to 6-membered heteroaryl (wherein 1-4 heteroaryl members are independently selected from N, O, and S), and 3- to 6-membered heterocycloalkyl (wherein 1-4 ring members are independently selected from N, O, and S); and each of R² and R³ is independently H, F, cyano, CH₃, or CF₃.

The present disclosure also provides, in various embodiments, a compound of formula (I) or (II) that is a compound of formula (IB), (IC), or (ID):

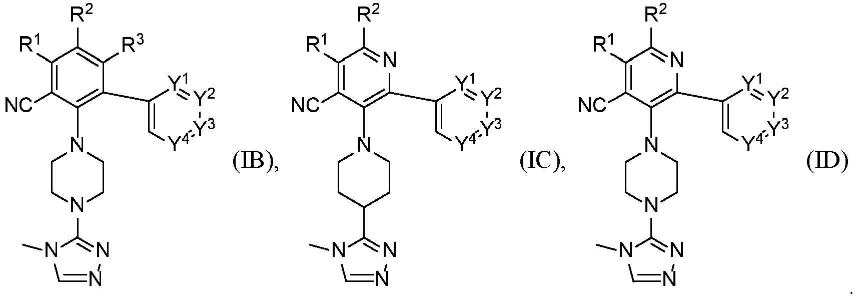

In formulae (IB), (IC), and (ID), per some embodiments, R¹ is selected from the group consisting of H, halo, C₁-C₆-alkoxy, C₆-C₁₀-aryl, C₃-C₆-cycloalkyl, 5- to 6-membered heteroaryl (wherein 1-4 heteroaryl members are independently selected from N, O, and S) and 3- to 6-membered heterocycloalkyl (wherein 1-4 ring members are independently selected from N, O, and S); and each of R² and R³, when present, is independently H, F, cyano, CH₃, or CF₃.

In additional embodiments, optionally in reference to any of formulae (IA), (B), (IC), and (ID), one of Y¹, Y², Y³, and Y⁴ is N and each of the remaining three is CR⁵. For example, each of Y¹ and Y² is CH and Y³ is CF.

In some embodiments, two of Y¹, Y², Y³, and Y⁴ are N and each of the remaining two is CR⁵.

In still further embodiments, the ring containing Y¹, Y², Y³, and Y⁴ is: 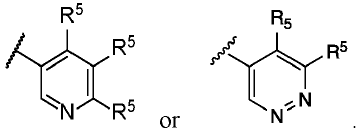 In an exemplary embodiment, the ring containing Y¹,

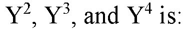

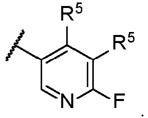

In additional embodiments, the present disclosure provides a compound or pharmaceutically acceptable salt and/or solvate thereof wherein the compound is one selected from Table 1.

**Table 1. Exemplary Compounds of the Present Disclosure.**

| | | |
|---|---|---|
| **1** | 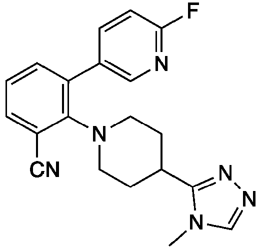 | 3-(6-fluoropyridin-3-yl)-2-[4-(4-methyl-4H-1,2,4-triazol-3-yl)piperidin-1-yl]benzonitrile |
| **2** | 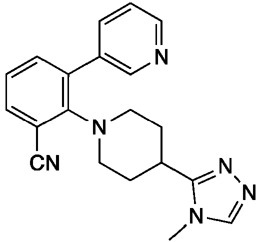 | 2-[4-(4-methyl-4H-1,2,4-triazol-3-yl)piperidin-1-yl]-3-(pyridin-3-yl)benzonitrile |
| **3** | 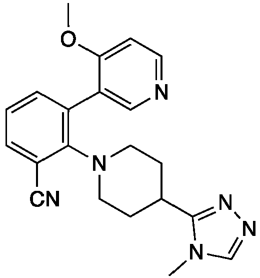 | 3-(4-methoxypyridin-3-yl)-2-[4-(4-methyl-4H-1,2,4-triazol-3-yl)piperidin-1-yl]benzonitrile |
| **4** | 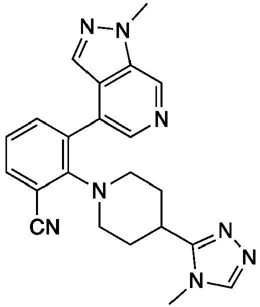 | 3-{ 1-methyl-1H-pyrazolo[3,4-c]pyridin-4-yl}-2-[4-(4-methyl-4H-1,2,4-triazol-3-yl)piperidin-1-yl]benzonitrile |
| **5** | 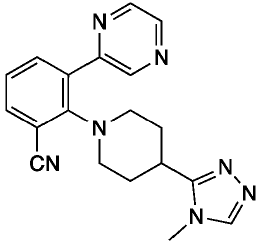 | 2-[4-(4-methyl-4H-1,2,4-triazol-3-yl)piperidin-1-yl]-3-(pyrazin-2-yl)benzonitrile |
| **6** | 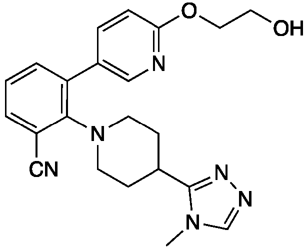 | 3-[6-(2-hydroxyethoxy)pyridin-3-yl]-2-[4-(4-methyl-4H-1,2,4-triazol-3-yl)piperidin-1-yl]benzonitrile |
| **7** | 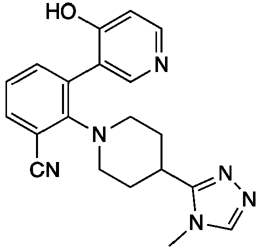 | 3-(4-hydroxypyridin-3-yl)-2-[4-(4-methyl-4H-1,2,4-triazol-3-yl)piperidin-1-yl]benzonitrile |
| **8** | 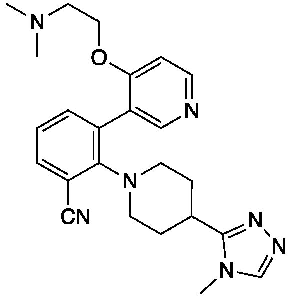 | 3-{ 4-[2-(dimethylamino)ethoxy]pyridin-3-yl}-2-[4-(4-methyl-4H-1,2,4-triazol-3-yl)piperidin-1-yl]benzonitrile |
| **9** | 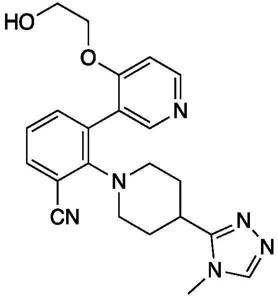 | 3-[4-(2-hydroxyethoxy)pyridin-3-yl]-2-[4-(4-methyl-4H-1,2,4-triazol-3-yl)piperidin-1-yl]benzonitrile |
| **10** | 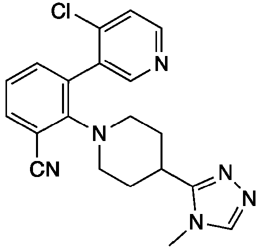 | 3-(4-chloropyridin-3-yl)-2-(4-(4-methyl-4H-1,2,4-triazol-3-yl)piperidin-1-yl)benzonitrile |
| **11** | 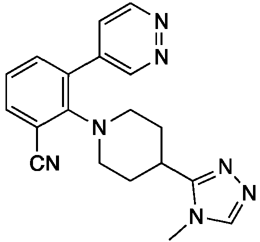 | 2-[4-(4-methyl-4H-1,2,4-triazol-3-yl)piperidin-1-yl]-3-(pyridazin-4-yl)benzonitrile |
| **12** | 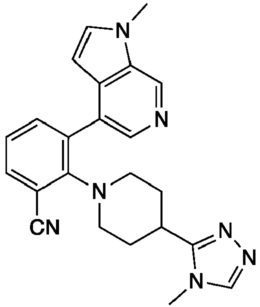 | 3-{1-methyl-1H-pyrrolo[2,3-c]pyridin-4-yl}-2-[4-(4-methyl-4H-1,2,4-triazol-3-yl)piperidin-1-yl]benzonitrile |
| **13** | 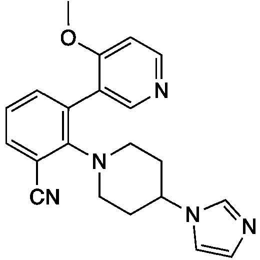 | 2-[4-(1H-imidazol-1-yl)piperidin-1-yl]-3-(4-methoxypyridin-3-yl)benzonitrile |
| **14** | 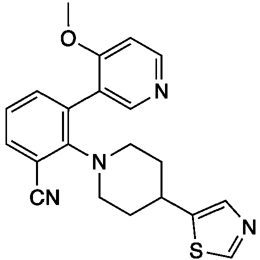 | 3-(4-methoxypyridin-3-yl)-2-[4-(1,3-thiazol-5-yl)piperidin-1-yl]benzonitrile |
| **15** | 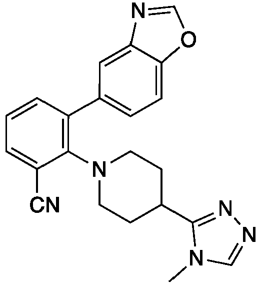 | 3-(1,3-benzoxazol-5-yl)-2-[4-(4-methyl-4H-1,2,4-triazol-3-yl)piperidin-1-yl]benzonitrile |
| **16** | 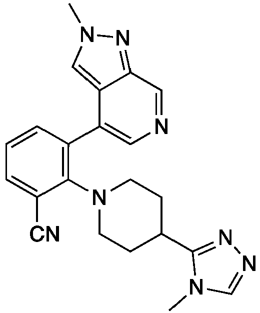 | 3-{2-methyl-2H-pyrazolo[3,4-c]pyridin-4-yl}-2-[4-(4-methyl-4H-1,2,4-triazol-3-yl)piperidin-1-yl]benzonitrile |
| **17** | 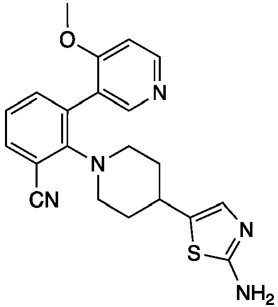 | 2-[4-(2-amino-1,3-thiazol-5-yl)piperidin-1-yl]-3-(4-methoxypyridin-3-yl)benzonitrile |
| **18** | 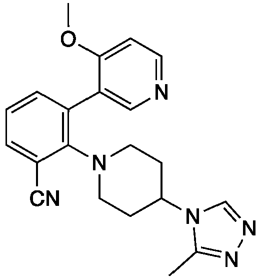 | 3-(4-methoxypyridin-3-yl)-2-[4-(3-methyl-4H-1,2,4-triazol-4-yl)piperidin-1-yl]benzonitrile |
| **19** | 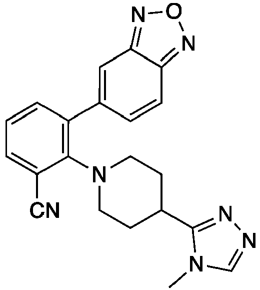 | 3-(2,1,3-benzoxadiazol-5-yl)-2-[4-(4-methyl-4H-1,2,4-triazol-3-yl)piperidin-1-yl]benzonitrile |
| **20** | 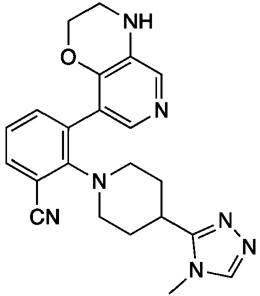 | 2-[4-(4-methyl-4H-1,2,4-triazol-3-yl)piperidin-1-yl]-3-{2H,3H,4H-pyrido[4,3-b][1,4]oxazin-8-yl} benzonitrile |
| **21** | 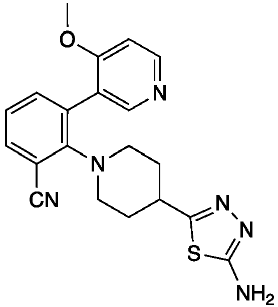 | 2-(4-(5-amino-1,3,4-thiadiazol-2-yl)piperidin-1-yl)-3-(4-methoxypyridin-3-yl)benzonitrile |
| **22** | 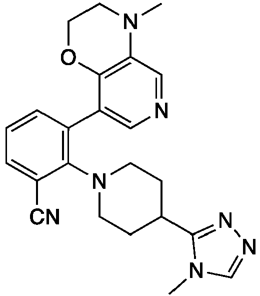 | 3-{4-methyl-2H,3H,4H-pyrido[4,3-b][1,4]oxazin-8-yl}-2-[4-(4-methyl-4H-1,2,4-triazol-3-yl)piperidin-1-yl]benzonitrile |
| **23** | 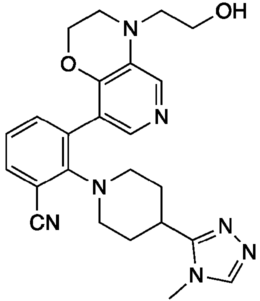 | 3-[4-(2-hydroxyethyl)-2H,3H,4H-pyrido[4,3-b][1,4]oxazin-8-yl]-2-[4-(4-methyl-4H-1,2,4-triazol-3-yl)piperidin-1-yl]benzonitrile |
| **24** | 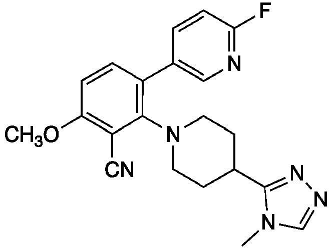 | 3-(6-fluoropyridin-3-yl)-6-methoxy-2-[4-(4-methyl-4H-1,2,4-triazol-3-yl)piperidin-1-yl]benzonitrile |
| **25** | 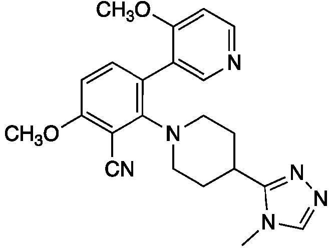 | 6-methoxy-3-(4-methoxypyridin-3-yl)-2-[4-(4-methyl-4H-1,2,4-triazol-3-yl)piperidin-1-yl]benzonitrile |
| **26** | 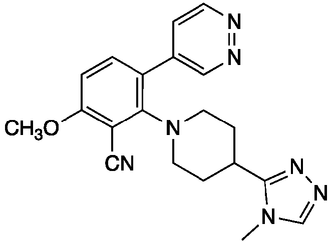 | 6-methoxy-2-[4-(4-methyl-4H-1,2,4-triazol-3-yl)piperidin-1-yl]-3-(pyridazin-4-yl)benzonitrile |
| **27** | 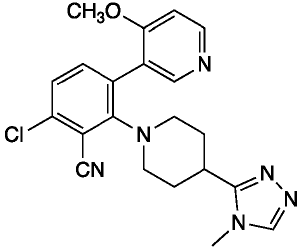 | 6-chloro-3-(4-methoxypyridin-3-yl)-2-[4-(4-methyl-4H-1,2,4-triazol-3-yl)piperidin-1-yl]benzonitrile |
| **28** | 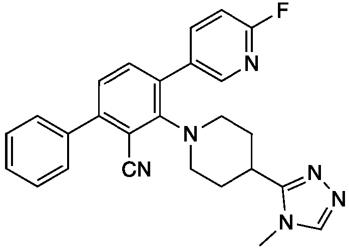 | 4-(6-fluoropyridin-3-yl)-3-[4-(4-methyl-4H-1,2,4-triazol-3-yl)piperidin-1-yl]-[1,1'-biphenyl]-2-carbonitrile |
| **29** | 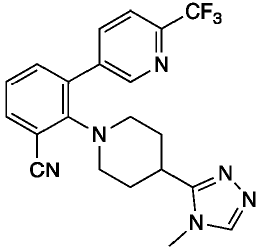 | 2-[4-(4-methyl-4H-1,2,4-triazol-3-yl)piperidin-1-yl]-3-[6-(trifluoromethyl)pyridin-3-yl]benzonitrile |
| **30** | 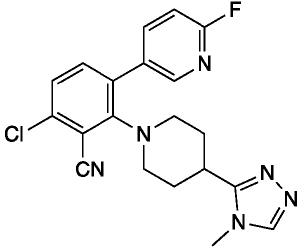 | 6-chloro-3-(6-fluoropyridin-3-yl)-2-[4-(4-methyl-4H-1,2,4-triazol-3-yl)piperidin-1-yl]benzonitrile |
| **31** | 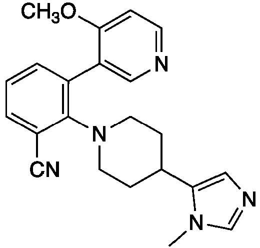 | 3-(4-methoxypyridin-3-yl)-2-[4-(1-methyl-1H-imidazol-5-yl)piperidin-1-yl]benzonitrile |
| **32** | 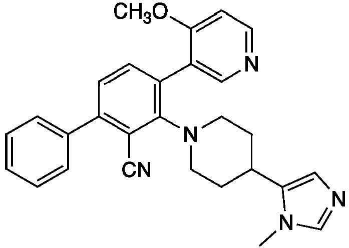 | 4-(4-methoxypyridin-3-yl)-3-[4-(4-methyl-4H-1,2,4-triazol-3-yl)piperidin-1-yl]-[1,1'-biphenyl]-2-carbonitrile |
| **33** | 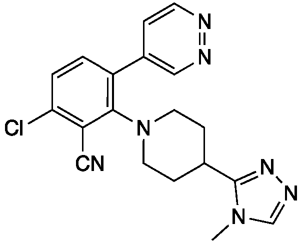 | 6-chloro-2-[4-(4-methyl-4H-1,2,4-triazol-3-yl)piperidin-1-yl]-3-(pyridazin-4-yl)benzonitrile |
| **34** | 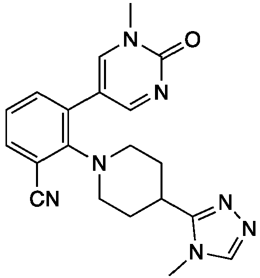 | 3-(1-methyl-2-oxo-1,2-dihydropyrimidin-5-yl)-2-[4-(4-methyl-4H-1,2,4-triazol-3-yl)piperidin-1-yl]benzonitrile |
| **35** | 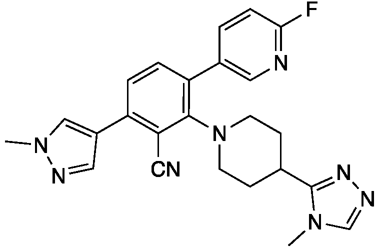 | 3-(6-fluoropyridin-3-yl)-6-(1-methyl-1H-pyrazol-4-yl)-2-[4-(4-methyl-4H-1,2,4-triazol-3-yl)piperidin-1-yl]benzonitrile |
| **36** | 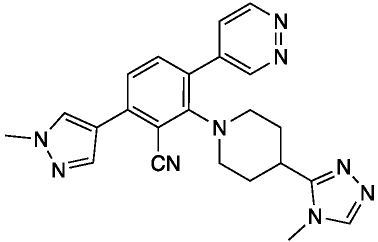 | 6-(1-methyl-1H-pyrazol-4-yl)-2-[4-(4-methyl-4H-1,2,4-triazol-3-yl)piperidin-1-yl]-3-(pyridazin-4-yl)benzonitrile |
| **37** | 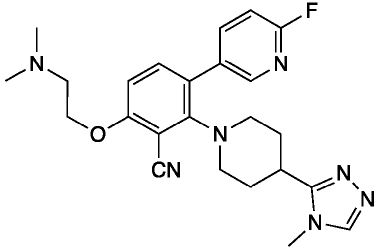 | 6-[2-(dimethylamino)ethoxy]-3-(6-fluoropyridin-3-yl)-2-[4-(4-methyl-4H-1,2,4-triazol-3-yl)piperidin-1-yl]benzonitrile |
| **38** | 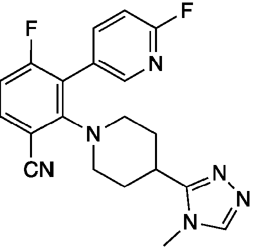 | 4-fluoro-3-(6-fluoropyridin-3-yl)-2-[4-(4-methyl-4H-1,2,4-triazol-3-yl)piperidin-1-yl]benzonitrile |
| **39** | 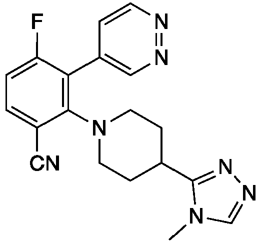 | 4-fluoro-2-[4-(4-methyl-4H-1,2,4-triazol-3-yl)piperidin-1-yl]-3-(pyridazin-4-yl)benzonitrile |
| **40** | 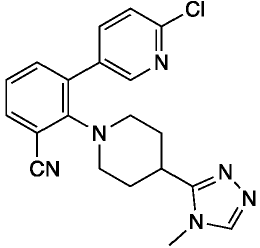 | 3-(6-chloropyridin-3-yl)-2-[4-(4-methyl-4H-1,2,4-triazol-3-yl)piperidin-1-yl]benzonitrile |
| **41** | 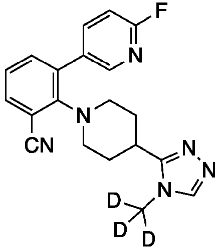 | 3-(6-fluoropyridin-3-yl)-2-(4-(4-(methyl-d3)-4H-1,2,4-triazol-3-yl)piperidin-1-yl)benzonitrile |
| **42** | 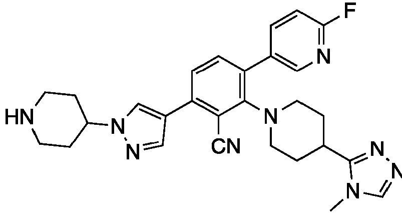 | 3-(6-fluoropyridin-3-yl)-2-(4-(4-methyl-4H-1,2,4-triazol-3-yl)piperidin-1-yl)-6-(1-(piperidin-4-yl)-1H-pyrazol-4-yl)benzonitrile |
| **43** | 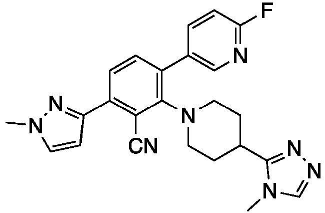 | 3-(6-fluoropyridin-3-yl)-6-(1-methyl-1H-pyrazol-3-yl)-2-(4-(4-methyl-4H-1,2,4-triazol-3-yl)piperidin-1-yl)benzonitrile |
| **44** | 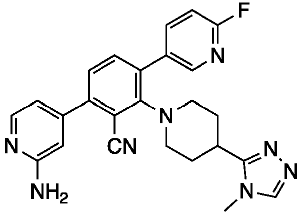 | 6-(2-aminopyridin-4-yl)-3-(6-fluoropyridin-3-yl)-2-(4-(4-methyl-4H-1,2,4-triazol-3-yl)piperidin-1-yl)benzonitrile |
| **45** | 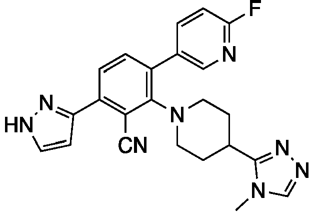 | 3-(6-fluoropyridin-3-yl)-2-(4-(4-methyl-4H-1,2,4-triazol-3-yl)piperidin-1-yl)-6-(1H-pyrazol-3-yl)benzonitrile |
| **46** | 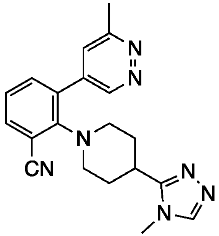 | 2-(4-(4-methyl-4H-1,2,4-triazol-3-yl)piperidin-1-yl)-3-(6-methylpyridazin-4-yl)benzonitrile |
| **47** | 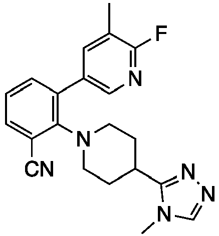 | 3-(6-fluoro-5-methylpyridin-3-yl)-2-(4-(4-methyl-4H-1,2,4-triazol-3-yl)piperidin-1-yl)benzonitrile |
| **48** | 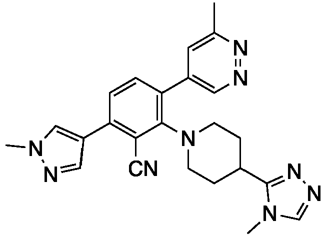 | 6-(1-methyl-1H-pyrazol-4-yl)-2-(4-(4-methyl-4H-1,2,4-triazol-3-yl)piperidin-1-yl)-3-(6-methylpyridazin-4-yl)benzonitrile |
| **49** | 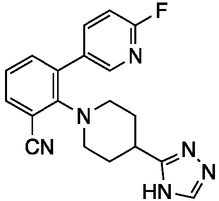 | 2-(4-(4H-1,2,4-triazol-3-yl)piperidin-1-yl)-3-(6-fluoropyridin-3-yl)benzonitrile |
| **50** | 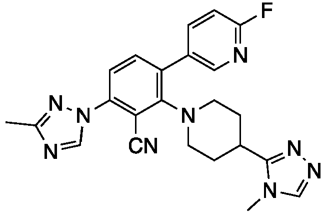 | 3-(6-fluoropyridin-3-yl)-6-(3-methyl-1H-1,2,4-triazol-1-yl)-2-(4-(4-methyl-4H-1,2,4-triazol-3-yl)piperidin-1-yl)benzonitrile |
| **51** | 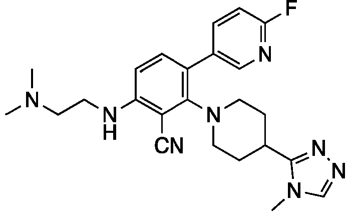 | 6-((2-(dimethylamino)ethyl)amino)-3-(6-fluoropyridin-3-yl)-2-(4-(4-methyl-4H-1,2,4-triazol-3-yl)piperidin-1-yl)benzonitrile |
| **52** | 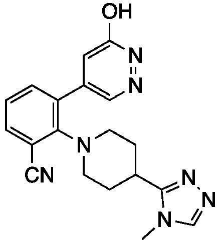 | 3-(6-hydroxypyridazin-4-yl)-2-(4-(4-methyl-4H-1,2,4-triazol-3-yl)piperidin-1-yl)benzonitrile |
| **53** | 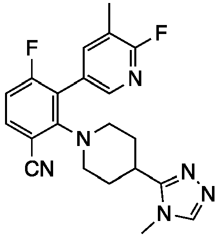 | 4-fluoro-3-(6-fluoro-5-methylpyridin-3-yl)-2-(4-(4-methyl-4H-1,2,4-triazol-3-yl)piperidin-1-yl)benzonitrile |
| **54** | 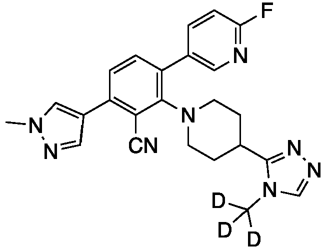 | 3-(6-fluoropyridin-3-yl)-6-(1-methyl-1H-pyrazol-4-yl)-2-(4-(4-(methyl-d3)-4H-1,2,4-triazol-3-yl)piperidin-1-yl)benzonitrile |
| **55** | 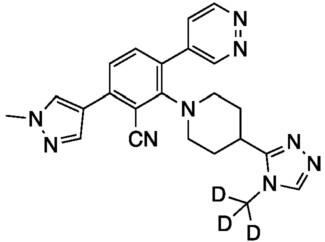 | 6-(1-methyl-1H-pyrazol-4-yl)-2-(4-(4-(methyl-d3)-4H-1,2,4-triazol-3-yl)piperidin-1-yl)-3-(pyridazin-4-yl)benzonitrile |
| **56** | 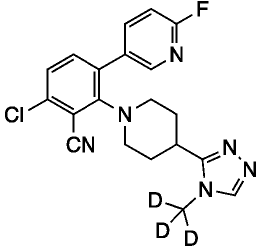 | 6-chloro-3-(6-fluoropyridin-3-yl)-2-(4-(4-(methyl-d3)-4H-1,2,4-triazol-3-yl)piperidin-1-yl)benzonitrile |
| **57** | 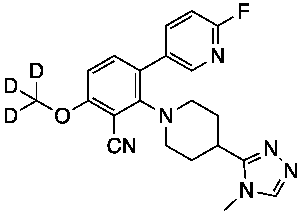 | 3-(6-fluoropyridin-3-yl)-6-(methoxy-d3)-2-(4-(4-methyl-4H-1,2,4-triazol-3-yl)piperidin-1-yl)benzonitrile |
| **58** | 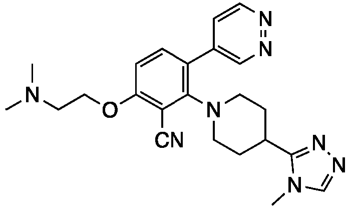 | 6-(2-(dimethylamino)ethoxy)-2-(4-(4-methyl-4H-1,2,4-triazol-3-yl)piperidin-1-yl)-3-(pyridazin-4-yl)benzonitrile |
| **59** | 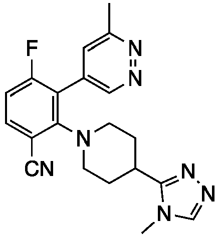 | 4-fluoro-2-(4-(4-methyl-4H-1,2,4-triazol-3-yl)piperidin-1-yl)-3-(6-methylpyridazin-4-yl)benzonitrile |
| **60** | 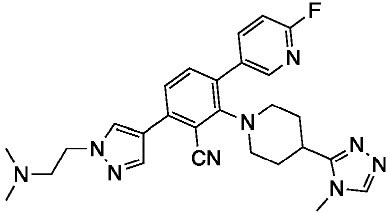 | 6-(1-(2-(dimethylamino)ethyl)-1H-pyrazol-4-yl)-3-(6-fluoropyridin-3-yl)-2-(4-(4-methyl-4H-1,2,4-triazol-3-yl)piperidin-1-yl)benzonitrile |
| **61** | 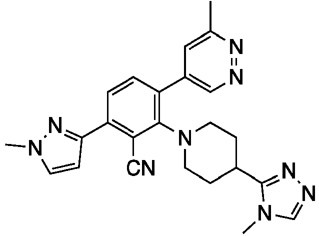 | 6-(1-methyl-1H-pyrazol-3-yl)-2-(4-(4-methyl-4H-1,2,4-triazol-3-yl)piperidin-1-yl)-3-(6-methylpyridazin-4-yl)benzonitrile |
| **62** | 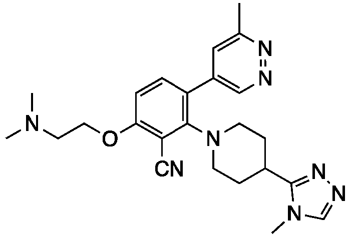 | 6-(2-(dimethylamino)ethoxy)-2-(4-(4-methyl-4H-1,2,4-triazol-3-yl)piperidin-1-yl)-3-(6-methylpyridazin-4-yl)benzonitrile |
| **63** | 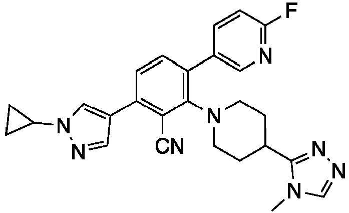 | 6-(1-cyclopropyl-1H-pyrazol-4-yl)-3-(6-fluoropyridin-3-yl)-2-(4-(4-methyl-4H-1,2,4-triazol-3-yl)piperidin-1-yl)benzonitrile |
| **64** | 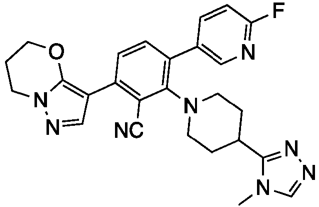 | 6-(6,7-dihydro-5H-pyrazolo[5,1-b][1,3]oxazin-3-yl)-3-(6-fluoropyridin-3-yl)-2-(4-(4-methyl-4H-1,2,4-triazol-3-yl)piperidin-1-yl)benzonitrile |
| **65** | 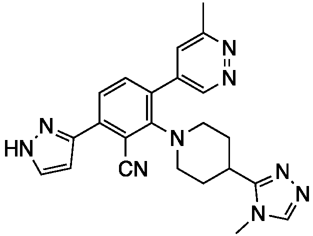 | 2-(4-(4-methyl-4H-1,2,4-triazol-3-yl)piperidin-1-yl)-3-(6-methylpyridazin-4-yl)-6-(1H-pyrazol-3-yl)benzonitrile |
| **66** | 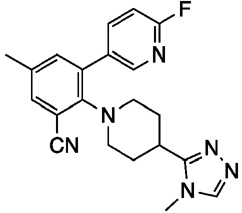 | 3-(6-fluoropyridin-3-yl)-5-methyl-2-(4-(4-methyl-4H-1,2,4-triazol-3-yl)piperidin-1-yl)benzonitrile |
| **67** | 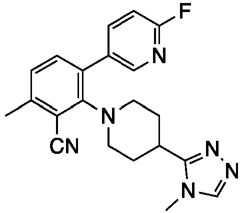 | 3-(6-fluoropyridin-3-yl)-6-methyl-2-(4-(4-methyl-4H-1,2,4-triazol-3-yl)piperidin-1-yl)benzonitrile |
| **68** | 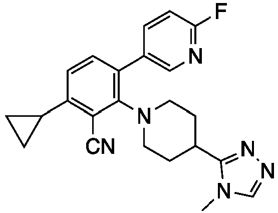 | 6-cyclopropyl-3-(6-fluoropyridin-3-yl)-2-(4-(4-methyl-4H-1,2,4-triazol-3-yl)piperidin-1-yl)benzonitrile |
| **69** | 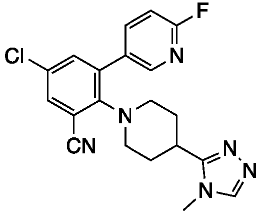 | 5-chloro-3-(6-fluoropyridin-3-yl)-2-(4-(4-methyl-4H-1,2,4-triazol-3-yl)piperidin-1-yl)benzonitrile |
| **70** | 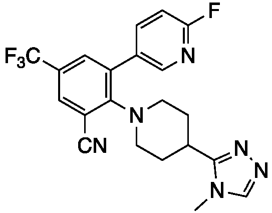 | 3-(6-fluoropyridin-3-yl)-2-(4-(4-methyl-4H-1,2,4-triazol-3-yl)piperidin-1-yl)-5-(trifluoromethyl)benzonitrile |
| **71** | 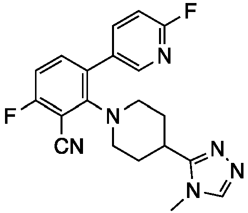 | 6-fluoro-3-(6-fluoropyridin-3-yl)-2-(4-(4-methyl-4H-1,2,4-triazol-3-yl)piperidin-1-yl)benzonitrile |
| **72** | 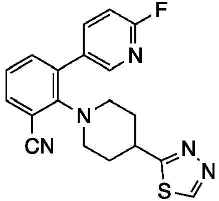 | 2-(4-(1,3,4-thiadiazol-2-yl)piperidin-1-yl)-3-(6-fluoropyridin-3-yl)benzonitrile |
| **73** | 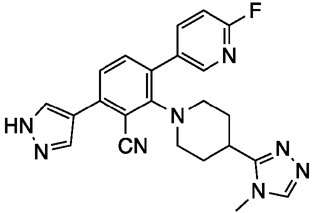 | 3-(6-fluoropyridin-3-yl)-2-(4-(4-methyl-4H-1,2,4-triazol-3-yl)piperidin-1-yl)-6-(1H-pyrazol-4-yl)benzonitrile |
| **74** | 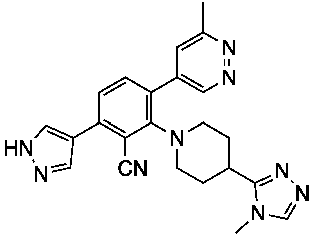 | 2-(4-(4-methyl-4H-1,2,4-triazol-3-yl)piperidin-1-yl)-3-(6-methylpyridazin-4-yl)-6-(1H-pyrazol-4-yl)benzonitrile |
| **75** | 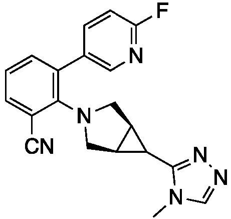 | 3-(6-fluoropyridin-3-yl)-2-((1R,5S)-6-(4-methyl-4H-1,2,4-triazol-3-yl)-3-azabicyclo[3.1.0]hexan-3-yl)benzonitrile |
| **76** | 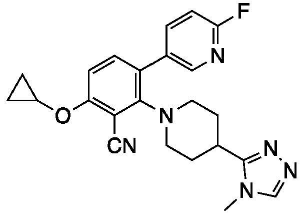 | 6-cyclopropoxy-3-(6-fluoropyridin-3-yl)-2-(4-(4-methyl-4H-1,2,4-triazol-3-yl)piperidin-1-yl)benzonitrile |
| **77** | 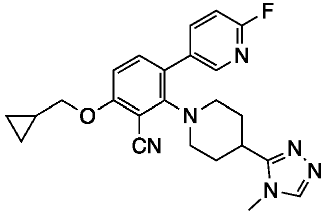 | 6-(cyclopropylmethoxy)-3-(6-fluoropyridin-3-yl)-2-(4-(4-methyl-4H-1,2,4-triazol-3-yl)piperidin-1-yl)benzonitrile |
| **78** | 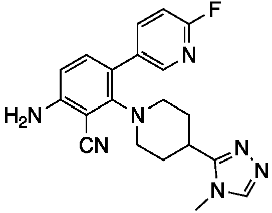 | 6-amino-3-(6-fluoropyridin-3-yl)-2-(4-(4-methyl-4H-1,2,4-triazol-3-yl)piperidin-1-yl)benzonitrile |
| **79** | 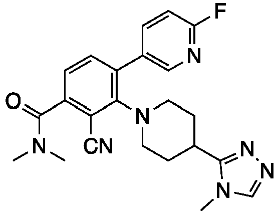 | 2-cyano-4-(6-fluoropyridin-3-yl)-N,N-dimethyl-3-(4-(4-methyl-4H-1,2,4-triazol-3-yl)piperidin-1-yl)benzamide |
| **80** | 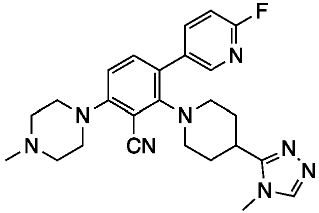 | 3-(6-fluoropyridin-3-yl)-2-(4-(4-methyl-4H-1,2,4-triazol-3-yl)piperidin-1-yl)-6-(4-methylpiperazin-1-yl)benzonitrile |
| **81** | 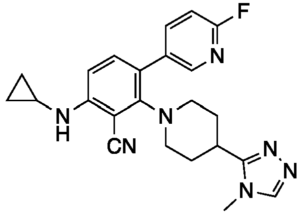 | 6-(cyclopropylamino)-3-(6-fluoropyridin-3-yl)-2-(4-(4-methyl-4H-1,2,4-triazol-3-yl)piperidin-1-yl)benzonitrile |
| **82** | 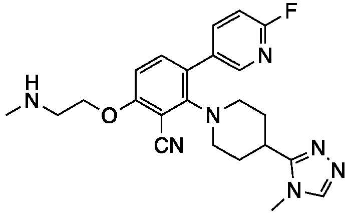 | 3-(6-fluoropyridin-3-yl)-2-(4-(4-methyl-4H-1,2,4-triazol-3-yl)piperidin-1-yl)-6-(2-(methylamino)ethoxy)benzonitrile |
| **83** | 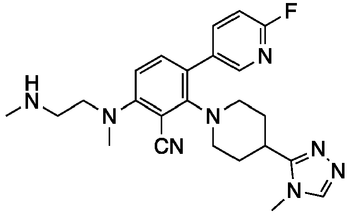 | 3-(6-fluoropyridin-3-yl)-6-(methyl(2-(methylamino)ethyl)amino)-2-(4-(4-methyl-4H-1,2,4-triazol-3-yl)piperidin-1-yl)benzonitrile |
| **84** | 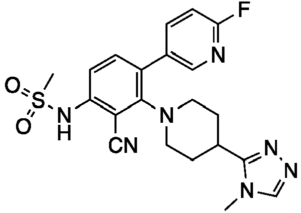 | N-(2-cyano-4-(6-fluoropyridin-3-yl)-3-(4-(4-methyl-4H-1,2,4-triazol-3-yl)piperidin-1-yl)phenyl)methanesulfonamide |
| **85** | 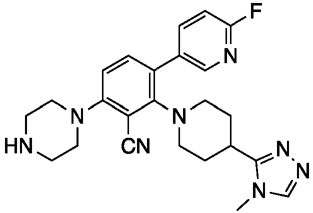 | 3-(6-fluoropyridin-3-yl)-2-(4-(4-methyl-4H-1,2,4-triazol-3-yl)piperidin-1-yl)-6-(piperazin-1-yl)benzonitrile |
| **86** | 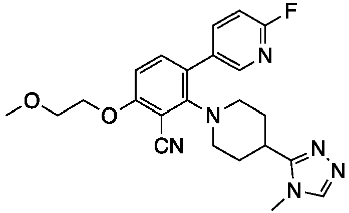 | 3-(6-fluoropyridin-3-yl)-6-(2-methoxyethoxy)-2-(4-(4-methyl-4H-1,2,4-triazol-3-yl)piperidin-1-yl)benzonitrile |
| **87** | 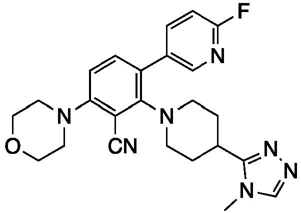 | 3-(6-fluoropyridin-3-yl)-2-(4-(4-methyl-4H-1,2,4-triazol-3-yl)piperidin-1-yl)-6-morpholinobenzonitrile |
| **88** | 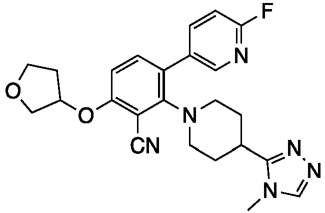 | 3-(6-fluoropyridin-3-yl)-2-(4-(4-methyl-4H-1,2,4-triazol-3-yl)piperidin-1-yl)-6-((tetrahydrofuran-3-yl)oxy)benzonitrile |
| | | Absolute stereochemistry not assigned (Isomer A) |
| **89** | 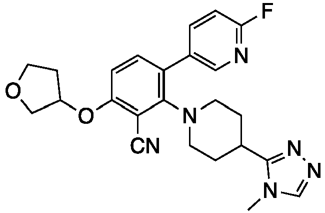 | 3-(6-fluoropyridin-3-yl)-2-(4-(4-methyl-4H-1,2,4-triazol-3-yl)piperidin-1-yl)-6-((tetrahydrofuran-3-yl)oxy)benzonitrile |
| | | Absolute stereochemistry not assigned (Isomer B) |
| **90** | 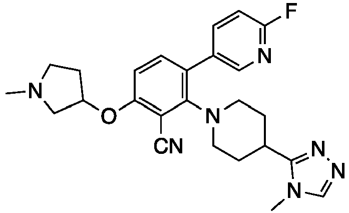 | 3-(6-fluoropyridin-3-yl)-2-(4-(4-methyl-4H-1,2,4-triazol-3-yl)piperidin-1-yl)-6-((1-methylpyrrolidin-3-yl)oxy)benzonitrile |
| | | Absolute stereochemistry not assigned (Isomer A) |
| **91** | 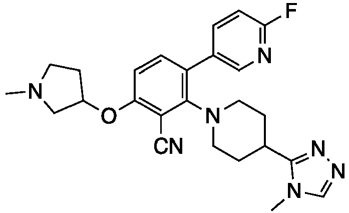 | 3-(6-fluoropyridin-3-yl)-2-(4-(4-methyl-4H-1,2,4-triazol-3-yl)piperidin-1-yl)-6-((1-methylpyrrolidin-3-yl)oxy)benzonitrile |
| | | Absolute stereochemistry not assigned (Isomer B) |
| **92** | 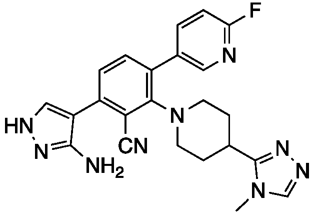 | 6-(3-amino-1H-pyrazol-4-yl)-3-(6-fluoropyridin-3-yl)-2-(4-(4-methyl-4H-1,2,4-triazol-3-yl)piperidin-1-yl)benzonitrile |
| **93** | 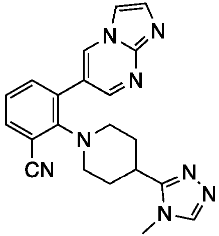 | 3-(imidazo[1,2-a]pyrimidin-6-yl)-2-(4-(4-methyl-4H-1,2,4-triazol-3-yl)piperidin-1-yl)benzonitrile |
| **94** | 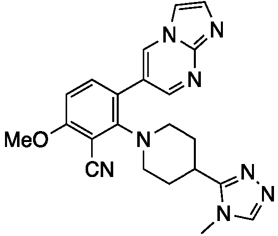 | 3-(imidazo[1,2-a]pyrimidin-6-yl)-6-methoxy-2-(4-(4-methyl-4H-1,2,4-triazol-3-yl)piperidin-1-yl)benzonitrile |
| **95** | 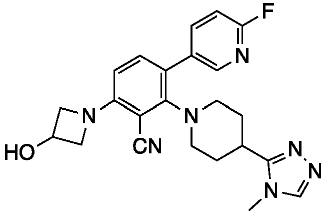 | 3-(6-fluoropyridin-3-yl)-6-(3-hydroxyazetidin-1-yl)-2-(4-(4-methyl-4H-1,2,4-triazol-3-yl)piperidin-1-yl)benzonitrile |
| **96** | 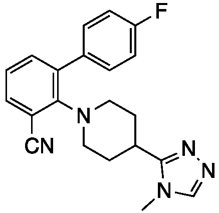 | 4'-fluoro-2-(4-(4-methyl-4H-1,2,4-triazol-3-yl)piperidin-1-yl)-[1,1'-biphenyl]-3-carbonitrile |
| **97** | 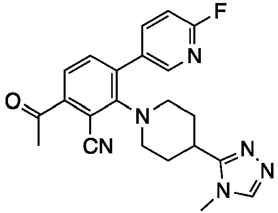 | 6-acetyl-3-(6-fluoropyridin-3-yl)-2-(4-(4-methyl-4H-1,2,4-triazol-3-yl)piperidin-1-yl)benzonitrile |
| **98** | 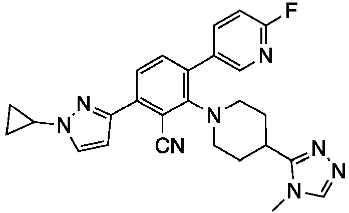 | 6-(1-cyclopropyl-1H-pyrazol-3-yl)-3-(6-fluoropyridin-3-yl)-2-(4-(4-methyl-4H-1,2,4-triazol-3-yl)piperidin-1-yl)benzonitrile |
| **99** | 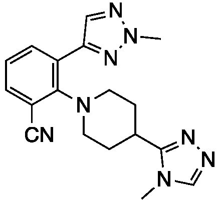 | 3-(2-methyl-2H-1,2,3-triazol-4-yl)-2-(4-(4-methyl-4H-1,2,4-triazol-3-yl)piperidin-1-yl)benzonitrile |
| **100** | 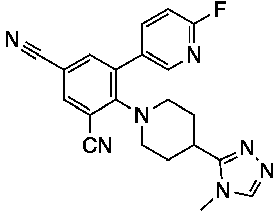 | 5-(6-fluoropyridin-3-yl)-4-(4-(4-methyl-4H-1,2,4-triazol-3-yl)piperidin-1-yl)isophthalonitrile |
| **101** | 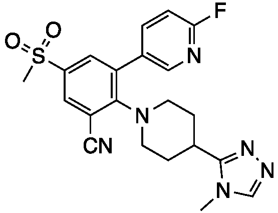 | 3-(6-fluoropyridin-3-yl)-2-(4-(4-methyl-4H-1,2,4-triazol-3-yl)piperidin-1-yl)-5-(methylsulfonyl)benzonitrile |
| **102** | 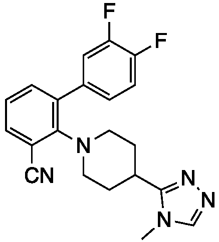 | 3',4'-difluoro-2-(4-(4-methyl-4H-1,2,4-triazol-3-yl)piperidin-1-yl)-[1,1'-biphenyl]-3-carbonitrile |
| **103** | 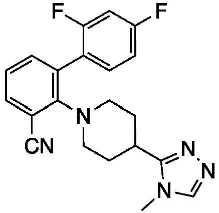 | 2',4'-difluoro-2-(4-(4-methyl-4H-1,2,4-triazol-3-yl)piperidin-1-yl)-[1,1'-biphenyl]-3-carbonitrile |
| **104** | 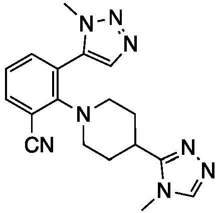 | 3-(1-methyl-1H-1,2,3-triazol-5-yl)-2-(4-(4-methyl-4H-1,2,4-triazol-3-yl)piperidin-1-yl)benzonitrile |
| **105** | 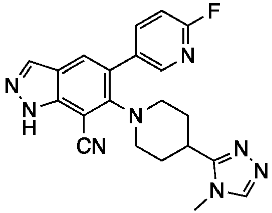 | 5-(6-fluoropyridin-3-yl)-6-(4-(4-methyl-4H-1,2,4-triazol-3-yl)piperidin-1-yl)-1H-indazole-7-carbonitrile |
| **106** | 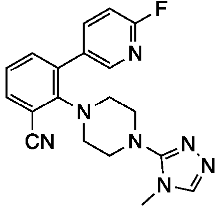 | 3-(6-fluoropyridin-3-yl)-2-(4-(4-methyl-4H-1,2,4-triazol-3-yl)piperazin-1-yl)benzonitrile |
| **107** | 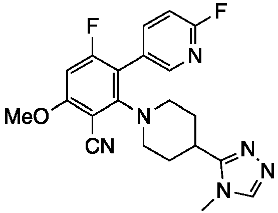 | 4-fluoro-3-(6-fluoropyridin-3-yl)-6-methoxy-2-(4-(4-methyl-4H-1,2,4-triazol-3-yl)piperidin-1-yl)benzonitrile |
| **108** | 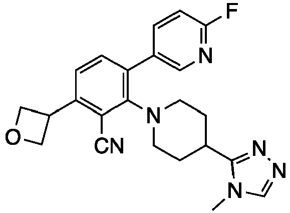 | 3-(6-fluoropyridin-3-yl)-2-(4-(4-methyl-4H-1,2,4-triazol-3-yl)piperidin-1-yl)-6-(oxetan-3-yl)benzonitrile |
| **109** | 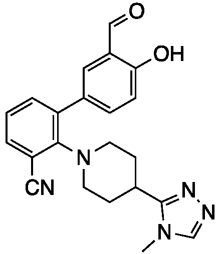 | 3'-formyl-4'-hydroxy-2-(4-(4-methyl-4H-1,2,4-triazol-3-yl)piperidin-1-yl)-[1,1'-biphenyl]-3-carbonitrile |
| **110** | 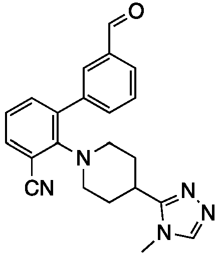 | 3'-formyl-2-(4-(4-methyl-4H-1,2,4-triazol-3-yl)piperidin-1-yl)-[1,1'-biphenyl]-3-carbonitrile |
| **111** | 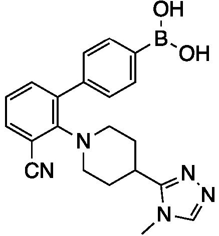 | (3'-cyano-2'-(4-(4-methyl-4H-1,2,4-triazol-3-yl)piperidin-1-yl)-[1,1'-biphenyl]-4-yl)boronic acid |
| **112** | 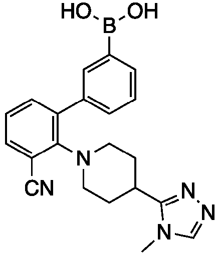 | (3'-cyano-2'-(4-(4-methyl-4H-1,2,4-triazol-3-yl)piperidin-1-yl)-[1,1'-biphenyl]-3-yl)boronic acid |
| **113** | 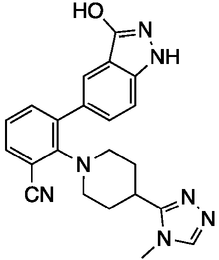 | 3-(3-hydroxy-1H-indazol-5-yl)-2-(4-(4-methyl-4H-1,2,4-triazol-3-yl)piperidin-1-yl)benzonitrile |
| **114** | 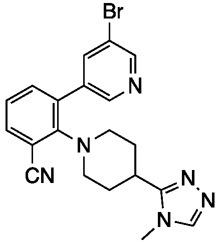 | 3-(5-bromopyridin-3-yl)-2-(4-(4-methyl-4H-1,2,4-triazol-3-yl)piperidin-1-yl)benzonitrile |
| **115** | 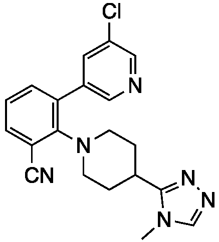 | 3-(5-chloropyridin-3-yl)-2-(4-(4-methyl-4H-1,2,4-triazol-3-yl)piperidin-1-yl)benzonitrile |
| **116** | 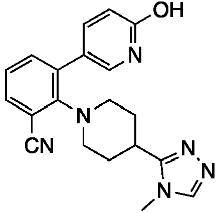 | 3-(6-hydroxypyridin-3-yl)-2-(4-(4-methyl-4H-1,2,4-triazol-3-yl)piperidin-1-yl)benzonitrile |
| **117** | 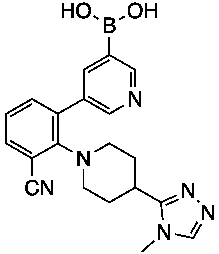 | (5-(3-cyano-2-(4-(4-methyl-4H-1,2,4-triazol-3-yl)piperidin-1-yl)phenyl)pyridin-3-yl)boronic acid |
| **118** | 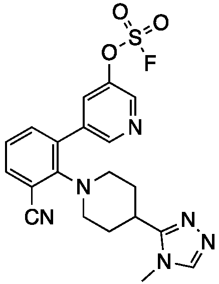 | 5-(3-cyano-2-(4-(4-methyl-4H-1,2,4-triazol-3-yl)piperidin-1-yl)phenyl)pyridin-3-yl sulfurofluoridate |
| **119** | 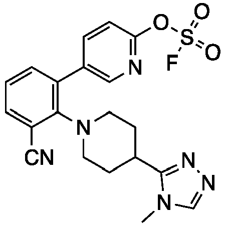 | 5-(3-cyano-2-(4-(4-methyl-4H-1,2,4-triazol-3-yl)piperidin-1-yl)phenyl)pyridin-2-yl sulfurofluoridate |
| **120** | 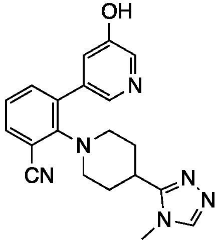 | 3-(5-hydroxypyridin-3-yl)-2-(4-(4-methyl-4H-1,2,4-triazol-3-yl)piperidin-1-yl)benzonitrile |
| **121** | 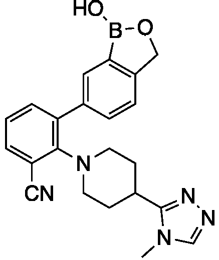 | 3-(1-hydroxy-1,3-dihydrobenzo[c][1,2]oxaborol-6-yl)-2-(4-(4-methyl-4H-1,2,4-triazol-3-yl)piperidin-1-yl)benzonitrile |
| **122** | 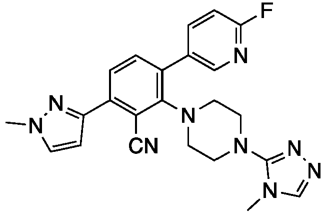 | 3-(6-fluoropyridin-3-yl)-6-(1-methyl-1H-pyrazol-3-yl)-2-(4-(4-methyl-4H-1,2,4-triazol-3-yl)piperazin-1-yl)benzonitrile |
| **123** | 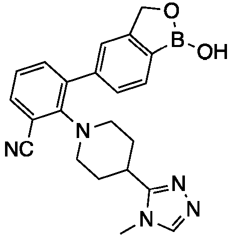 | 3-(1-hydroxy-1,3-dihydrobenzo[c][1,2]oxaborol-5-yl)-2-(4-(4-methyl-4H-1,2,4-triazol-3-yl)piperidin-1-yl)benzonitrile |
| **124** | 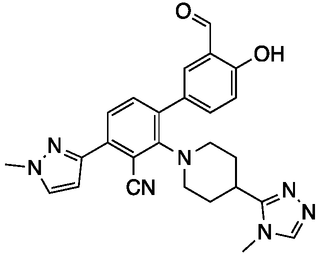 | 3'-formyl-4'-hydroxy-4-(1-methyl-1H-pyrazol-3-yl)-2-(4-(4-methyl-4H-1,2,4-triazol-3-yl)piperidin-1-yl)-[1,1'-biphenyl]-3-carbonitrile |
| **125** | 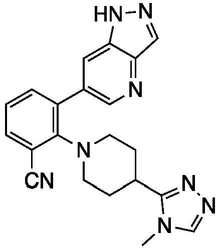 | 2-(4-(4-methyl-4H-1,2,4-triazol-3-yl)piperidin-1-yl)-3-(1H-pyrazolo[4,3-b]pyridin-6-yl)benzonitrile |
| **126** | 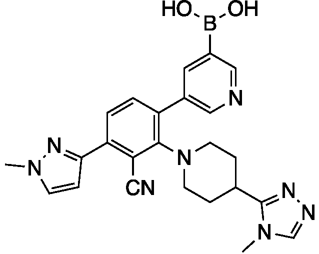 | (5-(3-cyano-4-(1-methyl-1H-pyrazol-3-yl)-2-(4-(4-methyl-4H-1,2,4-triazol-3-yl)piperidin-1-yl)phenyl)pyridin-3-yl)boronic acid |
| **127** | 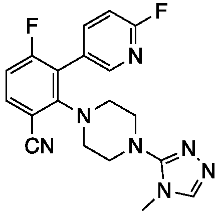 | 4-fluoro-3-(6-fluoropyridin-3-yl)-2-(4-(4-methyl-4H-1,2,4-triazol-3-yl)piperazin-1-yl)benzonitrile |
| **128** | 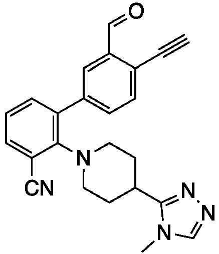 | 4'-ethynyl-3'-formyl-2-(4-(4-methyl-4H-1,2,4-triazol-3-yl)piperidin-1-yl)-[1,1'-biphenyl]-3-carbonitrile |
| **129** | 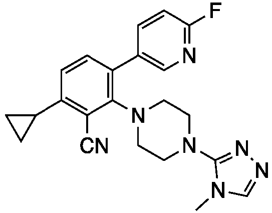 | 6-cyclopropyl-3-(6-fluoropyridin-3-yl)-2-(4-(4-methyl-4H-1,2,4-triazol-3-yl)piperazin-1-yl)benzonitrile |
| **130** | 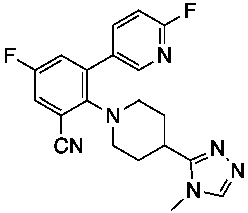 | 5-fluoro-3-(6-fluoropyridin-3-yl)-2-(4-(4-methyl-4H-1,2,4-triazol-3-yl)piperidin-1-yl)benzonitrile |
| **131** | 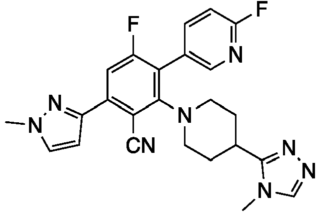 | 4-fluoro-3-(6-fluoropyridin-3-yl)-6-(1-methyl-1H-pyrazol-3-yl)-2-(4-(4-methyl-4H-1,2,4-triazol-3-yl)piperidin-1-yl)benzonitrile |
| **132** | 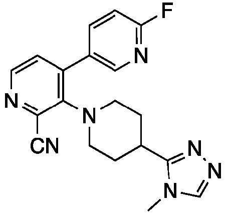 | 6-fluoro-3'-(4-(4-methyl-4H-1,2,4-triazol-3-yl)piperidin-1-yl)-[3,4'-bipyridine]-2'-carbonitrile |
| **133** | 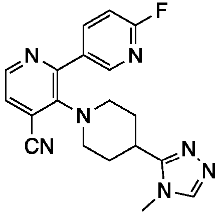 | 6'-fluoro-3-(4-(4-methyl-4H-1,2,4-triazol-3-yl)piperidin-1-yl)-[2,3'-bipyridine]-4-carbonitrile |
| **134** | 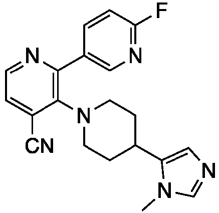 | 6'-fluoro-3-(4-(1-methyl-1H-imidazol-5-yl)piperidin-1-yl)-[2,3'-bipyridine]-4-carbonitrile |
| **135** | 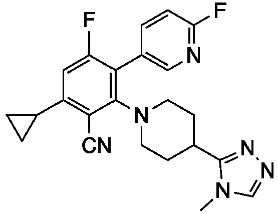 | 6-cyclopropyl-4-fluoro-3-(6-fluoropyridin-3-yl)-2-(4-(4-methyl-4H-1,2,4-triazol-3-yl)piperidin-1-yl)benzonitrile |
| **136** | 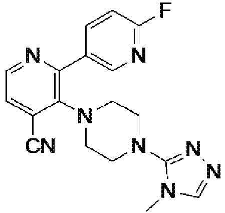 | 6'-fluoro-3-(4-(4-methyl-4H-1,2,4-triazol-3-yl)piperazin-1-yl)-[2,3'-bipyridine]-4-carbonitrile |
| **137** | 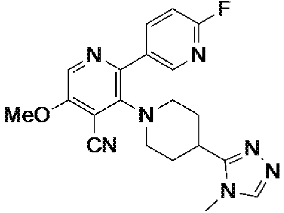 | 6'-fluoro-5-methoxy-3-(4-(4-methyl-4H-1,2,4-triazol-3-yl)piperidin-1-yl)-[2,3'-bipyridine]-4-carbonitrile |
| **138** | 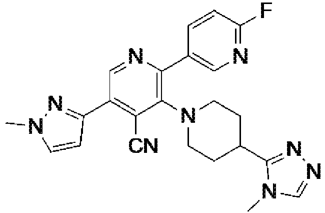 | 6'-fluoro-5-(1-methyl-1H-pyrazol-3-yl)-3-(4-(4-methyl-4H-1,2,4-triazol-3-yl)piperidin-1-yl)-[2,3'-bipyridine]-4-carbonitrile |
| **139** | 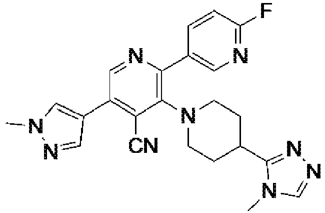 | 6'-fluoro-5-(1-methyl-1H-pyrazol-4-yl)-3-(4-(4-methyl-4H-1,2,4-triazol-3-yl)piperidin-1-yl)-[2,3'-bipyridine]-4-carbonitrile |

In additional embodiments, the present disclosure provides a compound or pharmaceutically acceptable salt and/or solvate thereof, wherein the compound is selected from Table 2.

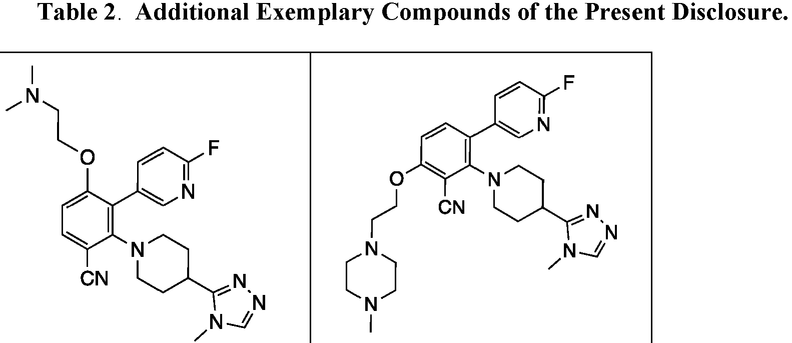

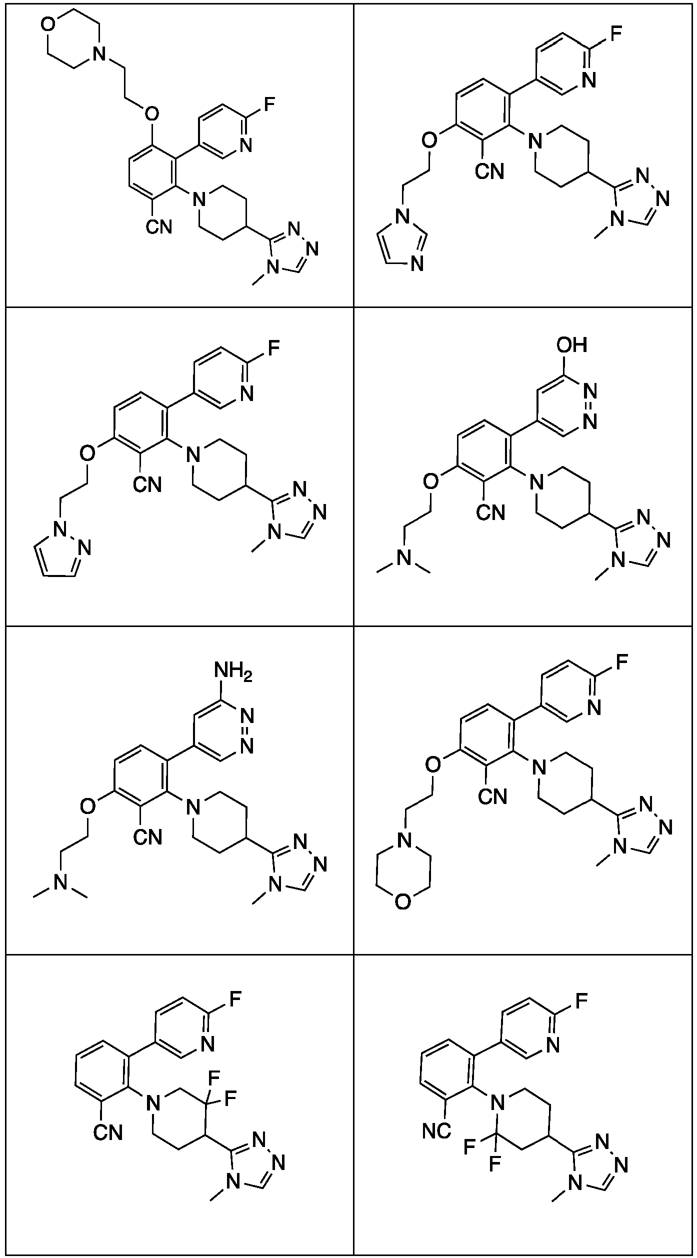

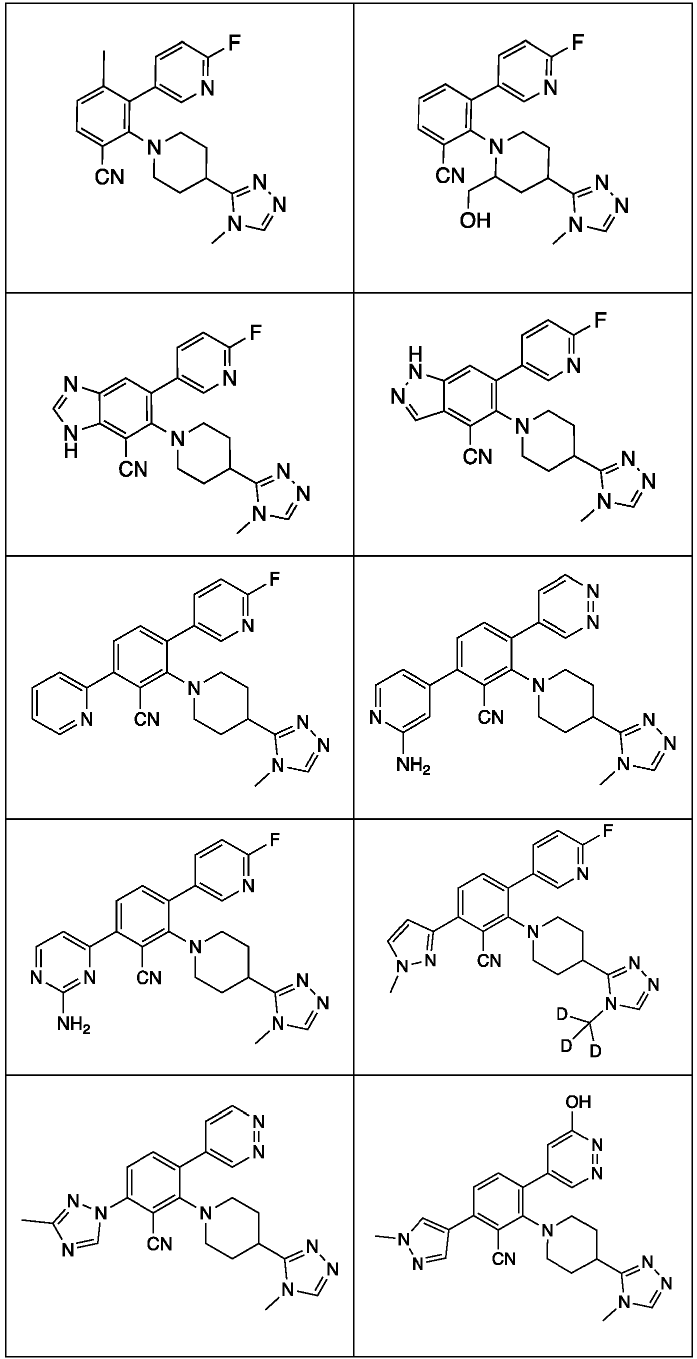

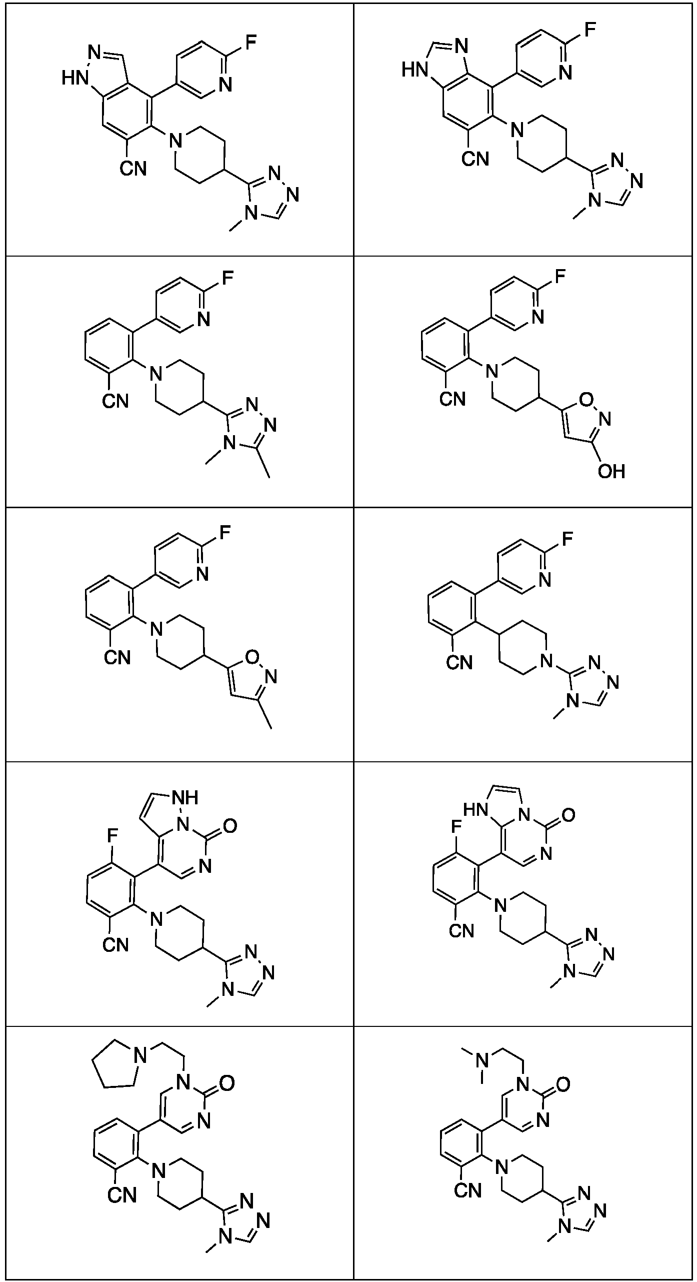

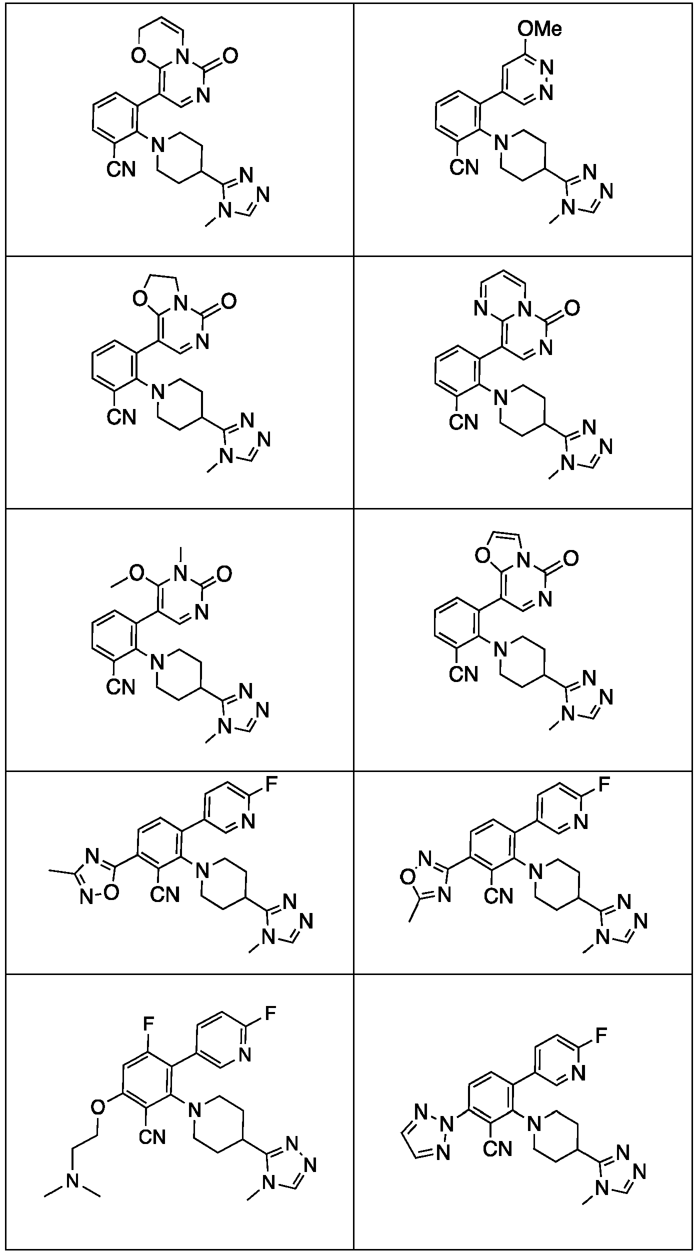

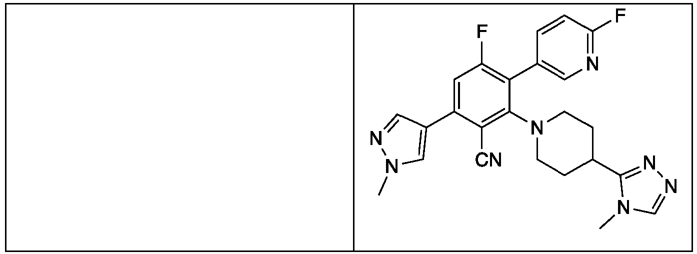

### PHARMACEUTICAL COMPOSITION

The disclosure also provides a pharmaceutical composition comprising a therapeutically effective amount of one or more compounds according to Formula I or Formula II, or a pharmaceutically acceptable salt, stereoisomer, isotopologue, and/or tautomer thereof in admixture with a pharmaceutically acceptable carrier. In some embodiments, the composition further contains, in accordance with accepted practices of pharmaceutical compounding, one or more additional therapeutic agents, pharmaceutically acceptable excipients, diluents, adjuvants, stabilizers, emulsifiers, preservatives, colorants, buffers, flavor imparting agents.

In one embodiment, the pharmaceutical composition comprises a compound selected from those illustrated in Tables 1 to 16 or a pharmaceutically acceptable salt, stereoisomer, isotopologue, and/or tautomer thereof, and a pharmaceutically acceptable carrier.

The pharmaceutical composition of the present disclosure is formulated, dosed, and administered in a fashion consistent with good medical practice. Factors for consideration in this context include the particular disorder being treated, the particular subject being treated, the clinical condition of the subject, the cause of the disorder, the site of delivery of the agent, the method of administration, the scheduling of administration, and other factors known to medical practitioners.

The "therapeutically effective amount" of a compound or a pharmaceutically acceptable salt, stereoisomer, isotopologue, and/or tautomer thereof that is administered is governed by such considerations, and is the minimum amount necessary to inhibit QPCTL, QPCT, or both. Such amount may be below the amount that is toxic to normal cells, or the subject as a whole. Generally, the initial therapeutically effective amount of a compound (or a pharmaceutically acceptable salt, stereoisomer, or tautomer thereof) of the present disclosure that is administered is in the range of about 0.01 to about 200 mg/kg or about 0.1 to about 20 mg/kg of patient body weight per day, with the typical initial range being about 0.3 to about 15 mg/kg/day. Oral unit dosage forms, such as tablets and capsules, may contain from about 0.1 mg to about 1000 mg of a compound (or a pharmaceutically acceptable salt, stereoisomer, or tautomer thereof) of the present disclosure. In another embodiment, such dosage forms contain from about 50 mg to about 500 mg of a compound (or a pharmaceutically acceptable salt, stereoisomer, or tautomer thereof) of the present disclosure. In yet another embodiment, such dosage forms contain from about 25 mg to about 200 mg of a compound (or a pharmaceutically acceptable salt, stereoisomer, or tautomer thereof) of the present disclosure. In still another embodiment, such dosage forms contain from about 10 mg to about 100 mg of a compound (or a pharmaceutically acceptable salt, stereoisomer, or tautomer thereof) of the present disclosure. In a further embodiment, such dosage forms contain from about 5 mg to about 50 mg of a compound (or a pharmaceutically acceptable salt, stereoisomer, or tautomer thereof) of the present disclosure. In any of the foregoing embodiments the dosage form can be administered once a day or twice per day.

In certain embodiments, the compound as described herein or a pharmaceutically acceptable salt or solvate thereof, is substantially pure, in that it contains less than about 5%, or less than about 2%, or less than about 1%, or less than about 0.5%, or less than about 0.1%, of other organic small molecules, such as unreacted intermediates or synthesis by-products that are created, for example, in one or more of the steps of a synthesis method.

The compositions of the present disclosure can be administered orally, topically, parenterally, by inhalation or spray or rectally in dosage unit formulations. The term parenteral as used herein includes subcutaneous injections, intravenous, intramuscular, intrasternal injection or infusion techniques.

Suitable oral compositions as described herein include without limitation tablets, troches, lozenges, aqueous or oily suspensions, dispersible powders or granules, emulsion, hard or soft capsules, syrups or elixirs.

In another aspect, also encompassed are pharmaceutical compositions suitable for single unit dosages that comprise a compound of the disclosure or its pharmaceutically acceptable stereoisomer, salt, or tautomer and a pharmaceutically acceptable carrier.

The compositions of the present disclosure that are suitable for oral use may be prepared according to any method known to the art for the manufacture of pharmaceutical compositions. For instance, liquid formulations of the compounds of the present disclosure contain one or more agents selected from the group consisting of sweetening agents, flavoring agents, coloring agents and preserving agents in order to provide pharmaceutically palatable preparations of a compound of the present disclosure.

For tablet compositions, a compound of the present disclosure in admixture with non-toxic pharmaceutically acceptable excipients is used for the manufacture of tablets. Examples of such excipients include without limitation inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known coating techniques to delay disintegration and absorption in the gastrointestinal tract and thereby to provide a sustained therapeutic action over a desired time period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

For aqueous suspensions, a compound of the present disclosure is admixed with excipients suitable for maintaining a stable suspension. Examples of such excipients include without limitation are sodium carboxymethylcellulose, methylcellulose, hydroxpropylmethylcellulose, sodium alginate, polyvinylpyrrolidone, gum tragacanth and gum acacia.

Oral suspensions can also contain dispersing or wetting agents, such as naturally-occurring phosphatide, for example, lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending a compound of the present disclosure in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol.

Sweetening agents such as those set forth above, and flavoring agents may be added to provide palatable oral preparations. These compositions may be preserved by the addition of an anti-oxidant such as ascorbic acid.

Dispersible powders and granules suitable for preparation of an aqueous suspension by the addition of water provide a compound of the present disclosure in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Pharmaceutical compositions of the present disclosure may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation reaction products of the said partial esters with ethylene oxide, for example polyoxyethylene sorbitan monoleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative, and flavoring and coloring agents. The pharmaceutical compositions may be in the form of a sterile injectable, an aqueous suspension or an oleaginous suspension. This suspension may be formulated according to the known art using those suitable dispersing or wetting agents and suspending agents which have been mentioned above. The sterile injectable preparation may also be sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of inj ectables.

A compound of the present disclosure can be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Compositions for parenteral administrations are administered in a sterile medium. Depending on the vehicle used and concentration the concentration of the drug in the formulation, the parenteral formulation can either be a suspension or a solution containing dissolved drug. Adjuvants such as local anesthetics, preservatives and buffering agents can also be added to parenteral compositions.

### METHODS OF USE

The compounds of the present disclosure are surprisingly potent inhibitors of glutaminyl-peptide cyclotransferase protein (QPCT) or glutaminyl-peptide cyclotransferase-like protein (QPCTL). The compounds are useful, in various embodiments, in a method of treating a disease in a patient suffering therefrom, wherein the disease is associated with expression of QPCT or QPCTL. The method comprises administering to the patient a compound or pharmaceutically acceptable salt and/or solvate thereof as described herein. The compound or pharmaceutically acceptable salt thereof is administered optionally in a pharmaceutical composition in accordance with the present disclosure, and by any of the routes of administration as described herein.

In some embodiments, the disease is a cancer, such as a leukemia or lymphoma. Examples of the leukemia or lymphoma include acute myeloid leukemia (AML), chronic myeloid leukemia (CML), acute lymphocytic leukemia (ALL), chronic lymphocytic leukemia (CLL), and non-Hodgkin's lymphoma (NHL), Burkitt lymphoma, hairy cell lymphoma (HCL), Waldenstrom macroglobulinemia, chronic lymphocytic leukemia/small lymphocytic lymphoma (CLL/SLL), diffuse large B cell lymphoma (DLBCL), B cell chronic lymphocytic leukemia (B-CLL), mantle cell lymphoma (MCL), follicular lymphoma (FL), marginal zone lymphoma (MZL), and pre-B acute lymphoblastic leukemia (pre-B ALL).

In additional embodiments, the cancer is selected from the group consisting of multiple myeloma (MM), ovarian cancer, gliomas, colon cancer, breast cancer, bladder cancer, gastric cancer, esophageal cancer, pancreatic cancer, liver cancer, small cell lung cancer (SCLC), non-small cell lung cancer (NSCLC), head and neck squamous cell cancer, mesothelioma, melanoma, glioma, glioblastoma, and pancreatic neuroendocrine tumors.

In still further embodiments, the cancer is selected from the group consisting of basal cell carcinoma, squamous cell carcinoma, renal cell carcinoma, invasive ductal carcinoma, adenocarcinoma, Merkel cell carcinoma, skin cancer, prostate cancer, colorectal cancer, soft tissue sarcoma, osteosarcoma, Ewing's sarcoma, chondrosarcoma, and myeloma.

The compounds of the present disclosure are potent inhibitors of QPCT, which is a druggable target in therapies for various neurogenerative diseases (M. Jimenez-Sanchez et al., Nat Chem Biol. 11(5) (2015) 347-354). These include, for example, Alzheimer's disease (A. Becker et al., BMC Neurosci 14 (2013) 108; M. Morawski et al., J Alzheimers Dis 39(2) (2014) 385-400), Parkinson's disease, amyotrophic lateral sclerosis, Friedreich ataxia, Huntington's disease, Lewy body dementia, and spinal muscular atrophy. In some embodiments, combination therapy is contemplated, wherein the compound of the present disclosure is administered in combination with an antibody that clears amyloid-beta (Aβ) plaque in the brain. Various monoclonal antibodies that bind different epitopes and conformations of Aβ are known in the art and suitable for this purpose, including but not limited to Bapineuzumab, Solanezumab, Gantenerumab, Crenezumab, Ponezumab, BAN2401, and Aducanumab (*See* C. H. van Dyck Biol. Psych. 83(4) (2018) 311 - 319).

In further embodiments, the disease is an inflammatory disease (*see, e.g.,* K. Bresser et al., Oncoimmunology 11(1) (2022) (https://doi.org/10.1080/2162402X.2022.2049486)). In other embodiments, the disease is an autoimmune disease (*see* N. Kanemitsu et al., Naunyn Schmiedebergs Arch Pharmacol. 394(4), 751 (2021)

In further embodiments, the disease is a cardiovascular disease. In an illustrative embodiment, the cardiovascular disease is atherosclerosis.

In some embodiments, optionally in combination with any other embodiment described herein, the compound of formula (I) or (II) or pharmaceutically acceptable salt and/or solvate thereof is administered in combination with an immune checkpoint inhibitor. Examples of immune checkpoint inhibitors include PD-1 inhibitors, PD-L1 inhibitors, CTLA-4 inhibitors, and LAG-3 inhibitors.

In further embodiments, the compound or pharmaceutically acceptable salt and/or solvate thereof is administered in combination with an opsonizing antibody. Opsonizing antibodies are well-known in the art, including IgG and IgM.

In additional embodiments, the present disclosure provides cormpounds for use in a method of inhibiting a glutaminyl-peptide cyclotransferase (QPCT) or glutaminyl-peptide cyclotransferase-like (QPCTL) enzyme. The method comprises contacting the enzyme with a compound or pharmaceutically acceptable salt and/or solvate thereof as described herein. In one embodiment, the contacting occurs *in vitro.* In another embodiment, the contacting occurs *in vivo.*

Also provided is a compound or pharmaceutically acceptable salt and/or solvate thereof as described herein for use in the treatment of a cancer, neurodegenerative disease, inflammatory disease, autoimmune disease, or a cardiovascular disease. The present disclosure also provides a use of a compound or pharmaceutically acceptable salt and/or solvate thereof as described herein in the manufacture of a medicament for the treatment of a cancer, neurodegenerative disease, inflammatory disease, autoimmune disease, or a cardiovascular disease.

Other embodiments and uses will be apparent to one skilled in the art in light of the present disclosure. The following examples are provided to illustrate and provide various embodiments and shall not be construed to limit the disclosure in any way.

### EXAMPLES

### Preparation of Compounds

The compounds used in the synthetic chemistry reactions described herein are made according to organic synthesis techniques known to those skilled in this art, starting from commercially available chemicals and/or from compounds described in the chemical literature. "Commercially available chemicals" are obtained from standard commercial sources including Acros Organics (Pittsburgh, PA), Aldrich Chemical (Milwaukee, WI, including Sigma Chemical and Fluka), Apin Chemicals Ltd. (Milton Park, UK), Avocado Research (Lancashire, U.K.), BDH Inc. (Toronto, Canada), Bionet (Cornwall, U.K.), Chemservice Inc. (West Chester, PA), Crescent Chemical Co. (Hauppauge, NY), Eastman Organic Chemicals, Eastman Kodak Company (Rochester, NY), Fisher Scientific Co. (Pittsburgh, PA), Fisons Chemicals (Leicestershire, UK), Frontier Scientific (Logan, UT), ICN Biomedicals, Inc. (Costa Mesa, CA), Key Organics (Cornwall, U.K.), Lancaster Synthesis (Windham, NH), Maybridge Chemical Co. Ltd. (Cornwall, U.K.), Parish Chemical Co. (Orem, UT), Pfaltz & Bauer, Inc. (Waterbury, CN), Polyorganix (Houston, TX), Pierce Chemical Co. (Rockford, IL), Riedel de Haen AG (Hanover, Germany), Spectrum Quality Product, Inc. (New Brunswick, NJ), TCI America (Portland, OR), Trans World Chemicals, Inc. (Rockville, MD), and Wako Chemicals USA, Inc. (Richmond, VA).

Suitable reference books and treatise that detail the synthesis of reactants useful in the preparation of compounds described herein, or provide references to articles that describe the preparation, include for example, "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992. Additional suitable reference books and treatise that detail the synthesis of reactants useful in the preparation of compounds described herein, or provide references to articles that describe the preparation, include for example, Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and analogous reactants are optionally identified through the indices of known chemicals prepared by the Chemical Abstract Service of the American Chemical Society, which are available in most public and university libraries, as well as through on-line databases (contact the American Chemical Society, Washington, D.C. for more details). Chemicals that are known but not commercially available in catalogs are optionally prepared by custom chemical synthesis houses, where many of the standard chemical supply houses (e.g., those listed above) provide custom synthesis services. A reference useful for the preparation and selection of pharmaceutical salts of the compounds described herein is P. H. Stahl & C. G. Wermuth "Handbook of Pharmaceutical Salts", Verlag Helvetica Chimica Acta, Zurich, 2002.

### General Synthetic Schemes

The compounds disclosed herein are prepared by a variety of synthetic routes including, but not limited to, the routes described below in Scheme I or II.

As shown below in Scheme I, suitably functionalized 2-fluoro-3-bromobenzonitriles can undergo nucleophilic aromatic substitution reactions with a substituted piperidine to provide the piperidinyl-substituted bromoarenes. A person of ordinary skill in the art will understand that various organic synthesis methods can be applied to prepare the substituted 2-fluoro-3-bromobenzonitriles.

Compounds of the present disclosure can be synthesized by various transition metal-mediated cross coupling reactions (e.g. Suzuki or Stille methods). As shown below in Scheme II, trialkylstannane arenes, heteroarylboronic acids, or heteroarylboronate esters can undergo cross coupling reactions with the intermediate cyano-bromoarenes under palladium catalysis to give final compounds.

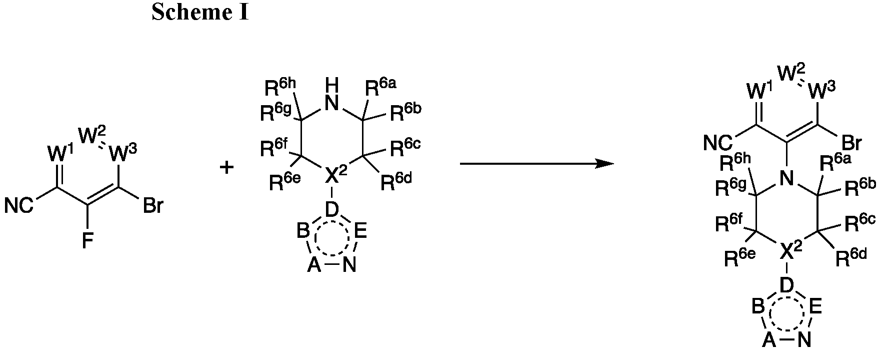

Scheme II illustrates the use of tetrakis(triphosphine)palladium(0) for the Stille-type reaction with the stannane reagent and [1,1'-bis(diphenylphosphino)ferrocene]dichloropalladium(II) for the Suzuki-type coupling with the heteroarylboronic acid. A person of ordinary skill in the art will understand that there are many palladium catalysts and reaction conditions that can be used for these types of cross coupling reactions.

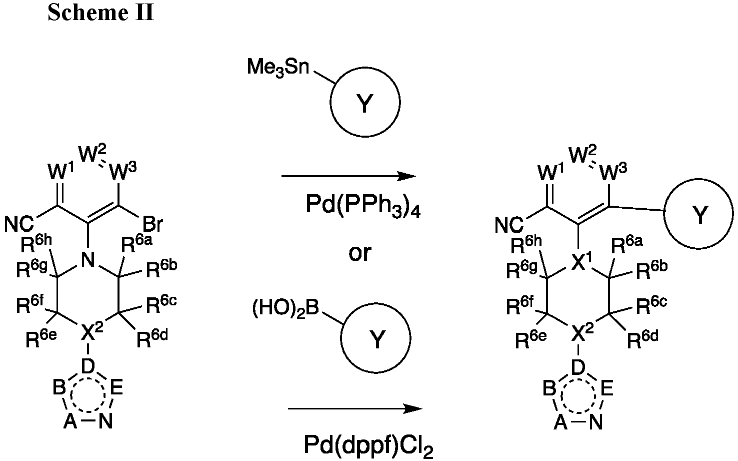

### I. Chemical Synthesis

As used below and throughout the present disclosure, the following abbreviations, unless otherwise indicated, shall be understood to have the following meanings:
- ACN: acetonitrile
- AcOH: acetic acid
- AMPhos: bis(di-tert-butyl(4-dimethylaminophenyl)phosphine)
- °C: degrees Celsius
- δ_{H}: chemical shift in parts per million downfield from tetramethylsilane
- d: day(s)
- DCM: dichloromethane
- DIAD: diisopropyl azodicarboxylate
- DIEA: diisopropylethylamine
- DMF: dimethylformamide
- DMF-DMA: dimethylformamide dimethyl acetal
- DMSO: dimethylsulfoxide
- dppf: 1,1'-bis(diphenylphosphino)ferrocene
- dcypf: 1,1'-bis(di-cyclohexylphosphino)ferrocene
- EA: ethyl acetate
- EtOAc: ethyl acetate
- EtOH: ethanol
- ESI: electrospray ionization
- Et: ethyl
- g: gram(s)
- h: hour(s)
- hr: hour(s)
- HPLC: high performance liquid chromatography
- Hz: hertz
- *J*: coupling constant (in NMR spectrometry)
- LCMS: liquid chromatography mass spectrometry
- *µ*: micro
- m: multiplet (spectral); meter(s); milli
- M: molar
- M⁺: parent molecular ion
- Me: methyl
- MeOH: methanol
- mg: milligram(s)
- MsCl: methanesulfonyl chloride
- MHz: megahertz
- min: minute(s)
- mol: mole(s); molecular (as in mol wt)
- mL: milliliter
- MS: mass spectrometry
- nm: nanometer(s)
- NMR: nuclear magnetic resonance
- pH: potential of hydrogen; a measure of the acidity or basicity
- PE: petroleum ether
- RT: room temperature
- s: singlet (spectral)
- t: triplet (spectral)
- SFC: supercritical fluid chromatography
- T: temperature
- TBAB: tetrabutylammonium bromide
- TBDMS: tert-butyldimethylsilyl
- TEA: triethylamine
- TFA: trifluoroacetic acid
- THF: tetrahydrofuran
- TPP: triphenylphosphine

### Synthesis of Intermediates

### Intermediate 1: 3-bromo-2-[4-(4-methyl-1,2,4-triazol-3-yl)piperidin-1-yl]benzonitrile

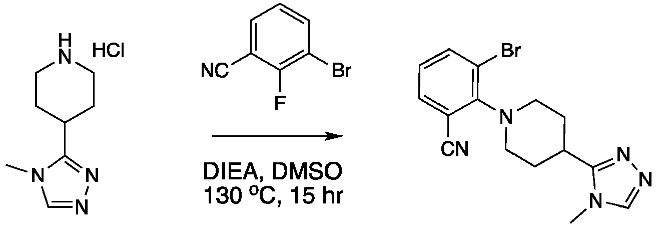

To a stirred solution of 4-(4-methyl-1,2,4-triazol-3-yl) piperidine hydrochloride (2 g, 9.9 mmol) and 3-bromo-2-fluorobenzonitrile (2.2 g, 11 mmol) in DMSO (80 mL) was added DIEA (5.1 g, 4 mmol). The resulting mixture was stirred for 15 h at 130 °C. The mixture was allowed to cool down to room temperature. The reaction was quenched with water at room temperature. The resulting mixture was extracted with EtOAc (3 x 100 mL). The combined organic layers were washed with brine (2 x 100 mL), dried over anhydrous Na₂SO₄. After filtration, the filtrate was concentrated under reduced pressure. The residue was purified by silica gel column chromatography, eluted with PE / EA (1:1) to afford 3-bromo-2-[4-(4-methyl-1,2,4-triazol-3-yl)piperidin-1-yl]benzonitrile (0.8 g) as a white solid. LCMS (ESI) m/z: 346, 348 [M+H]⁺.

### Intermediate 2: 3-bromo-2-[4-(imidazol-1-yl)piperidin-1-yl]benzonitrile

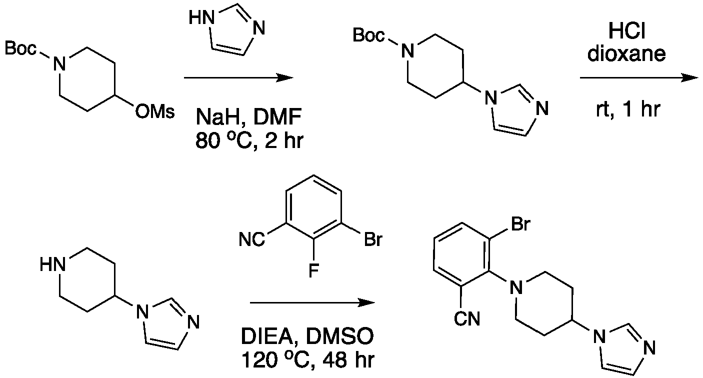

A solution of imidazole (2.4 g, 35.8 mmol) in DMF (120 mL) was treated with NaH (0.86 g, 35.8 mmol) for 25 min at 0 °C under nitrogen atmosphere followed by the addition of tert-butyl 4-(methanesulfonyloxy)piperidine-1-carboxylate (5 g, 17.8 mmol) at 0 °C. The resulting mixture was stirred for 2 h at 80 °C under nitrogen atmosphere. The resulting mixture was extracted with EtOAc (3 x 150 mL). The combined organic layers were washed with brine (2 x 80 mL), dried over anhydrous Na₂SO₄. After filtration, the filtrate was concentrated under reduced pressure. The residue was purified by silica gel column chromatography, eluted with CH₂Cl₂ / MeOH (5:1) to afford tert-butyl 4-(imidazol-1-yl)piperidine-1-carboxylate (1.5 g, 33%) as a yellow oil. LCMS (ESI) m/z: 252 [M+H]⁺.

A solution of tert-butyl 4-(imidazol-1-yl)piperidine-1-carboxylate (1.5 g, 5.9 mmol) in 4M HCl in 1,4-dioxane (25 mL) was stirred for 1 h at room temperature. The reaction mixture was evaporated and partitioned between saturated aqueous K₂CO₃ and EtOAc. The organic layer was dried over anhydrous Na₂SO₄, filtered, and was concentrated under reduced pressure, to afford 4-(imidazol-1-yl)piperidine (600 mg, 66%) as a light yellow oil. LCMS (ESI) m/z: 152 [M+H]⁺.

To a stirred solution of 4-(imidazol-1-yl)piperidine (600 mg, 3.96 mmol) in DMSO (150 mL) were added 3-bromo-2-fluorobenzonitrile (1190.4 mg, 5.95 mmol) and DIEA (2564.2 mg, 19.8 mmol) in portions at room temperature. The resulting mixture was stirred for 2 days at 120 °C under nitrogen atmosphere. The resulting mixture was extracted with EtOAc (3 x 150 mL). The combined organic layers were washed with brine (3 x 100 mL), dried over anhydrous Na₂SO₄. After filtration, the filtrate was concentrated under reduced pressure. The residue was purified by silica gel column chromatography, eluted with CH₂Cl₂ / MeOH (5:1) to afford 3-bromo-2-[4-(imidazol-1-yl)piperidin-1-yl]benzonitrile. LCMS (ESI) m/z: 331, 333 [M+H]⁺.

### Intermediate 3: 1-bromo-3-cyano-2-[4-(1,3-thiazol-5-yl)piperidin-1-yl]benzene

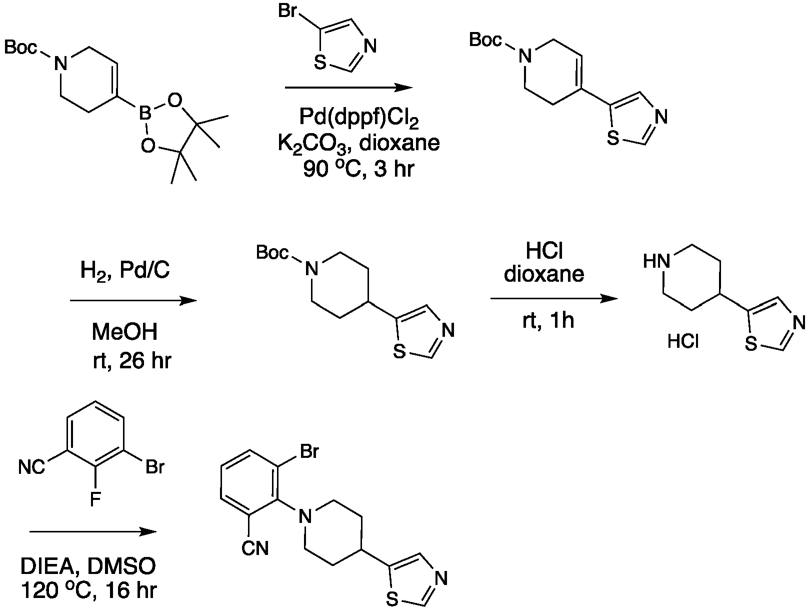

To a stirred solution of tert-butyl 4-(4,4,5,5-tetramethyl-1,3,2-dioxaborolan-2-yl)-3,6-dihydro-2H-pyridine-1-carboxylate (5 g, 16.2 mmol) and 5-bromo-1,3-thiazole (2652 mg, 16.2 mmol) in H₂O (12 mL) / dioxane (72 mL) were added K₂CO₃ (6704 mg, 48.5 mmol) and Pd(dppf)Cl₂ (1317 mg, 1.6 mmol) in portions at room temperature under nitrogen atmosphere. The resulting mixture was stirred for 3 h at 90 °C under nitrogen atmosphere. The reaction was quenched with water (100 mL) at room temperature. The resulting mixture was extracted with EtOAc (3 x 80 mL). The combined organic layers were washed with brine (2 x 30 mL), dried over anhydrous Na₂SO₄. After filtration, the filtrate was concentrated under reduced pressure. The residue was purified by silica gel column chromatography, eluted with MeOH/CH₂Cl₂ (0%-20%) to afford tert-butyl 4-(1,3-thiazol-5-yl)-3,6-dihydro-2H-pyridine-1-carboxylate (3.8 g, 88%) as a brown oil. LCMS (ESI) m/z: 267 [M+H]⁺.

To a stirred solution of tert-butyl 4-(1,3-thiazol-5-yl)-3,6-dihydro-2H-pyridine-1-carboxylate (3 g, 11.3 mmol) in MeOH (20 mL) was added Pd/C (1.5 g) in portions at room temperature under hydrogen atmosphere. The resulting mixture was stirred for 26 h at room temperature under hydrogen atmosphere. The resulting mixture was filtered, the filter cake was washed with MeOH (3 x 20 mL). The filtrate was concentrated under reduced pressure to afford tert-butyl 4-(1,3-thiazol-5-yl)piperidine-1-carboxylate (1.9 g, 63%) as a yellow oil. LCMS (ESI) m/z: 269 [M+H]⁺.

A mixture of tert-butyl 4-(1,3-thiazol-5-yl)-3,6-dihydro-2H-pyridine-1-carboxylate (1.9 g, 7.1 mmol) in HCl in 1,4-dioxane (20 mL) was stirred for 1 h at room temperature. The resulting mixture was concentrated under reduced pressure to afford 4-(1,3-thiazol-5-yl)piperidine hydrochloride (1.43 g, 98%) as an off-white solid. LCMS (ESI) m/z: 169 [M+H]⁺.

A mixture of 4-(1,3-thiazol-5-yl)piperidine hydrochloride (500 mg, 2.44 mmol), 1-bromo-2-fluoro-3-cyanobenzene (488.5 mg, 2.4 mmol) and DIEA (1262.7 mg, 9.8 mmol) in DMSO (5 mL) was stirred for 16 h at 120 ° C. The reaction was quenched with water (50 mL) at room temperature. The resulting mixture was extracted with EtOAc (3 x 30 mL). The combined organic layers were washed with brine (2 x 30 mL), dried over anhydrous Na₂SO₄. After filtration, the filtrate was concentrated under reduced pressure. The residue was purified by silica gel column chromatography, eluted with EtOAc/PE (40% - 60%) to afford 1-bromo-3-cyano-2-[4-(1,3-thiazol-5-yl)piperidin-1-yl]benzene (143.2 mg, 17%) as a yellow oil. LCMS (ESI) m/z: 348, 350 [M+H]⁺.

### Intermediate 4: 2-[4-(5-amino-1,3,4-thiadiazol-2-yl)piperidin-1-yl]-3-bromobenzonitrile

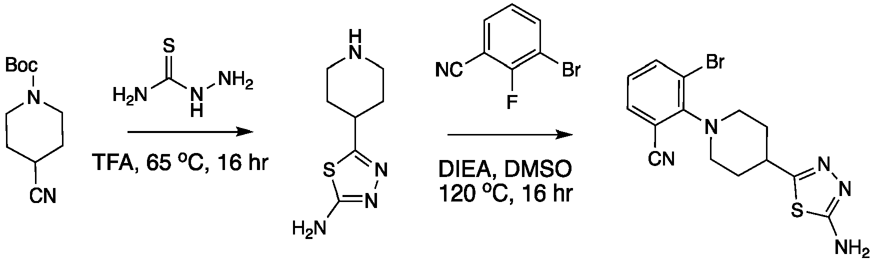

To a stirred solution of tert-butyl 4-cyanopiperidine-1-carboxylate (5 g, 23.7 mmol) in TFA (120 mL) was added thiosemicarbazide (3.2 g, 35.6 mmol) in portions at room temperature under nitrogen atmosphere. The resulting mixture was stirred at 65 °C overnight. The resulting mixture was concentrated under reduced pressure. The residue was purified by reverse flash chromatography with the following conditions: column, C18 silica gel; mobile phase, MeCN in water (0.1% FA), 1% to 10% gradient in 20 min; detector, UV 254 nm. to afford 5-(piperidin-4-yl)-1,3,4-thiadiazol-2-amine (4 g, 91%) as a colorless oil. LCMS (ESI) m/z: 185 [M+H]⁺.

To a stirred solution of 5-(piperidin-4-yl)-1,3,4-thiadiazol-2-amine (3 g, 16.3 mmol) in DMSO (60 mL) were added DIEA (8.4 g, 65.1 mmol) and 3-bromo-2-fluorobenzonitrile (2.3 g, 11.4 mmol) in portions at room temperature. The resulting mixture was stirred at 120 °C overnight. The resulting mixture was extracted with EtOAc (3 x 80 mL). The combined organic layers were washed with brine (3 x 60 mL), dried over anhydrous Na₂SO₄. After filtration, the filtrate was concentrated under reduced pressure. The residue was purified by silica gel column chromatography, eluted with CH₂Cl₂ / MeOH (5:1) to afford 2-[4-(5-amino-1,3,4-thiadiazol-2-yl)piperidin-1-yl]-3-bromobenzonitrile (450 mg, 8%) as a brown yellow solid. LCMS (ESI) m/z: 364, 366 [M+H]⁺.

### Intermediate 5: 3-bromo-2-[4-(3-methyl-1,2,4-triazol-4-yl)piperidin-1-yl]benzonitrile

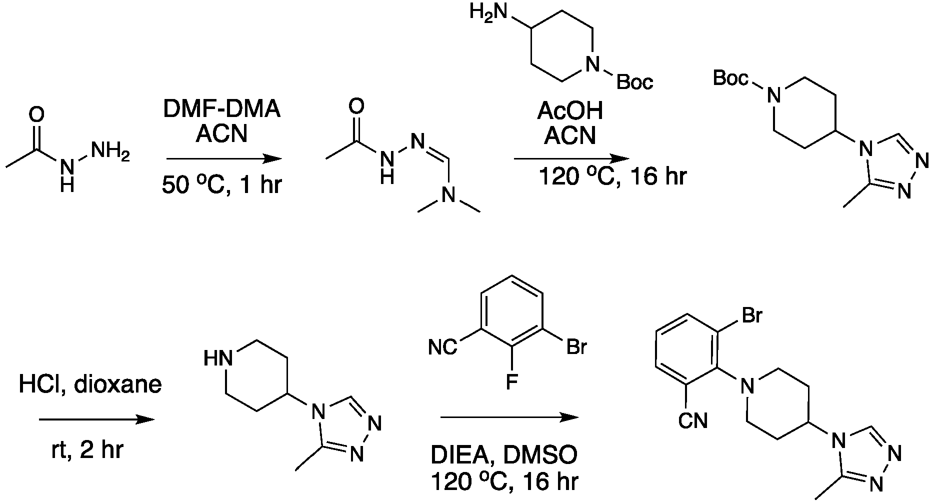

A mixture of acetohydrazide (5 g, 67.5 mmol) and DMF-DMA (8 g, 67.5 mmol) in ACN (70 mL) was stirred for 1 h at 50 ° C. The resulting mixture was concentrated under reduced pressure. The crude product mixture was used in the next step directly without further purification. LCMS (ESI) m/z: 130 [M+H]⁺.

A mixture of N'-[(1E)-(dimethylamino)methylidene]acetohydrazide (2 g, 15.5 mmol) and tert-butyl 4-aminopiperidine-1-carboxylate (3.7 g, 18.6 mmol) in AcOH (40 mL) and ACN (10 mL) was stirred for 16 h at 120° C .The reaction was quenched by the addition of water (150 mL) at room temperature. The resulting mixture was extracted with EtOAc (2 x 100 mL). The combined organic layers were washed with brine (80 mL), dried over anhydrous Na₂SO₄. After filtration, the filtrate was concentrated under reduced pressure. The residue was purified by silica gel column chromatography, eluted with EtOAc / PE (0-10%) to afford tert-butyl 4-(3-methyl-1,2,4-triazol-4-yl)piperidine-1-carboxylate (1.99 g, 48%) as a yellow oil. LCMS (ESI) m/z: 267 [M+H]⁺.

A mixture of tert-butyl 4-(3-methyl-1,2,4-triazol-4-yl)piperidine-1-carboxylate (1.9 g, 7.13 mmol) in HCl 1,4-dioxane (30 mL) was stirred for 2 h at room temperature The reaction was quenched by the addition of saturated aqueous K₂CO₃ (50 mL) at room temperature. The resulting mixture was extracted with EtOAc (2 x 100 mL). The combined organic layers were washed with brine (40 mL), dried over anhydrous Na₂SO₄. After filtration, the filtrate was concentrated under reduced pressure. The residue was purified by silica gel column chromatography, eluted with EtOAc / PE (20-30%) to afford 4-(3-methyl-1,2,4-triazol-4-yl)piperidine (1 g, 84%) as a yellow oil.

To a stirred mixture of 4-(3-methyl-1,2,4-triazol-4-yl)piperidine (1 g, 6 mmol) and 3-bromo-2-fluorobenzonitrile (1.18 g, 5.9 mmol) in DMSO (60 mL) was added DIEA (3.2 g, 24.7 mmol) dropwise at room temperature .The resulting mixture was stirred for 16 h at 120° C. The resulting mixture was extracted with EtOAc (2 x 100 mL). The combined organic layers were washed with brine (50 mL), dried over anhydrous Na₂SO₄. After filtration, the filtrate was concentrated under reduced pressure. The residue was purified by silica gel column chromatography, eluted with MeOH / CH₂Cl₂ (0-10%) to afford 3-bromo-2-[4-(3-methyl-1,2,4-triazol-4-yl)piperidin-1-yl]benzonitrile (200 mg, 10%) as a yellow oil. LCMS (ESI) m/z: 346, 348 [M+H]⁺.

### Intermediate 6: 3-bromo-6-chloro-2-[4-(4-methyl-1,2,4-triazol-3-yl)piperidin-1-yl]benzonitrile

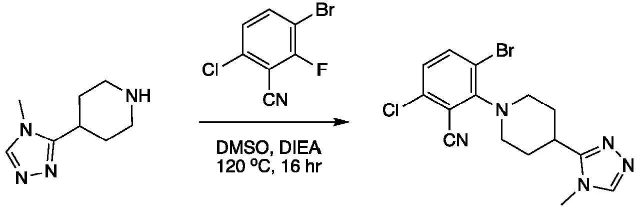

To a stirred solution of 4-(4-methyl-1,2,4-triazol-3-yl)piperidine (9 g, 54.1 mmol) in DMSO (150 mL) were added DIEA (48.9 g, 379 mmol) and 3-bromo-6-chloro-2-fluorobenzonitrile (8.9 g, 37.9 mmol) in portions at room temperature. The resulting mixture was stirred for overnight at 120 °C. The resulting mixture was extracted with EtOAc (3 x 200 mL). The combined organic layers were washed with brine (2 x 200 mL), dried over anhydrous Na₂SO₄. After filtration, the filtrate was concentrated under reduced pressure. The residue was purified by silica gel column chromatography, eluted with CH₂Cl₂ / MeOH (5:1) to afford 3-bromo-6-chloro-2-[4-(4-methyl-1,2,4-triazol-3-yl)piperidin-1-yl]benzonitrile (2.7 g, 13%) as a dark red solid. LCMS (ESI) m/z: 380, 382 [M+H]⁺.

### Intermediate 7: 3-bromo-6-methoxy-2-[4-(4-methyl-1,2,4-triazol-3-yl)piperidin-1-yl]benzonitrile

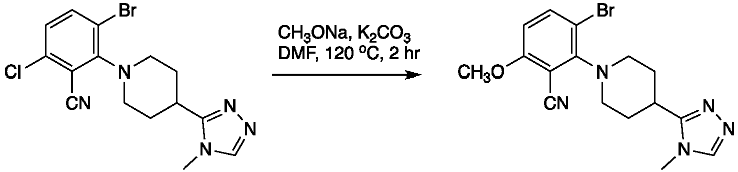

To a stirred solution of Intermediate 6 (500 mg, 1.3 mmol) in DMF (12 mL) were added K₂CO₃ (363 mg, 2.6 mmol) and CH₃ONa (212.9 mg, 3.9 mmol) in portions at room temperature. The resulting mixture was stirred for 2 h at 120 °C. The resulting mixture was extracted with EtOAc (3 x 100 mL). The combined organic layers were washed with brine (2 x 100 mL), dried over anhydrous Na₂SO₄. After filtration, the filtrate was concentrated under reduced pressure. The residue was purified by silica gel column chromatography, eluted with CH₂Cl₂ / MeOH (9:1) to obtain Intermediate 7 (350 mg, 71%). LCMS (ESI) m/z: 376, 378 [M+H]⁺.

### Intermediate 8: 3-bromo-2-[4-(3-methylimidazol-4-yl) piperidin-1-yl] benzonitrile

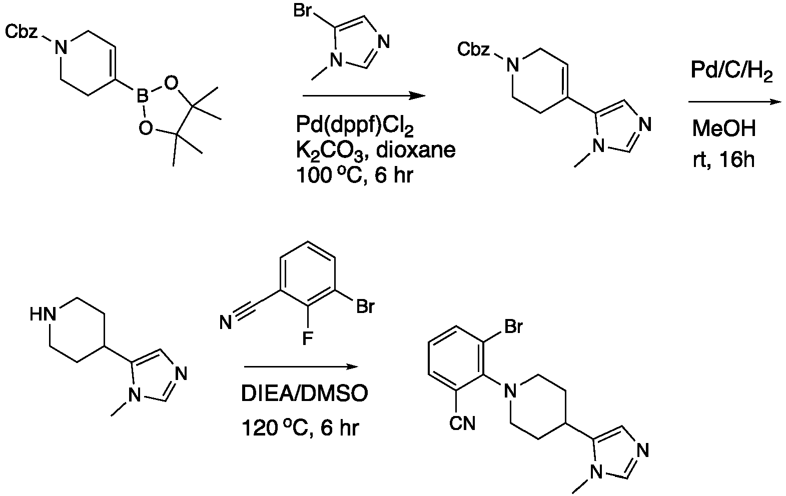

To a stirred solution of benzyl 4-(4,4,5,5-tetramethyl-1,3,2-dioxaborolan-2-yl)-3,6-dihydro-2H-pyridine-1-carboxylate (5 g, 14.6 mmol) in anhydrous 1,4-dioxane (50 mL) and H₂O (10 mL) was added K₂CO₃ (4 g, 29.1 mmol) and Pd(dppf)Cl₂ (1.07 g, 1.5 mmol) followed by 5-bromo-1-methylimidazole (2.3 g, 14.6 mmol) at room temperature. The reaction mixture was stirred at 100 °C for 6 h. After completion of reaction, the reaction mixture was quenched by addition of water (40 mL). The aqueous layer was extracted with ethyl acetate (300 mL). The combined organic phase was washed with brine (300 mL), dried over anhydrous sodium sulfate and concentrated under reduced pressure to give crude product which was further purified by column chromatography using (10% to 50% MeOH/DCM) to afford desired compound benzyl 4-(3-methylimidazol-4-yl)-3,6-dihydro-2H-pyridine-1-carboxylate (3 g). LCMS (ESI) m/z: 298 [M+H] ⁺.

A solution of benzyl 4-(3-methyl-1,2-dihydroimidazol-4-yl)-3,6-dihydro-2H-pyridine-1-carboxylate (3 g, 10 mmol) and Pd/C (2.7 g) in MeOH (50 mL) was stirred for 16 h at room temperature under hydrogen atmosphere. The resulting mixture was filtered, the filter cake was washed with MeOH (100 mL). The filtrate was concentrated under reduced pressure. The residue was purified by silica gel column chromatography, eluted with CH₂Cl₂/MeOH (12:1) to afford 4-(3-methyl-1,2-dihydroimidazol-4-yl) piperidine (2 g) as a brown solid. LCMS (ESI) m/z: 166 [M+H]⁺.

A solution of 4-(3-methylimidazol-4-yl) piperidine (400 mg, 2.4 mmol), 3-bromo-2-fluorobenzonitrile (581 mg, 2.9 mmol), and DIEA (938.6 mg, 7.26 mmol) in DMSO (20 mL) was stirred for 6 h at 120 °C. The resulting mixture was diluted with water (20 mL). The resulting mixture was extracted with EtOAc (2 x 10 mL). The combined organic layers were washed with water (3x10 mL), dried over anhydrous Na₂SO₄. After filtration, the filtrate was concentrated under reduced pressure. The residue was purified by silica gel column chromatography, eluted with CH₂Cl₂/MeOH (12: 1) to afford 3-bromo-2-[4-(3-methylimidazol-4-yl) piperidin-1-yl] benzonitrile (100 mg, 12%) as a yellow solid. LCMS (ESI) m/z: 345, 347 [M+H]⁺.

### Intermediate 9: 3-chloro-4-fluoro-2-[4-(4-methyl-1,2,4-triazol-3-yl) piperidin-1-yl] benzonitrile

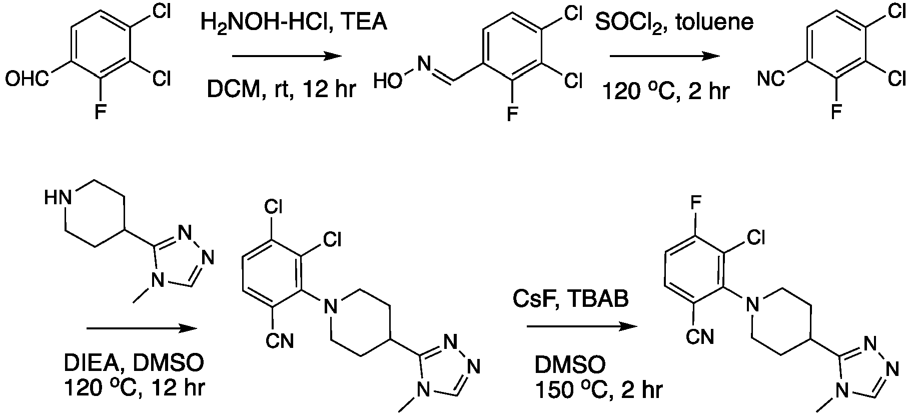

To a stirred solution of 3,4-dichloro-2-fluorobenzaldehyde (4 g, 20.7 mmol) in anhydrous DCM (50 mL) was added TEA (6.29 g, 62.2 mmol) and NH₂OH.HCl (10.8 mg, 0.16 mmol) at 0 °C. The reaction mixture was stirred at room temperature for 12 h. After completion of reaction, the reaction mixture was quenched by addition of water (50 mL). The resulting mixture was extracted with CH₂Cl₂ (300 mL) dried over anhydrous Na₂SO₄. After filtration, the filtrate was concentrated under reduced pressure. This resulted in (E)-N-[(3,4-dichloro-2-fluorophenyl) methylidene] hydroxylamine (3.2 g) as a brown solid. LCMS (ESI) m/z: 209 [M+H]⁺.

To a stirred solution of (E)-N-[(3,4-dichloro-2-fluorophenyl) methylidene] hydroxylamine (3.2 g, 15.38 mmol) in anhydrous toluene (50 mL) was added SOCl₂ (2.75 g, 23.08 mmol) at 0 °C. The reaction mixture was stirred at 120 °C for 2 h. After completion of reaction, the reaction mixture was quenched by addition of water (50 mL). The aqueous layer was extracted with ethyl acetate (300 mL), dried over anhydrous sodium sulfate and concentrated under reduced pressure to give crude product which was further purified by column chromatography using 5 to 20% EtOAc in PE gradient to afford desired compound 3,4-dichloro-2-fluorobenzonitrile (2.3 g). LCMS (ESI) m/z: 191 [M+H]⁺.

To a stirred solution of 4-(4-methyl-1,2,4-triazol-3-yl) piperidine (700 mg, 4.21 mmol) in anhydrous DMSO (50 mL) was added 3,4-dichloro-2-fluorobenzonitrile (720.1 mg, 3.79 mmol) and DIEA (2.18 g, 16.8 mmol) at room temperature. The reaction mixture was stirred at 120 °C for 12 h. After completion of reaction, the reaction mixture was quenched by addition of water (50 mL). The aqueous layer was extracted with ethyl acetate (300 mL). The combined organic phase was washed with brine (300 mL), dried over anhydrous sodium sulfate and concentrated under reduced pressure to give crude product which was further purified by column chromatography using 5 to 15 % MeOH in DCM gradient to afford desired compound 3,4-dichloro-2-[4-(4-methyl-1,2,4-triazol-3-yl) piperidin-1-yl] benzonitrile (500 mg). LCMS (ESI) m/z: 337 [M+H]⁺.

To a stirred solution of 3,4-dichloro-2-[4-(4-methyl-1,2,4-triazol-3-yl) piperidin-1-yl] benzonitrile (500 mg, 1.49 mmol) in anhydrous DMSO (50 mL) was added tetrabutylammonium bromide (47.9 mg, 0.15 mmol) and CsF (2.26 g, 14.9 mmol) at room temperature. The reaction mixture was stirred at 150 °C for a period of 2 h. After completion of reaction, the reaction mixture was quenched by addition of water (50 mL). The aqueous layer was extracted with ethyl acetate (300 mL). The combined organic phase was washed with brine (300 mL), dried over anhydrous sodium sulfate and concentrated under reduced pressure to give crude product which was further purified by column chromatography using 5 to 15% MeOH in DCM gradient to afford compound 3-chloro-4-fluoro-2-[4-(4-methyl-1,2,4-triazol-3-yl) piperidin-1-yl] benzonitrile (300 mg). LCMS (ESI) m/z: 320 [M+H]⁺.

### Intermediate 10: 3-bromo-2-{4-[4-(²H3)methyl-1,2,4-triazol-3-yl]piperidin-1-yl}benzonitrile

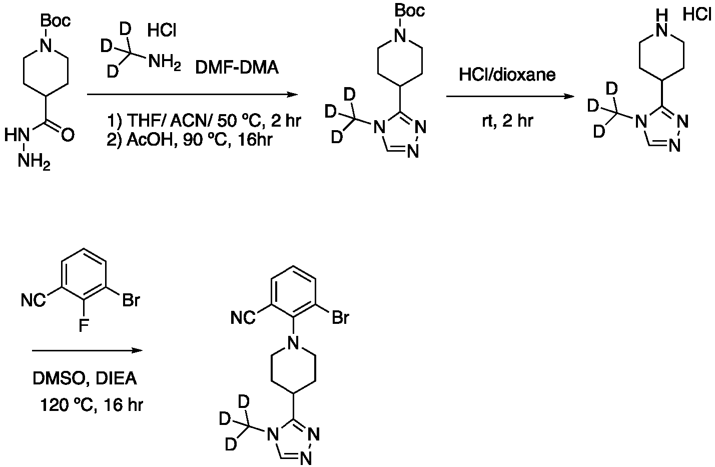

To a solution of tert-butyl 4-(hydrazinecarbonyl)piperidine-1-carboxylate (10.5 g, 43.1 mmol) in THF (50 mL) and ACN (200 mL) was added DMF-DMA (12.9 g, 107 mmol) dropwise at room temperature. The resulting mixture was stirred for 2 h at 50 °C. To the above mixture was added (2H3)methanamine hydrochloride (6.1 g, 86.3 mmol) and AcOH (25.9 g, 432 mmol) in portions over 20 min at 50 °C. The resulting mixture was stirred for additional 16 h at 90 °C. The resulting mixture was concentrated under reduced pressure and quenched by the addition of water (100 mL) at room temperature. The mixture was basified to pH 7 with saturated Na₂CO₃ (aq.). The resulting mixture was extracted with EtOAc (3 x 200 mL). The combined organic layers were washed with water (2 x 200 mL), dried over anhydrous Na₂SO₄. After filtration, the filtrate was concentrated under reduced pressure. The residue was purified by silica gel column chromatography, eluted with MeOH in CH₂Cl₂ (10%-15%) to afford tert-butyl 4-[4-(2H3)methyl-1,2,4-triazol-3-yl]piperidine-1-carboxylate (7 g, 60%) as a yellow oil. LCMS (ESI) m/z: 270 [M+H]⁺.

To tert-butyl 4-[4-(2H3)methyl-1,2,4-triazol-3-yl]piperidine-1-carboxylate (7 g, 25.9 mmol) was added HCl in 1,4-dioxane (100 mL) in portions at room temperature. The resulting mixture was stirred for 2 h at room temperature. The resulting mixture was concentrated under reduced pressure to afford 4-[4-(2H3)methyl-1,2,4-triazol-3-yl]piperidine hydrochloride (5g) as a white solid. The crude product mixture was used in the next step directly without further purification. LCMS (ESI) m/z: 170 [M+H]⁺.

To a stirred mixture of 4-[4-(2H3)methyl-1,2,4-triazol-3-yl]piperidine hydrochloride (3 g, 14.5 mmol) and 3-bromo-2-fluorobenzonitrile (3.2 g, 16.0 mmol ) in DMSO (50 mL) was added DIEA (9.4 g, 72.9 mmol) in portions at room temperature. The resulting mixture was stirred for 16 h at 120 °C. The residue was purified by reversed-phase flash chromatography with the following conditions: column, C18 silica gel; mobile phase, MeCN in water (0.1% FA), 40% to 50% gradient in 10 min; detector, UV 254 nm to afford 3-bromo-2-{4-[4-(2H3)methyl-1,2,4-triazol-3-yl]piperidin-1-yl}benzonitrile (300 mg, 6%) as a black oil. LCMS (ESI) m/z: 349, 351 [M+H]⁺.

### Intermediate 11: 3-bromo-2-[4-(4H-1,2,4-triazol-3-yl)piperidin-1-yl]benzonitrile

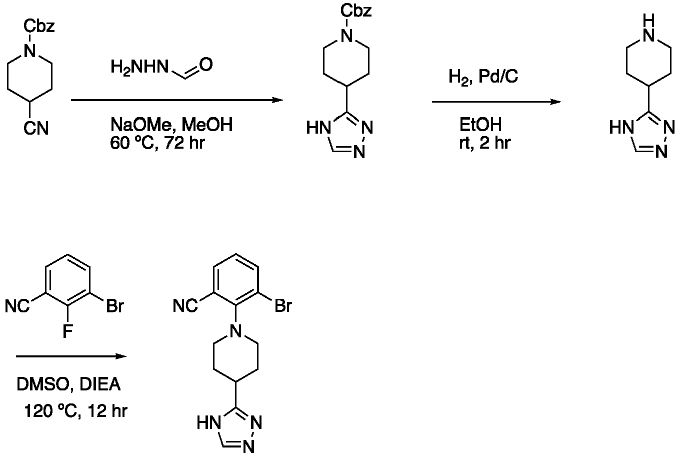

To a stirred solution of benzyl 4-cyanopiperidine-1-carboxylate (9.6 g, 39.3 mmol) and N-formylhydrazine (2.4 g, 40 mmol) in MeOH (20 mL) was added NaOMe (0.64 g) in portions at room temperature under nitrogen atmosphere. The resulting mixture was stirred for 72 h at 60 °C under nitrogen atmosphere. The resulting mixture was washed with acetic acid (3 mL). The resulting mixture was concentrated under reduced pressure. The residue was purified by silica gel column chromatography, eluted with PE / EA (1: 1) to afford benzyl 4-(4H-1,2,4-triazol-3-yl)piperidine-1-carboxylate (2.4 g, 21%) as a white solid. LCMS (ESI) m/z: 287 [M+H]⁺.

To a solution of benzyl 4-(4H-1,2,4-triazol-3-yl)piperidine-1-carboxylate (2.4 g, 8.4 mmol) in MeOH (10 mL) was added Pd/C (10%, Pd/C (0.19 g)) under nitrogen atmosphere. The mixture was hydrogenated at room temperature for 2 h under hydrogen atmosphere using a hydrogen balloon, filtered through a celite pad and concentrated under reduced pressure. The resulting filter cake was washed with MeOH (3 x 10 mL). The filtrate was concentrated under reduced pressure. The residue was purified by silica gel column chromatography, eluted with PE / EA (1: 1) to afford 4-(4H-1,2,4-triazol-3-yl)piperidine (640 mg, 46%) as a white solid. LCMS (ESI) m/z: 153 [M+H]⁺.

To a stirred solution of 4-(4H-1,2,4-triazol-3-yl)piperidine (640 mg, 4.2 mmol) and 3-bromo-2-fluorobenzonitrile (1093 mg, 5.5 mmol) in DMSO (5 mL) was added DIEA (1630 mg) dropwise at room temperature under nitrogen atmosphere. The resulting mixture was stirred for 12 h at 120 °C. The resulting mixture was extracted with EtOAc (3 x 50 mL). The combined organic layers were washed with brine (3 x 10 mL), dried over anhydrous Na₂SO₄. After filtration, the filtrate was concentrated under reduced pressure. The residue was purified by silica gel column chromatography, eluted with PE / EA (1: 1) to afford 3-bromo-2-[4-(4H-1,2,4-triazol-3-yl)piperidin-1-yl]benzonitrile (80 mg, 6%) as a white solid. LCMS (ESI) m/z: 332, 334 [M+H]⁺.

### Intermediate 12: 3-bromo-5-methyl-2-[4-(4-methyl-1,2,4-triazol-3-yl)piperidin-1-yl]benzonitrile

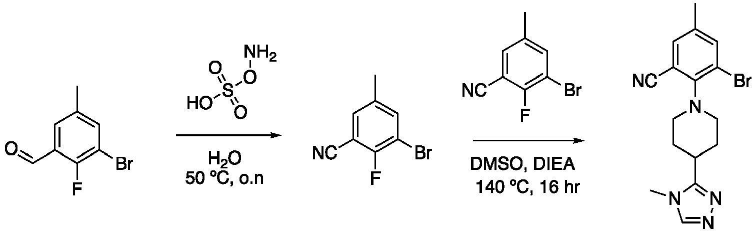

A solution of 3-bromo-2-fluoro-5-methylbenzaldehyde (1 g, 4.6 mmol) and hydroxylamine-o-sulfonic acid (0.89 g, 7.8 mmol) in H₂O (10 mL) was stirred for 10 min at room temperature and then stirred at 50 °C overnight under nitrogen atmosphere. The resulting mixture was extracted with EtOAc (3 x 70 mL). The combined organic layers were washed with brine (3 x 10 mL), dried over anhydrous Na₂SO₄. After filtration, the filtrate was concentrated under reduced pressure. The residue was purified by silica gel column chromatography, eluted with CH₂Cl₂ / MeOH (10:1) to afford 3-bromo-2-fluoro-5-methylbenzonitrile (879 mg, 89%) as a white solid. LCMS (ESI) m/z: 214, 216 [M+H]⁺.

To a stirred solution of 3-bromo-2-fluoro-5-methylbenzonitrile (879 mg, 4.1 mmol) and 4-(4-methyl-1,2,4-triazol-3-yl)piperidine (586.2 mg, 3.5 mmol) in DMSO (12 mL) was added DIEA (2681.1 mg, 20.7 mmol) dropwise at room temperature under nitrogen atmosphere. The resulting mixture was stirred for 16 h at 140 °C. The reaction was quenched by the addition of water (20 mL) at room temperature. The resulting mixture was extracted with EtOAc (3 x 50 mL). The combined organic layers were washed with brine (3 x 10 mL), dried over anhydrous Na₂SO₄. After filtration, the filtrate was concentrated under reduced pressure. The residue was purified by Prep-TLC (CH₂Cl₂ / MeOH 9:1) to afford 3-bromo-5-methyl-2-[4-(4-methyl-1,2,4-triazol-3-yl)piperidin-1-yl]benzonitrile (156 mg, 10%) as a white solid. LCMS (ESI) m/z: 360, 362 [M+H]⁺.

### Intermediate 13: 3-bromo-5-chloro-2-[4-(4-methyl-1,2,4-triazol-3-yl) piperidin-1-yl] benzonitrile

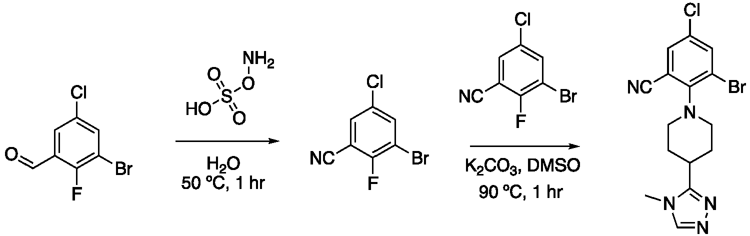

A solution of 3-bromo-5-chloro-2-fluorobenzaldehyde (1 g, 4.2 mmol) and aminooxysulfonic acid (0.81 g, 7.2 mmol) in water (10 mL) was stirred for 1 h at 50 °C. The resulting mixture was extracted with EtOAc (3 x 150 mL). The combined organic layers were washed with brine (3 x 50 mL), dried over anhydrous Na₂SO₄. After filtration, the filtrate was concentrated under reduced pressure. The residue was purified by silica gel column chromatography, eluted with PE / EA (1: 1) to afford 3-bromo-5-chloro-2-fluorobenzonitrile (900 mg, 91%) as a yellow solid. LCMS (ESI) m/z: 234, 236 [M+H]⁺.

A solution of 3-bromo-5-chloro-2-fluorobenzonitrile (730 mg, 3.1 mmol), 4-(4-methyl-1,2,4-triazol-3-yl) piperidine (517.6 mg, 3.1 mmol) and K₂CO₃ (2151.6 mg, 15.6 mmol) in DMSO (5 mL) was stirred for 1 h at 90 °C. The residue was purified by reverse-phase flash chromatography with the following conditions: column, C18 silica gel; mobile phase, MeCN in water (0.1% FA), 10% to 50% gradient in 10 min; detector, UV 254 nm to afford 3-bromo-5-chloro-2-[4-(4-methyl-1,2,4-triazol-3-yl) piperidin-1-yl] benzonitrile (160 mg, 13%) as a brown oil. LCMS (ESI) m/z: 380, 382 [M+H]⁺.

### Intermediate 14: 3-bromo-2-[4-(4-methyl-1,2,4-triazol-3-yl)piperidin-1-yl]-5-(trifluoromethyl)benzonitrile

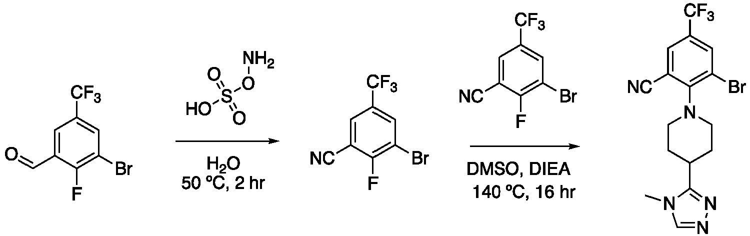

A solution of 3-bromo-2-fluoro-5-(trifluoromethyl) benzaldehyde (500 mg, 1.8 mmol) and (aminooxy)sulfonic acid (354.7 mg, 3.1mmol) in H₂O (10 mL) was stirred for 2 h at 50 °C under nitrogen atmosphere. The resulting mixture was extracted with EtOAc (2 x 100 mL). The combined organic layers were washed with brine (2 x 10 mL), dried over anhydrous Na₂SO₄. After filtration, the filtrate was concentrated under reduced pressure. The residue was purified by silica gel column chromatography, eluted with PE / EA (0%-10%) to afford 3-bromo-2-fluoro-5-(trifluoromethyl) benzonitrile (200 mg) as a white solid. LCMS (ESI) m/z: 268, 270 [M+H]⁺.

To a stirred mixture of 3-bromo-2-fluoro-5-(trifluoromethyl)benzonitrile (500 mg, 1.87 mmol) and 4-(4-methyl-1,2,4-triazol-3-yl)piperidine (465 mg, 2.79 mmol) in DMSO (10 mL) was added DIEA (1205 mg, 9.33 mmol) in portions at room temperature. The resulting mixture was stirred at 140 °C overnight. The resulting mixture was extracted with EtOAc (3 x 150 mL). The combined organic layers were washed with brine (2 x10 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3-bromo-2-[4-(4-methyl-1,2,4-triazol-3-yl)piperidin-1-yl]-5-(trifluoromethyl)benzonitrile (180 mg) as a pink oil. LCMS (ESI) m/z: 414, 416 [M+H]⁺.

### Intermediate 15: 3-bromo-2-[4-(1,3,4-thiadiazol-2-yl) piperidin-1-yl] benzonitrile

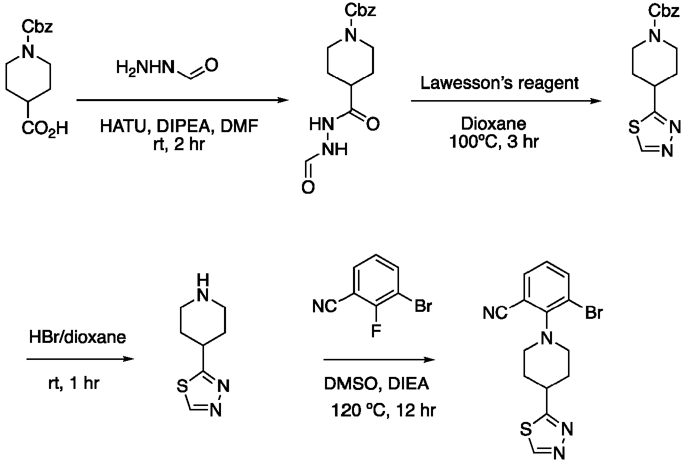

A solution of 1-[(benzyloxy) carbonyl] piperidine-4-carboxylic acid (5 g, 19 mmol), N-formylhydrazine (1.48 g, 24.7 mmol), HATU (8.66 g, 22.8 mmol) and DIPEA (6.38 g, 49.4 mmol) in DMF (50 mL) was stirred for 2 h at room temperature. The reaction was quenched with water at room temperature. The resulting mixture was extracted with EtOAc (5 x 100 mL). The combined organic layers were washed with brine (5 x 50 mL), dried over anhydrous Na₂SO₄. After filtration, the filtrate was concentrated under reduced pressure. The residue was purified by silica gel column chromatography, eluted with PE / EA (1:1) to afford benzyl 4-(N'-formylhydrazinecarbonyl) piperidine-1-carboxylate (4.5 g, 77%) as a white solid. LCMS (ESI) m/z: 306 [M+H]⁺.

A solution of benzyl 4-(N'-formylhydrazinecarbonyl) piperidine-1-carboxylate (4.50 g, 14.7 mmol) and Lawesson Reagent (6.56 g, 16.2 mmol) in dioxane (30 mL) was stirred for 3 h at 100 °C. The reaction was quenched with water at room temperature. The resulting mixture was extracted with EtOAc (3 x 150 mL). The combined organic layers were washed with brine (3 x 50 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm to afford benzyl 4-(1,3,4-thiadiazol-2-yl) piperidine-1-carboxylate (210 mg, 5%) as a yellow oil. LCMS (ESI) m/z: 304 [M+H]⁺.

A solution of benzyl 4-(1,3,4-thiadiazol-2-yl) piperidine-1-carboxylate (210 mg, 0.69 mmol) in dioxane (1 mL) and HBr (2 mL) was stirred for 1 h at room temperature. The resulting mixture was concentrated under reduced pressure. The crude product was used for the next step without further purification. LCMS (ESI) m/z: 170 [M+H]⁺.

A solution of 4-(1,3,4-thiadiazol-2-yl) piperidine (150 mg, 0.89 mmol), 3-bromo-2-fluorobenzonitrile (177 mg, 0.89 mmol) and DIEA (572.7 mg, 4.43 mmol) in DMSO (4 mL) was heated at 120 °C overnight. The resulting mixture was concentrated under reduced pressure. The residue was purified by reversed-phase flash chromatography with the following conditions: column, C18 silica gel; mobile phase, MeCN in water (0.1% NH₃.H₂O), 10% to 50% gradient in 10 min; detector, UV 254 nm to afford 3-bromo-2-[4-(1,3,4-thiadiazol-2-yl) piperidin-1-yl] benzonitrile (50 mg, 16%) as a yellow oil. LCMS (ESI) m/z: 349, 351 [M+H]⁺.

### Intermediate 16: 3-bromo-2-[(1R,5S)-6-(4-methyl-1,2,4-triazol-3-yl)-3-azabicyclo[3. 1.0]hexan-3-yl]benzonitrile

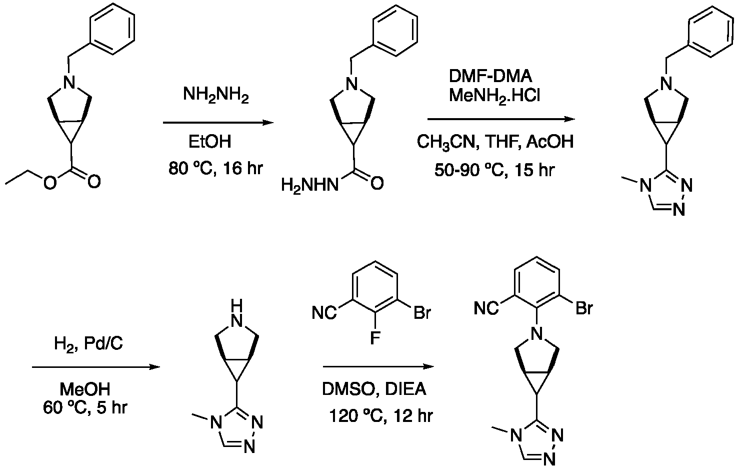

A solution of ethyl (1R,5S)-3-benzyl-3-azabicyclo[3.1.0]hexane-6-carboxylate (3 g, 12.2 mmol) and NH₂NH₂.H₂O (18.4 g, 366.9 mmol) in EtOH (30 mL) was stirred for 16 h at 80 °C. The resulting mixture was concentrated under reduced pressure. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm to afford (1R,5S)-3-benzyl-3-azabicyclo[3.1.0]hexane-6-carbohydrazide (2.5 g, 88%) as a white solid. LCMS (ESI) m/z: 232 [M+H]⁺.

A solution of (1R,5S)-3-benzyl-3-azabicyclo[3.1.0]hexane-6-carbohydrazide (1 g, 4.3 mmol) and DMF-DMA (1.29 g, 10.8 mmol) in MeCN (1.7 mL, 32.4 mmol), THF (5 mL) was stirred for 1 h at 50 °C. To the above mixture was added CH₃NH₂-HCl (0.35 g, 5.2 mmol), HOAc (2.6 g, 43.2 mmol) dropwise over 10 min at room temperature. The resulting mixture was stirred for additional 15 h at 90 °C. The resulting mixture was concentrated under reduced pressure. The residue was purified by silica gel column chromatography, eluted with CH₂Cl₂ / MeOH (9:1) to afford (1R,5S)-3-benzyl-6-(4-methyl-1,2,4-triazol-3-yl)-3-azabicyclo[3.1.0]hexane (1 g, 91%) as a light yellow oil. LCMS (ESI) m/z: 255 [M+H]⁺.

A solution of (1R,5S)-3-benzyl-6-(4-methyl-1,2,4-triazol-3-yl)-3-azabicyclo[3.1.0]hexane (1 g, 3.9 mmol) and Pd/C (1 g, 9.4 mmol) in MeOH (50 mL) was stirred for 5 h at 60 °C under hydrogen atmosphere (5 atm). The resulting mixture was filtered through celite and the filtrate was concentrated under reduced pressure to afford the desired product as a light yellow oil (660 mg). The crude product was used in the next step directly without further purification. LCMS (ESI) m/z: 165 [M+H]⁺.

A solution of (1R,5S)-6-(4-methyl-1,2,4-triazol-3-yl)-3-azabicyclo[3.1.0]hexane (600 mg, 3.6 mmol), 3-bromo-2-fluorobenzonitrile (877 mg, 4.4 mmol) and DIEA (1416.7 mg, 10.9 mmol) in DMSO (10 mL) was stirred for 12 h at 120 °C. The resulting mixture was extracted with EtOAc (3 x 50 mL). The combined organic layers were washed with brine (2 x 50 mL), dried over anhydrous Na₂SO₄. After filtration, the filtrate was concentrated under reduced pressure. The residue was purified by silica gel column chromatography, eluted with PE / EA (1:1) to afford 3-bromo-2-[(1R,5S)-6-(4-methyl-1,2,4-triazol-3-yl)-3-azabicyclo[3.1.0]hexan-3-yl]benzonitrile (90 mg, 4%) as a light yellow oil. LCMS (ESI) m/z: 344, 346 [M+H]⁺.

### Intermediate 17: 5-chloro-4-[4-(4-methyl-1,2,4-triazol-3-yl)piperidin-1-yl]benzene-1,3-dicarbonitrile

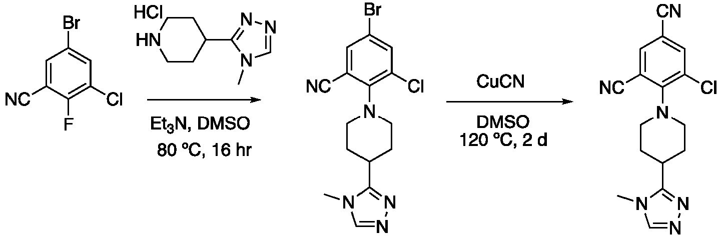

To a stirred solution of 5-bromo-3-chloro-2-fluorobenzonitrile (3 g, 12.8 mmol) in DMSO (150 mL) were added TEA (10.8 g, 106.6 mmol) and 4-(4-methyl-1,2,4-triazol-3-yl)piperidine (3.5 g, 21.3 mmol) in portions at room temperature under nitrogen atmosphere. The resulting mixture was stirred at 80 °C overnight. The resulting mixture was extracted with EtOAc (3 x 300 mL). The combined organic layers were washed with brine (2 x 150 mL), dried over anhydrous Na₂SO₄. After filtration, the filtrate was concentrated under reduced pressure. The residue was purified by silica gel column chromatography, eluted with CH₂Cl₂ / MeOH (8:1) to afford 5-bromo-3-chloro-2-[4-(4-methyl-1,2,4-triazol-3-yl)piperidin-1-yl]benzonitrile (2.3 g, 28%) as a yellow solid.

A mixture of 5-bromo-3-chloro-2-[4-(4-methyl-1,2,4-triazol-3-yl)piperidin-1-yl]benzonitrile (500 mg, 1.31 mmol) and CuCN (176.5 mg, 1.97 mmol) in DMSO (10 mL) was stirred for 2 days at 120 °C under nitrogen atmosphere. The resulting mixture was extracted with EtOAc (3 x 100 mL). The combined organic layers were washed with brine (2 x 100 mL), dried over anhydrous Na₂SO₄. After filtration, the filtrate was concentrated under reduced pressure. The residue was purified by silica gel column chromatography, eluted with CH₂Cl₂ / MeOH (9:1) to afford 5-chloro-4-[4-(4-methyl-1,2,4-triazol-3-yl)piperidin-1-yl]benzene-1,3-dicarbonitrile (160 mg, 37%) as a yellow solid. LCMS (ESI) m/z: 327 [M+H]⁺.

### Intermediate 18: 3-chloro-5-methanesulfonyl-2-[4-(4-methyl-1,2,4-triazol-3-yl)piperidin-1-yl]benzonitrile

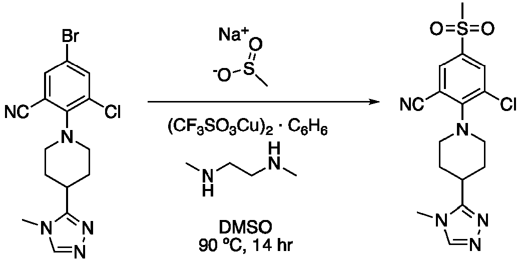

To a stirred solution of 5-bromo-3-chloro-2-[4-(4-methyl-1,2,4-triazol-3-yl)piperidin-1-yl]benzonitrile (300 mg, 0.79 mmol) in DMSO (5 mL) were added methyl[2-(methylamino)ethyl]amine (55.6 mg, 0.63 mmol), sodium methanesulfinate (80.4 mg, 0.79 mmol) and Copper (I) trifluoromethanesulfonate benzene complex (39.7 mg, 0.08 mmol) in portions at room temperature under nitrogen atmosphere. The resulting mixture was stirred at 90 °C overnight. The residue was purified by reversed-phase flash chromatography with the following conditions: column, C18 silica gel; mobile phase, MeCN in water (0.1% FA), 20% to 40% gradient in 20 min; detector, UV 254 nm. to afford 3-chloro-5-methanesulfonyl-2-[4-(4-methyl-1,2,4-triazol-3-yl)piperidin-1-yl]benzonitrile (190 mg, 63%) as a yellow solid. LCMS (ESI) m/z: 380 [M+H]⁺.

### Intermediate 19: 5-bromo-6-[4-(4-methyl-1,2,4-triazol-3-yl)piperidin-1-yl]-1H-indazole-7-carbonitrile

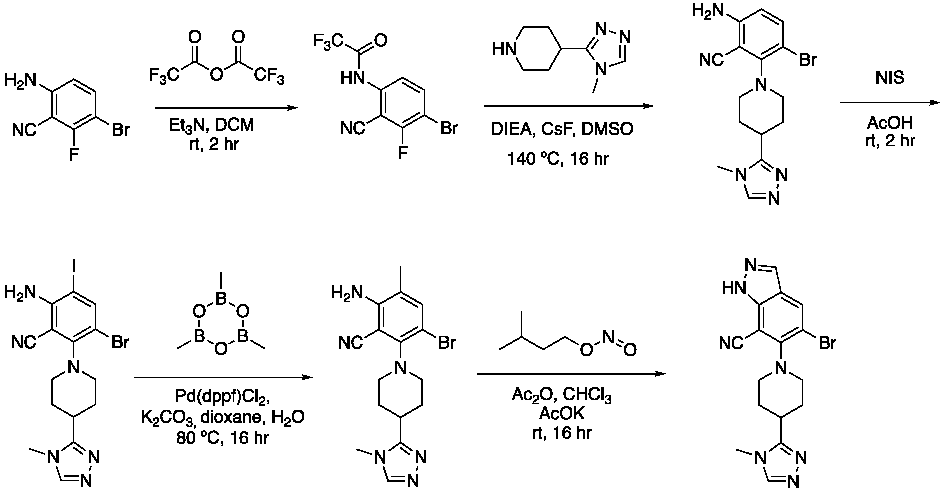

A solution of 6-amino-3-bromo-2-fluorobenzonitrile (5 g, 23.2 mmol), trifluoroacetic anhydride (5.9 g, 27.9 mmol) and TEA (7.1 g, 69.7 mmol) in DCM (25 mL) was stirred for 2 h at room temperature. The resulting mixture was concentrated under reduced pressure. The residue was purified by silica gel column chromatography, eluted with PE / EA (1:1) to afford N-(4-bromo-2-cyano-3-fluorophenyl)-2,2,2-trifluoroacetamide (5 g, 68%) as a light yellow solid. LCMS (ESI) m/z: 311, 313 [M+H]⁺.

A solution of N-(4-bromo-2-cyano-3-fluorophenyl)-2,2,2-trifluoroacetamide (6 g, 19.3 mmol), 4-(4-methyl-1,2,4-triazol-3-yl)piperidine (6.4 g, 38.6 mmol) and CsF (8.8 g, 57.9 mmol) in DMSO (25 mL) was stirred for 16 h at 140 °C. The resulting mixture was extracted with EtOAc (3 x 200 mL). The combined organic layers were washed with brine (2 x 200 mL), dried over anhydrous Na₂SO₄. After filtration, the filtrate was concentrated under reduced pressure. The residue was purified by silica gel column chromatography, eluted with CH₂Cl₂ / MeOH (9:1) to afford 6-amino-3-bromo-2-[4-(4-methyl-1,2,4-triazol-3-yl)piperidin-1-yl]benzonitrile (600 mg, 7%) as a light yellow oil. LCMS (ESI) m/z: 361, 363 [M+H]⁺.

A solution of 6-amino-3-bromo-2-[4-(4-methyl-1,2,4-triazol-3-yl)piperidin-1-yl]benzonitrile (600 mg, 1.6 mmol) and NIS (373.7 mg, 1.6 mmol) in AcOH (15 mL) was stirred for 2 h at room temperature. The resulting mixture was concentrated under reduced pressure. The residue was purified by silica gel column chromatography, eluted with CH₂Cl₂ / MeOH (8:1) to afford 2-amino-5-bromo-3-iodo-6-[4-(4-methyl-1,2,4-triazol-3-yl)piperidin-1-yl]benzonitrile (900 mg, 75%) as a light brown oil. LCMS (ESI) m/z: 487, 489 [M+H]⁺.

A solution of 2-amino-5-bromo-3-iodo-6-[4-(4-methyl-1,2,4-triazol-3-yl)piperidin-1-yl]benzonitrile (900 mg, 1.84 mmol), trimethyl-1,3,5,2,4,6-trioxatriborinane (278.3 mg, 2.22 mmol), K₂CO₃ (510.7 mg, 3.7 mmol) and Pd(dppf)Cl₂ (135.2 mg, 0.18 mmol) in 1,4-dioxane (20 mL) was stirred for 16 h at 80 °C under nitrogen atmosphere. The resulting mixture was concentrated under reduced pressure. The residue was purified by silica gel column chromatography, eluted with CH₂Cl₂ / MeOH (8:1) to afford 2-amino-5-bromo-3-methyl-6-[4-(4-methyl-1,2,4-triazol-3-yl)piperidin-1-yl]benzonitrile (600 mg, 86%) as a light yellow oil. LCMS (ESI) m/z: 375, 377 [M+H]⁺.

A solution of 2-amino-5-bromo-3-methyl-6-[4-(4-methyl-1,2,4-triazol-3-yl)piperidin-1-yl]benzonitrile (600 mg, 1.6 mmol) in CHCl₃ (10 mL) was treated with Ac₂O (359.1 mg, 3.5 mmol) for 10 min at 0 °C followed by the addition of isoamyl nitrite (430.8 mg, 3.7 mmol), AcOK (47.1 mg, 0.48 mmol) in portions at room temperature. The resulting mixture was stirred for 16 h at room temperature. The resulting mixture was concentrated under reduced pressure. The residue was purified by silica gel column chromatography, eluted with CH₂Cl₂ / MeOH (8:1) to afford 5-bromo-6-[4-(4-methyl-1,2,4-triazol-3-yl)piperidin-1-yl]-1H-indazole-7-carbonitrile (50 mg, 8%) as a light yellow oil. LCMS (ESI) m/z: 386, 388 [M+H]⁺.

### Intermediate 20: 3-bromo-2-[4-(4-methyl-1,2,4-triazol-3-yl)piperazin-1-yl]benzonitrile

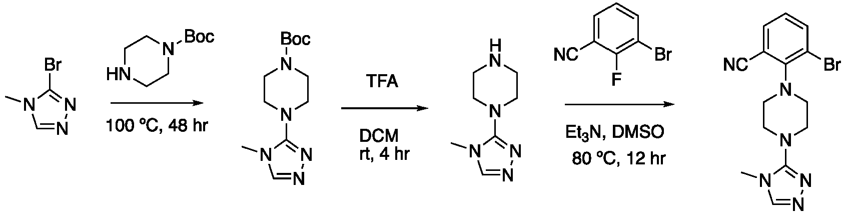

A mixture of 3-bromo-4-methyl-1,2,4-triazole (1 g, 6.2 mmol) in tert-butyl piperazine-1-carboxylate (5 g, 26.8 mmol) was stirred for 2 days at 90 °C. The residue was purified by silica gel column chromatography, eluted with CH₂Cl₂ / MeOH (0%-10%) and then by Prep-HPLC with the following conditions (Column: Xselect CSH F-Phenyl OBD column 30*250 mm, 5µm; Mobile Phase A: water (0.1% FA), Mobile Phase B: ACN; Flow rate: 60 mL/min mL/min; Gradient: 28% B to 40% B in 10 min; Wave Length: 254 nm/220 nm; RT1(min): 12.62) to afford tert-butyl 4-(4-methyl-1,2,4-triazol-3-yl)piperazine-1-carboxylate (1.2 g) as a white oil. LCMS (ESI) m/z: 268 [M+H]⁺.

A mixture of tert-butyl 4-(4-methyl-1,2,4-triazol-3-yl)piperazine-1-carboxylate (1.2 g, 4.48 mmol) in TFA (2 mL) and DCM (10 mL) was stirred for 4 h at room temperature. The mixture was concentrated under reduced pressure and the crude product was used in the next step directly without further purification. LCMS (ESI) m/z: 168 [M+H]⁺.

To a stirred mixture of 1-(4-methyl-1,2,4-triazol-3-yl)piperazine (100 mg, 0.6 mmol) and 3-bromo-2-fluorobenzonitrile (179.4 mg, 0.9 mmol) in DMSO (5 mL) was added TEA (302.6 mg, 3 mmol) in portions at room temperature. The resulting mixture was stirred for overnight at 90 °C. To the reaction water (20 mL) was added and the resulting mixture was extracted with EtOAc (3 x 100 mL). The combined organic layers were washed with brine (2 x 15 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3-bromo-2-[4-(4-methyl-1,2,4-triazol-3-yl)piperazin-1-yl]benzonitrile (70 mg) as a brown solid. LCMS (ESI) m/z: 347, 349 [M+H]⁺.

### Intermediate 21: 3-bromo-4-chloro-6-methoxy-2-[4-(4-methyl-1,2,4-triazol-3-yl)piperidin-1-yl]benzonitrile

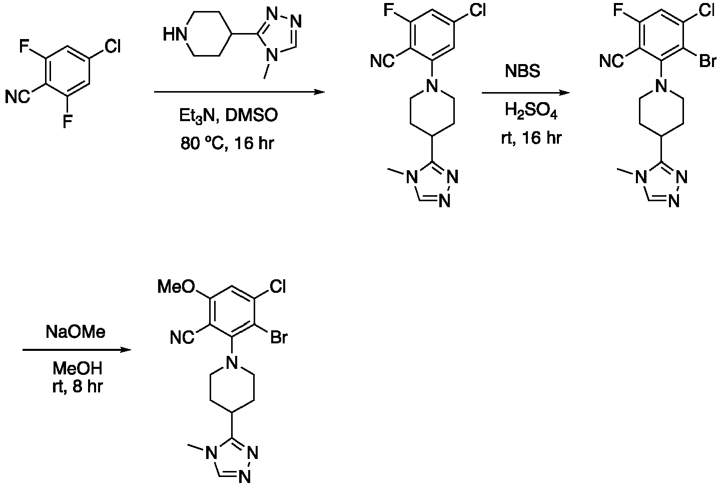

To a stirred mixture of 4-chloro-2,6-difluorobenzonitrile (10 g, 57.6 mmol) and 4-(4-methyl-1,2,4-triazol-3-yl)piperidine (14.4 g, 86.4 mmol) in DMSO (30 mL) was added TEA (17.5 g, 172.9 mmol) in portions at room temperature. The resulting mixture was stirred for 16 h at 80 °C. To the reaction water (500 mL) was added and extracted with EtOAc (3 x 800 mL). The combined organic layers were washed with brine (2 x100 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 4-chloro-2-fluoro-6-[4-(4-methyl-1,2,4-triazol-3-yl)piperidin-1-yl]benzonitrile (15 g) as a brown solid. LCMS (ESI) m/z: 320 [M+H]⁺.

To a stirred mixture of 4-chloro-2-fluoro-6-[4-(4-methyl-1,2,4-triazol-3-yl)piperidin-1-yl]benzonitrile (15 g, 47 mmol) in H₂SO₄ (50 mL) was added NBS (16.7 g, 93.8 mmol) at 0 °C. The resulting mixture was stirred for 16h at room temperature. To the reaction mixture water (600 mL) was added at 0 °C. The mixture was basified to pH 8 with saturated Na₂CO₃ (aq.). The resulting mixture was extracted with EtOAc (3 x 1L). The combined organic layers were washed with brine (2 x150 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3-bromo-4-chloro-6-fluoro-2-[4-(4-methyl-1,2,4-triazol-3-yl)piperidin-1-yl]benzonitrile (8 g) as a light yellow solid. LCMS (ESI) m/z: 398, 400 [M+H]⁺.

A mixture of 3-bromo-4-chloro-6-fluoro-2-[4-(4-methyl-1,2,4-triazol-3-yl)piperidin-1-yl]benzonitrile (1 g, 2.5 mmol) and CH₃ONa (203.3 mg, 3.8 mmol) in MeOH (15 mL) was stirred for 8 h at room temperature. The residue was purified by silica gel column chromatography, eluted with CH₂Cl₂ / MeOH (0%-10%) to afford 3-bromo-4-chloro-6-methoxy-2-[4-(4-methyl-1,2,4-triazol-3-yl)piperidin-1-yl]benzonitrile (750 mg) as an off-white solid. LCMS (ESI) m/z: 410, 412 [M+H]⁺.

### Intermediate 22: 3-bromo-6-chloro-2-[4-(4-methyl-1,2,4-triazol-3-yl)piperazin-1-yl]benzonitrile

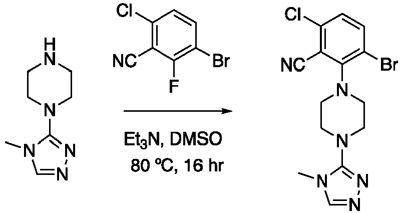

To a stirred mixture of 1-(4-methyl-1,2,4-triazol-3-yl)piperazine (500 mg, 3 mmol) and 3-bromo-6-chloro-2-fluorobenzonitrile (1051.5 mg, 4.48 mmol) in DMSO (10 mL) was added TEA (1512.9 mg, 14.9 mmol) in portions at room temperature. The resulting mixture was stirred over night at 80 °C. The reaction was quenched by the addition of water (100 mL) at room temperature. The resulting mixture was extracted with EtOAc (3 x 150 mL). The combined organic layers were washed with brine (2 x15 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3-bromo-6-chloro-2-[4-(4-methyl-1,2,4-triazol-3-yl)piperazin-1-yl]benzonitrile (150 mg) as a brown solid. LCMS (ESI) m/z: 381, 383 [M+H]⁺.

### Intermediate 23: 3-chloro-4-fluoro-2-[4-(4-methyl-1,2,4-triazol-3-yl)piperazin-1-yl]benzonitrile

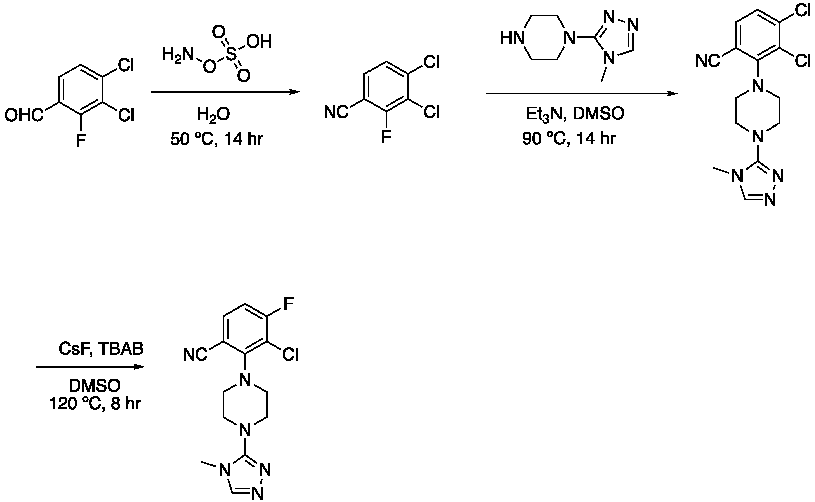

A mixture of 3,4-dichloro-2-fluorobenzaldehyde (5 g, 25.9 mmol) and aminooxysulfonic acid (5.3 g, 46.6 mmol) in H₂O (150 mL) was stirred overnight at 50 °C. The precipitated solids were collected by filtration and washed with water (3 x 100 mL). The residue was purified by silica gel column chromatography, eluted with PE / EA (0%-10%) to afford 3,4-dichloro-2-fluorobenzonitrile (3.3 g, 67%) as an off-white solid. LCMS (ESI) m/z: 191 [M+H]⁺.

To a stirred mixture of 3,4-dichloro-2-fluorobenzonitrile (852.2 mg, 4.48 mmol) and 1-bromo-2-fluorobenzene (31.4 mg, 0.18 mmol) in DMSO (10 mL) was added TEA (1512.9 mg, 14.9 mmol) in portions at room temperature. The resulting mixture was stirred overnight at 90 °C. The reaction was quenched by the addition of water (150 mL) at room temperature. The resulting mixture was extracted with EtOAc (3 x 160 mL). The combined organic layers were washed with brine (2 x 20 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3,4-dichloro-2-[4-(4-methyl-1,2,4-triazol-3-yl)piperazin-1-yl]benzonitrile (180 mg, 18%) as a light brown solid. LCMS (ESI) m/z: 338 [M+H]⁺.

To a stirred mixture of 3,4-dichloro-2-[4-(4-methyl-1,2,4-triazol-3-yl)piperazin-1-yl]benzonitrile (80 mg, 0.24 mmol) and CsF (360.4 mg, 2.37 mmol) in DMSO (8 mL) was added TBAB (7.6 mg, 0.024 mmol) in portions at room temperature. The resulting mixture was stirred for 8 h at 120 °C. The reaction was quenched by the addition of water (100 mL) at room temperature. The resulting mixture was extracted with EtOAc (3 x 110 mL). The combined organic layers were washed with brine (2 x 10 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3-chloro-4-fluoro-2-[4-(4-methyl-1,2,4-triazol-3-yl)piperazin-1-yl]benzonitrile (50 mg, 66%) as a light yellow solid. LCMS (ESI) m/z: 321 [M+H]⁺.

### Intermediate 24: 3-bromo-6-chloro-2-[4-(4-methyl-1,2,4-triazol-3-yl)piperazin-1-yl]benzonitrile

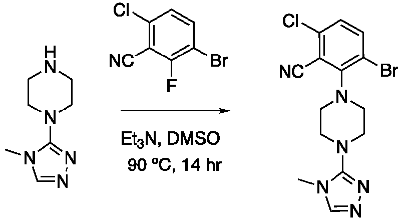

To a stirred mixture of 1-(4-methyl-1,2,4-triazol-3-yl)piperazine (300 mg, 1.79 mmol) and 3-bromo-6-chloro-2-fluorobenzonitrile (630.9 mg, 2.69 mmol) in DMSO (10 mL) was added TEA (907.7 mg, 8.97 mmol) in portions at room temperature. The resulting mixture was stirred overnight at 90 °C. The reaction was quenched by the addition of water (80 mL) at room temperature. The resulting mixture was extracted with EtOAc (3 x 90 mL). The combined organic layers were washed with brine (2 x 20 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3-bromo-6-chloro-2-[4-(4-methyl-1,2,4-triazol-3-yl)piperazin-1-yl]benzonitrile (120 mg, 17%) as a brown oil. LCMS (ESI) m/z: 381, 383 [M+H]⁺.

### Intermediate 25: 3-bromo-5-fluoro-2-[4-(4-methyl-1,2,4-triazol-3-yl)piperidin-1-yl]benzonitrile

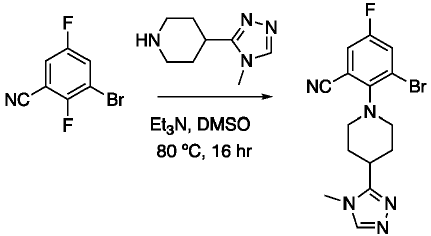

A solution of 3-bromo-2,5-difluorobenzonitrile (1 g, 4.6 mmol), 4-(4-methyl-1,2,4-triazol-3-yl)piperidine (0.9 g, 5.5 mmol) and TEA (1.4 g, 13.8 mmol) in DMSO (25 mL) was stirred for 16 h at 80 °C. The resulting mixture was diluted with water (100 mL) and was extracted with EtOAc (3 x 100 mL). The combined organic layers were washed with brine (2 x 100 mL), dried over anhydrous Na₂SO₄. After filtration, the filtrate was concentrated under reduced pressure. The residue was purified by silica gel column chromatography, eluted with CH₂Cl₂ / MeOH (9:1) to afford 3-bromo-5-fluoro-2-[4-(4-methyl-1,2,4-triazol-3-yl)piperidin-1-yl]benzonitrile (140 mg, 7%) as a light yellow oil. LCMS (ESI) m/z: 364, 366 [M+H]⁺.

### Intermediate 26: 6-bromo-4-chloro-3-(6-fluoropyridin-3-yl)-2-[4-(4-methyl-1,2,4-triazol-3-yl)piperidin-1-yl]benzonitrile

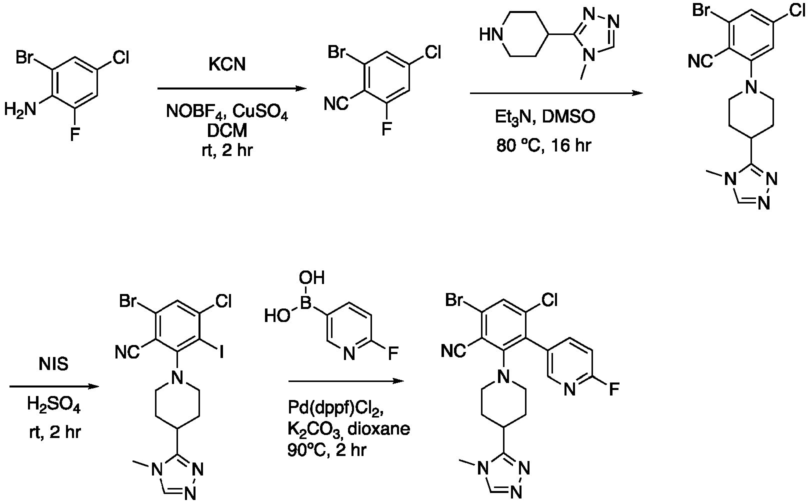

A solution of 2-bromo-4-chloro-6-fluoroaniline (25 g, 111.4 mmol), KCN (14.5 g, 222.7 mmol), NOBF₄ (13 g, 111.4 mmol) and CuSO₄ (53.3 g, 334.1 mmol) in DCM (50 mL) was stirred for 2 h at room temperature. The reaction was quenched with Fe₂SO₄ sat. sodium hyposulfite (aq.) at room temperature. The mixture was basified to pH 10 with saturated NaHCO₃ (aq.). The resulting mixture was diluted with water (200 mL) and extracted with CH₂Cl₂ (3 x 200 mL). The combined organic layers were washed with brine (2 x 200 mL), dried over anhydrous Na₂SO₄. After filtration, the filtrate was concentrated under reduced pressure. The residue was purified by silica gel column chromatography, eluted with PE / EA (1:1) to afford 2-bromo-4-chloro-6-fluorobenzonitrile (10 g, 38%) as a light yellow oil. LCMS (ESI) m/z: 234, 236 [M+H]⁺.

A solution of 2-bromo-4-chloro-6-fluorobenzonitrile (5 g, 21.3 mmol), 4-(4-methyl-1,2,4-triazol-3-yl)piperidine (4.2 g, 25.6 mmol) and TEA (6.5 g, 64 mmol) in DMSO (25 mL) was stirred for 16 h at 80 °C. The resulting mixture was diluted with water (200 mL) and extracted with EtOAc (3 x 200 mL). The combined organic layers were washed with brine (2 x 200 mL), dried over anhydrous Na₂SO₄. After filtration, the filtrate was concentrated under reduced pressure. The residue was purified by silica gel column chromatography, eluted with CH₂Cl₂ / MeOH (9:1) to afford 2-bromo-4-chloro-6-[4-(4-methyl-1,2,4-triazol-3-yl)piperidin-1-yl]benzonitrile (1.1 g, 11%) as a light yellow oil. LCMS (ESI) m/z: 380, 382 [M+H]⁺.

A solution of 2-bromo-4-chloro-6-[4-(4-methyl-1,2,4-triazol-3-yl)piperidin-1-yl]benzonitrile (1.1 g, 2.9 mmol) and NIS (0.65 g, 2.9 mmol) in H₂SO₄ (20 mL) was stirred for 2 h at room temperature. The mixture was basified to pH 10 with NaOH. The resulting mixture was diluted with water (200 mL) and extracted with EtOAc (3 x 200 mL). The combined organic layers were washed with brine (2 x 200 mL), dried over anhydrous Na₂SO₄. After filtration, the filtrate was concentrated under reduced pressure. The residue was purified by silica gel column chromatography, eluted with CH₂Cl₂ / MeOH (9:1) to afford 6-bromo-4-chloro-3-iodo-2-[4-(4-methyl-1,2,4-triazol-3-yl)piperidin-1-yl]benzonitrile (400 mg, 23%) as a light yellow solid. LCMS (ESI) m/z: 506, 508 [M+H]⁺.

A solution of 6-bromo-4-chloro-3-iodo-2-[4-(4-methyl-1,2,4-triazol-3-yl)piperidin-1-yl]benzonitrile (400 mg, 0.79 mmol), 6-fluoropyridin-3-ylboronic acid (0.09 g, 0.63 mmol), K₂CO₃ (0.33 g, 2.37 mmol) and Pd(dppf)Cl₂ (0.06 g, 0.08 mmol) in 1,4-dioxane (16 mL), H₂O (2 mL) was stirred for 2 h at 90 °C under nitrogen atmosphere. The resulting mixture was concentrated under reduced pressure. The residue was purified by silica gel column chromatography, eluted with CH₂Cl₂ / MeOH (9:1) to afford 6-bromo-4-chloro-3-(6-fluoropyridin-3-yl)-2-[4-(4-methyl-1,2,4-triazol-3-yl)piperidin-1-yl]benzonitrile (270 mg, 68%) as a light yellow oil. LCMS (ESI) m/z: 475, 477 [M+H]⁺.

### Intermediate 27: iodo-3-[4-(4-methyl-1,2,4-triazol-3-yl)piperidin-1-yl]pyridine-2-carbonitrile

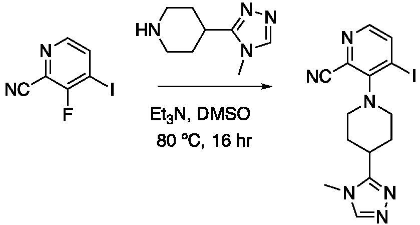

To a stirred solution of 4-(4-methyl-1,2,4-triazol-3-yl)piperidine (2 g, 12 mmol) and 3-fluoro-4-iodopyridine-2-carbonitrile (3.6 g, 14.4 mmol) in DMSO (50 mL) was added TEA (6.1 g, 60.1 mmol) in portions at room temperature. The resulting mixture was stirred for 16 h at 80 °C. To the reaction mixture of water (10 mL) was added at room temperature and extracted with EtOAc (3 x 50 mL). The combined organic layers were washed with water (2 x 50 mL), dried over anhydrous Na₂SO₄. After filtration, the filtrate was concentrated under reduced pressure. The residue was purified by silica gel column chromatography, eluted with MeOH in CH₂Cl₂ (0%-10%) to afford 4-iodo-3-[4-(4-methyl-1,2,4-triazol-3-yl)piperidin-1-yl]pyridine-2-carbonitrile (200 mg, 4%) as a red solid. LCMS (ESI) m/z: 395 [M+H]⁺.

### Intermediate 28: 2-chloro-3-[4-(4-methyl-1,2,4-triazol-3-yl)piperidin-1-yl]pyridine-4-carbonitrile

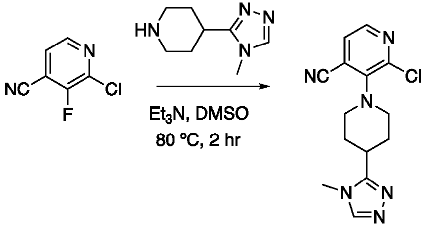

To a stirred mixture of 2-chloro-3-fluoropyridine-4-carbonitrile (2 g, 12.8 mmol) and 4-(4-methyl-1,2,4-triazol-3-yl)piperidine (4.2 g, 25.5 mmol) in DMSO (40 mL) was added TEA (6.5 g, 63.9 mmol) dropwise at room temperature. The resulting mixture was stirred for 2 h at 80 C. Water was added at room temperature and extracted with EtOAc (2 x 150 mL). The combined organic layers were washed with brine (100 mL), dried over anhydrous Na₂SO₄. After filtration, the filtrate was concentrated under reduced pressure. The residue was purified by silica gel column chromatography, eluted with MeOH /CH₂Cl₂ (0-10%) to afford 2-chloro-3-[4-(4-methyl-1,2,4-triazol-3-yl)piperidin-1-yl]pyridine-4-carbonitrile (1 g, 26%) as a red solid. LCMS (ESI) m/z: 303 [M+H]⁺.

### Intermediate 29: 2-chloro-3-[4-(1-methyl-5-imidazolyl)-1-piperidyl]isonicotinonitrile

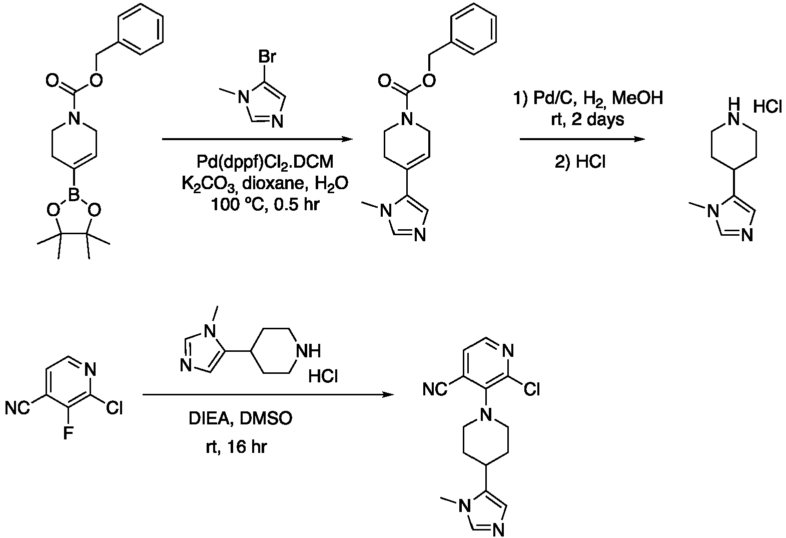

A mixture of benzyl 4-(4,4,5,5-tetramethyl-1,3,2-dioxaborolan-2-yl)-1,2,3,6-tetrahydro-1-pyridinecarboxylate (2 g, 5.83 mmol), 5-bromo-1-methylimidazole (985 mg, 6.12 mmol), K₂CO₃ (1.61 g, 11.7 mmol), Pd(dppf)Cl_{2.} DCM (238 mg, 0.29 mmol) were dissolved in 1,4 dioxane (19.4 mL) and water (5 mL). The reaction was heated at 100 °C for 30 minutes. Reaction was quenched with water, extracted with EtOAc, dried with anhydrous Na₂SO₄. After filtration, the filtrate was concentrated under reduced pressure and purified by silica gel column chromatography (0-100% Hex : [25% EtOH in EtOAc]) to afford benzyl 4-(1-methyl-5-imidazolyl)-1,2,3,6-tetrahydro-1-pyridinecarboxylate (1.15 g, 66%) as a brown oil.

A solution of benzyl 4-(1-methyl-5-imidazolyl)-1,2,3,6-tetrahydro-1-pyridinecarboxylate (1.15 g, 3.87 mmol) was dissolved in MeOH (13 mL) and added Pd/C (250 mg). The reaction vessel was placed under a hydrogen atmosphere and stirred at room temperature over the weekend. The mixture was filtered over celite, washed with methanol. The solution was dried in vacuo to afford and orange residue. The residue was dissolved in Et₂O (50 mL) and MeOH (1 mL) and HCl gas was bubbled through the reaction mixture. The resultant precipitate was filtered and dried in vacuo to afford 1-methyl-5-(4-piperidyl)imidazole-hydrogen chloride (658 mg, 84%) as a tan solid. LCMS (ESI) m/z: 166 [M+H]⁺.

To a mixture of 1-methyl-5-(4-piperidyl)imidazole-hydrogen chloride (1/1) (0.1 g, 0.5 mmol), 2-chloro-3-fluoroisonicotinonitrile (85.4 mg, 0.54 mmol) in DMSO (2.1 mL) was added DIEA (192 mg, 1.5 mmol). The reaction was stirred at room temperature overnight. Solvent was removed in vacuo and the residue was purified by silica gel column chromatography (0-100%, Hex : [25% EtOH in EtOAc] to afford 2-chloro-3-[4-(1-methyl-5-imidazolyl)-1-piperidyl]isonicotinonitrile_(46 mg, 31%). LCMS (ESI) m/z: 302 [M+H]⁺.

### Intermediate 30: 2-chloro-3-[4-(4-methyl-1,2,4-triazol-3-yl)piperazin-1-yl]pyridine-4-carbonitrile

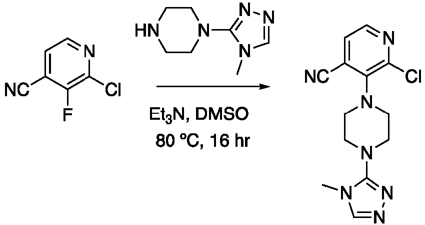

To a stirred mixture of 2-chloro-3-fluoropyridine-4-carbonitrile (200 mg, 1.28 mmol) and 1-(4-methyl-1,2,4-triazol-3-yl)piperazine (320.5 mg, 1.92 mmol) in DMSO (5 mL) was added TEA (646.4 mg, 6.39 mmol) in portions at room temperature. The resulting mixture was stirred overnight at 80 °C under nitrogen atmosphere. The reaction was quenched by the addition of water (50 mL) at room temperature. The resulting mixture was extracted with EtOAc (3 x 60 mL). The combined organic layers were washed with brine (2 x 10 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 2-chloro-3-[4-(4-methyl-1,2,4-triazol-3-yl)piperazin-1-yl]pyridine-4-carbonitrile (210 mg) as a green solid. LCMS (ESI) m/z: 304 [M+H]⁺.

### Intermediate 31: 2-chloro-5-methoxy-3-[4-(4-methyl-1,2,4-triazol-3-yl)piperidin-1-yl]pyridine-4-carbonitrile

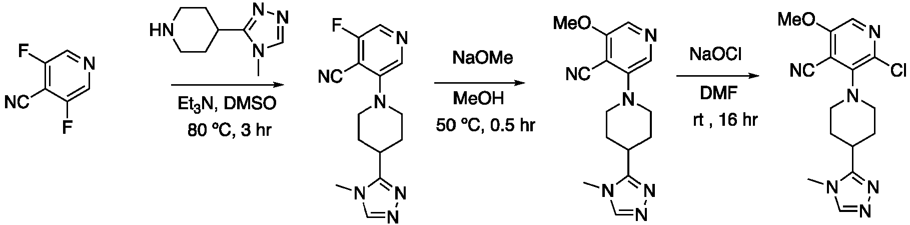

A solution of 3,5-difluoropyridine-4-carbonitrile (5 g, 35.7 mmol), 4-(4-methyl-1,2,4-triazol-3-yl)piperidine (5.93 g, 35.7 mmol) and TEA (18.1 g, 178.4 mmol) in DMSO (25 mL) was stirred for 3 h at 80 °C. The resulting mixture was diluted with water (100 mL) and extracted with EtOAc (3 x 100 mL). The combined organic layers were washed with brine (2 x 100 mL), dried over anhydrous Na₂SO₄. After filtration, the filtrate was concentrated under reduced pressure. The residue was purified by silica gel column chromatography, eluted with CH₂Cl₂ / MeOH (9:1) to afford 3-fluoro-5-[4-(4-methyl-1,2,4-triazol-3-yl)piperidin-1-yl]pyridine-4-carbonitrile (2.5 g, 24%) as a light yellow oil. LCMS (ESI) m/z: 287 [M+H]⁺.

A solution of 3-fluoro-5-[4-(4-methyl-1,2,4-triazol-3-yl)piperidin-1-yl]pyridine-4-carbonitrile (1 g, 3.49 mmol) and sodium methoxide (0.23 g, 4.19 mmol) in MeOH (15 mL) was stirred for 30 min at 50 °C. The resulting mixture was diluted with water (80 mL) and extracted with EtOAc (3 x 80 mL). The combined organic layers were washed with brine (2 x 80 mL), dried over anhydrous Na₂SO₄. After filtration, the filtrate was concentrated under reduced pressure. The residue was purified by silica gel column chromatography, eluted with CH₂Cl₂ / MeOH (9:1) to afford 3-methoxy-5-[4-(4-methyl-1,2,4-triazol-3-yl)piperidin-1-yl]pyridine-4-carbonitrile (440 mg, 42%) as a light yellow oil. LCMS (ESI) m/z: 299 [M+H]⁺.

A solution of 3-methoxy-5-[4-(4-methyl-1,2,4-triazol-3-yl)piperidin-1-yl]pyridine-4-carbonitrile (440 mg, 1.47 mmol) and sodium hypochlorite (109.8 mg, 1.47 mmol) in DMF (10 mL) was stirred for 16 h at room temperature under nitrogen atmosphere. The resulting mixture was diluted with water (30 mL) and extracted with EtOAc (3 x 30 mL). The combined organic layers were washed with brine (2 x 30 mL), dried over anhydrous Na₂SO₄. After filtration, the filtrate was concentrated under reduced pressure. The residue was purified by silica gel column chromatography, eluted with CH₂Cl₂ / MeOH (9:1) to afford 2-chloro-5-methoxy-3-[4-(4-methyl-1,2,4-triazol-3-yl)piperidin-1-yl]pyridine-4-carbonitrile (110 mg, 22%) as a light yellow oil.

### Synthesis of Example Compounds

### Example 1: 3-(6-fluoropyridin-3-yl)-2-[4-(4-methyl-4H-1,2,4-triazol-3-yl)piperidin-1-yl]benzonitrile

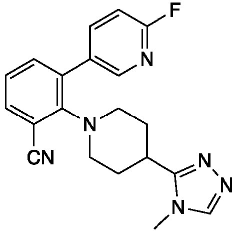

The title compound was prepared using the following procedure:

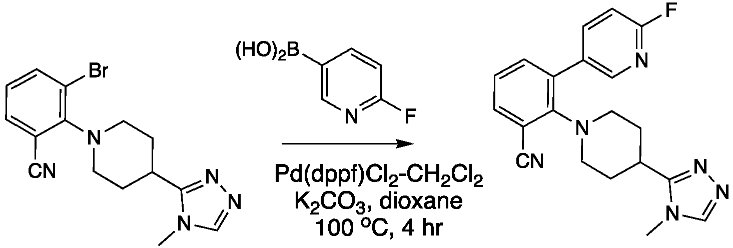

To a stirred solution of Intermediate 1 (80 mg, 0.2 mmol) and K₂CO₃ (64 mg, 0.5 mmol) in 1,4-dioxane (3 mL) were added Pd(dppf)Cl₂.CH₂Cl₂ (19 mg, 0.1 mmol) and 6-fluoropyridin-3-ylboronic acid (36 mg, 0.3 mmol) at room temperature under nitrogen atmosphere. The final reaction mixture was heated for 4 h at 100 °C. The reaction was quenched by the addition of water (30 mL) at room temperature. The resulting mixture was extracted with EtOAc (3 x 50 mL). The combined organic layers were washed with brine (3 x 50 mL), dried over anhydrous Na₂SO₄. After filtration, the filtrate was concentrated under reduced pressure. The crude product was purified by reverse phase flash with the following conditions (Column: X Bridge Shield RP18 OBD Column, 19*150 mm, 5µm; Mobile Phase A: water (10 mmol/L NH₄HCO₃), Mobile Phase B: ACN; Flow rate: 25 mL/min; Gradient: 15% B to 35% B in 7 min, 35% B; Wavelength: 220 nm; to afford Example 1. LCMS (ESI) m/z: 363.05 [M+H]⁺.

### Example 2: 2-[4-(4-methyl-4H-1,2,4-triazol-3-yl)piperidin-1-yl]-3-(pyridin-3-yl)benzonitrile

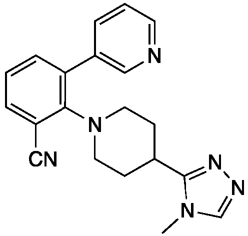

The title compound was prepared using the following procedure:

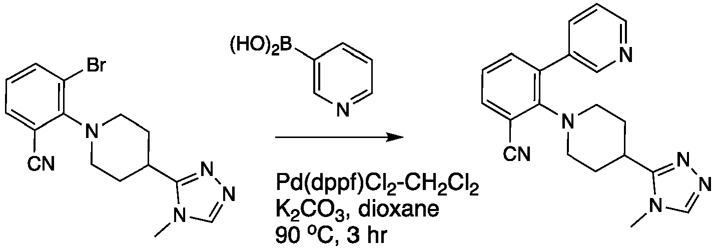

To a stirred mixture of Intermediate 1 (20 mg, 0.06 mmol) and pyridin-3-ylboronic acid (8.5 mg, 0.07 mmol) in dioxane (3 mL) /H₂O (0.5 mL) were added K₂CO₃ (24.0 mg, 0.2 mol) and Pd(dppf)Cl₂.CH₂Cl₂ (4.7 mg, 0.006 mmol,) in portions at room temperature under nitrogen atmosphere. The resulting mixture was stirred for 3 h at 90 °C under nitrogen atmosphere. The reaction was quenched with water (50 mL) at room temperature. The resulting mixture was extracted with EtOAc (3 x 20 mL). The combined organic layers were washed with brine (2 x 20 mL), dried over anhydrous Na₂SO₄. After filtration, the filtrate was concentrated under reduced pressure. The crude product (10 mg) was purified by Prep-HPLC with the following conditions (Column: YMC-Actus Triart C18 ExRS, 30*150 mm, 5µm; Mobile Phase A: water (10 mmol/L NH₄HCO₃), Mobile Phase B: ACN; Flow rate: 60 mL/min; Gradient: 27% B to 42% B in 7 min, 42% B; Wavelength: 220 nm; RT1(min): 4.65; to afford Example 2. LCMS (ESI) m/z: 345.15 [M+H]⁺.

### Example 3: 3-(4-methoxypyridin-3-yl)-2-[4-(4-methyl-4H-1,2,4-triazol-3-yl)piperidin-1-yl]benzonitrile

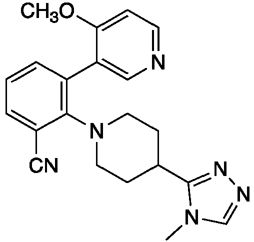

The title compound was prepared using the following procedure:

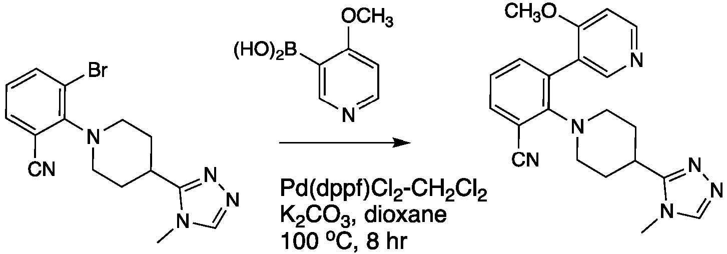

To a stirred mixture of Intermediate 1 (40 mg, 0.12 mmol) and 4-methoxypyridin-3-ylboronic acid (21.2 mg, 0.1 mmol) in dioxane (5 mL)/H₂O (0.8 mL) were added K₂CO₃ (47.9 mg, 0.3 mmol) and Pd(dppf)Cl₂.CH₂Cl₂ (9.4 mg, 0.01 mmol) at room temperature under nitrogen atmosphere. The resulting mixture was stirred for 8 h at 100 °C under nitrogen atmosphere. The reaction was quenched with water (50 mL) at room temperature. The resulting mixture was extracted with EtOAc (3 x 20 mL). The combined organic layers were washed with brine (2 x 20 mL), dried over anhydrous Na₂SO₄. After filtration, the filtrate was concentrated under reduced pressure. The crude product (10 mg) was purified by Prep-HPLC with the following conditions (Column: XBridge Shield RP18 OBD Column, 19*150 mm, 5µm; Mobile Phase A: water (10 mmol/L NH₄HCO₃), Mobile Phase B: ACN; Flow rate: 25 mL/min; Gradient: 34% B to 58% B in 8 min, 58% B; Wavelength: 220 nm; RT1(min): 7.35; to afford Example 3. LCMS (ESI) m/z: 375.10 [M+H]⁺.

### Example 4: 3-{ 1-methyl-1H-pyrazolo[3,4-c]pyridin-4-yl}-2-[4-(4-methyl-4H-1,2,4-triazol-3-yl)piperidin-1-yl]benzonitrile

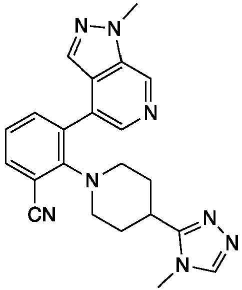

The title compound was prepared using the following procedure:

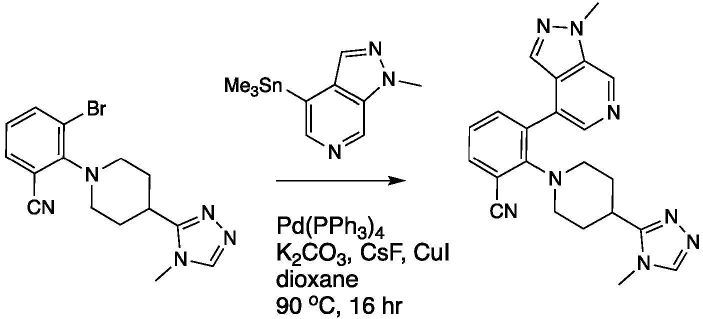

To a stirred mixture of 1-methyl-4-(trimethylstannyl)pyrazolo[3,4-c]pyridine (20 mg, 0.07 mmol) and Intermediate 1 (25.7 mg, 0.08 mmol) in dioxane (5 mL) were added Pd(PPh₃)₄ (7.8 mg, 0.007 mmol), K₂CO₃ (28.0 mg, 0.2 mmol), CsF (10.3 mg, 0.07 mmol) and CuI (12.9 mg, 0.07 mmol) at room temperature under nitrogen atmosphere. The resulting mixture was stirred for 16 h at 90° C under nitrogen atmosphere. The reaction was quenched with water (40 mL) at room temperature. The resulting mixture was extracted with EtOAc (3 x 15 mL). The combined organic layers were washed with water (2 x 15 mL), dried over anhydrous Na₂SO₄. After filtration, the filtrate was concentrated under reduced pressure. The crude product (10 mg) was purified by Prep-HPLC with the following conditions (Column: Xselect CSH C18 OBD Column 30* 150mm 5µm, n; Mobile Phase A: water (0.1% FA), Mobile Phase B: MeOH; Flow rate: 60 mL/min; Gradient: 34% B to 60% B in 9 min, 60% B; Wavelength: 254/220 nm; RT1(min): 6.73; to afford Example 4. LCMS (ESI) m/z: 399.15 [M+H]⁺.

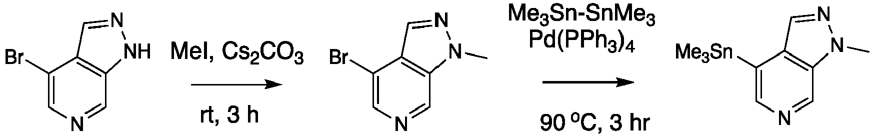

To a stirred mixture of 4-bromo-1H-pyrazolo[3,4-c]pyridine (500 mg, 2.5 mmol) and Cs₂CO₃ (2.1 g, 6.3 mmol) in DMF (20 mL) was added CH₃I (394.2 mg, 2.8 mmol) in portions at room temperature. The resulting mixture was stirred for 3 h at room temperature. The reaction was quenched with water (100 mL) at room temperature. The resulting mixture was extracted with EtOAc (3 x 50 mL). The combined organic layers were washed with brine (2 x 50 mL), dried over anhydrous Na₂SO₄. After filtration, the filtrate was concentrated under reduced pressure. The residue was purified by silica gel column chromatography, eluted with EA / PE (60%-90%) to afford 4-bromo-1-methylpyrazolo[3,4-c]pyridine (280 mg, 52%) as a light yellow solid. LCMS (ESI) m/z: 212, 214 [M+H]⁺.

To a stirred mixture of 4-bromo-1-methylpyrazolo[3,4-c]pyridine (100.0 mg, 0.5 mmol) and hexamethyldistannane (185.4 mg, 0.6 mmol) in dioxane (10 mL) was added Pd(PPh₃)₄ (54.5 mg, 0.05 mmol) in portions at room temperature under nitrogen atmosphere. The resulting mixture was stirred for 3 h at 90 ° C under nitrogen atmosphere. The reaction was quenched with water (80 mL) at room temperature. The resulting mixture was extracted with EtOAc (3 x 30 mL). The combined organic layers were washed with brine (2 x 30 mL), dried over anhydrous Na₂SO₄. After filtration, the filtrate was concentrated under reduced pressure. The residue was purified by silica gel column chromatography, eluted with DCM / MeOH (0%-12%) to afford 1-methyl-4-(trimethylstannyl)pyrazolo[3,4-c]pyridine (89 mg, 63.8%) as a brown solid. LCMS (ESI) m/z: 298.0 [M+H]⁺.

### Example 5: 2-[4-(4-methyl-4H-1,2,4-triazol-3-yl)piperidin-1-yl]-3-(pyrazin-2-yl)benzonitrile

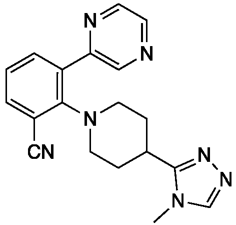

The title compound was prepared using the following procedure:

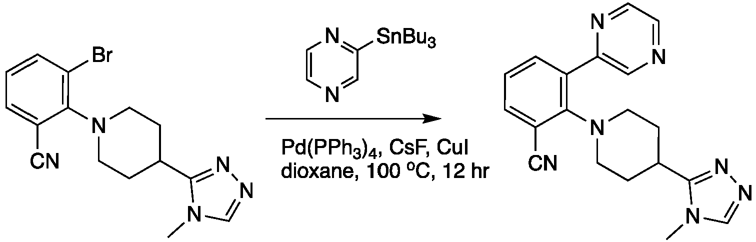

To a stirred solution of Intermediate 1 (50 mg, 0.14 mmol) and 2-(tributylstannyl)pyrazine (53 mg, 0.15 mmol) in anhydrous 1,4-dioxane (10 mL) and H₂O (1 mL) was added CuI (55 mg, 0.29 mmol) and CsF (43.9 mg, 0.29 mmol) followed by catalytic amount of Pd(PPh₃)₄ (16.7 mg, 0.014 mmol) at room temperature. The resulting mixture was stirred for 12 h at 100 °C. The reaction was quenched by the addition of water (30 mL) at room temperature. The resulting mixture was extracted with EtOAc (3 x 50 mL). The combined organic layers were washed with brine (3 x 50 mL), dried over anhydrous Na₂SO₄. After filtration, the filtrate was concentrated under reduced pressure. The crude product was purified by reverse phase flash with the following conditions (Column: YMC-Actus Triart C18 ExRS, 30*150 mm, 5µm; Mobile Phase A: water (10 mmol/L NH₄HCO₃), Mobile Phase B: ACN; Flow rate: 60 mL/min; Gradient: 25% B to 60% B in 7 min, 60% B; Wavelength: 220 nm; RT1(min): 6.88) to afford Example 5. LCMS (ESI) m/z: 346.05 [M+H]⁺.

### Example 6: 3-[6-(2-hydroxyethoxy)pyridin-3-yl]-2-[4-(4-methyl-4H-1,2,4-triazol-3-yl)piperidin-1-yl]benzonitrile

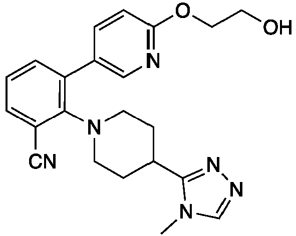

The title compound was prepared using the following procedure:

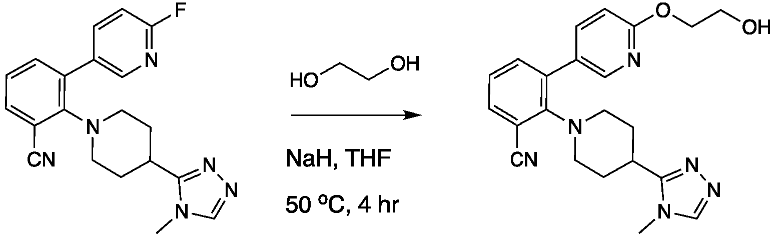

A solution of ethylene glycol (11 mg, 0.18 mmol) and NaH (4 mg, 0.2 mmol) in THF (10 mL) was stirred for 30 min at 0 °C under N₂ atmosphere. To the above mixture was added 3-(6-fluoropyridin-3-yl)-2-[4-(4-methyl-1,2,4-triazol-3-yl)piperidin-1-yl]benzonitrile (60 mg, 0.2 mmol) over 5 min at 0 °C. The resulting mixture was stirred for additional 4 h at 50 °C. The reaction was quenched by the addition of water (30 mL) at room temperature. The resulting mixture was extracted with EtOAc (3 x 50 mL). The combined organic layers were washed with brine (3 x 50 mL), dried over anhydrous Na₂SO₄. After filtration, the filtrate was concentrated under reduced pressure. The crude product was purified by reverse phase flash with the following conditions (Column: X Bridge Shield RP18 OBD Column, 19*150 mm, 5µm; Mobile Phase A: water (10 mmol/L NH₄HCO₃), Mobile Phase B: ACN; Flow rate: 25 mL/min; Gradient: 18% B to 45% B in 7 min, 45% B; Wavelength: 220 nm; RT1(min): 6.47; to afford Example 6. LCMS (ESI) m/z: 405.10 [M+H]⁺.

### Example 7: 3-(4-hydroxypyridin-3-yl)-2-[4-(4-methyl-4H-1,2,4-triazol-3-yl)piperidin-1-yl]benzonitrile

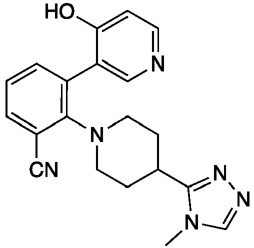

The title compound was prepared using the following procedure:

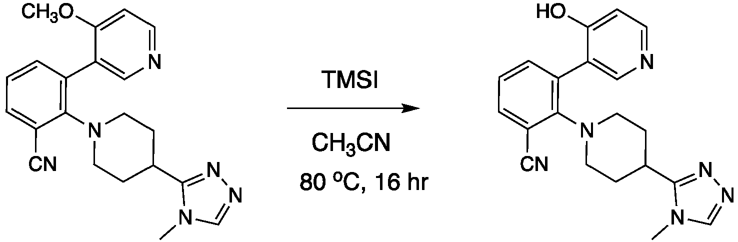

A mixture of 3-(4-methoxypyridin-3-yl)-2-(4-(4-methyl-4H-1,2,4-triazol-3-yl)piperidin-1-yl)benzonitrile (40 mg, 0.11 mmol) and TMSI (213.7 mg, 1.07 mmol) in acetonitrile (5 mL) was stirred overnight at 80 °C under nitrogen atmosphere. The residue was basified to pH 10 with saturated aqueous Na₂CO₃. The resulting mixture was extracted with CH₂Cl₂ (3 x 100 mL). The combined organic layers were washed with brine (2 x 50 mL), dried over anhydrous Na₂SO₄. After filtration, the filtrate was concentrated under reduced pressure. The crude product (5 mg) was purified by Prep-HPLC with the following conditions (Column: YMC-Actus Triart C18, 30*150 mm, 5µm; Mobile Phase A: water (0.1% FA), Mobile Phase B: ACN; Flow rate: 60 mL/min; Gradient: 20% B to 40% B in 7 min, 40% B; Wavelength: 254/2220 nm; RT1(min): 6.42; to afford Example 7. LCMS (ESI) m/z: 361.10 [M+H]⁺.

### Example 8: 3-{4-[2-(dimethylamino)ethoxy]pyridin-3-yl}-2-[4-(4-methyl-4H-1,2,4-triazol-3-yl)piperidin-1-yl]benzonitrile

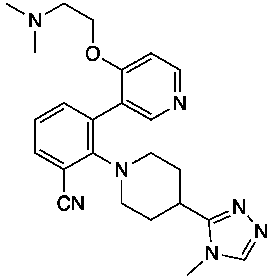

The title compound was prepared using the following procedure:

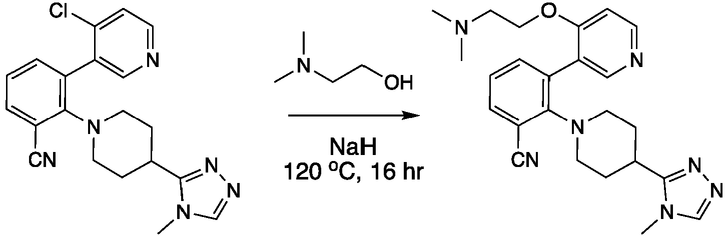

A solution of NaH (4 mg, 0.2 mmol) in dimethylaminoethanol (3 mL, 0.1 mmol) was stirred for 30 min at 0 °C under nitrogen atmosphere. To the above mixture was added 3-(4-chloropyridin-3-yl)-2-[4-(4-methyl-1,2,4-triazol-3-yl) piperidin-1-yl] benzonitrile (25 mg, 0.1 mmol) at room temperature. The resulting mixture was stirred at 120 °C overnight. The reaction was quenched by the addition of water (2 mL) at room temperature. After filtration, the filtrate was concentrated under reduced pressure. The crude product was purified by reverse phase flash with the following conditions (Column: YMC-Actus Triart C18, 30*150 mm, 5µm; Mobile Phase A: water (0.1%FA), Mobile Phase B: ACN; Flow rate: 60 mL/min; Gradient: 25% B to 45% B in 7 min, 45% B; Wavelength: 254/220 nm; RT1(min): 5.92; to afford Example 8. LCMS (ESI) m/z: 432.10 [M+H]⁺.

### Example 9: 3-[4-(2-hydroxyethoxy)pyridin-3-yl]-2-[4-(4-methyl-4H-1,2,4-triazol-3-yl)piperidin-1-yl]benzonitrile

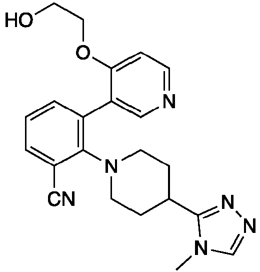

The title compound was prepared using the following procedure:

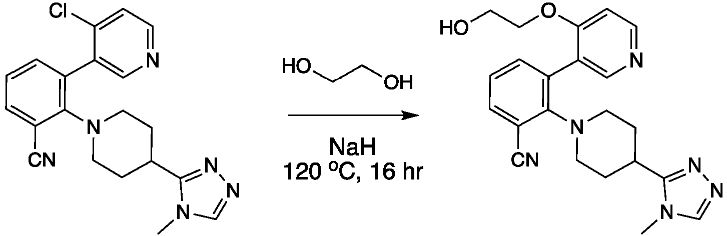

A solution of NaH (4 mg, 0.2 mmol) in ethylene glycol (3 mL, 0.1 mmol) was stirred for 30 min at 0 °C under nitrogen atmosphere. To the above mixture was added 3-(4-chloropyridin-3-yl)-2-[4-(4-methyl-1,2,4-triazol-3-yl) piperidin-1-yl] benzonitrile (25 mg, 0.1 mmol) over 2 min at room temperature. The resulting mixture was stirred at 120 °C overnight. The reaction was quenched by the addition of water (2 mL) at room temperature. After filtration, the filtrate was concentrated under reduced pressure. The crude product was purified by reverse phase flash with the following conditions (Column: X Bridge BEH130 Prep C18 OBD Column, 19*150 mm, 5µm; Mobile Phase A: water (0.05% NH₃.H₂O), Mobile Phase B: ACN; Flow rate: 60 mL/min; Gradient: 18% B to 30% B in 7 min, 30% B; Wavelength: 220 nm; RT1(min): 6.42; to afford Example 9. LCMS (ESI) m/z: 405.05 [M+H]⁺.

### Example 10: 3-(4-chloropyridin-3-yl)-2-[4-(4-methyl-4H-1,2,4-triazol-3-yl)piperidin-1-yl]benzonitrile

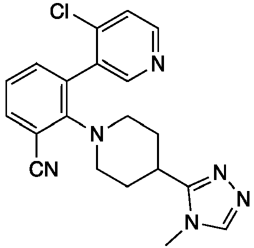

The title compound was prepared using the following procedure:

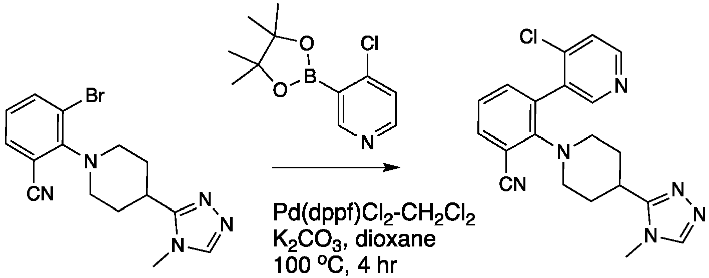

To a stirred solution of Intermediate 1 (50 mg, 0.14 mmol) and K₂CO₃ (40 mg, 0.5 mmol) in 1,4-dioxane (3 mL) were added Pd(dppf)Cl₂.CH₂Cl₂ (16 mg, 0.1 mmol) and 4-chloro-3-(4,4,5,5-tetramethyl-1,3,2-dioxaborolan-2-yl) pyridine (40 mg, 0.3 mmol) at room temperature under nitrogen atmosphere. The final reaction mixture was heated at 100 °C for 4 h. The reaction was quenched by the addition of water (30 mL) at room temperature. The resulting mixture was extracted with EtOAc (3 x 50 mL). The combined organic layers were washed with brine (3 x 50 mL), and dried over anhydrous Na₂SO₄. After filtration, the filtrate was concentrated under reduced pressure. The crude product was purified by reverse phase flash with the following conditions (Column: X Bridge Shield RP18 OBD Column, 19*150 mm, 5µm; Mobile Phase A: water (10 mmol/L NH₄HCO₃), Mobile Phase B: ACN; Flow rate: 25 mL/min; Gradient: 18% B to 45% B in 7 min, 45% B; Wavelength: 220 nm; RT1(min): 6.47; to afford Example 10. LCMS (ESI) m/z: 379.00 [M+H]⁺.

### Example 11: 2-[4-(4-methyl-4H-1,2,4-triazol-3-yl)piperidin-1-yl]-3-(pyridazin-4-yl)benzonitrile

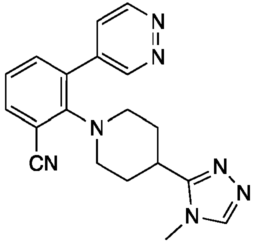

The title compound was prepared using the following procedure:

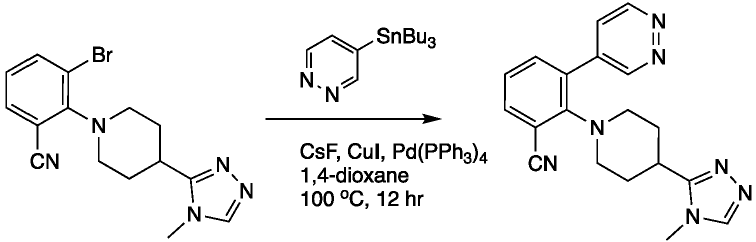

To a stirred solution of Intermediate 1 (50 mg, 0.14 mmol) and 4-(tributylstannyl)pyridazine (63.9 mg, 0.17 mmol) in anhydrous 1,4-dioxane (10 mL) and H₂O (1 mL) was added CuI (55 mg, 0.29 mmol) and CsF (43.9 mg, 0.29 mmol) followed by catalytic amount of Pd(PPh₃)₄ (16.7 mg, 0.014 mmol) at room temperature. The resulting mixture was stirred for 12 h at 100 °C. The reaction was quenched by the addition of water (30 mL) at room temperature. The resulting mixture was extracted with EtOAc (3 x 50 mL). The combined organic layers were washed with brine (3 x 50 mL), dried over anhydrous Na₂SO₄. After filtration, the filtrate was concentrated under reduced pressure. The crude product was purified by reverse phase flash with the following conditions (Column: YMC-Actus Triart C18 ExRS, 30*150 mm, 5µm; Mobile Phase A: water (10 mmol/L NH₄HCO₃), Mobile Phase B: ACN; Flow rate: 60 mL/min; Gradient: 25% B to 60% B in 7 min, 60% B; Wavelength: 220 nm; RT1(min): 6.88;) to afford Example 11. LCMS (ESI) m/z: 346.05 [M+H]⁺.

### Example 12: 3-{1-methyl-1H-pyrrolo[2,3-c]pyridin-4-yl}-2-[4-(4-methyl-4H-1,2,4-triazol-3-yl)piperidin-1-yl]benzonitrile

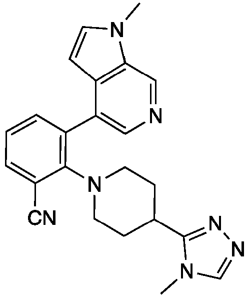

The title compound was prepared using the following procedure:

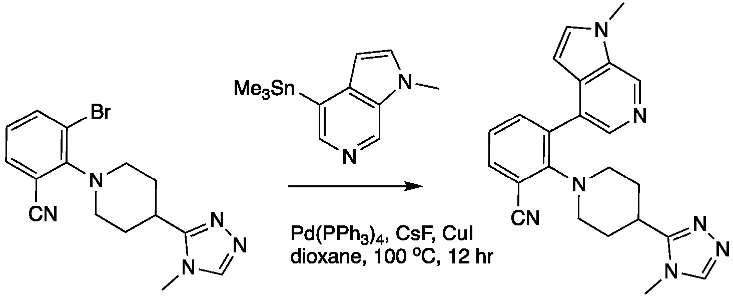

To a stirred solution Intermediate 1 (100 mg, 0.29 mmol) and 1-methyl-4-(trimethylstannyl)pyrrolo[2,3-c]pyridine (102.2 mg, 0.35 mmol) in anhydrous 1,4-dioxane (10 mL) and H₂O (1 mL) was added CuI (110 mg, 0.58 mmol) and CsF (87.8 mg, 0.58 mmol) followed by catalytic amount of Pd(PPh₃)₄ (33.4 mg, 0.03 mmol) at room temperature. The resulting mixture was stirred for 12 h at 100 °C. The reaction was quenched by the addition of water (30 mL) at room temperature. The resulting mixture was extracted with EtOAc (3 x 50 mL). The combined organic layers were washed with brine (3 x 50 mL), dried over anhydrous Na₂SO₄. After filtration, the filtrate was concentrated under reduced pressure. The crude product was purified by reverse phase flash with the following conditions (Column: X select CSH C18 OBD Column 30*150mm 5µm, n; Mobile Phase A: water (0.1% FA), Mobile Phase B: ACN; Flow rate: 60 mL/min; Gradient: 10% B to 33% B in 7 min, 33% B; Wavelength: 220 nm; RT1(min): 6.32) to afford Example 12. LCMS (ESI) m/z: 398.10 [M+H]⁺.

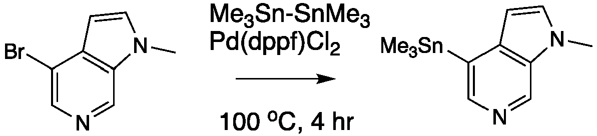

A solution of hexamethyldistannane (372 mg, 1 mmol) in 1,4-dioxane (10 mL) was treated with Pd(dppf)Cl₂ (77 mg, 0.1 mmol) for 5 min at room temperature under nitrogen atmosphere followed by the addition of 4-bromo-1-methylpyrrolo[2,3-c] pyridine (200 mg, 1 mmol) at room temperature. The reaction mixture was stirred at 100°C for a period of 4 h. The reaction was quenched with water (20 mL) at room temperature. The resulting mixture was extracted with EtOAc (3 x 50 mL). The combined organic layers were washed with brine (3 x 50 mL), dried over anhydrous Na₂SO₄. The residue was purified by silica gel column chromatography, eluted with CH₃CN/H₂O (8:1) to afford 4-(trimethylstannyl)-1H-pyrrolo[2,3-c] pyridine (110 mg) as a brown oil.

### Example 13: 2-[4-(1H-imidazol-1-yl)piperidin-1-yl]-3-(4-methoxypyridin-3-yl)benzonitrile

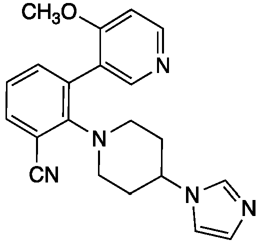

The title compound was prepared using the following procedure:

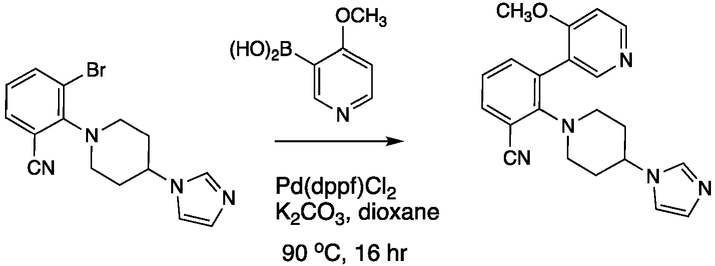

To a stirred solution of Intermediate 2 (300 mg, 0.91 mmol) in dioxane (12 mL) and H₂O (2 mL) were added 4-methoxypyridin-3-yl boronic acid (277 mg, 1.81 mmol), K₂CO₃ (250.4 mg, 1.81 mmol) and Pd(dppf)Cl₂ (66.3 mg, 0.091 mmol) in portions at room temperature under nitrogen atmosphere. The resulting mixture was stirred overnight at 90 °C. The resulting mixture was concentrated under reduced pressure. The residue was purified by silica gel column chromatography, eluted with CH₂Cl₂/MeOH (6: 1) to afford a dark red solid as a crude product. The crude product (100 mg) was purified by Prep-HPLC with the following conditions (Column: XBridge Prep OBD C18 Column, 30*150 mm, 5µm; Mobile Phase A: water (10 mmol/L NH₄HCO₃), Mobile Phase B: ACN; Flow rate: 60 mL/min; Gradient: 25% B to 50% B in 7 min, 50% B; Wavelength: 220 nm; RT1(min): 5.98; to afford Example 13. LCMS (ESI) m/z: 360.05 [M+H]⁺.

### Example 14: 3-(4-methoxypyridin-3-yl)-2-[4-(1,3-thiazol-5-yl)piperidin-1-yl]benzonitrile

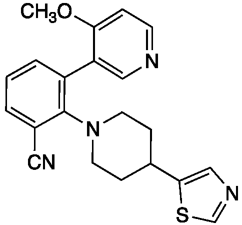

The title compound was prepared using the following procedure:

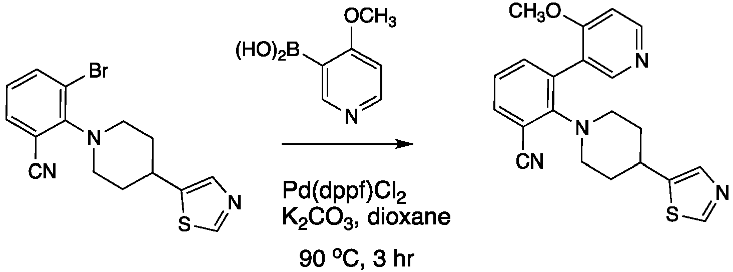

To a stirred solution of Intermediate 3 (133 mg, 0.39 mmol) and 4-methoxypyridin-3-ylboronic acid (63.9 mg, 0.39 mmol) in dioxane (6 mL)/ H₂O (1 mL) were added Pd(dppf)Cl₂ (28.0 mg, 0.04 mmol) and K₂CO₃ (157.8 mg, 1.15 mmol) in portions at room temperature under nitrogen atmosphere. The resulting mixture was stirred for 3 h at 90 ° C. The resulting mixture was extracted with EtOAc (3 x 20 mL). The combined organic layers were washed with brine (2 x 20 mL), dried over anhydrous Na₂SO₄. After filtration, the filtrate was concentrated under reduced pressure. The crude product (80 mg) was purified by Prep-HPLC with the following conditions (Column: XBridge Prep OBD C18 Column, 30*150 mm, 5 µm; Mobile Phase A: water (10 mmol/L NH₄HCO₃), Mobile Phase B: ACN; Flow rate: 60 mL/min; Gradient: 30% B to 55% B in 8 min, 55% B; Wavelength: 220 nm; RT1(min): 8.10; to afford Example 14. LCMS (ESI) m/z: 377.05 [M+H]⁺.

### Example 15: 3-(1,3-benzoxazol-5-yl)-2-[4-(4-methyl-4H-1,2,4-triazol-3-yl)piperidin-1-yl]benzonitrile

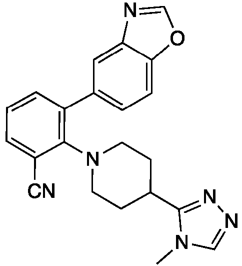

The title compound was prepared using the following procedure:

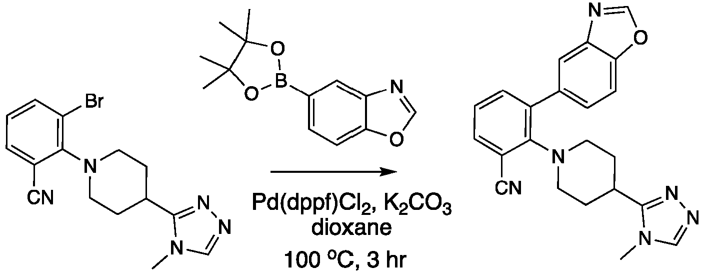

A mixture of Intermediate 1 (60.0 mg, 0.17 mmol), K₂CO₃ (71.8 mg, 0.5 mmol), Pd(dppf)Cl₂ (14.1 mg, 0.02 mmol), and 5-(4,4,5,5-tetramethyl-1,3,2-dioxaborolan-2-yl)-1,3-benzoxazole (46.7 mg, 0.2 mmol) in 1,4-dioxane (3.0 mL) /H₂O (0.5 mL) was stirred for 3 h at 100 °C under nitrogen atmosphere. The reaction was quenched with water (25 mL) at room temperature. The resulting mixture was extracted with EtOAc (3 x 20 mL). The combined organic layers were washed with brine (2 x 20 mL), dried over anhydrous Na₂SO₄. After filtration, the filtrate was concentrated under reduced pressure. The crude product (45 mg) was purified by Prep-HPLC with the following conditions (Column: XBridge Shield RP18 OBD Column, 19*150 mm, 5µm; Mobile Phase A: water (10 mmol/L NH₄HCO₃), Mobile Phase B: ACN; Flow rate: 25 mL/min; Gradient: 27% B to 53% B in 7 min, 53% B; Wavelength: 220 nm; RT1(min): 5.84; to afford Example 15. LCMS (ESI) m/z: 385.05 [M+H]⁺.

### Example 16: 3-{2-methyl-2H-pyrazolo[3,4-c]pyridin-4-yl}-2-[4-(4-methyl-4H-1,2,4-triazol-3-yl)piperidin-1-yl]benzonitrile

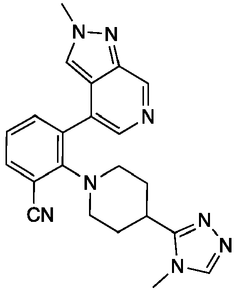

The title compound was prepared using the following procedure:

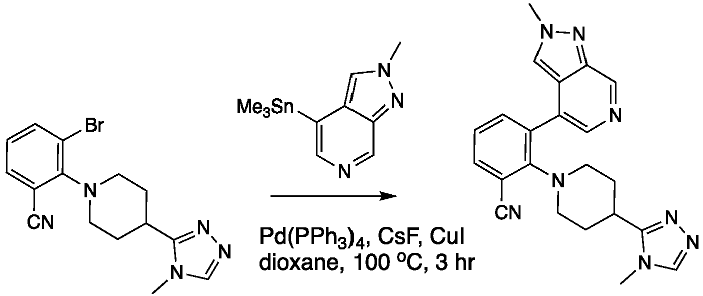

To a stirred mixture of 2-methyl-4-(trimethylstannyl)pyrazolo[3,4-c]pyridine (50 mg, 0.17 mmol) and Intermediate 1 (58.5 mg, 0.17 mmol) in dioxane (3 mL) were added Pd(PPh₃)₄ (19.6 mg, 0.017 mmol), CsF (25.7 mg, 0.17 mmol) and CuI (32.2 mg, 0.17 mmol) in portions at room temperature under nitrogen atmosphere. The resulting mixture was stirred for 3 h at 100 °C under nitrogen atmosphere. The reaction was quenched with water (30 mL) at room temperature. The resulting mixture was extracted with EtOAc (3 x 20 mL). The combined organic layers were washed with brine (2 x 20 mL), dried over anhydrous Na₂SO₄. After filtration, the filtrate was concentrated under reduced pressure. The crude product (12 mg) was purified by Prep-HPLC with the following conditions (Column: YMC-Actus Triart C18 ExRS, 30*150 mm, 5µm; Mobile Phase A: water (10 mmol/L NH₄HCO₃), Mobile Phase B: ACN; Flow rate: 60 mL/min; Gradient: 15% B to 40% B in 7 min, 40% B; Wavelength: 254/220 nm; RT1(min): 6.08; to afford Example 16. LCMS (ESI) m/z: 399.05 [M+H]⁺.

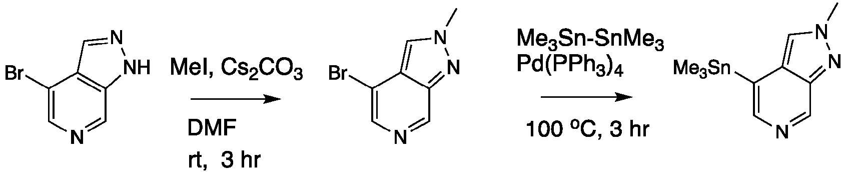

To a stirred mixture of 4-bromo-1H-pyrazolo[3,4-c]pyridine (500 mg, 2.5 mmol) and Cs₂CO₃ (2.1 g, 6.3 mmol) in DMF (20 mL) was added CH₃I (394.2 mg, 2.8 mmol) in portions at room temperature. The resulting mixture was stirred for 3 h at room temperature. The reaction was quenched with water (80 mL) at room temperature. The resulting mixture was extracted with EtOAc (3 x 50 mL). The combined organic layers were washed with brine (2 x 50 mL), dried over anhydrous Na₂SO₄. After filtration, the filtrate was concentrated under reduced pressure. The residue was purified by silica gel column chromatography, eluted with EtOAc/PE (60%-90%) to afford 4-bromo-2-methylpyrazolo[3,4-c]pyridine (130 mg, 24%) as a light yellow solid.

To a stirred mixture of 4-bromo-2-methyl-1H,7aH-pyrazolo[3,4-c]pyridine (80 mg, 0.4 mmol) and hexamethyldistannane (148.3 mg, 0.45 mmol) in dioxane (4 mL) was added Pd(PPh₃)₄ (43.6 mg, 0.04 mmol) in portions at room temperature under nitrogen atmosphere. The resulting mixture was stirred for 3 h at 100 °C under nitrogen atmosphere. The reaction was quenched with water (40 mL) at room temperature. The resulting mixture was extracted with EtOAc (3 x 30 mL). The combined organic layers were washed with brine (2 x 30 mL), dried over anhydrous Na₂SO₄. After filtration, the filtrate was concentrated under reduced pressure. The residue was purified by silica gel column chromatography, eluted with MeOH / CH₂Cl₂ (2%-10%) to afford 2-methyl-4-(trimethylstannyl)pyrazolo[3,4-c]pyridine (50 mg, 45%) as a yellow solid.

### Example 17: 2-[4-(2-amino-1,3-thiazol-5-yl)piperidin-1-yl]-3-(4-methoxypyridin-3-yl)benzonitrile

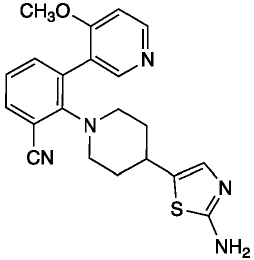

The title compound was prepared using the following procedure:

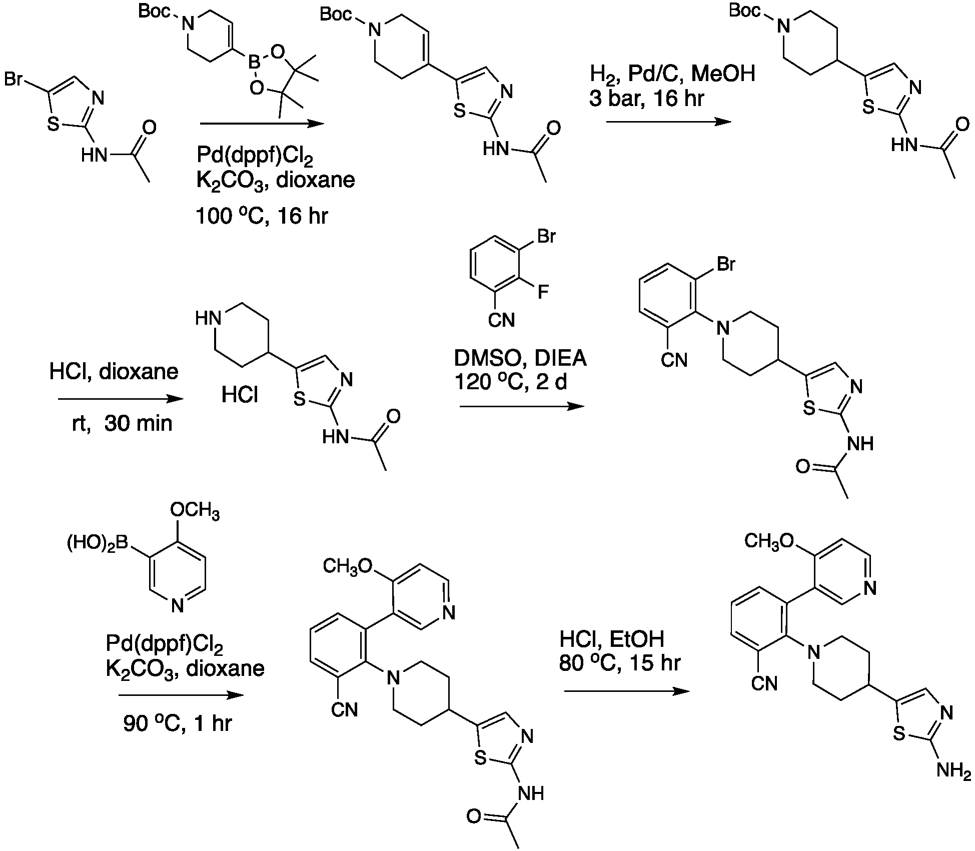

A solution of N-(5-bromo-1,3-thiazol-2-yl)acetamide (2 g, 9.05 mmol), Pd(dppf)Cl₂ (0.66 g, 0.91 mmol) , K₂CO₃ (3.75 g, 27.14 mmol) and tert-butyl 4-(4,4,5,5-tetramethyl -1,3,2-dioxaborolan-2-yl)-3,6-dihydro-2H-pyridine-1-carboxylate (3.36 g, 10.86 mmol) in dioxane (10 mL) and water (1 mL) was stirred for overnight at 100 °C under nitrogen atmosphere. The resulting mixture was extracted with EtOAc (2 x 100 mL). The combined organic layers were washed with water (20 mL), dried over anhydrous Na₂SO₄. After filtration, the filtrate was concentrated under reduced pressure. The residue was purified by silica gel column chromatography, eluted with PE / EA (1:1) to afford tert-butyl 4-(2-acetamido-1,3-thiazol-5-yl)- 3,6-dihydro-2H-pyridine-1-carboxylate (1 g, 34%) as a black solid. LCMS (ESI) m/z: 324 [M+H]⁺.

A solution of tert-butyl 4-(2-acetamido-1,3-thiazol-5-yl)-3,6-dihydro-2H-pyridine-1-carboxylate (560 mg, 1.73 mmol) and Pd/C (200 mg) in MeOH (50 mL) was stirred for overnight at room temperature under hydrogen. The resulting mixture was filtered, the filter cake was washed with MeOH (2 x 20 mL). The filtrate was concentrated under reduced pressure. The residue was purified by silica gel column chromatography, eluted with PE / EA (1:1) to afford tert-butyl 4-(2-acetamido-1,3-thiazol-5-yl) piperidine-1-carboxylate (440 mg, 78%) as a light yellow solid. LCMS (ESI) m/z: 326 [M+H]⁺.

A solution of tert-butyl 4-(2-acetamido-1,3-thiazol-5-yl) piperidine-1-carboxylate (440 mg, 1.35 mmol) in HCl/1,4-dioxane (10 mL) was stirred for 30 min at room temperature. The resulting mixture was concentrated under reduced pressure. The crude product was used in the next step directly without further purification.

A solution of N-[5-(piperidin-4-yl)-1,3-thiazol-2-yl] acetamide hydrochloride (300 mg, 1.33 mmol), DIEA (860.5 mg, 6.66 mmol) and 3-bromo-2-fluorobenzonitrile (319.6 mg, 1.60 mmol) in DMSO (10 mL) was stirred for 2 days at 120 °C. The resulting mixture was diluted with water (30 mL). The resulting mixture was extracted with EtOAc (2 x 50 mL). The combined organic layers were washed with water (2x10 mL), dried over anhydrous Na₂SO₄. After filtration, the filtrate was concentrated under reduced pressure. The residue was purified by silica gel column chromatography, eluted with CH₂Cl₂ / MeOH (10:1) to afford N-{5-[1-(2-bromo-6-cyanophenyl) piperidin-4-yl]-1,3-thiazol-2-yl } acetamide (150 mg, 28%) as a black solid. LCMS (ESI) m/z: 405, 407 [M+H]⁺.

A solution of N-{5-[1-(2-bromo-6-cyanophenyl) piperidin-4-yl]-1,3-thiazol-2-yl} acetamide (20 mg, 0.05 mmol), Pd(dppf)Cl₂ (3.61 mg, 0.005 mmol), K₂CO₃ (20.5 mg, 0.15 mmol) and 4-methoxypyridin-3-ylboronic acid (9.1 mg, 0.06 mmol) in dioxane (5 mL) and water (0.5 mL) was stirred for 1 h at 90 °C under nitrogen atmosphere. The resulting mixture was extracted with EtOAc (2 x 10 mL). The combined organic layers were washed with water (2 x 10 mL), dried over anhydrous Na₂SO₄. After filtration, the filtrate was concentrated under reduced pressure. The crude product (10 mg) was purified by Prep-HPLC with the following conditions (Column: Sunfire prep C18 column, 30*150 mm, 5µm; Mobile Phase A: water (0.1%FA), Mobile Phase B: ACN; Flow rate: 60 mL/min; Gradient: 11% B to 19% B in 7 min, 19% B; Wavelength: 254/220 nm; RT1(min): 4.82) to afford N-(5-{1-[2-cyano- 6-(4-methoxypyridin-3-yl)phenyl]piperidin-4-yl}-1,3-thiazol-2-yl)acetamide (1.9 mg, 8.9%) as a light yellow solid. LCMS (ESI) m/z: 434 [M+H]⁺.

A solution of N-(5-{1-[2-cyano-6-(4-methoxypyridin-3-yl)phenyl]piperidin-4-yl}-1,3-thiazol-2-yl)acetamide (20 mg, 0.046 mmol), conc HCl (3 mL) and EtOH (5 mL) was stirred overnight at 80 °C. The mixture was basified to pH 8 with saturated Na₂CO₃ (aq.). The resulting mixture was extracted with EtOAc (2 x 10 mL). The combined organic layers were washed with water (9 mL), dried over anhydrous Na₂SO₄. After filtration, the filtrate was concentrated under reduced pressure. The crude product (20 mg) was purified by Prep-HPLC with the following conditions (Column: XBridge Shield RP18 OBD Column, 30*150 mm, 5µm; Mobile Phase A: water (10 mmol/L NH₄HCO₃), Mobile Phase B: ACN; Flow rate: 60 mL/min; Gradient: 30% B to 45% B in 7 min, 45% B; Wavelength: 254/220 nm; RT1(min): 6.43) to afford Example 17. LCMS (ESI) m/z: 392.05 [M+H]⁺.

### Example 18: 3-(4-methoxypyridin-3-yl)-2-[4-(3-methyl-4H-1,2,4-triazol-4-yl)piperidin-1-yl]benzonitrile

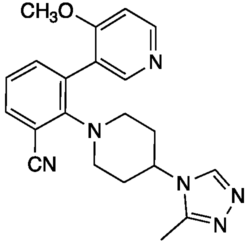

The title compound was prepared using the following procedure:

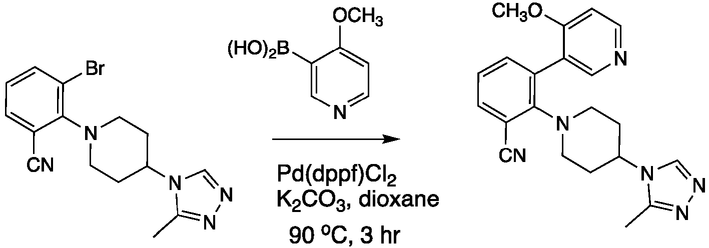

To a stirred mixture of Intermediate 5 (200 mg, 0.58 mmol) and 4-methoxypyridin-3-ylboronic acid (132.5 mg, 0.87 mmol) in 1,4-dioxane (10 mL) and H₂O (1 mL) were added K₂CO₃ (239.5 mg, 1.73 mmol) and Pd(dppf)Cl₂ (42.3 mg, 0.058 mmol) in portions at room temperature under nitrogen atmosphere. The resulting mixture was stirred for 3 h at 90° C under nitrogen atmosphere. The reaction was quenched by the addition of water (30 mL) at room temperature. The resulting mixture was extracted with EtOAc (2 x 50 mL). The combined organic layers were washed with brine (20 mL), dried over anhydrous Na₂SO₄ and evaporated to dryness. The crude product (50 mg) was purified by Prep-HPLC with the following conditions (Column: YMC-Actus Triart C18, 30*150 mm, 5 µm; Mobile Phase A: water (0.1% FA), Mobile Phase B: ACN; Flow rate: 60 mL/min; Gradient: 12% B to 40% B in 9 min, 40% B; Wavelength: 220/254 nm; RT1(min): 6.10; to afford Example 18. LCMS (ESI) m/z: 375.05 [M+H]⁺.

### Example 19: 3-(2,1,3-benzoxadiazol-5-yl)-2-[4-(4-methyl-4H-1,2,4-triazol-3-yl)piperidin-1-yl]benzonitrile

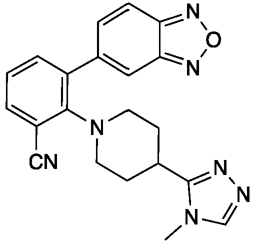

The title compound was prepared using the following procedure:

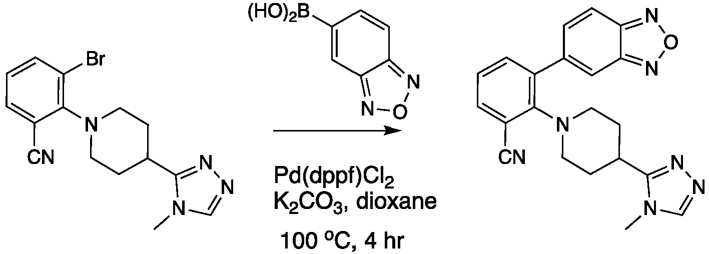

To a stirred mixture of Intermediate 1 (60 mg, 0.17 mmol) and 2,1,3-benzoxadiazol-5-ylboronic acid (42.6 mg, 0.26 mmol) in 1,4-dioxane (10 mL) and H₂O (1 mL) were added K₂CO₃ (71.8 mg, 0.52 mmol) and Pd(dppf)Cl₂ (12.7 mg, 0.017 mmol) in portions at room temperature under nitrogen atmosphere. The resulting mixture was stirred for 4 h at 100° C under nitrogen atmosphere. The resulting mixture was extracted with EtOAc (2 x 60 mL). The combined organic layers were washed with brine (1 x 20 mL), dried over anhydrous Na₂SO₄. The crude product (50 mg) was purified by Prep-HPLC with the following conditions (Column: Sunfire prep C18 column, 30*150 mm, 5 µm; Mobile Phase A: water (0.1% FA), Mobile Phase B: ACN; Flow rate: 60 mL/min; Gradient: 28% B to 50% B in 8 min, 50% B; Wavelength: 254/220 nm; RT1(min): 6.95; to afford Example 19. LCMS (ESI) m/z: 386.05 [M+H]⁺.

### Example 20: 2-[4-(4-methyl-4H-1,2,4-triazol-3-yl)piperidin-1-yl]-3-{2H,3H,4H-pyrido[4,3-b][1,4]oxazin-8-yl}benzonitrile

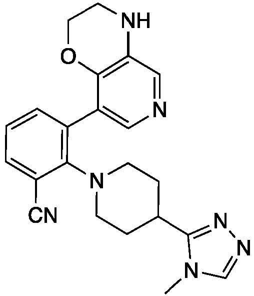

The title compound was prepared using the following procedure:

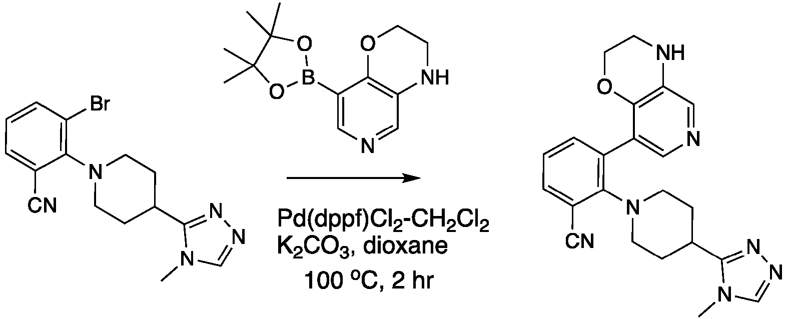

To a stirred solution of 8-(4,4,5,5-tetramethyl-1,3,2-dioxaborolan-2-yl)-2H,3H,4H-pyrido[4,3-b][1,4]oxazine (40 mg, 0.15 mmol) and K₂CO₃ (38 mg, 0.29 mmol) in 1,4-dioxane/H₂O (10:1) were added Pd(dppf)Cl₂CH₂Cl₂ (11 mg, 0.1 mmol) and Intermediate 1 (48 mg, 0.14 mmol) at room temperature under nitrogen atmosphere. The resulting mixture was stirred for 2 h at 100 °C. The reaction was quenched by the addition of water (5 mL) at room temperature. The crude product was purified by reverse phase flash with the following conditions (Column: X select CSH F-Phenyl OBD column, 19*250 mm, 5µm; Mobile Phase A: water (0.1% FA), Mobile Phase B: ACN; Flow rate: 25 mL/min; Gradient: 2% B to 22% B in 9 min, 22% B; Wavelength: 220 nm; RT1(min): 7.20; to afford Example 20. LCMS (ESI) m/z: 402.05 [M+H]⁺.

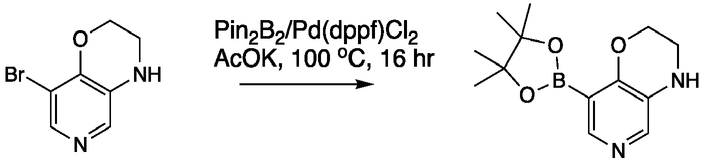

To a stirred solution of 8-bromo-2H,3H,4H-pyrido[4,3-b] [1,4] oxazine (900 mg, 4.18 mmol) in 1,4-dioxane (50 mL) was added Pd(dppf)Cl₂ (342 mg, 0.42 mmol), AcOK (1232 mg, 12.6 mmol) and bis(pinacolato)diboron (218 mg, 0.84 mmol) at room temperature under nitrogen atmosphere. The resulting mixture was stirred for 16 h at 100 °C under nitrogen atmosphere. The reaction was quenched by the addition of water (50 mL) at room temperature. The resulting mixture was extracted with EtOAc (3 x 100 mL). The combined organic layers were washed with brine (3 x 100 mL), dried over anhydrous Na₂SO₄. The residue was purified by reversed-phase flash chromatography with the following conditions: column, C18 silica gel; mobile phase, MeCN in water (0.1% FA), 10% to 50% gradient in 10 min; detector, UV 254 nm to afford desired compound (600 mg) as a brown solid. LCMS (ESI) m/z: 263 [M+H]⁺.

### Example 21: 2-[4-(5-amino-1,3,4-thiadiazol-2-yl)piperidin-1-yl]-3-(4-methoxypyridin-3-yl)benzonitrile

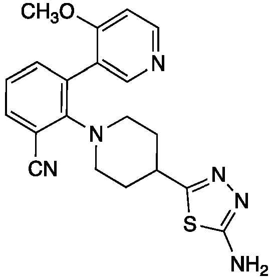

The title compound was prepared using the following procedure:

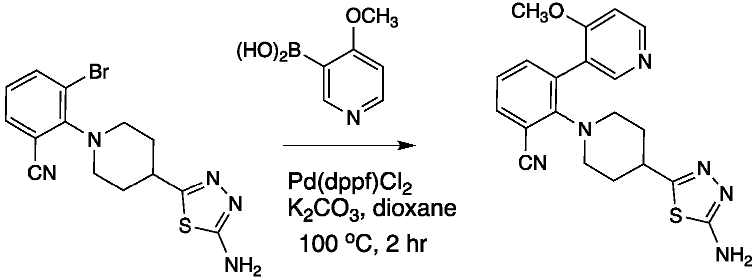

To a stirred solution of Intermediate 4 (200 mg, 0.55 mmol) in dioxane (15 mL) and H₂O (2 mL) were added K₂CO₃ (151.7 mg, 1.1 mmol), 4-methoxypyridin-3-ylboronic acid (167.9 mg, 1.01 mmol) and Pd(dppf)Cl₂ (40.2 mg, 0.06 mmol) in portions at room temperature under nitrogen atmosphere. The resulting mixture was stirred for 2 h at 100 °C. The resulting mixture was concentrated under reduced pressure. The residue was purified by silica gel column chromatography, eluted with CH₂Cl₂/MeOH (9:1) to afford a dark red solid as a crude product. The crude product (100 mg) was purified by Prep-HPLC with the following conditions (Column: XBridge Shield RP18 OBD Column, 19*150 mm, 5µm; Mobile Phase A: water (10 mmol/L NH₄HCO₃), Mobile Phase B: ACN; Flow rate: 25 mL/min; Gradient: 24% B to 48% B in 7 min, 48% B; Wavelength: 220 nm; RT1(min): 6.27; to afford Example 21. LCMS (ESI) m/z: 393.00 [M+H]⁺.

### Example 22: 3-{4-methyl-2H,3H,4H-pyrido[4,3-b][1,4]oxazin-8-yl}-2-[4-(4-methyl-4H-1,2,4-triazol-3-yl)piperidin-1-yl]benzonitrile

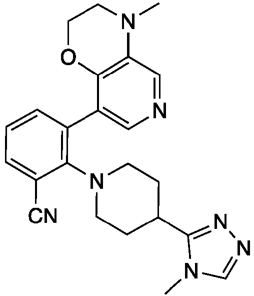

The title compound was prepared using the following procedure:

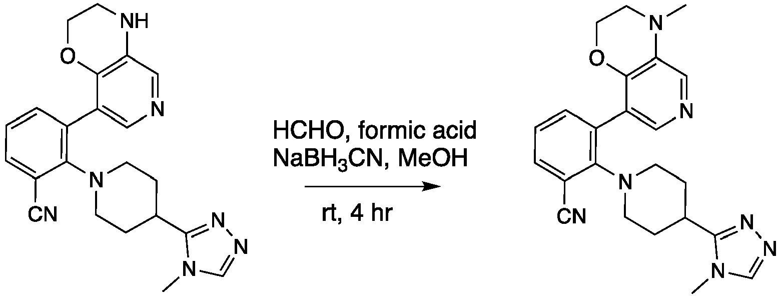

To a stirred solution of 2-[4-(4-methyl-4H-1,2,4-triazol-3-yl)piperidin-1-yl]-3-{2H,3H,4H-pyrido[4,3-b][1,4]oxazin-8-yl}benzonitrile (25 mg, 0.06 mmol) in anhydrous MeOH (5 mL) was added HCHO (4 mg, 0.13 mmol) and NaBH₃CN (12 mg, 0.19 mmol) followed by catalytic amount of HCOOH (3 mg, 0.07 mmol) at room temperature. The reaction mixture was stirred at room temperature for a period of 4 h. After completion of reaction, the reaction mixture was quenched by addition of water (5 mL) and extracted with EtOAc. The extract was washed with brine, dried over Na₂SO₄, and evaporated to dryness. The crude product was purified by reverse phase flash with the following conditions (Column: X select CSH C18 OBD Column 30*150mm 5µm, n; Mobile Phase A: water (0.1% FA), Mobile Phase B: ACN; Flow rate: 60 mL/min; Gradient: 10% B to 40% B in 7 min, 40% B; Wavelength: 220 nm; RT1(min): 5.62; to afford Example 22. LCMS (ESI) m/z: 416.10 [M+H]⁺.

### Example 23: 3-[4-(2-hydroxyethyl)-2H,3H,4H-pyrido[4,3-b][1,4]oxazin-8-yl]-2-[4-(4-methyl-4H-1,2,4-triazol-3-yl)piperidin-1-yl]benzonitrile

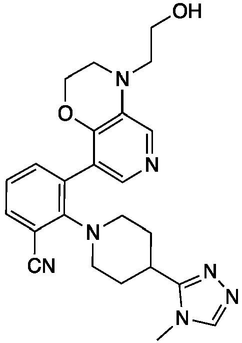

The title compound was prepared using the following procedure:

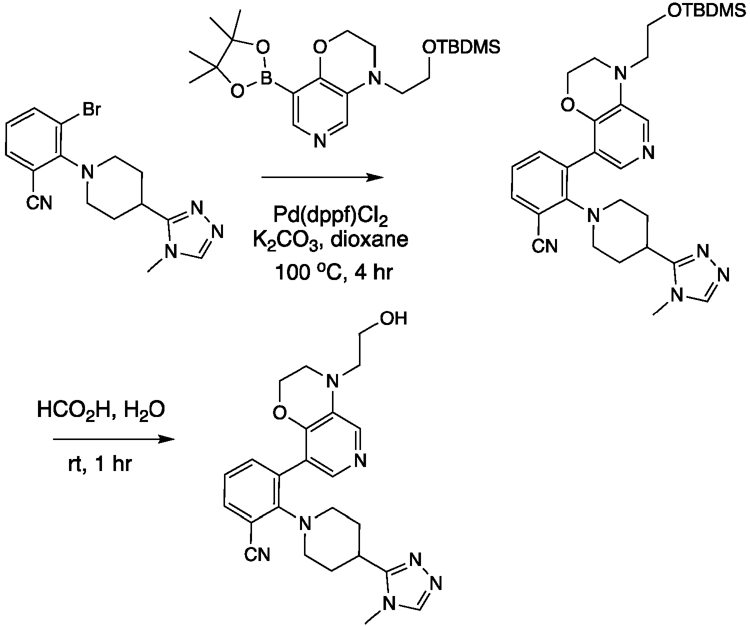

To a stirred solution of Intermediate 1 (20 mg, 0.058 mmol) in anhydrous 1,4-dioxane (5 mL) and H₂O (0.5 mL) was added K₂CO₃ (15.9 mg, 0.12 mmol) and 4-{2-[(tertbutyldimethylsilyl) oxy] ethyl}-2H,3H-pyrido[4,3-b] [1,4] oxazin-8-ylboronic acid (21.5 mg, 0.064 mmol) followed by catalytic amount of Pd(dppf)Cl₂ (4.71 mg, 0.006 mmol) at 0 °C. The reaction mixture was stirred at 100 °C for a period of 4 h. After completion of reaction, the reaction mixture was quenched by addition of water (5 mL). The aqueous layer was extracted with ethyl acetate (100 mL), dried over anhydrous sodium sulfate and concentrated under reduced pressure to give crude product which was further purified by column chromatography using 5% to 20% MeOH in DCM gradient to afford desired compound 3-(4-{2-[(tert-butyldimethylsilyl) oxy] ethyl}-2H,3H-pyrido[4,3-b][1,4] oxazin-8-yl)-2-[4-(4-methyl-1,2,4-triazol-3-yl) piperidin-1-yl] benzonitrile (10 mg).

To a stirred solution of 3-(4-{2-[(tert-butyldimethylsilyl) oxy] ethyl}-2H,3H-pyrido[4,3-b] [1,4] oxazin-8-yl)-2-[4-(4-methyl-1,2,4-triazol-3-yl) piperidin-1-yl] benzonitrile (10 mg, 0.018 mmol) added HCOOH (1.6 mg, 0.036 mmol) and H₂O (5 mL) at room temperature under nitrogen atmosphere. The final reaction mixture was stirred at room temperature for 1 h. The crude product was purified by reverse phase flash with the following conditions (Column: X select CSH F-Phenyl OBD column, 19*250 mm, 5µm;Mobile Phase A: water (0.1% FA), Mobile Phase B: ACN; Flow rate: 25 mL/min; Gradient: 2% B to 22% B in 9 min, 22% B; Wavelength: 220 nm; RT1(min): 7.20; to afford Example 23. LCMS (ESI) m/z: 446.05 [M+H]⁺.

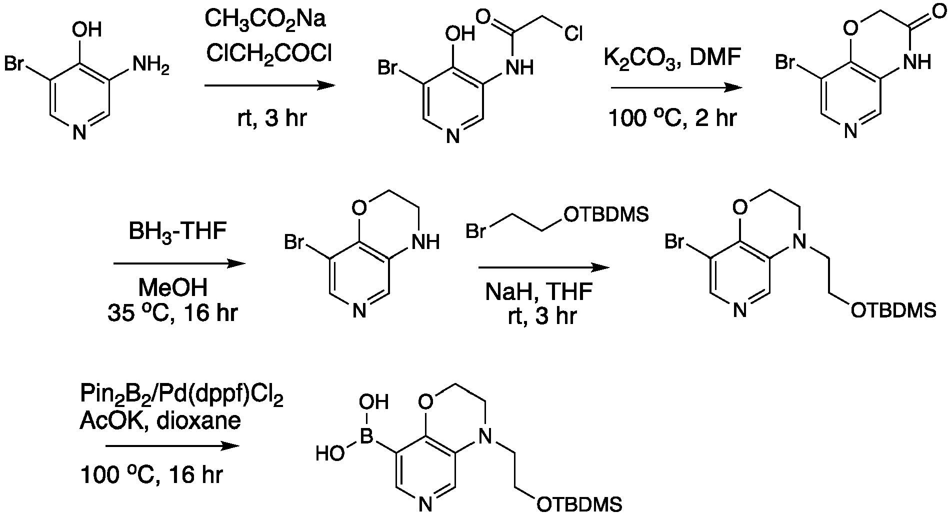

A solution of 3-amino-5-bromopyridin-4-ol (6 g, 31.7 mmol) in acetone (100 mL) was treated with NaOAc (5.2 g, 63.4 mmol) for 5 min at 0 °C under nitrogen atmosphere followed by the addition of chloroacetyl chloride (4.3 g, 38 mmol) dropwise at 0 °C. The resulting mixture was stirred for 3 h at room temperature under nitrogen atmosphere. The reaction was quenched with water (50 mL) at room temperature. The resulting mixture was extracted with EtOAc (3 x 200 mL). The combined organic layers were washed with brine (3 x 200 mL), dried over anhydrous Na₂SO₄. After filtration, the filtrate was concentrated under reduced pressure. This resulted in N-(5-bromo-4-hydroxypyridin-3-yl)-2-chloroacetamide (4 g) as a brown solid. LCMS (ESI) m/z: 265 [M+H]⁺.

A solution of N-(5-bromo-4-hydroxypyridin-3-yl)-2-chloroacetamide (4 g, 15.1 mmol) in DMF (10 mL) was treated with K₂CO₃ (4.2 g, 30.1 mmol) for 5 min at 0 °C. The resulting mixture was stirred for 2 h at 100 °C under nitrogen atmosphere. The reaction was quenched with water (20 mL) at room temperature. The resulting mixture was extracted with EtOAc (3 x 100 mL). The combined organic layers were washed with brine (3 x 100 mL), dried over anhydrous Na₂SO₄. After filtration, the filtrate was concentrated under reduced pressure. This resulted in 8-bromo-2H,4H,4aH,8aH-pyrido[4,3-b] [1,4] oxazin-3-one (3 g) as a brown solid. LCMS (ESI) m/z: 229, 231 [M+H]⁺.

A solution of 8-bromo-2H,4H,4aH,8aH-pyrido[4,3-b] [1,4] oxazin-3-one (4 g, 17.3 mmol) in borane-THF (70 mL) was stirred for 5 min at 0 °C under nitrogen atmosphere. The resulting mixture was stirred for additional 16 h at 35 °C. The reaction was quenched with MeOH (7 mL) for 30 min at 60 °C. The reaction was quenched with water (30 mL) at room temperature. The resulting mixture was extracted with EtOAc (3 x 100 mL). The combined organic layers were washed with brine (3 x 100 mL), dried over anhydrous Na₂SO₄. After filtration, the filtrate was concentrated under reduced pressure to obtain 8-bromo-2H,3H,4H-pyrido[4,3-b] [1,4]oxazine (2.5 g) as a brown solid. LCMS (ESI) m/z: 215, 217 [M+H]⁺.

To a stirred solution of 8-bromo-2H,3H,4H-pyrido[4,3-b] [1,4] oxazine (30 mg, 0.14 mmol) in anhydrous THF (5 mL) was added NaH (6.7 mg, 0.28 mmol) at 0 °C for a period of 30 min. A mixture of (2-bromoethoxy)(tert-butyl)dimethylsilane (50.1 mg, 0.21 mmol) was stirred for 3 h at room temperature under nitrogen atmosphere. After completion of reaction, the reaction mixture was quenched by addition of water (5 mL), dried over anhydrous sodium sulfate and concentrated under reduced pressure to give crude product which was further purified by column chromatography using 5% to 40% EtOAc in PE gradient to afford desired compound 8-bromo-4-{2-[(tert-butyldimethylsilyl) oxy] ethyl}-2H,3H-pyrido[4,3-b] [1,4] oxazine (40 mg). LCMS (ESI) m/z: 373, 375 [M+H]⁺.

To a stirred solution of 8-bromo-4-{2-[(tert-butyldimethylsilyl) oxy] ethyl}-2H,3H-pyrido[4,3-b] [1,4] oxazine (40 mg, 0.11 mmol) in anhydrous 1,4-dioxane (5 mL) was added AcOK (19.3 mg, 0.32 mmol) and bis(pinacolato)diboron (54.4 mg, 0.21 mmol) followed by catalytic amount of Pd(dppf)Cl₂ (8.7 mg, 0.01 mmol) at 0 °C. The reaction mixture was stirred at 100 °C for a period of 16 h. After completion of reaction, the reaction mixture was quenched by addition of water (5 mL). The aqueous layer was extracted with ethyl acetate (100 mL), dried over anhydrous sodium sulfate and concentrated under reduced pressure to give crude product which was further purified by column chromatography using 1% to 10% MeOH in DCM gradient to afford desired compound 4-{2-[(tertbutyldimethylsilyl)oxy]ethyl}-2H,3H-pyrido[4,3-b][1,4]oxazin-8-ylboronic acid (20 mg). LCMS (ESI) m/z: 339 [M+H]⁺.

### Example 24: 3-(6-fluoropyridin-3-yl)-6-methoxy-2-[4-(4-methyl-4H-1,2,4-triazol-3-yl)piperidin-1-yl]benzonitrile

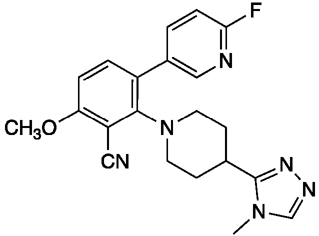

The title compound was prepared using the following procedure:

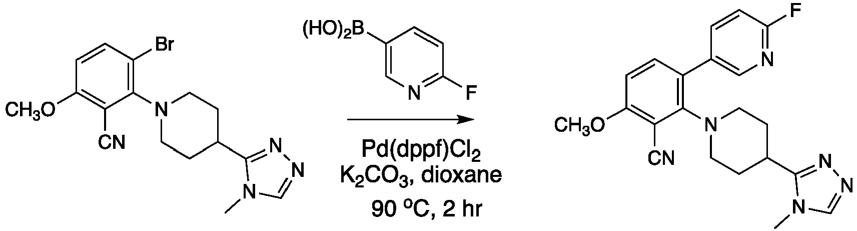

To a stirred solution of Intermediate 7 (100 mg, 0.27 mmol) in dioxane (8 mL) and H₂O (1 mL) were added K₂CO₃ (73.5 mg, 0.53 mmol), 6-fluoropyridin-3-ylboronic acid (74.9 mg, 0.53 mmol) and Pd(dppf)Cl₂ (19.4 mg, 0.03 mmol) in portions at room temperature under nitrogen atmosphere. The resulting mixture was stirred for 2 h at 90 °C under nitrogen atmosphere. The resulting mixture was concentrated under reduced pressure. The residue was purified by silica gel column chromatography, eluted with CH₂Cl₂ / MeOH (7: 1) to afford a brown solid as a crude product. The crude product (80 mg) was purified by Prep-HPLC with the following conditions (Column: YMC-Actus Triart C18 ExRS, 30*150 mm, 5µm;Mobile Phase A: water (10 mmol/L NH₄HCO₃), Mobile Phase B: ACN; Flow rate: 60 mL/min; Gradient: 26% B to 43% B in 7 min, 43% B; Wavelength: 254/220 nm; RT1(min): 6.38; to afford Example 24. LCMS (ESI) m/z: 393.05 [M+H]⁺.

### Example 25: 6-methoxy-3-(4-methoxypyridin-3-yl)-2-[4-(4-methyl-4H-1,2,4-triazol-3-yl)piperidin-1-yl]benzonitrile

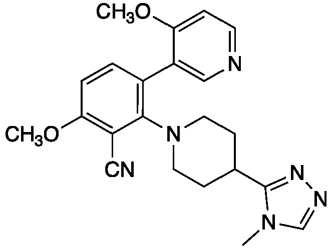

The title compound was prepared using the following procedure:

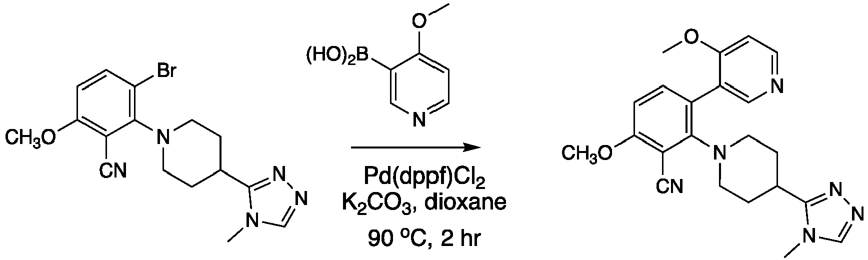

To a stirred solution of Intermediate 7 (100 mg, 0.27 mmol) in dioxane (7 mL) and H₂O (1 mL) were added K₂CO₃ (73.5 mg, 0.53 mmol) 4-methoxypyridin-3-ylboronic acid (81.3 mg, 0.53 mmol) and Pd(dppf)Cl₂ (19.4 mg, 0.027 mmol) in portions at room temperature under nitrogen atmosphere. The resulting mixture was stirred for 2 h at 90 °C. The resulting mixture was concentrated under reduced pressure. The residue was purified by silica gel column chromatography, eluted with CH₂Cl₂/MeOH (6: 1) to afford a tan solid as a crude product. The crude product (50 mg) was purified by Prep-HPLC with the following conditions (Column: Sunfire prep C18 column, 30*150 mm, 5µm;Mobile Phase A: water (0.1% FA), Mobile Phase B: ACN; Flow rate: 60 mL/min; Gradient: 9% B to 18% B in 8 min, 18% B; Wavelength: 254/220 nm; RT1(min): 5.73; to afford Example 25. LCMS (ESI) m/z: 405.10 [M+H]⁺.

### Example 26: 6-methoxy-2-[4-(4-methyl-4H-1,2,4-triazol-3-yl)piperidin-1-yl]-3-(pyridazin-4-yl)benzonitrile

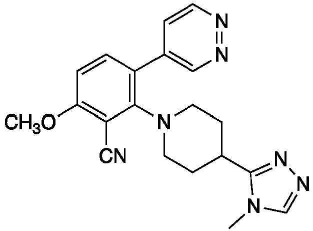

The title compound was prepared using the following procedure:

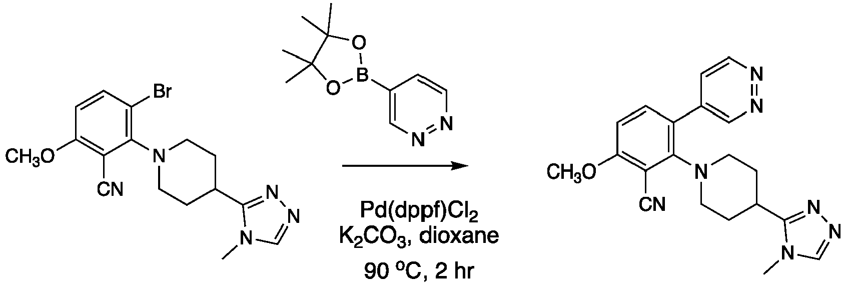

To a stirred solution of Intermediate 7 (50 mg, 0.133 mmol) in dioxane (2 mL) and H₂O (1 mL) were added K₂CO₃ (36.7 mg, 0.27 mmol) 4-(4,4,5,5-tetramethyl-1,3,2-dioxaborolan-2-yl)pyridazine (54.8 mg, 0.27 mmol) and Pd(dppf)Cl₂ (19.4 mg, 0.027 mmol) in portions at room temperature under nitrogen atmosphere. The resulting mixture was stirred for 2 h at 90 °C. The resulting mixture was concentrated under reduced pressure. The residue was purified by silica gel column chromatography, eluted with CH₂Cl₂ / MeOH (5:1) to afford a white solid as a crude product. The crude product (40 mg) was purified by Prep-HPLC with the following conditions (Column: XBridge Prep Phenyl OBD Column, 19*150 mm, 5µm;Mobile Phase A: water (10 mmol/L NH₄HCO₃), Mobile Phase B: MeOH-HPLC; Flow rate: 25 mL/min; Gradient: 39% B to 69% B in 7 min, 69% B; Wavelength: 254/220 nm; RT1(min): 7.13; to afford Example 26. LCMS (ESI) m/z: 376.05 [M+H]⁺.

### Example 27: 6-chloro-3-(4-methoxypyridin-3-yl)-2-[4-(4-methyl-4H-1,2,4-triazol-3-yl)piperidin-1-yl]benzonitrile

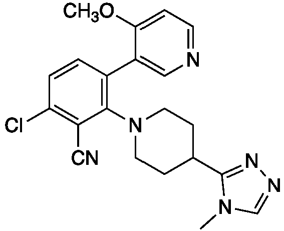

The title compound was prepared using the following procedure:

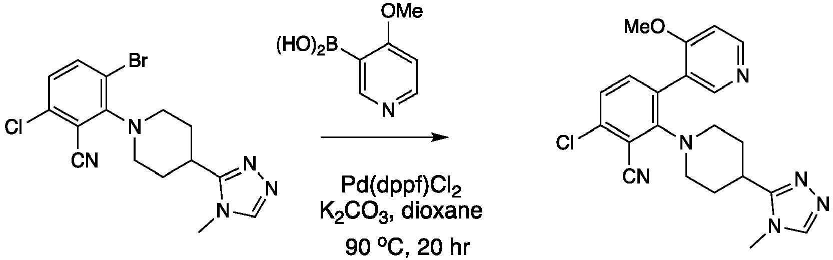

To a stirred mixture of Intermediate 6 (0.5 g, 1.31 mmol), K₂CO₃ (0.36 g, 2.63 mmol) in 1,4-dioxane (5 mL) and H₂O (0.5 mL) were added (4-methoxypyridin-3-yl)boronic acid (0.16 g, 1.05 mmol) and Pd(dppf)Cl₂ (0.10 g, 0.13 mmol) at room temperature under N₂ atmosphere. The mixture was stirred for 20 h at 90 °C under nitrogen atmosphere. The crude product (10 mg) was purified by Prep-HPLC with the following conditions (Column: Sunfire prep C18 column, 30*150 mm, 5µm; Mobile Phase A: water (0.1% FA), Mobile Phase B: ACN; Flow rate: 60 mL/min; Gradient: 5% B to 28% B in 8 min, 28% B; Wavelength: 254/220 nm; RT1(min): 7.00; to afford Example 27. LCMS (ESI) m/z: 409.00 [M+H] ⁺.

### Example 28: 4-(6-fluoropyridin-3-yl)-3-[4-(4-methyl-4H-1,2,4-triazol-3-yl)piperidin-1-yl]-[1,1'-biphenyl]-2-carbonitrile

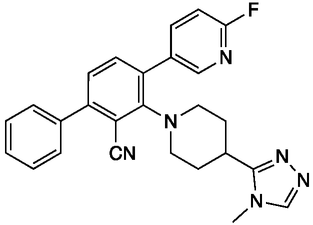

The title compound was prepared using the following procedure:

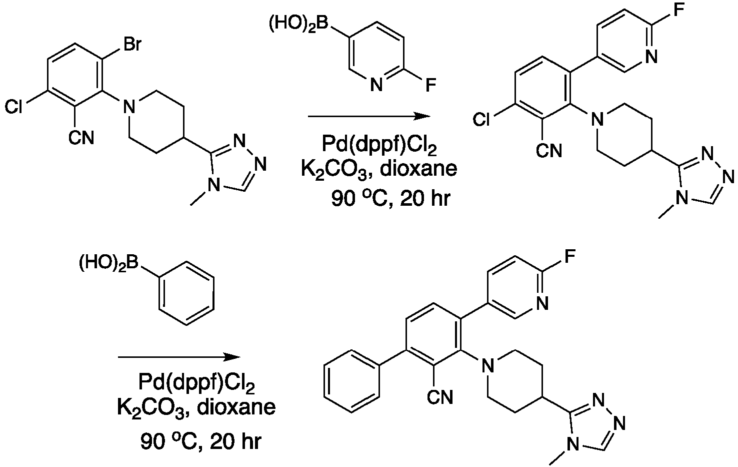

To a stirred solution of Intermediate 6 (200 mg, 0.52 mmol), K₂CO₃ (145 mg, 1.05 mmol) in dioxane (5 mL) and H₂O (0.5 mL) were added (6-fluoropyridin-3-yl) boronic acid (74 mg, 0.52 mmol) and Pd(dppf)Cl₂ (115.3 mg, 0.16 mmol) at room temperature under nitrogen atmosphere. The resulting mixture was stirred for 20 h at 90 °C under nitrogen atmosphere. The crude product (150 mg) was purified by Prep-HPLC with the following conditions (Column: XBridge Prep Phenyl OBD Column, 19*150 mm, 5µm; Mobile Phase A: water (10 mmol/L NH₄HCO₃), Mobile Phase B: MeOH-HPLC; Flow rate: 25 mL/min; Gradient: 48% B to 69% B in 10 min, 69% B; Wavelength: 220 nm; RT1(min): 8.50; to afford 6-chloro-3-(6-fluoropyridin-3-yl)-2-(4-(4-methyl-4H-1,2,4-triazol-3-yl)piperidin-1-yl)benzonitrile. LCMS (ESI) m/z: 409 [M+H] ⁺.

To a stirred solution of 6-chloro-3-(6-fluoropyridin-3-yl)-2-(4-(4-methyl-4H-1,2,4-triazol-3-yl)piperidin-1-yl)benzonitrile (100 mg, 0.25 mmol) and K₂CO₃ (69.6 mg, 0.5 mmol) in dioxane (5 mL) and H₂O (0.5 mL) were added phenylboronic acid (61.4 mg, 0.5 mmol) and Pd(dppf)Cl₂ (55.3 mg, 0.076 mmol) at room temperature under nitrogen atmosphere. The resulting mixture was stirred for 20 h at 90 °C. The resulting mixture was extracted with EtOAc (3 x 30mL). The combined organic layers were washed with brine (1x10 mL), dried over anhydrous Na₂SO₄. After filtration, the filtrate was concentrated under reduced pressure. The crude product (20 mg) was purified by Prep-HPLC with the following conditions (Column: XBridge Prep Phenyl OBD Column, 19*150 mm, 5µm; Mobile Phase A: water (10 mmol/L NH₄HCO₃), Mobile Phase B: ACN; Flow rate: 25 mL/min; Gradient: 25% B to 55% B in 7 min, 55% B; Wavelength: 254/220 nm; RT1(min): 6.52; to afford Example 28. LCMS (ESI) m/z: 439.05 [M+H] ⁺.

### Example 29: 2-[4-(4-methyl-4H-1,24-triazol-3-yl)piperidin-1-yl]-3-[6-(trifluoromethyl)pyridin-3-yl]benzonitrile

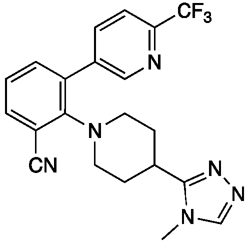

The title compound was prepared using the following procedure:

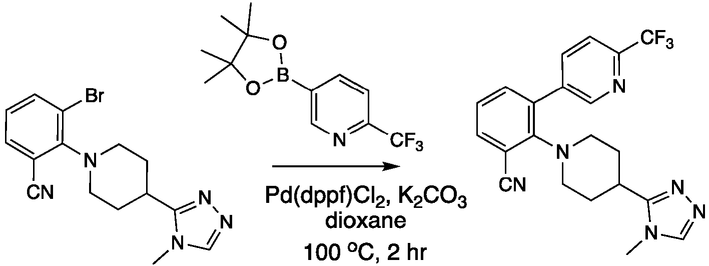

To a stirred solution of Intermediate 1 (100 mg, 0.29 mmol) in anhydrous 1,4-dioxane/H₂O (10:1) was added K₂CO₃ (80 mg, 0.58 mmol) and 5-(4,4,5,5-tetramethyl-1,3,2-dioxaborolan-2-yl)-2-(trifluoromethyl)pyridine (87 mg, 0.32 mmol) followed by Pd(dppf)Cl₂ (24 mg, 0.03 mmol) at room temperature. The reaction mixture was stirred at 100 °C for 2 h. After completion of reaction, the reaction mixture was quenched by addition of water (20 mL). The aqueous layer was extracted with ethyl acetate (100 mL). The combined organic phase was washed with brine (100 mL), The resulting mixture was concentrated under reduced pressure. The residue product was purified by reverse phase flash with the following conditions (Column: X Bridge Prep Phenyl OBD Column, 19*150 mm, 5µm; Mobile Phase A: water (0.05% NH₃.H₂O), Mobile Phase B: ACN; Flow rate: 25 mL/min; Gradient: 16% B to 37% B in 10 min, 37% B; Wavelength: 220 nm; RT1(min): 8.62; to afford Example 29. LCMS (ESI) m/z: 413.05 [M+H] ⁺.

### Example 30: 6-chloro-3-(6-fluoropyridin-3-yl)-2-[4-(4-methyl-4H-1,2,4-triazol-3-yl)piperidin-1-yl]benzonitrile

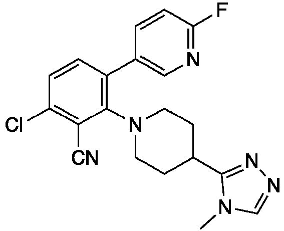

The title compound was prepared using the following procedure:

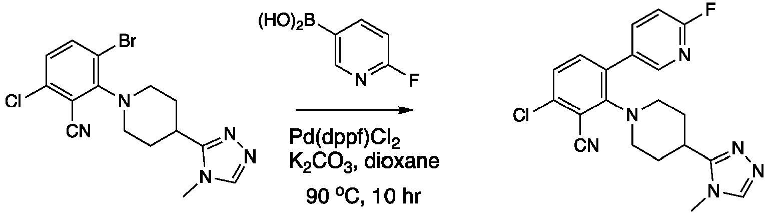

To a stirred solution of Intermediate 6 (200 mg, 0.52 mmol), K₂CO₃ (145.2 mg, 1.05 mmol) in dioxane (5 mL) and H₂O (0.5 mL) were added (6-fluoropyridin-3-yl)boronic acid (74 mg, 0.52 mmol) and Pd(dppf)Cl₂ (115.3 mg, 0.16 mmol) at room temperature under nitrogen atmosphere. The resulting mixture was stirred for 10 h at 90 °C. The crude product (150 mg) was purified by Prep-HPLC with the following conditions (Column: XBridge Prep Phenyl OBD Column, 19*150 mm, 5µm; Mobile Phase A: water (10 mmol/L NH₄HCO₃), Mobile Phase B: MeOH-HPLC; Flow rate: 25 mL/min; Gradient: 48% B to 69% B in 10 min, 69% B; Wavelength: 220 nm; RT1(min): 8.50; to afford Example 30. LCMS (ESI) m/z: 397.00 [M+H]⁺.

### Example 31: 3-(4-methoxypyridin-3-yl)-2-[4-(1-methyl-1H-imidazol-5-yl)piperidin-1-yl]benzonitrile

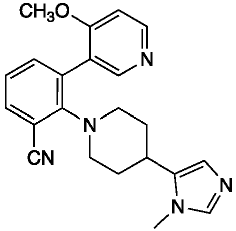

The title compound was prepared using the following procedure:

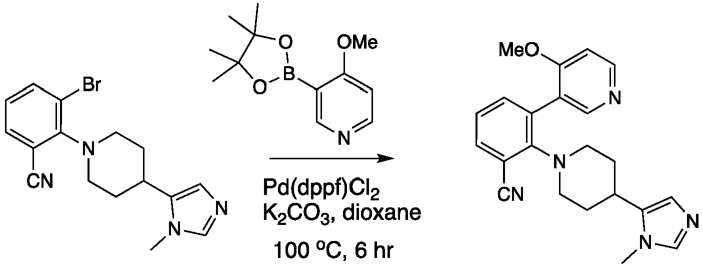

To a stirred solution of Intermediate 8 (100 mg, 0.29 mmol) in anhydrous 1,4-dioxane (10 mL) and H₂O (1 mL) was added K₂CO₃ (80 mg, 0.58 mmol) and Pd(dppf)Cl₂ (1.07 g, 1.5 mmol) followed by 4-methoxy-3-(4,4,5,5-tetramethyl-1,3,2-dioxaborolan-2-yl) pyridine (68 mg, 0.29 mmol) at room temperature. The reaction mixture was stirred at 100 °C for a period of 6 h. After completion of reaction, the reaction mixture was quenched by addition of water (5 mL). The crude product was purified by reverse phase flash with the following conditions (Column: Sunfire prep C18 column, 30*150 mm, 5µm; Mobile Phase A: water (0.1% FA), Mobile Phase B: ACN; Flow rate: 60 mL/min; Gradient: 15% B to 50% B in 7 min, 50% B; Wavelength: 254/220 nm; RT1(min): 3.88; to afford Example 31. LCMS (ESI) m/z: 374.35 [M+H]⁺.

### Example 32: 4-(4-methoxypyridin-3-yl)-3-[4-(4-methyl-4H-1,2,4-triazol-3-yl)piperidin-1-yl]-[1,1'-biphenyl]-2-carbonitrile

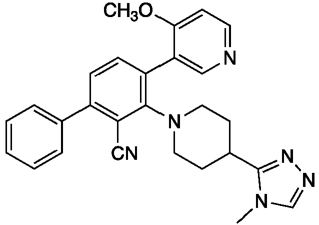

The title compound was prepared using the following procedure:

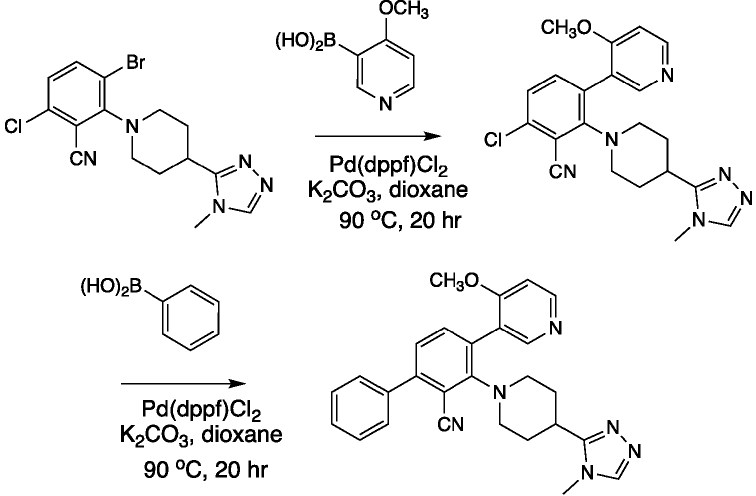

To a stirred mixture of Intermediate 6 (0.5 g, 1.31 mmol) and K₂CO₃ (0.36 g, 2.62 mmol) in 1,4-dioxane (5 mL) and H₂O (0.5 mL) were added (4-methoxypyridin-3-yl)boronic acid (0.16 g, 1 mmol) and Pd(dppf)Cl₂ (0.10 g, 0.13 mmol) at room temperature under nitrogen atmosphere. The mixture was stirred for 20 h at 90 °C. The crude product (10 mg) was purified by Prep-HPLC with the following conditions (Column: Sunfire prep C18 column, 30*150 mm, 5µm; Mobile Phase A: water (0.1% FA), Mobile Phase B: ACN; Flow rate: 60 mL/min; Gradient: 5% B to 28% B in 8 min, 28% B; Wavelength: 254/220 nm; RT1(min): 7.00; to afford 6-chloro-3-(4-methoxypyridin-3-yl)-2-(4-(4-methyl-4H-1,2,4-triazol-3-yl)piperidin-1-yl)benzonitrile. LCMS (ESI) m/z: 409 [M+H] ⁺.

To a stirred solution of 6-chloro-3-(4-methoxypyridin-3-yl)-2-(4-(4-methyl-4H-1,2,4-triazol-3-yl) piperidin-1-yl)benzonitrile (50 mg, 0.12 mmol) and K₂CO₃ (33.8 mg, 0.24 mmol) in dioxane (0.7 mL) were added phenyl boronic acid (14.9 mg, 0.12 mmol) and Pd(dppf)Cl₂ (26.8 mg, 0.04 mmol) at room temperature under nitrogen atmosphere. The mixture was stirred for 20 h at 90 °C. The crude product (5 mg) was purified by Prep-HPLC with the following conditions (Column: XBridge Shield RP18 OBD Column, 19*150 mm, 5µm; Mobile Phase A: water (0.1% FA), Mobile Phase B: ACN; Flow rate: 25 mL/min; Gradient: 31% B to 52% B in 8 min, 52% B; Wavelength: 220 nm; RT1(min): 7.70; to afford Example 32. LCMS (ESI) m/z: 451.10 [M+H]⁺.

### Example 33: 6-chloro-2-[4-(4-methyl-4H-1,2,4-triazol-3-yl)piperidin-1-yl]-3-(pyridazin-4-yl)benzonitrile

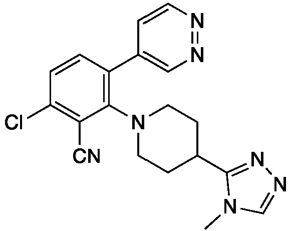

The title compound was prepared using the following procedure:

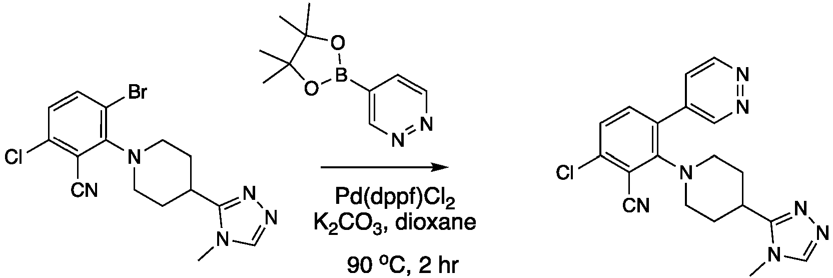

To a stirred solution of Intermediate 6 (100 mg, 0.26 mmol) in dioxane (2 mL) and H₂O (0.5 mL) were added K₂CO₃ (72.6 mg, 0.53 mmol), 4-(4,4,5,5-tetramethyl-1,3,2-dioxaborolan-2-yl)pyridazine (64.9 mg, 0.32 mmol) and Pd(dppf)Cl₂ (38.4 mg, 0.053 mmol) in portions at room temperature under nitrogen atmosphere. The resulting mixture was stirred for 2 h at 90 °C. The resulting mixture was concentrated under reduced pressure. The residue was purified by silica gel column chromatography, eluted with CH₂Cl₂/MeOH (6:1) to afford a red solid as a crude product. The crude product (20 mg) was purified by Prep-HPLC with the following conditions (Column: XBridge Prep Phenyl OBD Column, 19*150 mm, 5µm; Mobile Phase A: water (10 mmol/L NH₄HCO₃), Mobile Phase B: MeOH-HPLC; Flow rate: 25 mL/min; Gradient: 39% B to 69% B in 7 min, 69% B; Wavelength: 254/220 nm; RT1(min): 7.13; to afford Example 33. LCMS (ESI) m/z: 380.00 [M+H]⁺.

### Example 34: 3-(1-methyl-2-oxo-1,2-dihydropyrimidin-5-yl)-2-[4-(4-methyl-4H-1,2,4-triazol-3-yl)piperidin-1-yl]benzonitrile

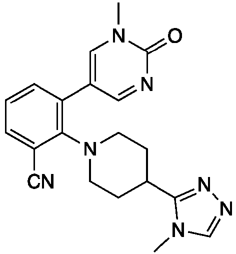

The title compound was prepared using the following procedure.

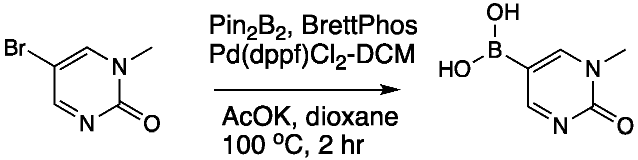

To a stirred solution of 5-bromo-1-methylpyrimidin-2-one (2 g, 10.6 mmol) and 4,4,5,5-tetramethyl-2-(4,4,5,5-tetramethyl-1,3,2-dioxaborolan-2-yl)-1,3,2-dioxaborolane (5.4 g, 21.2 mmol) in dioxane (30 mL) were added AcOK (3.1 g, 31.7 mmol), dicyclohexyl[3,6-dimethoxy-2',4',6'-tris(propan-2-yl)-[1,1'-biphenyl]-2-yl] phosphane (1.1 g, 2.1 mmol) and Pd(dppf)Cl₂.CH₂Cl₂ (43.1 mg, 0.01 mmol) in portions at room temperature under nitrogen atmosphere. The resulting mixture was stirred for 2 h at 100 °C. The reaction was quenched with water (100 mL) at room temperature. The resulting mixture was extracted with EtOAc (3 x 100 mL). The combined organic layers were washed with brine (2 x 50 mL), dried over anhydrous Na₂SO₄. After filtration, the filtrate was concentrated under reduced pressure. The residue was purified by silica gel column chromatography, eluted with EtOAc/petroleum ether (70%-90%) to afford 1-methyl-2-oxopyrimidin-5-ylboronic acid (400 mg, 25%) as a light yellow solid.

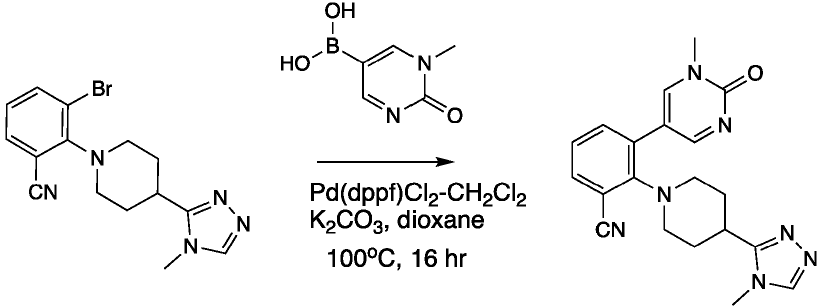

To a stirred solution of 1-methyl-2-oxopyrimidin-5-ylboronic acid (88.9 mg, 0.6 mmol) and Intermediate 1 (100 mg, 0.3 mmol) in dioxane (5 mL) /H₂O (0.5 mL) were added K₂CO₃ (119.8 mg, 0.9 mmol) and Pd(dppf)Cl₂.CH₂Cl₂ (23.5 mg, 0.03 mmol) in portions at room temperature under nitrogen atmosphere. The resulting mixture was stirred overnight at 100 °C under nitrogen atmosphere. The reaction was quenched with water (30 mL) at room temperature. The aqueous layer was extracted with EtOAc (3 x 30 mL). The crude product (50 mg) was purified by Prep-HPLC with the following conditions (Column: YMC-Actus Triart C18 ExRS, 30*150 mm, 5µm; Mobile Phase A: water (10 mmol/L NH₄HCO₃), Mobile Phase B: ACN; Flow rate: 60 mL/min; Gradient: 5% B to 25% B in 9 min, 25% B; Wavelength: 254/220 nm; RT1(min): 10.80; to afford Example 34. LCMS (ESI) m/z: 376.05 [M+H]⁺.

### Example 35: 3-(6-fluoropyridin-3-yl)-6-(1-methyl-1H-pyrazol-4-yl)-2-[4-(4-methyl-4H-1,2,4-triazol-3-yl)piperidin-1-yl]benzonitrile

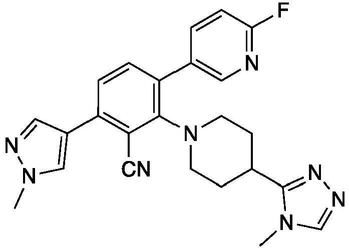

The title compound was prepared using the following procedure:

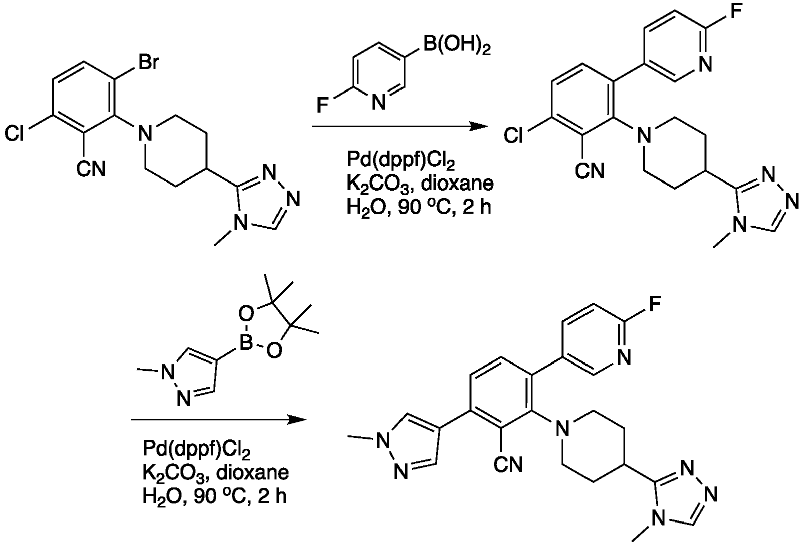

To a stirred solution of Intermediate 6 (300 mg, 0.79 mmol) in dioxane (3 mL) and H₂O (1 mL) were added K₂CO₃ (218 mg, 1.58 mmol) 6-fluoropyridin-3-ylboronic acid (88.8 mg, 0.63 mmol) and Pd(dppf)Cl₂ (115.3 mg, 0.16 mmol) in portions at room temperature under nitrogen atmosphere. The resulting mixture was stirred for 2 h at 90 °C under nitrogen atmosphere. The resulting mixture was concentrated under reduced pressure. The residue was purified by silica gel column chromatography, eluted with CH₂Cl₂ / MeOH (6:1) to afford 6-chloro-3-(6-fluoropyridin-3-yl)-2-[4-(4-methyl-1,2,4-triazol-3-yl)piperidin-1-yl]benzonitrile (200 mg, 64%) as a red solid. LCMS (ESI) m/z: 397 [M+H]⁺.

To a stirred solution of 6-chloro-3-(6-fluoropyridin-3-yl)-2-[4-(4-methyl-1,2,4-triazol-3-yl)piperidin-1-yl]benzonitrile (150 mg, 0.38 mmol) in dioxane (3 mL) and H₂O (1 mL) were added K₂CO₃ (104.5 mg, 0.76 mmol) 1-methyl-4-(4,4,5,5-tetramethyl-1,3,2-dioxaborolan-2-yl)pyrazole (157.3 mg, 0.76 mmol) and Pd(dppf)Cl₂ (55.3 mg, 0.076 mmol) in portions at room temperature under nitrogen atmosphere. The resulting mixture was stirred for 2 h at 90 °C under nitrogen atmosphere. Desired product could be detected by LCMS. The resulting mixture was concentrated under reduced pressure. The residue was purified by reversed-phase flash chromatography with the following conditions: column, C18 silica gel; mobile phase, MeCN in water (10mmol/L NH₄HCO₃), 10% to 50% gradient in 10 min; detector, UV 254 nm, to afford a light brown solid as a crude product. The crude product (25 mg) was purified by Prep-HPLC with the following conditions (Column: XBridge Prep Phenyl OBD Column, 19*100 mm, 5 *µ* m; Mobile Phase A: water (10 mmol/L NH₄HCO₃), Mobile Phase B: ACN; Flow rate: 25 mL/min; Gradient: 15% B to 35% B in 8 min, 35% B; Wavelength: 254/220 nm; RT1(min): 9.88) to afford Example 35. LCMS: (ES, m/z): 443.10 [M+H]⁺.

### Example 36: 6-(1-methyl-1H-pyrazol-4-yl)-2-[4-(4-methyl-4H-1,2,4-triazol-3-yl)piperidin-1-yl]-3-(pyridazin-4-yl)benzonitrile

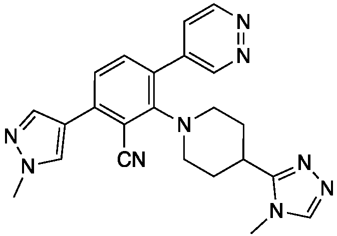

The title compound was prepared using the following procedure:

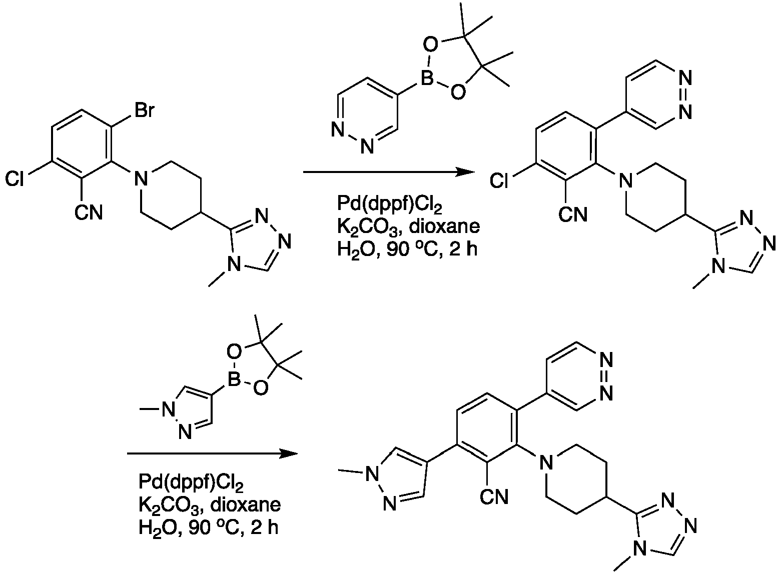

To a stirred solution of Intermediate 6 (100 mg, 0.26 mmol) in dioxane (3 mL) and H₂O (1 mL) were added K₂CO₃ (72.6 mg, 0.53 mmol) 4-(4,4,5,5-tetramethyl-1,3,2-dioxaborolan-2-yl)pyridazine (64.9 mg, 0.32 mmol) and Pd(dppf)Cl₂ (38.4 mg, 0.053 mmol) in portions at room temperature. The resulting mixture was stirred for 2 h at 90 °C under nitrogen atmosphere. Desired product could be detected by LCMS. The resulting mixture was concentrated under reduced pressure. The residue was purified by silica gel column chromatography, eluted with CH₂Cl₂ / MeOH (6: 1) to afford 6-chloro-2-[4-(4-methyl-1,2,4-triazol-3-yl)piperidin-1-yl]-3-(pyridazin-4-yl)benzonitrile (50 mg, 50%) as a red solid. LCMS (ESI) m/z: 380 [M+H]⁺.

To a stirred solution of 6-chloro-2-[4-(4-methyl-1,2,4-triazol-3-yl)piperidin-1-yl]-3-(pyridazin-4-yl)benzonitrile (50 mg, 0.13 mmol) in dioxane (2 mL) and H₂O (0.5 mL) were added K₂CO₃ (36.4 mg, 0.26 mmol) 1-methyl-4-(4,4,5,5-tetramethyl-1,3,2-dioxaborolan-2-yl)pyrazole (54.8 mg, 0.26 mmol) and Pd(dppf)Cl₂ (19.3 mg, 0.026 mmol) in portions at room temperature under nitrogen atmosphere. The resulting mixture was stirred for 2 h at 90 °C under nitrogen atmosphere. Desired product could be detected by LCMS. The resulting mixture was concentrated under reduced pressure. The residue was purified by reversed-phase flash chromatography with the following conditions: column, C18 silica gel; mobile phase, MeCN in water (10 mmol/L NH₄HCO₃), 15% to 40% gradient in 20 min; detector, UV 254 nm, to afford a black solid as a crude product. The crude product (60 mg) was purified by Prep-HPLC with the following conditions (Column: XBridge Shield RP18 OBD Column, 30*150 mm, 5µm; Mobile Phase A: Water (10 mmol/L NH₄HCO₃), Mobile Phase B: MeOH--HPLC; Flow rate: 50 mL/min; Gradient: 31% B to 41% B in 8 min, 41% B; Wavelength: 254/220 nm; RT1(min): 5.00) to afford Example 36. LCMS (ESI) m/z: 426.05 [M+H]⁺.

### Example 37: 6-[2-(dimethylamino)ethoxy]-3-(6-fluoropyridin-3-yl)-2-[4-(4-methyl-4H-1,2,4-triazol-3-yl)piperidin-1-yl]benzonitrile

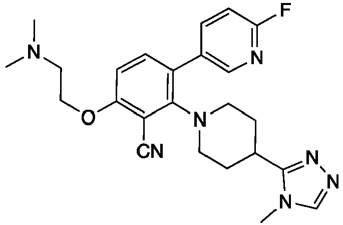

The title compound was prepared using the following procedure:

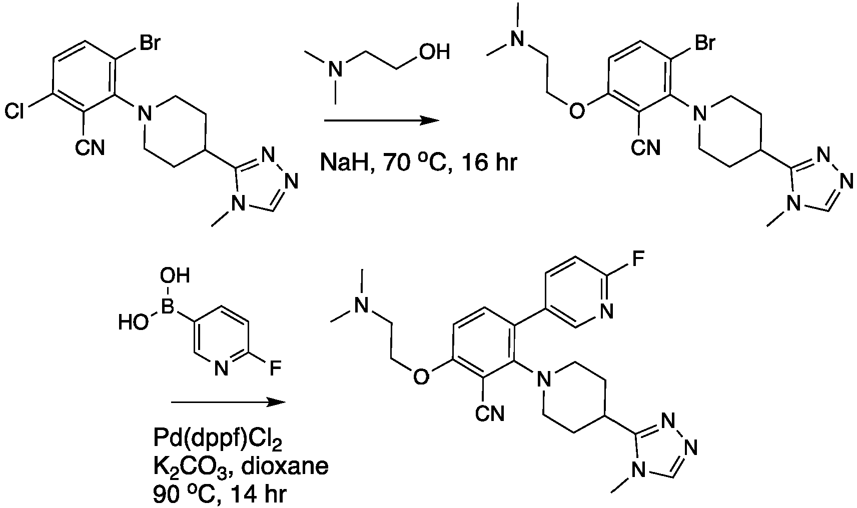

To a stirred solution of Intermediate 6 (1.4 g, 3.7 mmol) in dimethylaminoethanol (393.4 mg, 4.4 mmol) was added NaH (189 mg, 7.9 mmol) in portions at 0 °C under nitrogen atmosphere. The resulting mixture was stirred overnight at 70 °C under nitrogen atmosphere. The resulting mixture was filtered, the filter cake was washed with MeCN (3 x 20 mL). The filtrate was concentrated under reduced pressure. The residue was purified by reversed-phase flash chromatography with the following conditions: column, C18 silica gel; mobile phase, MeCN in water (10mmol/L NH₄HCO₃), 10% to 50% gradient in 10 min; detector, UV 254 nm. To afford 3-bromo-6-[2-(dimethylamino)ethoxy]-2-[4-(4-methyl-1,2,4-triazol-3-yl)piperidin-1-yl]benzonitrile (430 mg, 26.4%) as a brown viscous oil.

To a stirred solution of 3-bromo-6-[2-(dimethylamino)ethoxy]-2-[4-(4-methyl-1,2,4-triazol-3-yl)piperidin-1-yl]benzonitrile (250 mg, 0.6 mmol) and 6-fluoropyridin-3-ylboronic acid (89.4 mg, 0.6 mmol) in dioxane (5 mL)/H₂O (0.5 mL) were added Pd(dppf)Cl₂CH₂Cl₂ (47 mg, 0.06 mmol) and K₂CO₃ (239.2 mg, 1.7 mmol) in portions at room temperature under nitrogen atmosphere. The resulting mixture was stirred for overnight at 90 °C under nitrogen atmosphere. The resulting mixture was filtered, the filter cake was washed with MeCN (3 x 20 mL). The filtrate was concentrated under reduced pressure. The crude product (100 mg) was purified by Prep-HPLC with the following conditions (Column: YMC-Actus Triart C18 ExRS, 30*150 mm, 5µm; Mobile Phase A: water (10 mmol/L NH₄HCO₃+0.1%NH₃.H₂O), Mobile Phase B: ACN; Flow rate: 60 mL/min; Gradient: 12% B to 32% B in 9 min, 32% B; Wavelength: 254/220 nm; RT1(min): 11.82) to afford Example 37. LCMS (ESI) m/z: 450.10 [M+H]⁺.

### Example 38: 4-fluoro-3-(6-fluoropyridin-3-yl)-2-[4-(4-methyl-4H-1,2,4-triazol-3-yl)piperidin-1-yl]benzonitrile

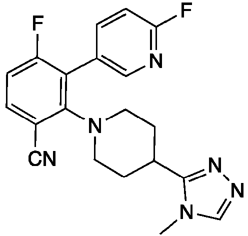

The title compound was prepared using the following procedure:

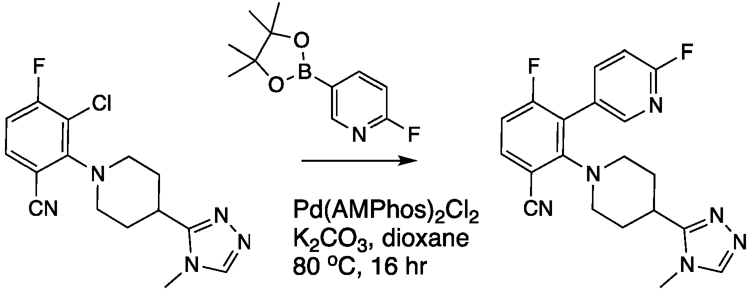

To a stirred solution of Intermediate 9 (150 mg, 0.47 mmol) in anhydrous 1,4-dioxane (10 mL) and H₂O (2mL) was added K₂CO₃ (129.7 mg, 0.94 mmol) and 2-fluoro-5-(4,4,5,5-tetramethyl-1,3,2-dioxaborolan-2-yl) pyridine (115.1 mg, 0.52 mmol) followed by catalytic amount of Pd(AMPhos)₂Cl₂ (33.2 mg, 0.05 mmol) at room temperature. The reaction mixture was stirred at 80 °C for 16 h. After completion of reaction, the reaction mixture was quenched by addition of water (20 mL). The aqueous layer was extracted with ethyl acetate (100 mL). The combined organic phase was washed with brine (100 mL), The resulting mixture was concentrated under reduced pressure. The residue product was purified by reverse phase flash with the following conditions (Column: X Bridge Prep Phenyl OBD Column, 19*150 mm, 5µm; Mobile Phase A: water (10 mmol/L NH₄HCO₃), Mobile Phase B: ACN; Flow rate: 25 mL/min; Gradient: 25% B to 40% B in 8 min, 40% B; Wavelength: 254/220 nm; RT1(min): 6.93) to afford Example 38. LCMS (ESI) m/z) 381.05 [M+H]⁺.

### Example 39: 4-fluoro-2-[4-(4-methyl-4H-1,2,4-triazol-3-yl)piperidin-1-yl]-3-(pyridazin-4-yl)benzonitrile

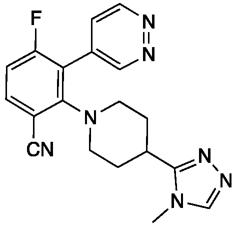

The title compound was prepared using the following procedure:

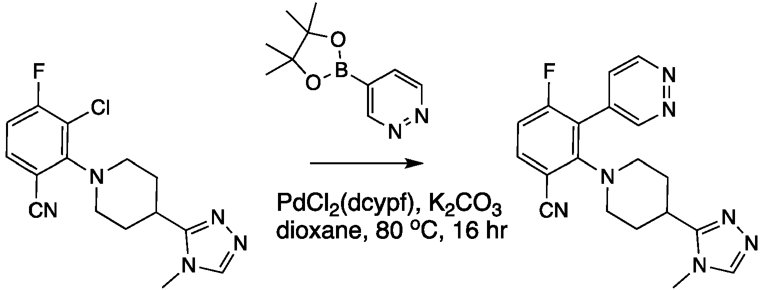

To a stirred solution of Intermediate 9 (50 mg, 0.156 mmol) in anhydrous 1,4-dioxane (5 mL) and H₂O (1 mL) was added K₂CO₃ (129.7 mg, 0.94 mmol) and 4-(4,4,5,5-tetramethyl-1,3,2-dioxaborolan-2-yl) pyridazine (35.4 mg, 0.17 mmol) followed by catalytic amount of PdCl₂(dcypf) (11.8 mg, 0.016 mmol) at room temperature. The reaction mixture was stirred at 80 °C for 16 h. After completion of reaction, the reaction mixture was quenched by addition of water (20 mL). The aqueous layer was extracted with ethyl acetate (100 mL). The combined organic phase was washed with brine (100 mL), The resulting mixture was concentrated under reduced pressure. The residue product was purified by reverse phase flash with the following conditions (Column: X Select CSH Fluoro Phenyl, 30*250 mm, 5µm; Mobile Phase A: water (0.1% FA), Mobile Phase B: MeOH-HPLC; Flow rate: 60 mL/min; Gradient: 20% B to 38% B in 10 min, 38% B; Wavelength: 254/220 nm; RT1(min) to afford Example 39. LCMS (ESI) m/z: 364.05 [M+H]⁺.

### Example 40: 3-(6-chloropyridin-3-yl)-2-[4-(4-methyl-4H-1,2,4-triazol-3-yl)piperidin-1-yl]benzonitrile

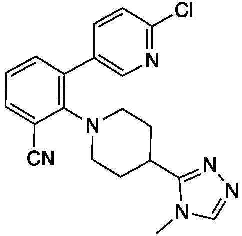

The title compound was prepared using the following procedure:

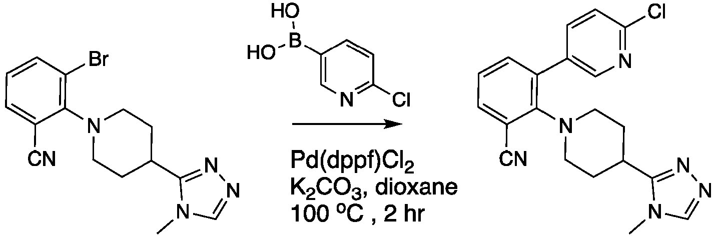

To a stirred solution of Intermediate 1 (50 mg, 0.14 mmol) in anhydrous 1,4-dioxane (5 mL) and H₂O (0.5 mL) was added K₂CO₃ (39.9 mg, 0.29 mmol) and 6-chloropyridin-3-ylboronic acid (25 mg, 0.16 mmol) followed by catalytic amount of Pd(dppf)Cl₂ (11.8 mg, 0.014 mmol) at room temperature. The reaction mixture was stirred at 100 °C for a period of 2 h. After completion of reaction, the reaction mixture was quenched by addition of water (20 mL). The aqueous layer was extracted with ethyl acetate (100 mL). The combined organic phase was washed with brine (100 mL), The resulting mixture was concentrated under reduced pressure. The residue product was purified by reverse phase flash with the following conditions (Column: YMC-Actus Triart C18 ExRS, 30*150 mm, 5µm; Mobile Phase A: water (10 mmol/L NH₄HCO₃ + 0.1% NH₃.H₂O), Mobile Phase B: ACN; Flow rate: 60 mL/min; Gradient: 21% B to 40% B in 9 min, 40% B; Wavelength: 254/220 nm; RT1(min): 10.48) to afford Example 40. LCMS (ESI) m/z: 379.00 [M+H]⁺.

### Example 41: 3-(6-fluoropyridin-3-yl)-2-(4-(4-(methyl-d3)-4H-1,2,4-triazol-3-yl)piperidin-1-yl)benzonitrile

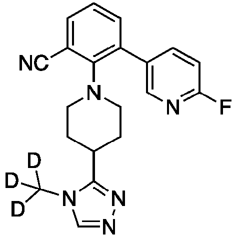

The title compound was prepared using the following procedure:

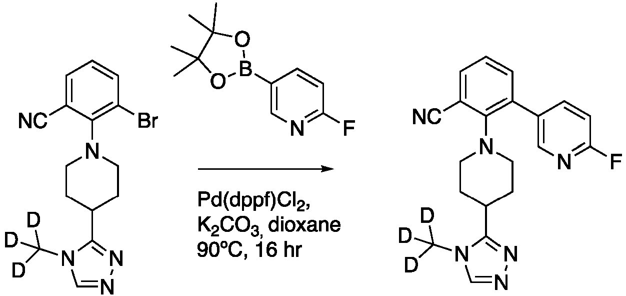

To a stirred solution of Intermediate 10 (100 mg, 0.29 mmol) and 2-fluoro-5-(4,4,5,5-tetramethyl-1,3,2-dioxaborolan-2-yl)pyridine (76.6 mg, 0.34 mmol) in dioxane (20 mL) were added Pd(dppf)Cl₂.CH₂Cl₂ (23.3 mg, 0.03 mmol) and K₂CO₃ (79 mg, 0.57 mmol) in portions at room temperature under nitrogen atmosphere. The resulting mixture was stirred for 16 h at 90 °C under nitrogen atmosphere. The reaction was quenched by the addition of water (5 mL) at room temperature. The resulting mixture was extracted with EtOAc (3 x 20 mL). The combined organic layers were washed with water (2 x 20 mL), dried over anhydrous Na₂SO₄. After filtration, the filtrate was concentrated under reduced pressure. The crude product was purified by Prep-HPLC with the following conditions (Column: Xselect CSH C18 OBD Column 30*150mm 5 *µ* m, n; Mobile Phase A: water (0.1% FA), Mobile Phase B: ACN; Flow rate: 60 mL/min; Gradient: 16% B to 34% B in 9 min, 34% B; Wave Length: 254/220 nm; RT1(min): 9.38; to afford Example 41 (48.4 mg, 44%). LCMS (ESI) m/z: 366.05 [M+H]⁺.

### Example 43: 3-(6-fluoropyridin-3-yl)-6-(1-methyl-1H-pyrazol-3-yl)-2-(4-(4-methyl-4H-1,2,4-triazol-3-yl)piperidin-1-yl)benzonitrile

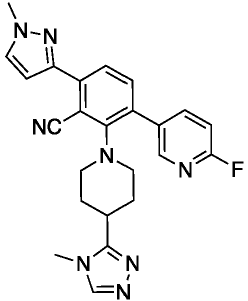

The title compound was prepared using the following procedure:

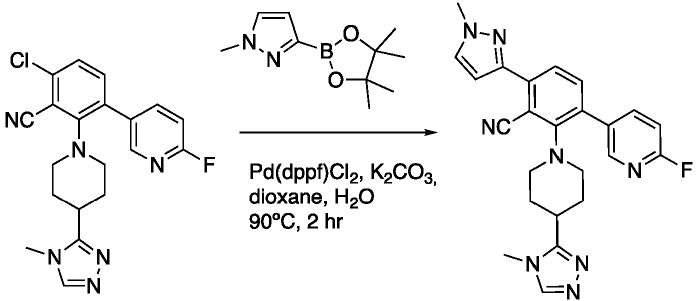

To a stirred solution of Example 30 (150 mg, 0.38 mmol) in dioxane (5 mL) and H₂O (1 mL) were added K₂CO₃ (104.5 mg, 0.76 mmol), 1-methyl-3-(4,4,5,5-tetramethyl-1,3,2-dioxaborolan-2-yl)pyrazole (118 mg, 0.57 mmol) and Pd(dppf)Cl₂ (27.7 mg, 0.038 mmol) in portions at room temperature under nitrogen atmosphere. The resulting mixture was stirred for 2 h at 90 °C. The resulting mixture was concentrated under reduced pressure. The residue was purified by silica gel column chromatography, eluted with CH₂Cl₂ / MeOH (9:1) to afford a red solid as a crude product. The crude product was purified by Prep-HPLC with the following conditions (Column: XBridge Prep OBD C18 Column, 30*150 mm, 5µm; Mobile Phase A: water (10 mmol/L NH₄HCO₃), Mobile Phase B: ACN; Flow rate: 60 mL/min; Gradient: 20% B to 36% B in 10 min, 36% B; Wave Length: 220/254 nm; RT1(min): 11.22; to afford Example 43 (22.8 mg, 13%). LCMS (ESI) m/z: 443.05 [M+H]⁺.

### Example 49: 2-(4-(4H-1,2,4-triazol-3-yl)piperidin-1-yl)-3-(6-fluoropyridin-3-yl)benzonitrile

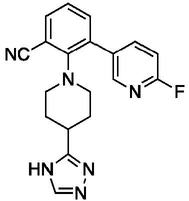

The title compound was prepared using the following procedure:

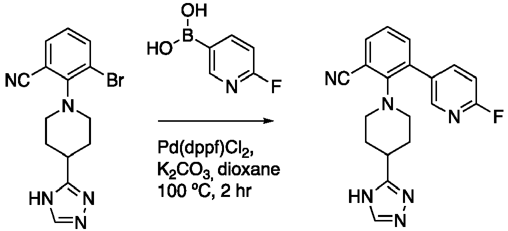

To a stirred solution of Intermediate 11 (51 mg, 0.15 mmol) and 6-fluoropyridin-3-ylboronic acid (28.1 mg, 0.2 mmol) in 1,4-dioxane (5 mL) and water (1 mg) were added Cs₂CO₃ (100 mg) and Pd(dppf)Cl₂.CH₂Cl₂ (12.5 mg) in portions at room temperature under nitrogen atmosphere. The resulting mixture was stirred for 2 h at 100 °C. The resulting mixture was extracted with EtOAc (3 x 10 mL). The combined organic layers were washed with brine (3 x 5 mL), dried over anhydrous Na₂SO₄. After filtration, the filtrate was concentrated under reduced pressure. The crude product was purified by Prep-HPLC with the following conditions (Column: XBridge Prep Phenyl OBD Column, 19*150 mm, 5µm; Mobile Phase A: water (10 mmol/L NH₄HCO₃), Mobile Phase B: MeOH-HPLC; Flow rate: 50 mL/min; Gradient: 56% B to 72% B in 8 min, 72% B; Wave Length: 254/220 nm; RT1(min): 6.47; to afford Example 49 (2.7 mg, 4%). LCMS (ESI) m/z: 349.00 [M+H]⁺.

### Example 50: 3-(6-fluoropyridin-3-yl)-6-(3-methyl-1H-1,2,4-triazol-1-yl)-2-(4-(4-methyl-4H-1,2,4-triazol-3-yl)piperidin-1-yl)benzonitrile

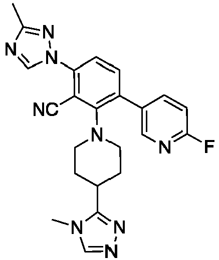

The title compound was prepared using the following procedure:

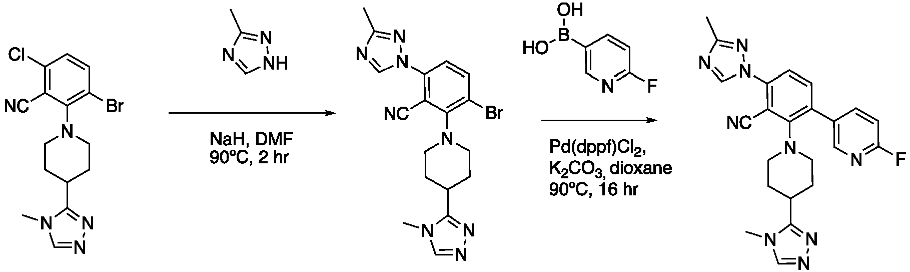

To a stirred solution of Intermediate 6 (300 mg, 0.7 mmol) and 3-methyl-1H-1,2,4-triazole (85.1 mg) in DMF (10 mL) was added NaH (28.4 mg) in portions at room temperature under nitrogen atmosphere. The resulting mixture was stirred for 2 h at 90 °C under nitrogen atmosphere. The reaction was quenched by the addition of water (10 mL) at room temperature. The resulting mixture was extracted with EtOAc (3 x 20 mL). The combined organic layers were washed with brine (3 x 10 mL), dried over anhydrous Na₂SO₄. After filtration, the filtrate was concentrated under reduced pressure. The residue was purified by silica gel column chromatography, eluted with CH₂Cl₂ / MeOH (9:1) to afford 3-bromo-6-(3-methyl-1,2,4-triazol-1-yl)-2-[4-(4-methyl-1,2,4-triazol-3-yl)piperidin-1-yl]benzonitrile (117 mg) as a yellow solid. LCMS (ESI) m/z: 427, 429 [M+H]⁺.

To a stirred solution of 3-bromo-6-(3-methyl-1,2,4-triazol-1-yl)-2-[4-(4-methyl-1,2,4-triazol-3-yl)piperidin-1-yl]benzonitrile (100 mg, 0.23 mmol) and 6-fluoropyridin-3-ylboronic acid (42.9 mg, 0.3 mmol) in 1,4-dioxane (10 mL) and H₂O (1 mL) were added K₂CO₃ (64.7 mg,) and Pd(dppf)Cl₂.CH₂Cl₂ (19.1 mg) in portions at room temperature under nitrogen atmosphere. The resulting mixture was stirred overnight at 90 °C under nitrogen atmosphere. The reaction was quenched by the addition of water (20 mL) and extracted with EtOAc (3 x 30 mL). The combined organic layers were washed with brine (3 x 10 mL), dried over anhydrous Na₂SO₄. After filtration, the filtrate was concentrated under reduced pressure. The residue was purified by silica gel column chromatography, eluted with CH₂Cl₂ / MeOH (10:1) to afford Example 50 (32.7 mg, 31%). LCMS (ESI) m/z: 444.05 [M+H]⁺.

### Example 51: 6-((2-(dimethylamino)ethyl)amino)-3-(6-fluoropyridin-3-yl)-2-(4-(4-methyl-4H-1,2,4-triazol-3-yl)piperidin-1-yl)benzonitrile

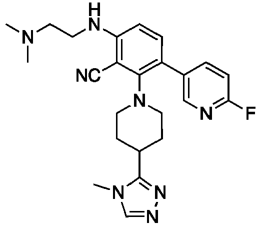

The title compound was prepared using the following procedure:

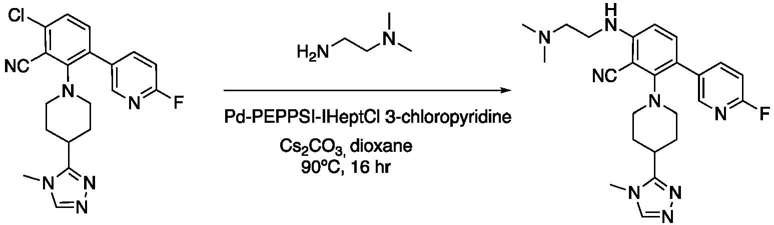

A solution of Example 30 (50 mg, 0.13 mmol) in 1,4-dioxane (1.5 mL) was treated with Cs₂CO₃ (82.1 mg, 0.25 mmol) for 5 min at room temperature under nitrogen atmosphere. To the above mixture were added Pd-PEPPSI-IHeptCl 3-chloropyridine (12.3 mg, 0.013 mmol) and (2-aminoethyl)dimethylamine (22.2 mg, 0.25 mmol) for 5 min at room temperature under nitrogen atmosphere. The resulting mixture was stirred overnight at 90 °C under nitrogen atmosphere. The resulting mixture was concentrated under reduced pressure. The residue was purified by Prep-TLC (CH₂Cl₂ / MeOH 9: 1) and then re-purified by Prep-HPLC with the following conditions (Column: YMC-Actus Triart C₁₈ ExRS, 30*150 mm, 5µm; Mobile Phase A: water (10 mmol/L NH₄HCO₃), Mobile Phase B: ACN; Flow rate: 60 mL/min; Gradient: 20% B to 45% B in 7 min, 45% B; Wave Length: 254/220 nm; RT1(min): 6.72; to afford Example 51 (6.8 mg, 12%). LCMS (ESI) m/z: 449.20 [M+H]⁺.

### Example 57: 3-(6-fluoropyridin-3-yl)-6-(methoxy-d3)-2-(4-(4-methyl-4H-1,2,4-triazol-3-yl)piperidin-1-yl)benzonitrile

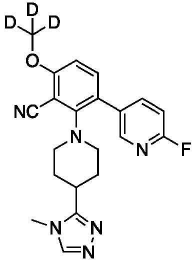

The title compound was prepared using the following procedure:

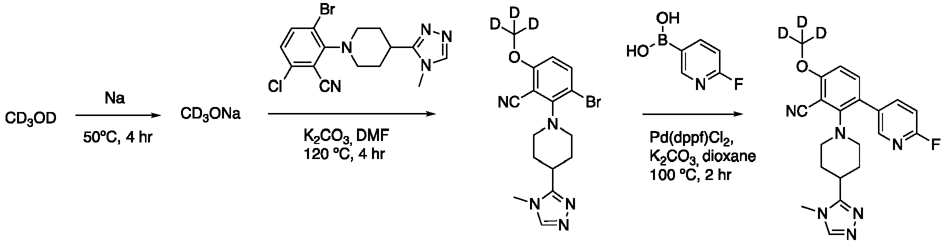

A mixture of sodium (2 g, 87 mmol) in (²H3)methanol (20 mL) was stirred for 4 h at 50 °C under nitrogen atmosphere. The resulting mixture was concentrated under vacuum. This resulted in (²H3)methoxysodium (3.5 g, 70%) as a white solid.

To a stirred solution of Intermediate 6 (150 mg, 0.39 mmol) and K₂CO₃ (109 mg, 0.79 mmol) in DMF (5 mL) were added sodium (²H3)methanolate (27 mg, 0.47 mmol) in portions at room temperature. The resulting mixture was stirred for 4 h at 120 °C. The resulting mixture was extracted with EtOAc (2 x 150 mL). The combined organic layers were washed with brine (200 mL), dried over anhydrous Na₂SO₄. After filtration, the filtrate was concentrated under reduced pressure. The residue was purified by silica gel column chromatography, eluted with PE / EA (1:1) to afford 3-bromo-6-(2H3)methoxy-2-[4-(4-methyl-1,2,4-triazol-3-yl)piperidin-1-yl]benzonitrile (70 mg, 47%) as a light yellow oil. LCMS (ESI) m/z: 379, 381 [M+H]⁺.

To a stirred mixture of 3-bromo-6-(2H3)methoxy-2-[4-(4-methyl-1,2,4-triazol-3-yl)piperidin-1-yl]benzonitrile (70 mg, 0.18 mmol) and 6-fluoropyridin-3-ylboronic acid (33.8 mg, 0.24 mmol) in dioxane (10 mL) and H₂O (1 mL) were added K₂CO₃ (51 mg, 0.37 mmol) and Pd(dppf)Cl₂ (13.5 mg, 0.02 mmol) in portions at room temperature under nitrogen atmosphere. The resulting mixture was stirred for 2 h at 100 °C, filtered, the filter cake was washed with MeOH (2 x 10 mL). The filtrate was concentrated under reduced pressure. The crude product was purified by Prep-HPLC with the following conditions (Column: YMC-Actus Triart C18 ExRS, 30*150 mm, 5m; Mobile Phase A: water (10 mmol/L NH₄HCO₃), Mobile Phase B: ACN; Flow rate: 60 mL/min; Gradient: 20% B to 45% B in 7 min, 45% B; Wave Length: 254/220 nm; RT1(min): 6.72; to afford Example 57 (26.5 mg, 36%). LCMS (ESI) m/z: 396.05 [M+H]⁺.

### Example 58: 6-(2-(dimethylamino)ethoxy)-2-(4-(4-methyl-4H-1,2,4-triazol-3-yl)piperidin-1-yl)-3-(pyridazin-4-yl)benzonitrile

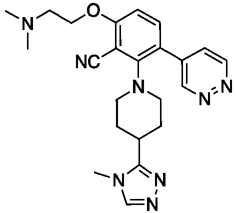

The title compound was prepared using the following procedure:

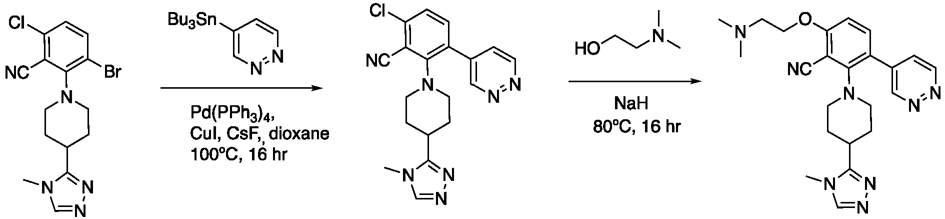

A solution of Intermediate 6 (2.3 g, 6.0 mmol), 4-(tributylstannyl)pyridazine (2.23 g, 6.04 mmol), Pd(PPh₃)₄ (0.70 g, 0.60 mmol), CuI (1.15 g, 6.04 mmol) and CsF (0.92 g, 6.04 mmol) in dioxane (10 mL) was stirred overnight at 100 °C under nitrogen atmosphere. The resulting mixture was concentrated under reduced pressure. The residue was purified by silica gel column chromatography, eluted with CH₂Cl₂ / MeOH (9:1) to afford 6-chloro-2-[4-(4-methyl-1,2,4-triazol-3-yl)piperidin-1-yl]-3-(pyridazin-4-yl)benzonitrile (240 mg, 10%) as a brown solid. LCMS (ESI) m/z: 380 [M+H]⁺.

A solution of 6-chloro-2-[4-(4-methyl-1,2,4-triazol-3-yl)piperidin-1-yl]-3-(pyridazin-4-yl)benzonitrile (100 mg, 0.26 mmol) and NaH (12.6 mg, 0.53 mmol) in dimethylaminoethanol (2 mL) was stirred overnight at 80 °C. The resulting mixture was extracted with EtOAc (2 x 50 mL). The combined organic layers were washed with water (2 x 10 mL), dried over anhydrous Na₂SO₄. After filtration, the filtrate was concentrated under reduced pressure. The crude product was purified by Prep-HPLC with the following conditions (Column: XBridge Shield RP18 OBD Column, 19*250 mm, 10µm; Mobile Phase A: water (10 mmol/L NH₄HCO₃), Mobile Phase B: ACN; Flow rate: 60 mL/min; Gradient: 17% B to 42% B in 10 min, 42% B; Wave Length: 220/254 nm; RT1(min): 9.82; to afford Example 58 (2.8 mg, 4%). LCMS (ESI) m/z: 433.35 [M+H]⁺.

### Example 59: 4-fluoro-2-(4-(4-methyl-4H-1,2,4-triazol-3-yl)piperidin-1-yl)-3-(6-methylpyridazin-4-yl)benzonitrile

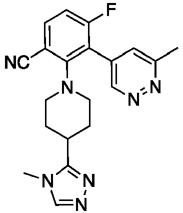

The title compound was prepared using the following procedure:

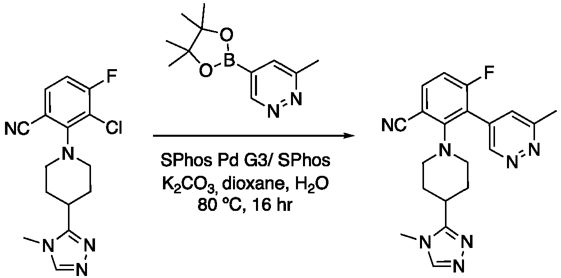

To a stirred solution of Intermediate 9 (50 mg, 0.16 mmol) in anhydrous 1,4-dioxane (5 mL) and H₂O (1 mL) was added K₂CO₃ (43.2 mg, 0.31 mmol) and 3-methyl-5-(4,4,5,5-tetramethyl-1,3,2-dioxaborolan-2-yl) pyridazine (41.3 mg, 0.19 mmol) followed by catalytic amount of Sphos Pd G₃ (24.7 mg, 0.016 mmol) and Sphos (12.6 mg, 0.016 mmol) at room temperature. The reaction mixture was stirred at 80 °C for a period of 16 h. After completion of reaction, the reaction mixture was quenched by addition of water (20 mL). The aqueous layer was extracted with ethyl acetate (100 mL). The combined organic phase was washed with brine (100 mL), The resulting mixture was concentrated under reduced pressure. The crude product was purified by reverse phase flash with the following conditions (Column: X Bridge Prep Phenyl OBD Column, 19*150 mm, 5µm; Mobile Phase A: water (0.05% TFA), Mobile Phase B: ACN; Flow rate: 50 mL/min; Gradient: 18% B to 34% B in 8 min, 34% B; Wave Length: 254/220 nm; RT1(min): 7.75; to afford Example 59 (3.2 mg, 5%). LCMS (ESI) m/z: 378.00 [M+H]⁺.

### Example 64: 6-(6,7-dihydro-5H-pyrazolo[5,1-b][1,3]oxazin-3-yl)-3-(6-fluoropyridin-3-yl)-2-(4-(4-methyl-4H-1,2,4-triazol-3-yl)piperidin-1-yl)benzonitrile

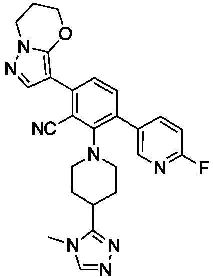

The title compound was prepared using the following procedure:

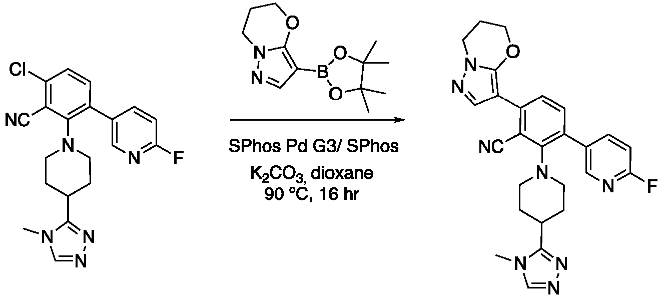

A mixture of Example 30 (70 mg, 0.18 mmol) and K₂CO₃ (48.8 mg, 0.35 mmol) in dioxane (1.6 mL), H₂O (0.4 mL) was stirred for 5 min at room temperature under nitrogen atmosphere. To the above mixture were added SPhos Pd G3 (13.8 mg, 0.018 mmol), Sphos (7.2 mg, 0.018 mmol), 3-(4,4,5,5-tetramethyl-1,3,2-dioxaborolan-2-yl)-5H,6H,7H-pyrazolo[3,2-b] [1,3] oxazine (52.9 mg, 0.21 mmol) at room temperature. The resulting mixture was heated at 90 ° C overnight. The residue was purified by silica gel column chromatography, eluted with CH₂Cl₂ / MeOH (9:1) which was further purified by Prep-HPLC with the following conditions (Column: XBridge Prep Phenyl OBD Column, 19*250 mm, 5 µ m; Mobile Phase A: water (10 mmol/L NH₄HCO₃ + 0.1% NH₃·H₂O), Mobile Phase B: ACN; Flow rate: 50 mL/min; Gradient: 26% B to 43% B in 8 min, 43% B; Wave Length: 254/220 nm; RT1(min): 8.12; to afford Example 64 (2.1 mg, 2%). LCMS (ESI) m/z: 485.25 [M+H]⁺.

### Example 66: 3-(6-fluoropyridin-3-yl)-5-methyl-2-(4-(4-methyl-4H-1,2,4-triazol-3-yl)piperidin-1-yl)benzonitrile

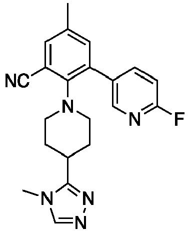

The title compound was prepared using the following procedure:

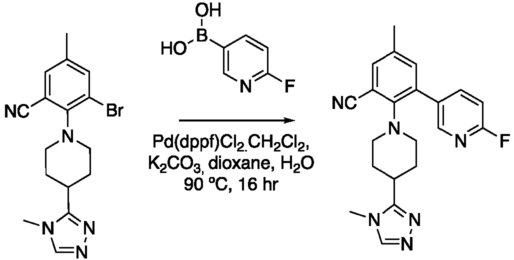

To a stirred solution of Intermediate 12 (50 mg, 0.14 mmol) and 6-fluoropyridin-3-ylboronic acid (25.4 mg, 0.18 mmol) in 1,4-dioxane (5 mL) and H₂O (0.5 mL) were added K₂CO₃ (38.4 mg, 0.28 mmol) and Pd(dppf)Cl₂CH₂Cl₂ (11.3 mg, 0.014 mmol) in portions at room temperature under nitrogen atmosphere. The resulting mixture was stirred overnight at 90 °C under nitrogen atmosphere. The reaction was quenched by the addition of water (5 mL) at room temperature. The resulting mixture was extracted with EtOAc (3 x 25 mL). The combined organic layers were washed with brine (3 x 10 mL), dried over anhydrous Na₂SO₄. After filtration, the filtrate was concentrated under reduced pressure. The residue was purified by silica gel column chromatography, eluted with CH₂Cl₂ / MeOH (9:1) and then further purified by Prep-HPLC with the following conditions (Column: XBridge Shield RP18 OBD Column, 30*150 mm, 5µm; Mobile Phase A: water (10 mmol/L NH₄HCO₃), Mobile Phase B: ACN; Flow rate: 60 mL/min; Gradient: 29% B to 46% B in 10 min; Wave Length: 254/220 nm; RT1(min): 8.50) to afford Example 66 (1 mg, 2%). LCMS (ESI) m/z: 377.05 [M+H]⁺.

### Example 69: 5-chloro-3-(6-fluoropyridin-3-yl)-2-(4-(4-methyl-4H-1,2,4-triazol-3-yl)piperidin-1-yl)benzonitrile

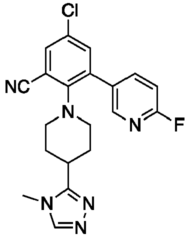

The title compound was prepared using the following procedure:

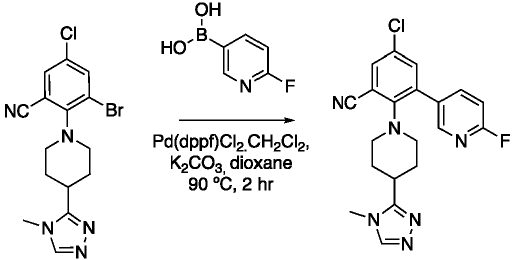

A solution of Intermediate 13 (160 mg, 0.42 mmol), K₂CO₃ (174.3 mg, 1.26 mmol) and Pd(dppf)Cl₂CH₂Cl₂ (34.2 mg, 0.042 mmol) in dioxane (5 mL) was stirred for 2 h at 90 °C under nitrogen atmosphere. The resulting mixture was concentrated under reduced pressure. The residue was purified by reversed-phase flash chromatography with the following conditions: column, C18 silica gel; mobile phase, MeCN in water (0.10% FA), 10% to 50% gradient in 10 min; detector, UV 254 nm and then by Prep-HPLC with the following conditions (Column: Xselect CSH F-Phenyl OBD column, 30*250 mm, 5µm; Mobile Phase A: water (0.05% FA), Mobile Phase B: ACN; Flow rate: 60 mL/min; Gradient: 5% B to 18% B in 10 min, 18% B; Wave Length: 254/220 nm; RT1(min): 10.68; to afford Example 69 (6.4 mg, 4%). LCMS (ESI) m/z: 397.05 [M+H]⁺.

### Example 70: 3-(6-fluoropyridin-3-yl)-2-(4-(4-methyl-4H-1,2,4-triazol-3-yl)piperidin-1-yl)-5-(trifluoromethyl)benzonitrile

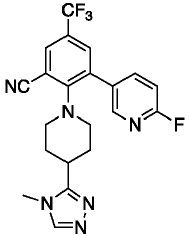

The title compound was prepared using the following procedure:

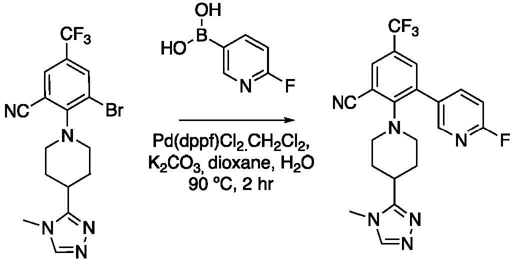

To a stirred mixture of Intermediate 14 (50 mg, 0.12 mmol), 6-fluoropyridin-3-ylboronic acid (20.4 mg, 0.14 mmol) in H₂O (1 mL) and 1,4-dioxane (4 mL) were added K₂CO₃ (33.4 mg, 0.24 mmol) and Pd(dppf)Cl₂.CH₂Cl₂ (10 mg) in portions at room temperature. The resulting mixture was stirred for 2 h at 90 °C under nitrogen atmosphere. The residue was purified by Prep-TLC (10% MeOH / CH₂Cl₂). The crude product was purified by Prep-HPLC with the following conditions (Column: YMC-Actus Triart C18 ExRS, 30*150 mm, 5µm; Mobile Phase A: water (10 mmol/L NH₄HCO₃+ 0.1% NH₃.H₂O), Mobile Phase B: ACN; Flow rate: 60 mL/min; Gradient: 25% B to 45% B in 10 min; Wave Length: 254/220 nm; RT1(min): 12.27) to afford Example 70 (6 mg, 12%). LCMS (ESI) m/z: 431.15 [M+H]⁺.

### Example 71: 6-fluoro-3-(6-fluoropyridin-3-yl)-2-(4-(4-methyl-4H-1,2,4-triazol-3-yl)piperidin-1-yl)benzonitrile

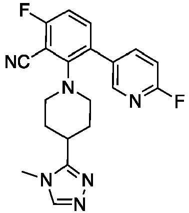

The title compound was prepared using the following procedure:

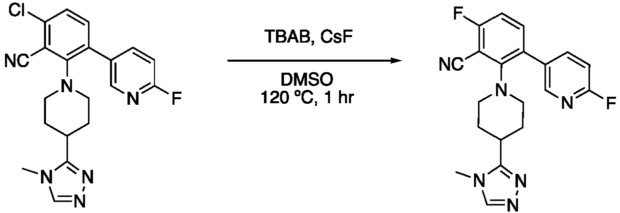

A solution of Example 30 (60 mg, 0.15 mmol), CsF (229.6 mg, 1.51 mmol) and TBAB (4.87 mg, 0.015 mmol) in DMSO (3 mL) was stirred for 1 h at 120 °C. The reaction was quenched with water at room temperature. The resulting mixture was extracted with EtOAc (3 x 20 mL). The combined organic layers were washed with brine (3 x 5 mL), dried over anhydrous Na₂SO₄. The residue was purified by reversed-phase flash chromatography with the following conditions: column, C18 silica gel; mobile phase, MeCN in water (0.1% FA), 10% to 50% gradient in 10 min; detector, UV 254 nm followed by Prep-HPLC with the following conditions (Column: Xselect CSH F-Phenyl OBD column, 30*250 mm, 5µm; Mobile Phase A: water (0.05% FA), Mobile Phase B: ACN; Flow rate: 60 mL/min; Gradient: 5% B to 18% B in 10 min, 18% B; Wave Length: 254/220 nm; RT1(min): 10.68; to afford Example 71 (2.2 mg, 4%). LCMS (ESI) m/z: 381.05 [M+H]⁺.

### Example 72: 2-(4-(1,3,4-thiadiazol-2-yl)piperidin-1-yl)-3-(6-fluoropyridin-3-yl)benzonitrile

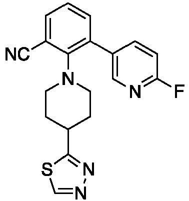

The title compound was prepared using the following procedure:

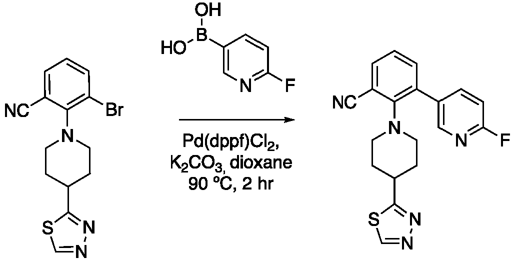

A solution of 3 Intermediate 15 (160 mg, 0.46 mmol), 6-fluoropyridin-3-ylboronic acid (71 mg, 0.50 mmol), K₂CO₃ (126.6 mg, 0.92 mmol) and Pd(dppf)Cl₂ (33.5 mg, 0.05 mmol) in dioxane (2 mL) was stirred for 2 h at 90 °C under nitrogen atmosphere. The reaction was quenched with water at room temperature. The resulting mixture was extracted with EtOAc (2 x 20 mL). The combined organic layers were washed with brine (2 x 5 mL), dried over anhydrous Na₂SO₄. After filtration, the filtrate was concentrated under reduced pressure. The crude product was purified by Prep-HPLC with the following conditions (Column: Xselect CSH F-Phenyl OBD column, 30*250 mm, 5µm; Mobile Phase A: water (0.05% FA), Mobile Phase B: ACN; Flow rate: 60 mL/min; Gradient: 5% B to 18% B in 10 min, 18% B; Wave Length: 254/220 nm; RT1(min): 10.68; to afford Example 72 (3 mg, 2%). LCMS (ESI) m/z: 366.00 [M+H]⁺.

### Example 75: 3-(6-fluoropyridin-3-yl)-2-((1R,5S)-6-(4-methyl-4H-1,2,4-triazol-3-yl)-3-azabicyclo[3.1.0]hexan-3-yl)benzonitrile

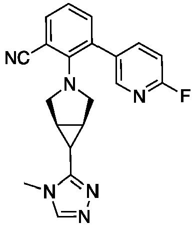

The title compound was prepared using the following procedure:

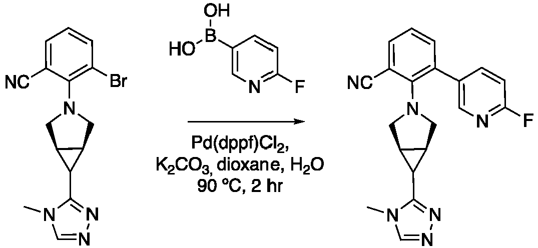

A solution of Intermediate 16 (90 mg, 0.26 mmol), 6-fluoropyridin-3-ylboronic acid (44.2 mg, 0.31 mmol), K₂CO₃ (108.4 mg, 0.78 mmol) and Pd(dppf)Cl₂ (19.1 mg, 0.026 mmol) in 1,4-dioxane (12 mL) and H₂O (1.2 mL) was stirred for 2 h at 90 °C under nitrogen atmosphere. The reaction was quenched with water at room temperature. The resulting mixture was extracted with EtOAc (3 x 50 mL). The combined organic layers were washed with brine (2 x 50 mL), dried over anhydrous Na₂SO₄. After filtration, the filtrate was concentrated under reduced pressure. The crude product was purified by Prep-HPLC with the following conditions (Column: XBridge Prep Phenyl OBD Column, 19*250 mm, 5µm; Mobile Phase A: water (10 mmol/L NH₃.H₂O), Mobile Phase B: ACN; Flow rate: 50 mL/min; Gradient: 13% B to 30% B in 8 min; Wave Length: 254/220 nm; RT1(min): 9.32; to afford Example 75 (10.8 mg, 11%). LCMS (ESI) m/z: 361.10 [M+H]⁺.

### Example 76: 6-cyclopropoxy-3-(6-fluoropyridin-3-yl)-2-(4-(4-methyl-4H-1,2,4-triazol-3-yl)piperidin-1-yl)benzonitrile

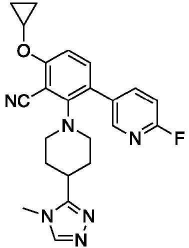

The title compound was prepared using the following procedure:

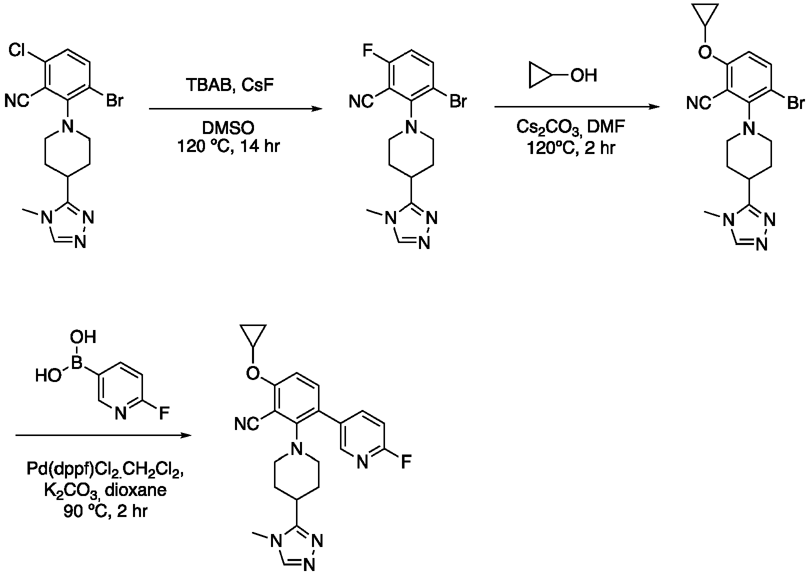

A solution of Intermediate 6 (1 g, 2.63 mmol), TBAB (0.08 g, 0.26 mmol) and CsF (3.99 g, 26.3 mmol) in DMSO (5 mL) was stirred overnight at 120 °C. The reaction was quenched with water at room temperature. The resulting mixture was extracted with EtOAc (3 x 300 mL). The combined organic layers were washed with brine (3 x 150 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm to afford 3-bromo-6-fluoro-2-[4-(4-methyl-1,2,4-triazol-3-yl) piperidin-1-yl] benzonitrile (500 mg, 52%) as a yellow oil. LCMS (ESI) m/z: 364, 366 [M+H]⁺.

A solution of 3-bromo-6-fluoro-2-[4-(4-methyl-1,2,4-triazol-3-yl) piperidin-1-yl] benzonitrile (150 mg, 0.41 mmol), cyclopropanol (35.9 mg, 0.62 mmol) and Cs₂CO₃ (201.3 mg, 0.62 mmol) in DMF (4 mL) was stirred for 2 h at 120 °C. The reaction was quenched with water at room temperature. The resulting mixture was extracted with EtOAc (3 x 100 mL). The combined organic layers were washed with brine (3 x 50 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm to afford 3-bromo-6-cyclopropoxy-2-[4-(4-methyl-1,2,4-triazol-3-yl) piperidin-1-yl] benzonitrile (60 mg, 36%) as a yellow oil. LCMS (ESI) m/z: 402, 404 [M+H]⁺.

A solution of 3-bromo-6-cyclopropoxy-2-[4-(4-methyl-1,2,4-triazol-3-yl) piperidin-1-yl] benzonitrile (50 mg, 0.12 mmol), K₂CO₃ (34.3 mg, 0.25 mmol) and Pd(dppf)Cl₂CH₂Cl₂ (10.1 mg, 0.01 mmol) in dioxane (2 mL) was stirred for 2 h at 90 °C under nitrogen atmosphere. The reaction was quenched with water at room temperature. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm. The crude product was purified by Prep-HPLC with the following conditions (Column: Xselect CSH F-Phenyl OBD column, 30*250 mm, 5µm; Mobile Phase A: water (0.05% FA), Mobile Phase B: ACN; Flow rate: 60 mL/min; Gradient: 5% B to 18% B in 10 min, 18% B; Wave Length: 254/220 nm; RT1(min): 10.68; to afford Example 76 (4.4 mg, 8%). LCMS (ESI) m/z: 419.25 [M+H]⁺.

### Example 78: 6-amino-3-(6-fluoropyridin-3-yl)-2-(4-(4-methyl-4H-1,2,4-triazol-3-yl)piperidin-1-yl)benzonitrile

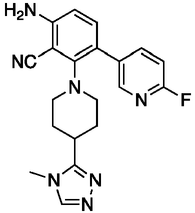

The title compound was prepared using the following procedure:

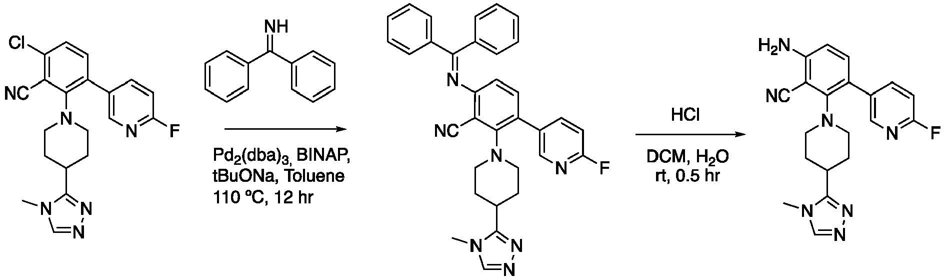

A mixture of Example 30 (130 mg, 0.33 mmol), Pd₂(dba)₃ (45 mg, 0.049 mmol), BINAP (61.2 mg, 0.098 mmol) and t-BuONa (47.2 mg, 0.49 mmol) in toluene (6 mL) was stirred for 12 h at 110 °C under nitrogen atmosphere. The resulting mixture was concentrated under reduced pressure. The residue was purified by silica gel column chromatography, eluted with CH₂Cl₂ / MeOH (12:1) to afford 6-[(diphenylmethylidene)amino]-3-(6-fluoropyridin-3-yl)-2-[4-(4-methyl-1,2,4-triazol-3-yl)piperidin-1-yl]benzonitrile (70 mg, 39%) as orange oil. The crude product was used in the next step directly without further purification. LCMS (ESI) m/z: 542 [M+H]⁺.

Into a round-bottom flask were added 6-[(diphenylmethylidene)amino]-3-(6-fluoropyridin-3-yl)-2-[4-(4-methyl-1,2,4-triazol-3-yl)piperidin-1-yl]benzonitrile (60 mg, 0.11 mmol), HCl (1 mL), DCM (6 mL) and H₂O (6 mL) at room temperature. The mixture was stirred for 0.5 h at room temperature. The mixture was basified to pH 8 with saturated NaHCO₃ (aq.). The resulting mixture was extracted with DCM (3 x 20 mL). The combined organic layers were washed with water (20 mL), dried over anhydrous Na₂SO₄. After filtration, the filtrate was concentrated under reduced pressure. The residue was purified by silica gel column chromatography, eluted with CH₂Cl₂ / MeOH (10:1) and then repurified by Prep-HPLC with the following conditions (Column: Sunfire prep C18 column, 30*150 mm, 5µm; Mobile Phase A: water (0.1% NH₄HCO₃), Mobile Phase B: ACN; Flow rate: 60 mL/min; Gradient: 25% B to 42% B in 9 min, 42% B; Wave Length: 254/220 nm; RT1(min): 8.97 ) to afford Example 78 (5.1 mg, 12%). LCMS (ESI) m/z: 378.25 [M+H]⁺.

### Example 79: 2-cyano-4-(6-fluoropyridin-3-yl)-N,N-dimethyl-3-(4-(4-methyl-4H-1,2,4-triazol-3-yl)piperidin-1-yl)benzamide

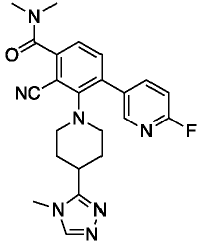

The title compound was prepared using the following procedure:

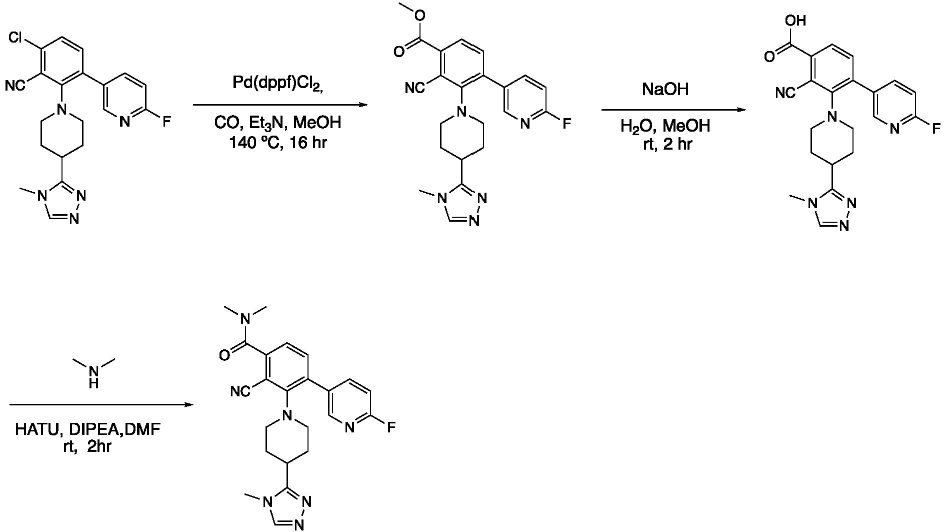

To a solution of Example 30 (150 mg, 0.38 mmol) in MeOH (5 mL) was added Pd(dppf)Cl₂ (27.7 mg, 0.038 mmol) and TEA (114.8 mg, 1.13 mmol) in a pressure tank. The mixture was purged with nitrogen for 10 min and then was pressurized to 20 atm with carbon monoxide at 140 °C overnight. The reaction mixture was cooled to room temperature and filtered to remove insoluble solids. The resulting mixture was extracted with EtOAc (2 x 80 mL). The combined organic layers were washed with brine (2 x 5 mL), dried over anhydrous Na₂SO₄. After filtration, the filtrate was concentrated under reduced pressure. The residue was purified by silica gel column chromatography, eluted with MeOH/CH₂Cl₂ (0%-10%) to afford methyl 2-cyano-4-(6-fluoropyridin-3-yl)-3-[4-(4-methyl-1,2,4-triazol-3-yl)piperidin-1-yl]benzoate (104 mg) as a red solid. LCMS (ESI) m/z: 421 [M+H]⁺.

A mixture of methyl 2-cyano-4-(6-fluoropyridin-3-yl)-3-[4-(4-methyl-1,2,4-triazol-3-yl)piperidin-1-yl]benzoate (90 mg, 0.21 mmol) and NaOH (17.1 mg, 0.43 mmol) in H₂O (3 mL) and MeOH (3 mL) was stirred for 2 h at room temperature. The residue was purified by Prep-TLC (10% MeOH/CH₂Cl₂) to afford 2-cyano-4-(6-fluoropyridin-3-yl)-3-[4-(4-methyl-1,2,4-triazol-3-yl)piperidin-1-yl]benzoic acid (40 mg) as a pink solid. LCMS (ESI) m/z: 407 [M+H]⁺.

To a stirred mixture of 2-cyano-4-(6-fluoropyridin-3-yl)-3-[4-(4-methyl-1,2,4-triazol-3-yl)piperidin-1-yl]benzoic acid (40 mg, 0.098 mmol) and dimethylamine (5.3 mg, 0.19 mmol) in DMF (3 mL) were added HATU (44.9 mg, 0.12 mmol) and DIEA (50.9 mg, 0.39 mmol) in portions at room temperature. The resulting mixture was stirred for 2 h at room temperature. The resulting mixture was extracted with EtOAc (2 x 80 mL). The combined organic layers were washed with brine (2 x10 mL), dried over anhydrous Na₂SO₄. After filtration, the filtrate was concentrated under reduced pressure. The crude product was purified by Prep-HPLC with the following conditions (Column: XBridge Prep Phenyl OBD Column, 19*250 mm, 5µm; Mobile Phase A: water (0.1% FA), Mobile Phase B: ACN; Flow rate: 50 mL/min; Gradient: 15% B to 32% B in 8 min, 32% B; Wave Length: 254/220 nm; RT1(min): 6.75; to afford Example 79 (10 mg, 23%). LCMS (ESI) m/z: 434.00 [M+H]⁺.

### Example 80: 3-(6-fluoropyridin-3-yl)-2-(4-(4-methyl-4H-1,2,4-triazol-3-yl)piperidin-1-yl)-6-(4-methylpiperazin-1-yl)benzonitrile

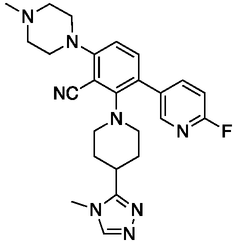

The title compound was prepared using the following procedure:

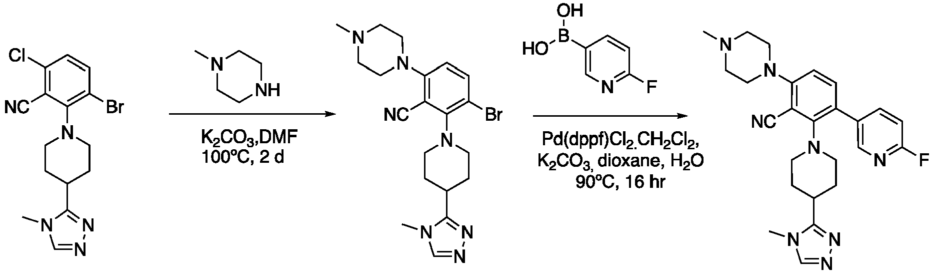

To a solution of Intermediate 6 (300 mg, 0.79 mmol) in 1-methylpiperazine (10 mL) was added K₂CO₃ (327 mg, 2.37 mmol) in portions at room temperature. The resulting mixture was stirred for 2 days at 100 °C. The resulting mixture was extracted with EtOAc (3 x 20 mL). The combined organic layers were washed with water (2 x 20 mL), dried over anhydrous Na₂SO₄. After filtration, the filtrate was concentrated under reduced pressure. The residue was purified by silica gel column chromatography, eluted with MeOH in CH₂Cl₂ (0%-10%) to afford 3-bromo-2-[4-(4-methyl-1,2,4-triazol-3-yl)piperidin-1-yl]-6-(4-methylpiperazin-1-yl)benzonitrile (100 mg, 28%) as a yellow solid. LCMS (ESI) m/z: 444, 446 [M+H]⁺.

To a stirred solution of 3-bromo-2-[4-(4-methyl-1,2,4-triazol-3-yl)piperidin-1-yl]-6-(4-methylpiperazin-1-yl)benzonitrile (100 mg, 0.22 mmol) and 6-fluoropyridin-3-ylboronic acid (63.4 mg, 0.45 mmol) in dioxane (10 mL) and H₂O (1 mL) were added Pd(dppf)Cl₂CH₂Cl₂ (18.3 mg, 0.023 mmol) and K₂CO₃ (93.3 mg, 0.68 mmol) in portions at room temperature under nitrogen atmosphere. The resulting mixture was stirred for 16 h at 90 °C under nitrogen atmosphere. The reaction was quenched by the addition of water (2 mL) at room temperature. The resulting mixture was extracted with EtOAc (2 x 20 mL). The combined organic layers were washed with water (10 mL), dried over anhydrous Na₂SO₄. After filtration, the filtrate was concentrated under reduced pressure. The crude product was purified by Prep-HPLC with the following conditions (Column: Aeris PEPTIDE 10 um XB-C18 Axia, 50 mm X 250 mm, 10 µ m; Mobile Phase A: water 0.1% NH₄HCO₃), Mobile Phase B: ACN; Flow rate: 100 mL/min; Gradient: 5% B to 35% B in 30 min, 20% B; Wave Length: 220/254 nm; RT1(min): 13.97; to afford Example 80 (10.5 mg, 10%). LCMS (ESI) m/z: 461.25 [M+H]⁺.

### Example 81: 6-(cyclopropylamino)-3-(6-fluoropyridin-3-yl)-2-(4-(4-methyl-4H-1,2,4-triazol-3-yl)piperidin-1-yl)benzonitrile

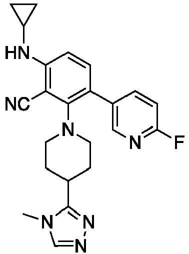

The title compound was prepared using the following procedure:

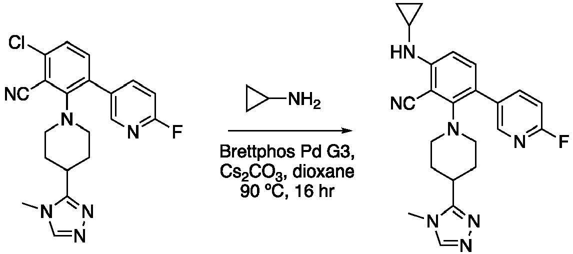

To a stirred solution Example 30 (200 mg, 0.5 mmol) and aminocyclopropane (43.2 mg, 0.76 mmol) in dioxane (20 mL) were added Cs₂CO₃ (494 mg, 1.51 mmol) and Brettphos Pd G3 (45.7 mg, 0.05 mmol) in portions at room temperature under nitrogen atmosphere. The resulting mixture was stirred for 16 h at 90 °C under nitrogen atmosphere. The reaction was quenched by the addition of water (5 mL) at room temperature. The resulting mixture was extracted with EtOAc (3 x 20 mL). The combined organic layers were washed with water (2 x 20 mL), dried over anhydrous Na₂SO₄. After filtration, the filtrate was concentrated under reduced pressure. The crude product was purified by Prep-HPLC with the following conditions (Column: Aeris PEPTIDE 5um XB-C18 Axia, 21.2 mm X 250 mm, 5 *µ* m; Mobile Phase A: water (10 mmol/L NH₄HCO₃+ 0.1% NH₃.H₂O), Mobile Phase B: ACN; Flow rate: 60 mL/min; Gradient: 22% B to 40% B in 10 min, 40% B; Wave Length: 220/254 nm; RT1(min): 13.97; to afford Example 81 (1.2 mg, 0.6%). LCMS (ESI) m/z: 418.30 [M+H]⁺.

### Example 82: 3-(6-fluoropyridin-3-yl)-2-(4-(4-methyl-4H-1,2,4-triazol-3-yl)piperidin-1-yl)-6-(2-(methylamino)ethoxy)benzonitrile

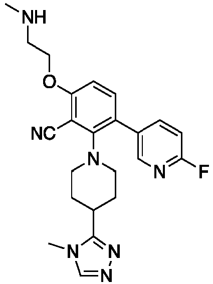

The title compound was prepared using the following procedure:

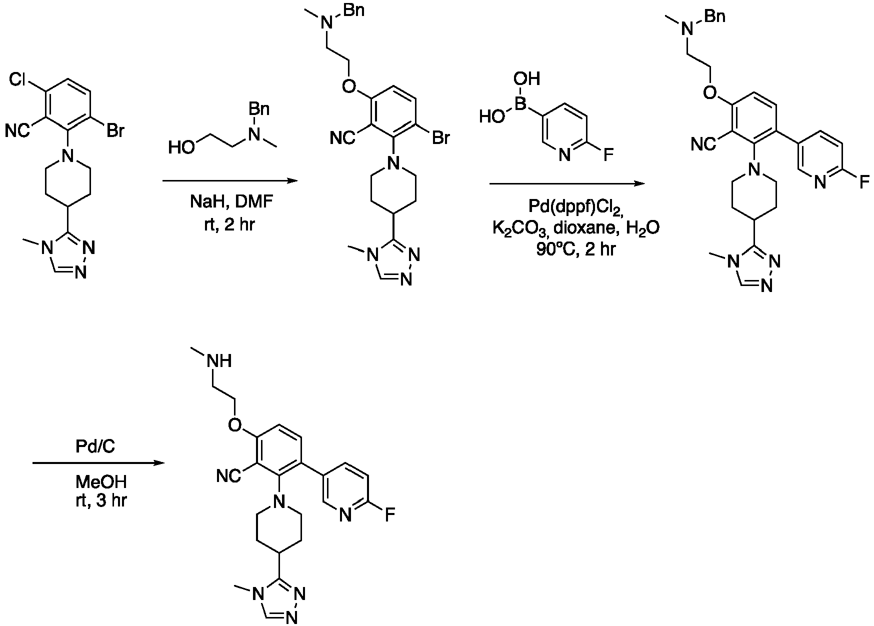

A mixture of 2-[benzyl(methyl)amino]ethanol (138.9 mg, 0.84 mmol) and NaH (30.3 mg, 1.26 mmol) in DMF (8 mL) was stirred for 30 min at 0 °C under nitrogen atmosphere. To the above mixture added Intermediate 6 (400 mg, 1.05 mmol) at 0 °C. The resulting mixture was stirred for additional 2 h at room temperature. The residue was purified by reversed-phase flash chromatography with the following conditions: column, C18 silica gel; mobile phase, MeCN in water (0.1% FA), 10% to 30% gradient in 15 min; detector, UV 254 nm, to afford 6-{2-[benzyl(methyl)amino]ethoxy}-3-bromo-2-[4-(4-methyl-1,2,4-triazol-3-yl)piperidin-1-yl]benzonitrile (370 mg, 69%). LCMS (ESI) m/z: 509, 511 [M+H]⁺.

To a stirred solution of 6-{2-[benzyl(methyl)amino]ethoxy}-3-bromo-2-[4-(4-methyl-1,2,4-triazol-3-yl)piperidin-1-yl]benzonitrile (200 mg, 0.39 mmol) in dioxane (4 mL) and H₂O (1 mL) were added K₂CO₃ (162.8 mg, 1.18 mmol) 6-fluoropyridin-3-ylboronic acid (83 mg, 0.59 mmol) and Pd(dppf)Cl₂ (28.7 mg, 0.039 mmol). The resulting mixture was stirred for 2 h at 90 °C under nitrogen atmosphere. The crude mixture was concentrated under reduced pressure. The residue was purified by reversed-phase flash chromatography with the following conditions: column, C18 silica gel; mobile phase, MeCN in water (0.1% FA), 20% to 40% gradient in 20 min; detector, UV 254 nm. to afford 6-{2-[benzyl(methyl)amino]ethoxy}-3-(6-fluoropyridin-3-yl)-2-[4-(4-methyl-1,2,4-triazol-3-yl)piperidin-1-yl]benzonitrile (160 mg, 77%) as a yellow oil. LCMS (ESI) m/z: 526 [M+H]⁺.

To a stirred solution of 6-{2-[benzyl(methyl)amino]ethoxy}-3-(6-fluoropyridin-3-yl)-2-[4-(4-methyl-1,2,4-triazol-3-yl)piperidin-1-yl]benzonitrile (100 mg, 0.19 mmol) in MeOH (10 mL) were added Pd/C (50.6 mg, 0.47 mmol) in portions at room temperature under nitrogen atmosphere. The resulting mixture was stirred for 3 h at room temperature under hydrogen atmosphere. The mixture was filtered, the filter cake was washed with MeOH (2 x 20 mL). The filtrate was concentrated under reduced pressure. The crude product was purified by Prep-HPLC with the following conditions (Column: XBridge Prep C18 OBD Column, 19*150 mm, 5µm; Mobile Phase A: water (10 mmol/L NH₄HCO₃), Mobile Phase B: ACN; Flow rate: 60 mL/min; Gradient: 6% B to 23% B in 10 min, 23% B; Wave Length: 200/220 nm; RT1(min): 12.73; to afford Example 82 (12.3 mg, 14%). LCMS (ESI) m/z: 436.10 [M+H]⁺.

### Example 84: N-(2-cyano-4-(6-fluoropyridin-3-yl)-3-(4-(4-methyl-4H-1,2,4-triazol-3-yl)piperidin-1-yl)phenyl)methanesulfonamide

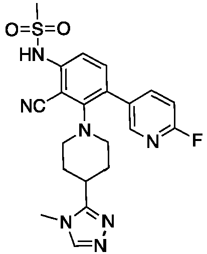

The title compound was prepared using the following procedure:

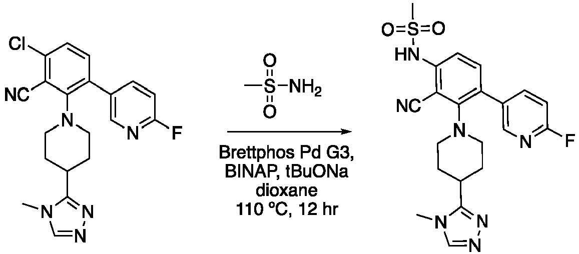

A mixture of Example 30 (100 mg, 0.25 mmol), methanesulfonamide (47.9 mg, 0.50 mmol), BINAP (15.7 mg, 0.025 mmol), t-BuONa (36.3 mg, 0.38 mmol) and BrettPhos Pd G3 (22.8 mg, 0.025 mmol) in 1,4-dioxane was stirred for 12 h at 110 °C under nitrogen atmosphere. The mixture was allowed to cool down to room temperature. The resulting mixture was concentrated under reduced pressure. The crude product was purified by Prep-HPLC with the following conditions (Column: Sunfire prep C18 column, 30*150 mm, 5µm; Mobile Phase A: water (0.1% FA), Mobile Phase B: ACN; Flow rate: 60 mL/min; Gradient: 25% B to 42% B in 9 min, 42% B; Wave Length: 254/220 nm; RT1(min): 8.97.) to afford Example 84 (12.5 mg, 11%). LCMS (ESI) m/z: 456.00 [M+H]⁺.

### Example 85: 3-(6-fluoropyridin-3-yl)-2-(4-(4-methyl-4H-1,2,4-triazol-3-yl)piperidin-1-yl)-6-(piperazin-1-yl)benzonitrile

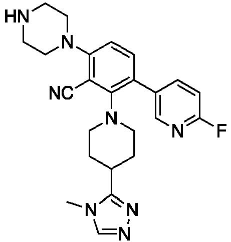

The title compound was prepared using the following procedure:

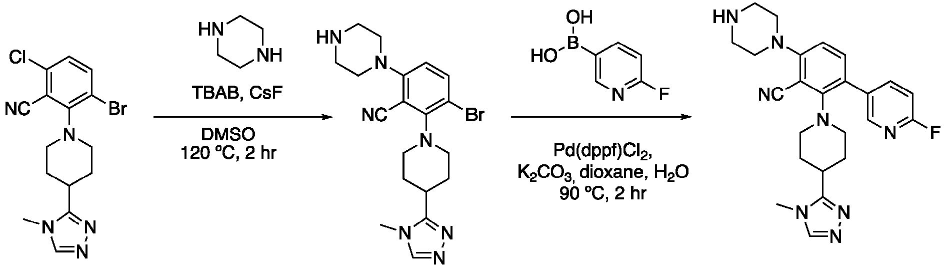

To a stirred solution of Intermediate 6 (300 mg, 0.79 mmol) in DMSO (10 mL) were added CsF (838 mg, 5.52 mmol), TBAB (25.4 mg, 0.079 mmol) and piperazine (135.7 mg, 1.58 mmol) in portions at room temperature. The resulting mixture was stirred for 2 h at 120 °C under nitrogen atmosphere. The residue was purified by reversed-phase flash chromatography with the following conditions: column, C18 silica gel; mobile phase, MeCN in water (0.1% FA), 10% to 40% gradient in 20 min; detector, UV 254 nm. to afford 3-bromo-2-[4-(4-methyl-1,2,4-triazol-3-yl)piperidin-1-yl]-6-(piperazin-1-yl)benzonitrile (240 mg, 71%) as a brown yellow oil.

To a stirred solution of 3-bromo-2-[4-(4-methyl-1,2,4-triazol-3-yl)piperidin-1-yl]-6-(piperazin-1-yl)benzonitrile (200 mg, 0.46 mmol) in dioxane (4 mL) and H₂O (1 mL) were added K₂CO₃ (192.7 mg, 1.39 mmol) 6-fluoropyridin-3-ylboronic acid (98.2 mg, 0.7 mmol) and Pd(dppf)Cl₂ (34 mg, 0.047 mmol) in portions at room temperature. The resulting mixture was stirred for 2 h at 90 °C under nitrogen atmosphere. The resulting mixture was concentrated under reduced pressure. The residue was purified by reversed-phase flash chromatography with the following conditions: column, C18 silica gel; mobile phase, MeCN in water (0.1% FA), 30% to 50% gradient in 25 min; detector, UV 254 nm. to afford a brown oil as a crude product. The crude product was purified by Prep-HPLC with the following conditions (Column: YMC-Actus Triart C18 ExRS, 30*150 mm, 5µm; Mobile Phase A: water (10 mmol/L NH₄HCO₃), Mobile Phase B: ACN; Flow rate: 60 mL/min; Gradient: 6% B to 23% B in 9 min, 23% B; Wave Length: 254/220 nm; RT1(min): 15.72; to afford Example 85 (56.3 mg, 27%). LCMS (ESI) m/z: 447.35 [M+H]⁺.

### Examples 90 and 91: 3-(6-fluoropyridin-3-yl)-2-(4-(4-methyl-4H-1,2,4-triazol-3-yl)piperidin-1-yl)-6-((1-methylpyrrolidin-3-yl)oxy)benzonitrile (Isomers A and B)

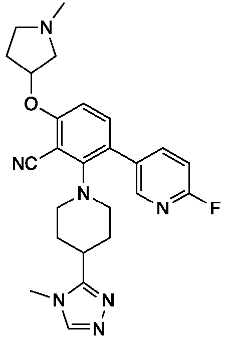

The title compounds were prepared using the following procedure:

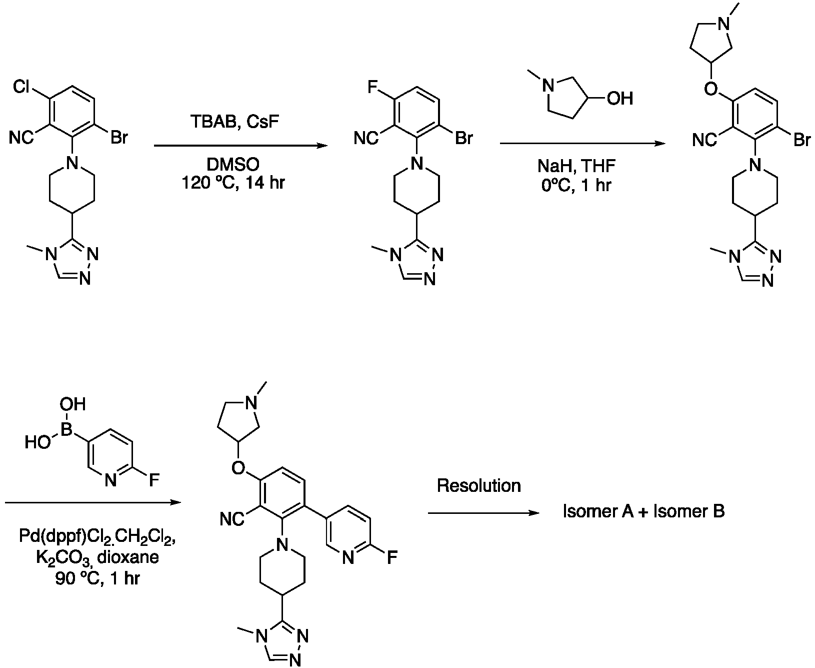

A solution of Intermediate 6 (1 g, 2.63 mmol), TBAB (0.08 g, 0.26 mmol) and CsF (3.99 g, 26.3 mmol) in DMSO (5 mL) was stirred overnight at 120 °C. The reaction was quenched with water (150 mL) at room temperature. The resulting mixture was extracted with EtOAc (3 x 300 mL). The combined organic layers were washed with brine (3 x 150 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm to afford 3-bromo-6-fluoro-2-[4-(4-methyl-1,2,4-triazol-3-yl) piperidin-1-yl] benzonitrile (500 mg, 52%) as a yellow oil.

A solution of 5-bromo-2-fluorobenzonitrile (200 mg, 1 mmol), 1-methylpyrrolidin-3-ol (61.1 mg, 0.6 mmol) and NaH (26.4 mg, 1.1 mmol) in THF (6 mL) was stirred for 1 h at 0 °C. The reaction was quenched with water at room temperature. The resulting mixture was filtered, the filter cake was washed with THF (3 x 10 mL). The filtrate was concentrated under reduced pressure. The resulting mixture was extracted with EtOAc (3 x 200 mL). The combined organic layers were washed with brine (3 x 100 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm to afford 3-bromo-2-[4-(4-methyl-1,2,4-triazol-3-yl) piperidin-1-yl]-6-[(1-methylpyrrolidin-3-yl) oxy] benzonitrile (190 mg, 78%) as a yellow solid.

A solution of 3-bromo-2-[4-(4-methyl-1,2,4-triazol-3-yl) piperidin-1-yl]-6-(pyrrolidin-3-yloxy) benzonitrile (100 mg, 0.23 mmol) and Pd(dppf)Cl₂CH₂Cl₂ (18.9 mg, 0.023 mmol) in dioxane (10 mL) was stirred for 1 h at 90 °C under nitrogen atmosphere. The reaction was quenched with water at room temperature. The resulting mixture was extracted with EtOAc (3 x 100 mL). The combined organic layers were washed with brine (3 x 50 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (0.1% NH₃.H₂O), 10% to 50% gradient in 10 min; detector, UV 254 nm. The crude product was purified by Prep-HPLC with the following conditions (Column: XBridge Prep C18 OBD Column, 19*150 mm, 5µm; Mobile Phase A: water (10 mmol/L NH₄HCO₃), Mobile Phase B: ACN; Flow rate: 60 mL/min; Gradient: 12% B to 30% B in 10 min; Wave Length: 220 nm; RT1(min): 12.55) to afford 3-(6-fluoropyridin-3-yl)-2-[4-(4-methyl-1,2,4-triazol-3-yl)piperidin-1-yl]-6-[(1-methylpyrrolidin-3-yl)oxy] benzonitrile (30 mg) as a white solid. The product was purified by Chiral resolution with the following conditions (Column: CHIRAL ART Cellulose-SZ, 4.6*50mm, 3µm; Mobile Phase A: Hex (0.1% DEA): EtOH=50: 50; Flow rate: 1 mL/min; Gradient: isocratic; Injection Volume: 5ul mL) to afford Example 90 (6.4 mg, 7%) and Example 91 (7.4 mg, 7%). LCMS (ESI) m/z: 462.40 [M+H]⁺.

### Example 92: 6-(3-amino-1H-pyrazol-4-yl)-3-(6-fluoropyridin-3-yl)-2-(4-(4-methyl-4H-1,2,4-triazol-3-yl)piperidin-1-yl)benzonitrile

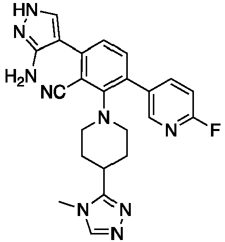

The title compound was prepared using the following procedure:

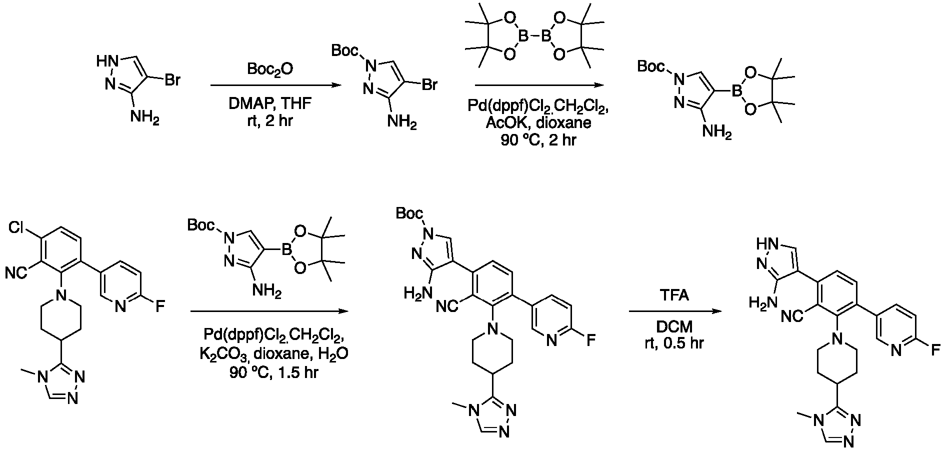

To a stirred mixture of 4-bromo-1H-pyrazol-3-amine (500 mg, 3.09 mmol) and di-tert-butyl dicarbonate (1347.3 mg, 6.17 mmol) in THF (15 mL) was added DMAP (37.7 mg, 0.31 mmol) in portions at room temperature. The resulting mixture was stirred for 2 h at room temperature. The reaction was quenched by the addition of water (50 mL) and extracted with EtOAc (3 x 150 mL). The combined organic layers were washed with brine (2 x 10 mL) and dried over anhydrous Na₂SO₄. After filtration, the filtrate was concentrated under reduced pressure. The residue was purified by silica gel column chromatography, eluted with CH₂Cl₂ / MeOH (0%-10%) to afford tert-butyl 3-amino-4-bromopyrazole-1-carboxylate (500 mg) as a white solid.

To a stirred mixture of tert-butyl 3-amino-4-bromopyrazole-1-carboxylate (200 mg, 0.76 mmol) and bis(pinacolato)diboron (775.1 mg, 3.05 mmol) in 1,4-dioxane (10 mL) was added AcOK (224.7 mg, 2.3 mmol) and Pd(dppf)Cl₂CH₂Cl₂ (62.2 mg, 0.076 mmol) in portions at room temperature. The resulting mixture was stirred for 2 h at 90 °C under nitrogen atmosphere. The reaction was quenched by the addition of water (20 mL) at room temperature. The resulting mixture was extracted with EtOAc (3 x 100 mL). The combined organic layers were washed with brine (2 x15 mL), dried over anhydrous Na₂SO₄. After filtration, the filtrate was concentrated under reduced pressure. The residue was purified by Prep-TLC (10% MeOH/ CH₂Cl₂) to afford tert-butyl 3-amino-4-(4,4,5,5-tetramethyl-1,3,2-dioxaborolan-2-yl)pyrazole-1-carboxylate (220 mg) as an off-white solid.

To a stirred mixture of tert-butyl 3-amino-4-(4,4,5,5-tetramethyl-1,3,2-dioxaborolan-2-yl)pyrazole-1-carboxylate (220 mg, 0.71 mmol) and Example 30 (225.9 mg, 0.57 mmol) in dioxane (5 mL) and H₂O (1 mL) were added K₂CO₃ (295 mg, 2.14 mmol) and Pd(dppf)Cl₂CH₂Cl₂ (52.2 mg, 0.064 mmol) in portions at room temperature. The resulting mixture was stirred for 1.5 h at 90 °C under nitrogen atmosphere. The residue was purified by silica gel column chromatography, eluted with MeOH/ CH₂Cl₂ (0%-10%) to afford tert-butyl 3-amino-4-[2-cyano-4-(6-fluoropyridin-3-yl)-3-[4-(4-methyl-1,2,4-triazol-3-yl)piperidin-1-yl]phenyl]pyrazole-1-carboxylate (317 mg) as a brown solid.

A mixture of tert-butyl 3-amino-4-[2-cyano-4-(6-fluoropyridin-3-yl)-3-[4-(4-methyl-1,2,4-triazol-3-yl)piperidin-1-yl]phenyl]pyrazole-1-carboxylate (300 mg, 0.55 mmol) in TFA (1 mL) and DCM (5 mL) was stirred for 0.5 h at room temperature. The residue was purified by reversed-phase flash chromatography with the following conditions: column, C18 silica gel; mobile phase, MeCN in water (0.1% TFA), 10% to 50% gradient in 10 min; detector, UV 254 nm. The crude product was purified by Prep-HPLC with the following conditions (Column: XBridge Shield RP18 OBD Column, 30*150 mm, 5µm; Mobile Phase A: water (10 mmol/L NH₄HCO₃), Mobile Phase B: ACN; Flow rate: 60 mL/min; Gradient: 4% B to 24% B in 8 min, 24% B; Wave Length: 254/220 nm; RT1(min): 11.58; to afford Example 92 (25 mg, 6%). LCMS (ESI) m/z: 444.30 [M+H]⁺.

### Example 93: 3-(imidazo[1,2-a]pyrimidin-6-yl)-2-(4-(4-methyl-4H-1,2,4-triazol-3-yl)piperidin-1-yl)benzonitrile

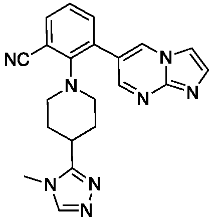

The title compound was prepared using the following procedure:

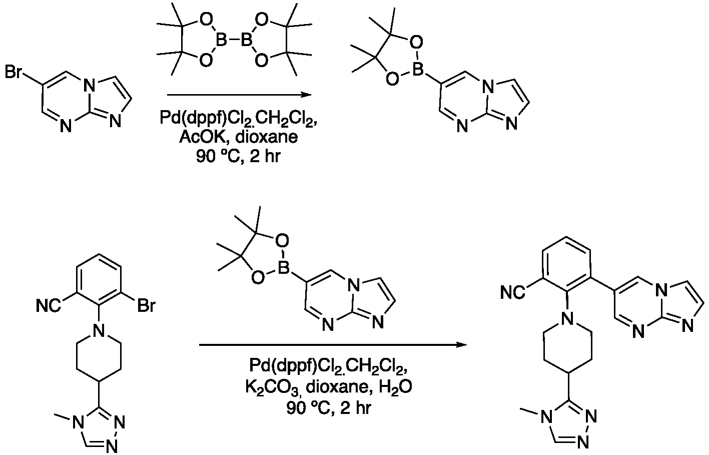

To a stirred solution of 6-bromoimidazo[1,2-a]pyrimidine (50 mg, 0.25 mmol) in 1,4-dioxane (5 mL) was added bis(pinacolato)diboron (96.2 mg, 0.38 mmol), Pd(dppf)Cl₂CH₂Cl₂ (20.6 mg, 0.025 mmol) and AcOK (74.3 mg, 0.76 mmol) in portions at room temperature. The resulting mixture was stirred for 2 h at 90 °C under nitrogen atmosphere. The resulting mixture was evaporated in vacuo and used in the next step directly without further purification.

To a stirred mixture of Intermediate 1 (50 mg, 0.14 mmol) and 6-(4,4,5,5-tetramethyl-1,3,2-dioxaborolan-2-yl)imidazo[1,2-a]pyrimidine (35.5 mg, 0.14 mmol) in H₂O (0.5 mL) was added K₂CO₃ (60 mg, 0.42 mmol) and Pd(dppf)Cl₂CH₂Cl₂ (11.8 mg, 0.014 mmol) in portions at room temperature. The resulting mixture was stirred for 2 h at 90 °C under nitrogen atmosphere. The crude product was purified by Prep-HPLC with the following conditions (Column: XBridge Shield RP18 OBD Column, 30*150 mm, 5 *µ* m; Mobile Phase A: water (10 mmol/L NH₄HCO₃), Mobile Phase B: ACN; Flow rate: 60 mL/min; Gradient: 4% B to 24% B in 8 min; Wave Length: 254/220 nm; RT1(min): 11.58) to afford Example 93 (43 mg, 77%). LCMS (ESI) m/z: 385.10 [M+H]⁺.

### Example 95: 3-(6-fluoropyridin-3-yl)-6-(3-hydroxyazetidin-1-yl)-2-(4-(4-methyl-4H-1,2,4-triazol-3-yl)piperidin-1-yl)benzonitrile

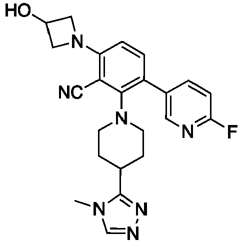

The title compound was prepared using the following procedure:

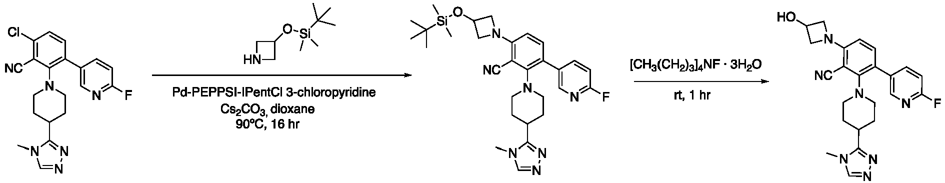

A mixture of Example 30 (200 mg, 0.5 mmol) and Cs₂CO₃ (328.4 mg, 1 mmol) in dioxane (5 mL) was stirred for 5 min at room temperature. To the above mixture were added Pd-PEPPSI-IPentCl 3-chloropyridine (49 mg, 0.05 mmol) and 3- [(tertbutyldimethylsilyl) oxy] azetidine (188.8 mg, 1 mmol). The resulting mixture was stirred overnight at 90 °C under nitrogen atmosphere. The reaction was quenched with water and extracted with EtOAc (400 mL). The combined organic layers were washed with brine (400 mL), dried over anhydrous Na₂SO₄. After filtration, the filtrate was concentrated under reduced pressure. The residue was purified by silica gel column chromatography, eluted with CH₂Cl₂ / MeOH (9:1) to afford 6-{3-[(tert-butyldimethylsilyl) oxy] azetidin-1-yl}-3-(6-fluoropyridin-3-yl)-2-[4-(4-methyl-1,2,4-triazol-3-yl) piperidin-1-yl] benzonitrile (196 mg, 71%) as a brown solid. LCMS (ESI) m/z: 548 [M+H]⁺.

Into a vial were added 6-{3-[(tert-butyldimethylsilyl) oxy] azetidin-1-yl}-3-(6-fluoropyridin-3-yl)-2-[4-(4-methyl-1,2,4-triazol-3-yl) piperidin-1-yl] benzonitrile (160 mg, 0.29 mmol) and Tetrabutylammonium fluoride trihydrate (184.3 mg, 0.58 mmol) in THF (5 mL) at room temperature. The resulting mixture was stirred for 1 h at room temperature under nitrogen atmosphere. The reaction was quenched with water and extracted with EtOAc (300 mL). The combined organic layers were washed with brine (300 mL), dried over anhydrous Na₂SO₄. After filtration, the filtrate was concentrated under reduced pressure. The crude product was purified by Prep-HPLC with the following conditions (Column: Sunfire prep C18 column, 30*150 mm, 5 *µ* m; Mobile Phase A: water (0.1% FA), Mobile Phase B: ACN; Flow rate: 60 mL/min mL/min; Gradient: isocratic 10% B -28% B in 10 min; Wave Length: 254 nm/220 nm; RT1(min): 12.62) to afford Example 95 (14.7 mg, 11%). LCMS (ESI) m/z: 434.05 [M+H]⁺.

### Example 97: 6-acetyl-3-(6-fluoropyridin-3-yl)-2-(4-(4-methyl-4H-1,2,4-triazol-3-yl)piperidin-1-yl)benzonitrile

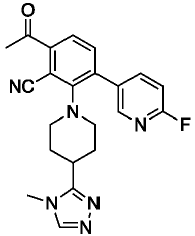

The title compound was prepared using the following procedure:

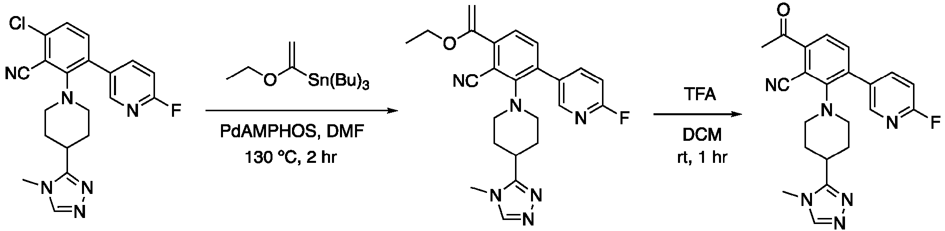

Into a vial were added Example 30 (320 mg, 0.81 mmol) and DMF (5 mL), PdAMPHOS (57.1 mg, 0.081 mmol) and tributyl(1-ethoxyethenyl) stannane (436.8 mg, 1.21 mmol). The resulting mixture was stirred for 2 h at 130 °C. The reaction was quenched with water and extracted with EtOAc (500 mL). The combined organic layers were washed with brine (500 mL), dried over anhydrous Na₂SO₄. After filtration, the filtrate was concentrated under reduced pressure. The residue was purified by silica gel column chromatography, eluted with CH₂Cl₂ / MeOH (9:1) to afford 6-(1-ethoxyethenyl)-3-(6-fluoropyridin-3-yl)-2-[4-(4-methyl-1,2,4-triazol-3-yl) piperidin-1-yl] benzonitrile (300 mg, 92%) as a brown solid. LCMS (ESI) m/z: 433 [M+H]⁺.

Into a vial were added 6-(1-ethoxyethenyl)-3-(6-fluoropyridin-3-yl)-2-[4-(4-methyl-1,2,4-triazol-3-yl) piperidin-1-yl] benzonitrile (300 mg, 0.69 mmol), TFA (1.2 mL) and DCM (12 mL) at room temperature. The resulting mixture was stirred for 1h at room temperature. The crude product was purified by Prep-HPLC with the following conditions (Column: Xselect CSH C18 OBD Column 30* 150mm 5µm; Mobile Phase A: water (0.1% FA), Mobile Phase B: ACN; Flow rate: 60 mL/min; Gradient: 10% B to 30% B in 7 min; Wave Length: 254/220 nm; RT1(min): 6.92) to afford Example 97 (85.5 mg, 30%). LCMS (ESI) m/z: 405.20 [M+H]⁺.

### Example 100: 5-(6-fluoropyridin-3-yl)-4-(4-(4-methyl-4H-1,2,4-triazol-3-yl)piperidin-1-yl)isophthalonitrile

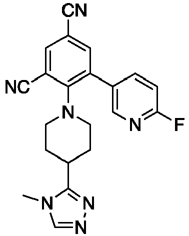

The title compound was prepared using the following procedure:

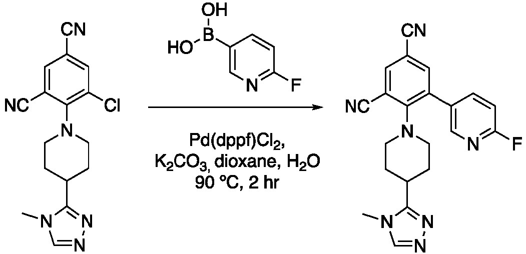

To a stirred solution of Intermediate 17 (200 mg, 0.61 mmol) in dioxane (5 mL) and H₂O (1 mL) were added K₂CO₃ (253.7 mg, 1.84 mmol), 6-fluoropyridin-3-ylboronic acid (172.5 mg, 1.22 mmol) and Pd(dppf)Cl₂ (44.8 mg, 0.061 mmol) in portions at room temperature. The resulting mixture was stirred for 2 h at 90 °C under nitrogen atmosphere. The resulting mixture was concentrated under reduced pressure. The residue was purified by silica gel column chromatography, eluted with CH₂Cl₂ / MeOH (8:1) to afford a brown solid which was repurified by Prep-HPLC with the following conditions (Column: XBridge Prep OBD C18 Column 30*150 mm, 5m; Mobile Phase A: water (10 mmol/L NH₄HCO₃ + 0.1% NH₃.H₂O), Mobile Phase B: ACN; Flow rate: 60 mL/min mL/min; Gradient: 9% B to25% B in 10 min; Wave Length: 254 nm/220 nm; RT1(min): 12.78) to afford Example 100 (19.3 mg, 8%). LCMS (ESI) m/z: 388.25 [M+H]⁺.

### Example 101: 3-(6-fluoropyridin-3-yl)-2-(4-(4-methyl-4H-1,2,4-triazol-3-yl)piperidin-1-yl)-5-(methylsulfonyl)benzonitrile

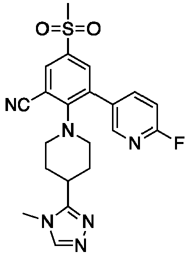

The title compound was prepared using the following procedure:

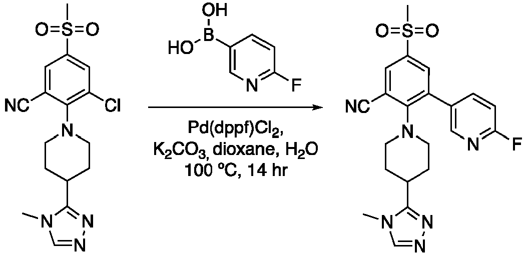

To a stirred solution of Intermediate 18 (160 mg, 0.42 mmol) in dioxane (6 mL) and H₂O (1.5 mL) were added K₂CO₃ (174.6 mg, 1.26 mmol), 6-fluoropyridin-3-ylboronic acid (71.2 mg, 0.5 mmol) and Pd(dppf)Cl₂ (30.8 mg, 0.042 mmol) in portions at room temperature. The resulting mixture was stirred overnight at 100 °C under nitrogen atmosphere. The resulting mixture was concentrated under reduced pressure. The residue was purified by silica gel column chromatography, eluted with CH₂Cl₂ / MeOH (10:1) to afford a brown solid which was further purified by Prep-HPLC with the following conditions (Column: XBridge Prep OBD C18 Column 30*150 mm, 5µm; Mobile Phase A: water (10 mmol/L NH₄HCO₃ + 0.1% NH₃.H₂O), Mobile Phase B: ACN; Flow rate: 60 mL/min mL/min; Gradient: 9% B to 25% B in 10 min; Wave Length: 254 nm/220 nm; RT1(min): 12.78) to afford Example 101 (69.6 mg, 37%). LCMS (ESI) m/z: 441.00 [M+H]⁺.

### Example 105: 5-(6-fluoropyridin-3-yl)-6-(4-(4-methyl-4H-1,2,4-triazol-3-yl)piperidin-1-yl)-1H-indazole-7-carbonitrile

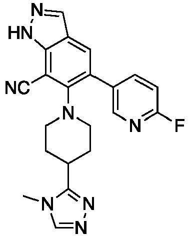

The title compound was prepared using the following procedure:

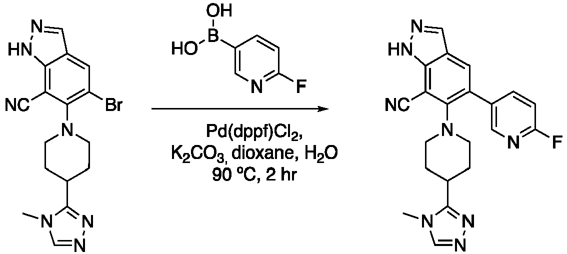

A solution of Intermediate 19 (50 mg, 0.13 mmol), 6-fluoropyridin-3-ylboronic acid (21.9 mg, 0.15 mmol), K₂CO₃ (53.7 mg, 0.39 mmol) and Pd(dppf)Cl₂ (9.5 mg, 0.013 mmol) in 1,4-dioxane (8 mL), H₂O (2 mL) was stirred for 2 h at 90 °C under nitrogen atmosphere. The resulting mixture was diluted with water (50 mL) and extracted with EtOAc (2 x 50 mL). The combined organic layers were washed with brine (2 x 50 mL), dried over anhydrous Na₂SO₄. After filtration, the filtrate was concentrated under reduced pressure. The crude product was purified by Prep-HPLC with the following conditions (Column: YMC-Actus Triart C18 ExRS 30*150 mm, 5 µm; Mobile Phase A: water (10 mmol/L NH₄HCO₃), Mobile Phase B: ACN; Flow rate: 60 mL/min mL/min; Gradient: 11% B to 28% B in 10 min; Wave Length: 254 nm/220 nm; RT1(min): 12.65) to afford Example 105 (0.9 mg, 2%). LCMS (ESI) m/z: 403.50 [M+H]⁺.

### Example 106: 3-(6-fluoropyridin-3-yl)-2-(4-(4-methyl-4H-1,2,4-triazol-3-yl)piperazin-1-yl)benzonitrile

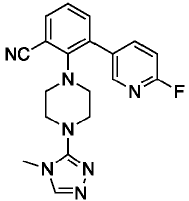

The title compound was prepared using the following procedure:

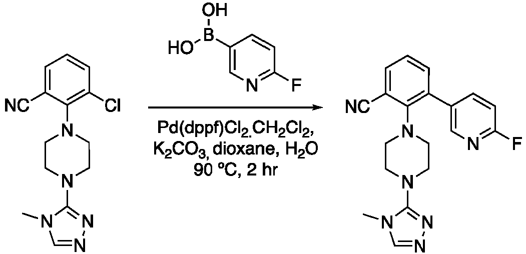

To a stirred mixture of Intermediate 20 (60 mg, 0.17 mmol) and 6-fluoropyridin-3-ylboronic acid (29.2 mg, 0.21 mmol) in H₂O (1 mL) and 1,4-dioxane (4 mL) were added K₂CO₃ (47.8 mg, 0.35 mmol) and Pd(dppf)Cl₂CH₂Cl₂ (14.1 mg, 0.017 mmol) in portions at room temperature. The resulting mixture was stirred for 2 h at 90 °C under nitrogen atmosphere. The residue was purified by Prep-TLC (10% MeOH / CH₂Cl₂). and was further purified by Prep-HPLC with the following conditions (Column: Sunfire prep C18 column, 30*150 mm, 5m; Mobile Phase A: water (0.1% FA), Mobile Phase B: ACN; Flow rate: 60 mL/min mL/min; Gradient: isocratic 10% B - 30% B in 10 min; Wave Length: 254 nm/220 nm; RT1(min): 11.68) to afford Example 106 (10 mg, 16%). LCMS (ESI) m/z: 364.05 [M+H]⁺.

### Example 107: 4-fluoro-3-(6-fluoropyridin-3-yl)-6-methoxy-2-(4-(4-methyl-4H-1,2,4-triazol-3-yl)piperidin-1-yl)benzonitrile

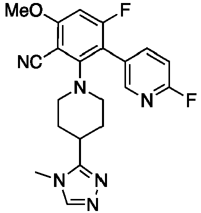

The title compound was prepared using the following procedure:

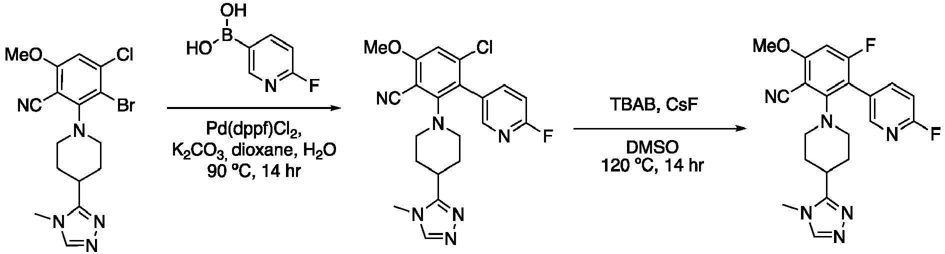

To a stirred mixture of Intermediate 21 (200 mg, 0.49 mmol) and 6-fluoropyridin-3-ylboronic acid (102.9 mg, 0.73 mmol) in 1,4-dioxane (4 mL) and H₂O (0.4 mL) were added K₂CO₃ (135 mg, 0.97 mmol) and Pd(dppf)Cl₂ (35.6 mg, 0.049 mmol) in portions at room temperature. The resulting mixture was stirred overnight at 90 °C under nitrogen atmosphere. The reaction was quenched by the addition of water (30 mL) at room temperature and was extracted with EtOAc (3 x 80 mL). The combined organic layers were washed with brine (2 x15 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (10 mmol/L NH₄HCO₃), 10% to 50% gradient in 10 min; detector, UV 254 nm. and then by Prep-HPLC with the following conditions (Column: Xselect CSH C18 OBD Column 30*150mm 5µm, n; Mobile Phase A: water (0.1% FA), Mobile Phase B: ACN; Flow rate: 60 mL/min mL/min; Gradient: 15% B to 45% B in 8 min; Wave Length: 254 nm/220 nm; RT1(min): 9.77) to afford 4-chloro-3-(6-fluoropyridin-3-yl)-6-methoxy-2-[4-(4-methyl-1,2,4-triazol-3-yl)piperidin-1-yl]benzonitrile) as a white solid.

To a stirred mixture of 4-chloro-3-(6-fluoropyridin-3-yl)-6-methoxy-2-[4-(4-methyl-1,2,4-triazol-3-yl)piperidin-1-yl]benzonitrile (25 mg, 0.059 mmol) and CsF (89 mg, 0.59 mmol) in DMSO (10 mL) was added TBAB (2 mg, 0.006 mmol) in portions at room temperature. The resulting mixture was stirred for 4 h at 120 °C. The crude product was purified by Prep-HPLC with the following conditions (Column: Xselect CSH C18 OBD Column 30*150mm 5µm, n; Mobile Phase A: water (0.1% FA), Mobile Phase B: ACN; Flow rate: 60 mL/min mL/min; Gradient: isocratic 15% B to 35% B in 10 min; Wave Length: 254 nm/220 nm; RT1(min): 12.1) to afford Example 107 (5 mg, 20%). LCMS (ESI) m/z: 411.25 [M+H]⁺.

### Example 108: 3-(6-fluoropyridin-3-yl)-2-(4-(4-methyl-4H-1,2,4-triazol-3-yl)piperidin-1-yl)-6-(oxetan-3-yl)benzonitrile

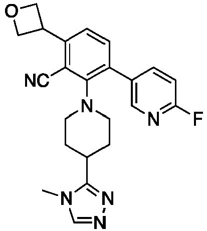

The title compound was prepared using the following procedure:

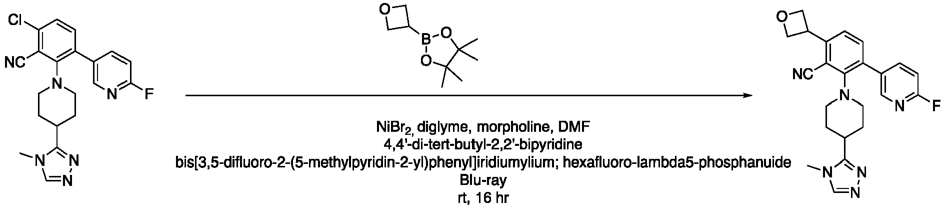

To a stirred mixture of Example 30 (70 mg, 0.18 mmol) and 4,4,5,5-tetramethyl-2-(oxetan-3-yl)-1,3,2-dioxaborolane (64.9 mg, 0.35 mmol) in DMF (3 mL) were added 4,4'-di-tert-butyl-2,2'-bipyridine; bis[3,5-difluoro-2-(5-methylpyridin-2-yl)phenyl]iridiumylium; hexafluoro-lambda5-phosphanuide (3.6 mg, 0.004 mmol) 1-methoxy-2-(2-methoxyethoxy)ethane; dibromonickel (3.1 mg, 0.009 mmol) 4,4'-di-tert-butyl-2,2'-bipyridine (2.4 mg, 0.009 mmol) and morpholine (30.7 mg, 0.35 mmol) in portions at room temperature. The resulting mixture was stirred overnight at room temperature under nitrogen atmosphere during condition of blu-ray. The resulting mixture was concentrated under reduced pressure. The crude product was purified by Prep-HPLC with the following conditions (Column: YMC-Actus Triart C18 ExRS 30*150 mm, 5 µm; Mobile Phase A: water (10 mmol/L NH₄HCO₃ + 0.05% NH₃H₂O), Mobile Phase B: ACN; Flow rate: 60 mL/min mL/min; Gradient: 17% B to 34% B in 10 min; Wave Length: 254 nm/220 nm; RT1(min): 11.63) to afford Example 108 (2.4 mg, 3%). LCMS (ESI) m/z: 419.20 [M+H]⁺.

### Example 113: 3-(3-hydroxy-1H-indazol-5-yl)-2-(4-(4-methyl-4H-1,2,4-triazol-3-yl)piperidin-1-yl)benzonitrile

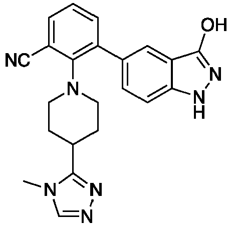

The title compound was prepared using the following procedure:

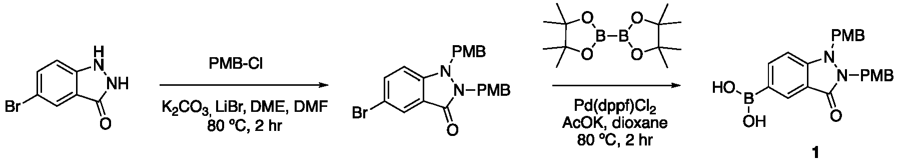

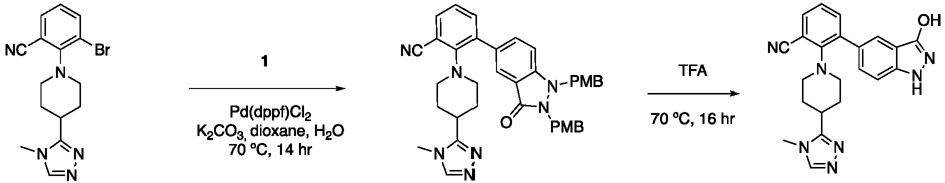

To a stirred mixture of 5-bromo-1,2-dihydro-3H-indazol-3-one (2.0 g, 9.4 mmol) and K₂CO₃ (3.9 g, 28.2 mmol) and 4-methoxybenzyl chloride (2.9 g, 18.8 mmol) in DME (20 mL), DMF (20 mL) were added lithium bromide (2.5 g, 28.2 mmol) in portions at room temperature under nitrogen atmosphere. The resulting mixture was stirred for 2 h at 80 °C under nitrogen atmosphere. The reaction was quenched by the addition of sat. NH₄Cl (aq.) (50 mL) at room temperature. The resulting mixture was extracted with EtOAc (3 x 200 mL). The combined organic layers were washed with water (2 x 30 mL), dried over anhydrous Na₂SO₄. After filtration, the filtrate was concentrated under reduced pressure. The residue was purified by silica gel column chromatography, eluted with PE / EA (2:3) to afford 5-bromo-1,2-bis(4-methoxybenzyl)-1,2-dihydro-3H-indazol-3-one (1.5 g, 48%) as a white solid.

To a stirred mixture of 5-bromo-1,2-bis(4-methoxybenzyl)-1,2-dihydro-3H-indazol-3-one (1.5 g, 3.3 mmol) and bis(pinacolato)diboron (1.3 g, 5.0 mmol) in dioxane (15 mL) were added Pd(dppf)Cl₂ (0.2 g, 0.3 mmol) and KOAc (1.0 g, 10.0 mmol) in portions at room temperature under nitrogen atmosphere. The resulting mixture was stirred for 2 h at 80 °C under nitrogen atmosphere. The reaction was quenched with water at room temperature. The resulting mixture was extracted with EtOAc (3 x 200 mL). The combined organic layers were washed with water (2 x 20 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in water (10 mmol/L NH₄HCO₃) 10% to afford (1,2-bis(4-methoxybenzyl)-3-oxo-2,3-dihydro-1H-indazol-5-yl)boronic acid (492 mg, 29%) as a brown viscous oil.

To a stirred mixture of Intermediate 1 (291 mg, 0.8 mmol) and (1,2-bis(4-methoxybenzyl)-3-oxo-2,3-dihydro-1H-indazol-5-yl)boronic acid (490.1 mg, 1.0 mmol) in dioxane (15 mL), H₂O (1.7 mL) were added Pd(dppf)Cl₂ (61.5 mg, 0.1 mmol) and K₂CO₃ (348.5 mg, 2.5 mmol) in portions at room temperature under nitrogen atmosphere. The resulting mixture was stirred overnight at 70 °C under nitrogen atmosphere. The resulting mixture was concentrated under reduced pressure. The residue was purified by reversed-phase flash chromatography with the following conditions: column, C18 silica gel; mobile phase, MeOH in water (0.1% FA), 10% to 50% gradient in 10 min; detector, UV 254 nm to afford 3-{1,2-bis[(p-methoxyphenyl)methyl]-3-oxo-1,2-dihydro-3H-indazol-5-yl}-2-[4-(4-methyl-4H-1,2,4-triazol-3-yl)-1-piperidyl]benzonitrile (225 mg, 42%) as a light yellow solid.

A solution of 3-{1,2-bis[(p-methoxyphenyl)methyl]-3-oxo-1,2-dihydro-3H-indazol-5-yl}-2-[4-(4-methyl-4H-1,2,4-triazol-3-yl)-1-piperidyl]benzonitrile (225 mg, 0.4 mmol) in TFA (10 mL) was stirred overnight at 70 °C under nitrogen atmosphere. The resulting mixture was concentrated under reduced pressure. The crude product was purified by Prep-HPLC with the following conditions (Column: XBridge Prep Phenyl OBD Column 19*250 mm, 5m; Mobile Phase A: water (0.1% FA), Mobile Phase B: ACN; Flow rate: 60 mL/min mL/min; Gradient: 13% B to31 % B in 10 min; Wave Length: 254 nm/220 nm; RT1(min): 6.81) to afford Example 113 (14.3 mg, 6%). LCMS (ESI) m/z: 400.20 [M+H]⁺.

### Example 117: (5-(3-cyano-2-(4-(4-methyl-4H-1,2,4-triazol-3-yl)piperidin-1-yl)phenyl)pyridin-3-yl)boronic acid

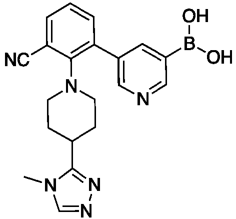

The title compound was prepared using the following procedure:

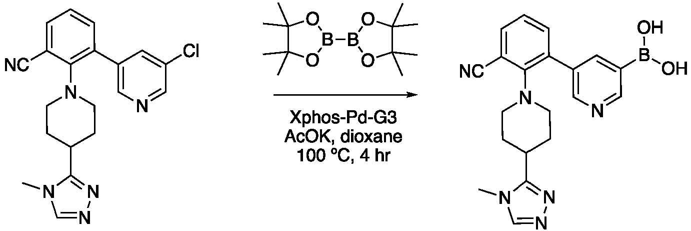

To a stirred mixture of Example 115 (90 mg, 0.24 mmol) and bis(pinacolato)diboron (72.4 mg, 0.28 mmol) in dioxane (2 mL) were added XPhos Pd G₃ (20.1 mg, 0.02 mmol) and KOAc (69.9 mg, 0.71 mmol) in portions at room temperature. The resulting mixture was stirred for 4 h at 100 °C under nitrogen atmosphere. The crude product was purified by Prep-HPLC with the following conditions (Column: XBridge Prep OBD C18 Column 30*150 mm, 5m; Mobile Phase A: water (10 mmol/L NH₄HCO₃ + 0.1% NH₃.H₂O), Mobile Phase B: ACN; Flow rate: 60 mL/min mL/min; Gradient: 2% B to 17% B in 10 min; Wave Length: 254 nm/220 nm; RT1(min): 13.8) to afford Example 117 (11.2 mg, 12%). LCMS (ESI) m/z: 389.15 [M+H]⁺.

### Example 118: 5-(3-cyano-2-(4-(4-methyl-4H-1,2,4-triazol-3-yl)piperidin-1-yl)phenyl)pyridin-3-yl sulfurofluoridate

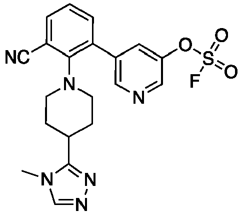

The title compound was prepared using the following procedure:

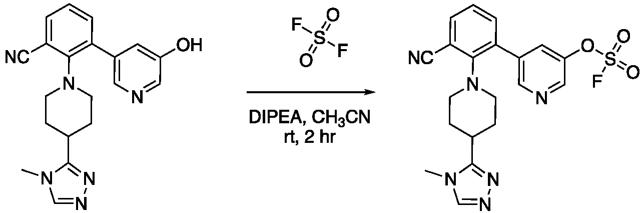

To a stirred solution of Example 120 (50 mg, 0.14 mmol) in anhydrous ACN (5 mL) was added DIEA (35.9 mg, 0.28 mmol) at room temperature under sulfonyl fluoride atmosphere. The reaction mixture was stirred at room temperature for a period of 2 h. The resulting mixture was extracted with EtOAc (50 mL). The combined organic layers were washed with brine (50 mL), dried over anhydrous Na₂SO₄. After filtration, the filtrate was concentrated under reduced pressure. The crude product was purified by reverse phase flash with the following conditions (Column: Xselect CSH F-Phenyl OBD column 30*250 mm, 5µm; Mobile Phase A: water (0.1% FA), Mobile Phase B: ACN; Flow rate: 60 mL/min mL/min; Gradient: 19% B to%37 B in 10 min; Wave Length: 254 nm/220 nm; RT1(min): 12.63) to afford Example 118 (3.1 mg, 5%). LCMS (ESI) m/z: 443.20 [M+H]⁺.

### Example 121: 3-(1-hydroxy-1,3-dihydrobenzo[c][1,2]oxaborol-6-yl)-2-(4-(4-methyl-4H-1,2,4-triazol-3-yl)piperidin-1-yl)benzonitrile

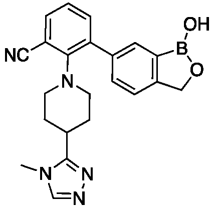

The title compound was prepared using the following procedure:

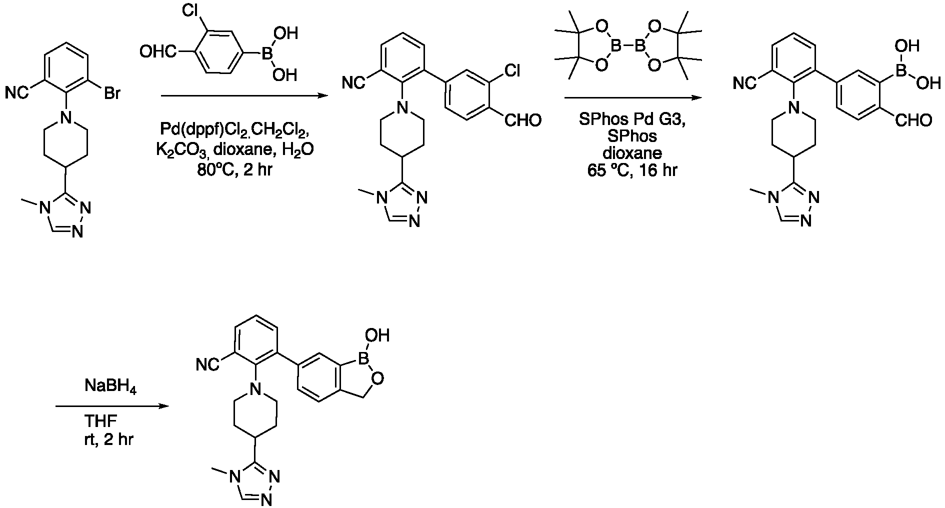

To a stirred mixture of Intermediate 1 (200 mg, 0.58 mmol) and 3-chloro-4-formylphenylboronic acid (127.8 mg, 0.69 mmol) in 1,4-dioxane (10 mL) and H₂O (1 mL) was added K₂CO₃ (159.7 mg, 1.16 mmol) and Pd(dppf)Cl₂CH₂Cl₂ (47.1 mg, 0.058 mmol) in portions at room temperature. The resulting mixture was stirred for 2 h at 80 °C under nitrogen atmosphere. The reaction was quenched by the addition of water (2 mL) at room temperature and extracted with EtOAc (50 mL). The combined organic layers were washed with brine (50 mL), dried over anhydrous Na₂SO₄. After filtration, the filtrate was concentrated under reduced pressure. The residue was purified by silica gel column chromatography, eluted with CH₂Cl₂ / MeOH (9:1) to afford 3'-chloro-4'-formyl-2-[4-(4-methyl-1,2,4-triazol-3-yl)piperidin-1-yl]-[1,1'-biphenyl]-3-carbonitrile (120 mg, 51%) as a brown solid.

To a stirred solution of 3'-chloro-4'-formyl-2-[4-(4-methyl-1,2,4-triazol-3-yl)piperidin-1-yl]-[1,1'-biphenyl]-3-carbonitrile (100 mg, 0.25 mmol) in anhydrous 1,4-dioxane (5 mL) was added bis(pinacolato)diboron (75.1 mg, 0.3 mmol) and potassium trifluoroacetate (15 mg, 0.098 mmol) followed by catalytic amount of SPhos Pd Gen.3 (19.2 mg, 0.025 mmol) and SPhos (10.1 mg, 0.025 mmol) at room temperature. The reaction mixture was stirred at 65 °C overnight. The reaction was quenched by the addition of water (2 mL) at room temperature. The resulting mixture was extracted with EtOAc (50 mL). The combined organic layers were washed with brine (50 mL), dried over anhydrous Na₂SO₄. After filtration, the filtrate was concentrated under reduced pressure. The residue was purified by silica gel column chromatography, eluted with CH₂Cl₂ / MeOH (9:1) to afford 3'-cyano-4-formyl-2'-[4-(4-methyl-1,2,4-triazol-3-yl)piperidin-1-yl]-[1,1'-biphenyl]-3-ylboronic acid (80 mg, 77%) as a brown solid.

To a stirred mixture of 3'-cyano-4-formyl-2'-[4-(4-methyl-1,2,4-triazol-3-yl)piperidin-1-yl]-[1,1'-biphenyl]-3-ylboronic acid (50 mg, 0.12 mmol) in THF (5 mL) was added NaBH₄ (9.1 mg, 0.24 mmol) in portions at room temperature. The resulting mixture was stirred for 2 h at room temperature under nitrogen atmosphere. The reaction mixture was purified by Prep-HPLC with the following conditions (Column: Sunfire prep C18 column, 30*150 mm, 5m; Mobile Phase A: water (0.1% FA), Mobile Phase B: ACN; Flow rate: 60 mL/min mL/min; Gradient: isocratic 10% B to 30% B in 10 min; Wave Length: 254 nm/220 nm; RT1(min): 15.75) to afford Example 121 (3.6 mg, 8%). LCMS (ESI) m/z: 400.30 [M+H]⁺.

### Example 122: 3-(6-fluoropyridin-3-yl)-6-(1-methyl-1H-pyrazol-3-yl)-2-(4-(4-methyl-4H-1,2,4-triazol-3-yl)piperazin-1-yl)benzonitrile

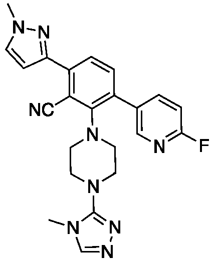

The title compound was prepared using the following procedure:

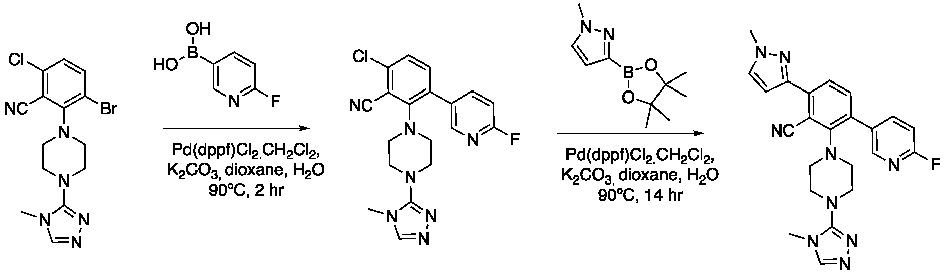

To a stirred mixture of Intermediate 22 (140 mg, 0.37 mmol) and 6-fluoropyridin-3-ylboronic acid (25.8 mg, 0.18 mmol) in H₂O (0.8 mL) and 1,4-dioxane (8 mL) were added K₂CO₃ (101.4 mg, 0.73 mmol) and Pd(dppf)Cl₂CH₂Cl₂ (30 mg, 0.037 mmol) in portions at room temperature. The resulting mixture was stirred for 2 h at 90 °C under nitrogen atmosphere. The residue was purified by silica gel column chromatography, eluted with CH₂Cl₂ / MeOH (0%-10%) to afford 6-chloro-3-(6-fluoropyridin-3-yl)-2-[4-(4-methyl-1,2,4-triazol-3-yl)piperazin-1-yl]benzonitrile (120 mg, 81%) as a light brown solid. LCMS (ESI) m/z: 398 [M+H]⁺.

To a stirred mixture of 6-chloro-3-(6-fluoropyridin-3-yl)-2-[4-(4-methyl-1,2,4-triazol-3-yl)piperazin-1-yl]benzonitrile (50 mg, 0.13 mmol) and 1-methyl-3-(4,4,5,5-tetramethyl-1,3,2-dioxaborolan-2-yl)pyrazole (31.4 mg, 0.15 mmol) in1,4-dioxane (4 mL) and H₂O (0.4 mL) were added K₂CO₃ (34.7 mg, 0.25 mmol) and Pd(dppf)Cl₂CH₂Cl₂ (9.2 mg, 0.013 mmol) in portions at room temperature. The resulting mixture was stirred overnight at 90 °C under nitrogen atmosphere. The residue was purified by Prep-TLC (CH₂Cl₂ / MeOH 9:1) and then repurified by Prep-HPLC with the following conditions (Column: XBridge Prep OBD C18 Column 30*150 mm, 5m; Mobile Phase A: water (10 mmol/L NH₄HCO₃), Mobile Phase B: ACN; Flow rate: 60 mL/min mL/min; Gradient: 14% B to 32% B in 10 min; Wave Length: 254 nm/220 nm; RT1(min): 12) to afford Example 122 (7 mg, 13%). LCMS (ESI) m/z: 444.30 [M+H]⁺.

### Example 125: 2-(4-(4-methyl-4H-1,2,4-triazol-3-yl)piperidin-1-yl)-3-(1H-pyrazolo[4,3-b]pyridin-6-yl)benzonitrile

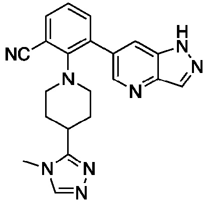

The title compound was prepared using the following procedure:

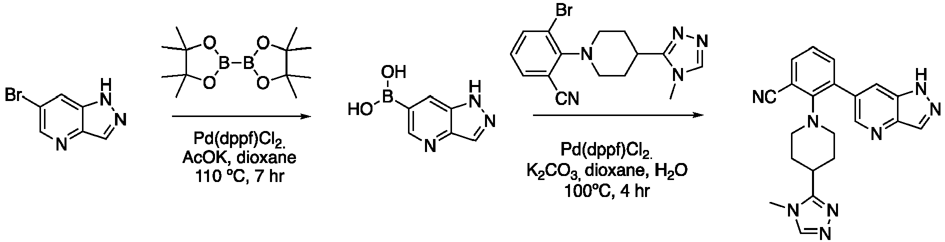

A mixture of 6-bromo-1H-pyrazolo[4,3-b]pyridine (500 mg, 2.52 mmol), bis(pinacolato)diboron (961.8 mg, 3.79 mmol), KOAc (743.4 mg, 7.57 mmol) and Pd(dppf)Cl₂ (184.7 mg, 0.25 mmol) in 1,4-dioxane (10 mL) was stirred for 7 h at 110 °C under nitrogen atmosphere. The resulting mixture was concentrated under reduced pressure. The reaction was quenched by the addition of water (50 mL) at room temperature. The resulting mixture was extracted with EtOAc (3 x 50 mL). The combined organic layers were washed with brine (50 mL), dried over anhydrous Na₂SO₄. After filtration, the filtrate was concentrated under reduced pressure. The residue was purified by silica gel column chromatography, eluted with PE/EA (10:1) to afford 1H-pyrazolo[4,3-b]pyridin-6-ylboronic acid) as a brown oil. LCMS (ESI) m/z: 164 [M+H]⁺.

A mixture of Intermediate 1 (70 mg, 0.20 mmol), K₂CO₃ (84.4 mg, 0.61 mmol), 1H-pyrazolo[4,3-b]pyridin-6-ylboronic acid (49.4 mg, 0.3 mmol) and Pd(dppf)Cl₂ (14.8 mg, 0.02 mmol) in water (0.2 mL), dioxane (2 mL) was stirred for 4 h at 100 °C under nitrogen atmosphere. The resulting mixture was concentrated under reduced pressure. The reaction was quenched by the addition of water (30 mL) at room temperature. The resulting mixture was extracted with EtOAc (3 x 50 mL). The combined organic layers were washed with brine (20 mL), dried over anhydrous Na₂SO₄. After filtration, the filtrate was concentrated under reduced pressure. The crude product was purified by Prep-HPLC to afford Example 125 (11 mg, 14%). LCMS (ESI) m/z: 385.15 [M+H]⁺.

### Example 126: (5-(3-cyano-4-(1-methyl-1H-pyrazol-3-yl)-2-(4-(4-methyl-4H-1,2,4-triazol-3-yl)piperidin-1-yl)phenyl)pyridin-3-yl)boronic acid

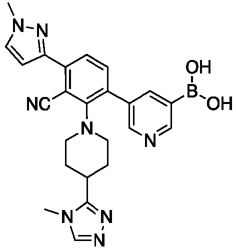

The title compound was prepared using the following procedure:

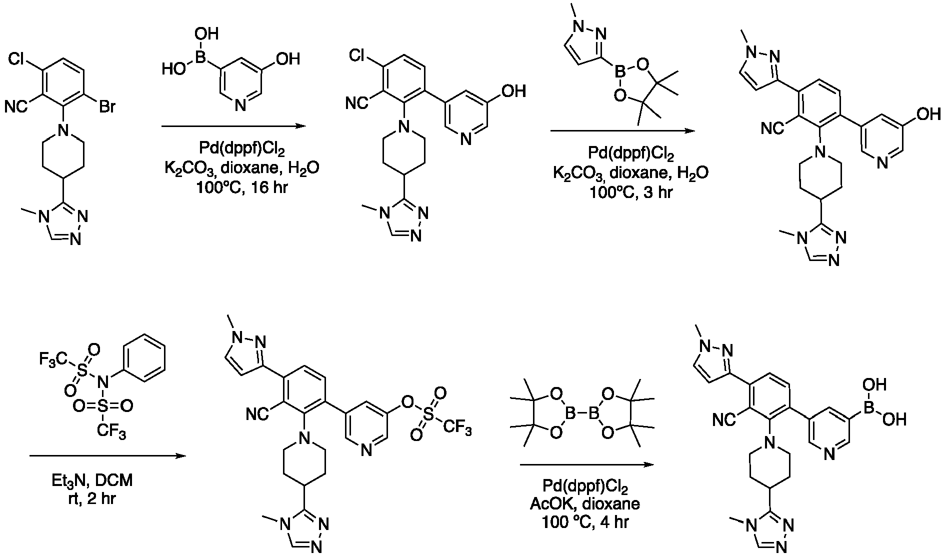

A mixture of Intermediate 6 (500 mg, 1.31 mmol), 5-hydroxypyridin-3-ylboronic acid (182.5 mg, 1.31 mmol) K₂CO₃ (453.8 mg, 3.28 mmol) and Pd(dppf)Cl₂ (96.1 mg, 0.13 mmol) in dioxane (10 mL), DMF (5 mL) and H₂O (1 mL) was stirred for overnight at 100 °C under nitrogen atmosphere. The resulting mixture was concentrated under reduced pressure. The reaction was quenched by the addition of water (30 mL) at room temperature. The resulting mixture was extracted with CH₂Cl₂ (3 x 50 mL). The combined organic layers were washed with brine (20 mL), dried over anhydrous Na₂SO₄. After filtration, the filtrate was concentrated under reduced pressure. The residue was purified by silica gel column chromatography, eluted with CH₂Cl₂/MeOH (12:1) to afford 6-chloro-3-(5-hydroxypyridin-3-yl)-2-[4-(4-methyl-1,2,4-triazol-3-yl)piperidin-1-yl]benzonitrile (342 mg, 66%) as a brown solid. LCMS (ESI) m/z: 395 [M+H]⁺.

A mixture of 6-chloro-3-(5-hydroxypyridin-3-yl)-2-[4-(4-methyl-1,2,4-triazol-3-yl)piperidin-1-yl]benzonitrile (300 mg, 0.76 mmol), 1-methyl-3-(4,4,5,5-tetramethyl-1,3,2-dioxaborolan-2-yl)pyrazolidine (193.4 mg, 0.91 mmol), Pd(dppf)Cl₂ (55.6 mg, 0.076 mmol) and K₂CO₃ (315 mg, 2.28 mmol) in dioxane (10 mL), H₂O (1 mL) was stirred for 3 h at 100 °C under nitrogen atmosphere. The resulting mixture was concentrated under reduced pressure. The reaction was quenched by the addition of water (30 mL) at room temperature. The resulting mixture was extracted with CH₂Cl₂ (3 x 50 mL). The combined organic layers were washed with brine (20 mL), dried over anhydrous Na₂SO₄. After filtration, the filtrate was concentrated under reduced pressure. The residue was purified by silica gel column chromatography, eluted with CH₂Cl₂/MeOH (10:1) to afford 3-(5-hydroxypyridin-3-yl)-2-[4-(4-methyl-1,2,4-triazol-3-yl)piperidin-1-yl]-6-(1-methylpyrazol-3 -yl)benzonitrile (180 mg, 54%) as a brown solid. LCMS (ESI) m/z: 441 [M+H]⁺.

A mixture of 3-(5-hydroxypyridin-3-yl)-2-[4-(4-methyl-1,2,4-triazol-3-yl)piperidin-1-yl]-6-(1-methylpyrazol-3-yl)benzonitrile (160 mg, 0.36 mmol), TEA (55.1 mg, 0.54 mmol) and 1,1,1-trifluoro-N-phenyl-N-trifluoromethanesulfonylmethanesulfonamide (155.7 mg, 0.44 mmol) in DCM (3 mL) was stirred for 2 h at room temperature. The reaction was quenched by the addition of water (20 mL) at room temperature. The resulting mixture was extracted with CH₂Cl₂ (3 x 20 mL). The combined organic layers were washed with brine (10 mL), dried over anhydrous Na₂SO₄. After filtration, the filtrate was concentrated under reduced pressure. The residue was purified by silica gel column chromatography, eluted with CH₂Cl₂ / MeOH (10:1) to afford 5-{3-cyano-2-[4-(4-methyl-1,2,4-triazol-3-yl)piperidin-1-yl]-4-(1-methylpyrazol-3-yl)phenyl}pyridin-3-yl trifluoromethanesulfonate (169 mg, 81%) as a light brown solid. LCMS (ESI) m/z: 573 [M+H]⁺.

A mixture of 5-{3-cyano-2-[4-(4-methyl-1,2,4-triazol-3-yl)piperidin-1-yl]-4-(1-methylpyrazol-3-yl)phenyl}pyridin-3-yl trifluoromethanesulfonate (120 mg, 0.21 mmol), bis(pinacolato)diboron (106.4 mg, 0.42 mmol), KOAc (82.3 mg, 0.84 mmol) and Pd(dppf)Cl₂ (15.3 mg, 0.021 mmol) in dioxane (2.5 mL) was stirred for 4 h at 100 °C under nitrogen atmosphere. The resulting mixture was concentrated under reduced pressure. The reaction was quenched by the addition of water (20 mL) at room temperature. The resulting mixture was extracted with EtOAc (3 x 20 mL). The combined organic layers were washed with brine (10 mL), dried over anhydrous Na₂SO₄. After filtration, the filtrate was concentrated under reduced pressure. The crude product was purified by Prep-HPLC with the following conditions (Column: Sunfire prep C18 column, 30*150 mm, 5m; Mobile Phase A: water (0.1% FA), Mobile Phase B: ACN; Flow rate: 60 mL/min mL/min; Gradient: 2% B to 17% B in 10 min; Wave Length: 254 nm/220 nm; RT1(min): 13.32) to afford Example 126 (2.9 mg, 3%). LCMS (ESI) m/z: 469.20 [M+H]⁺.

### Example 127: 4-fluoro-3-(6-fluoropyridin-3-yl)-2-(4-(4-methyl-4H-1,2,4-triazol-3-yl)piperazin-1-yl)benzonitrile

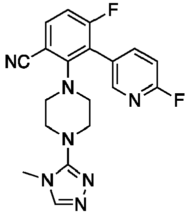

The title compound was prepared using the following procedure: 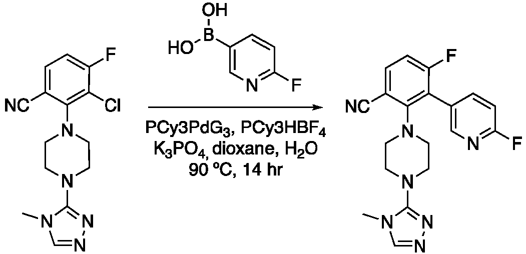 To a stirred mixture of Intermediate 23 (50 mg, 0.16 mmol) and 6-fluoropyridin-3-ylboronic acid (26.4 mg, 0.19 mmol) in 1,4-dioxane (5 mL) and H₂O (0.5 mL) were added K₃PO₄ (99.3 mg, 0.47 mmol) and PCy3pd G3 (10.1 mg, 0.015 mmol) and PCy3HBF₄ (5.7 mg, 0.016 mmol) in portions at room temperature. The resulting mixture was stirred overnight at 90 °C under nitrogen atmosphere. The residue was purified by Prep-TLC (10% MeOH / CH₂Cl₂) and then re purified by Prep-HPLC with the following conditions (Column: Xselect CSH F-Phenyl OBD column 30*250 mm, 5µm; Mobile Phase A: water (0.1% FA), Mobile Phase B: ACN; Flow rate: 60 mL/min mL/min; Gradient: 9% B to 27% B in 10 min; Wave Length: 254 nm/220 nm; RT1(min): 1) to afford Example 127 (7 mg, 11%). LCMS (ESI) m/z: 382.10 [M+H]⁺.

### Example 128: 4'-ethynyl-3'-formyl-2-(4-(4-methyl-4H-1,2,4-triazol-3-yl)piperidin-1-yl)-[1,1'-biphenyl]-3-carbonitrile

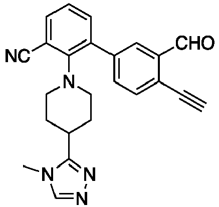

The title compound was prepared using the following procedure:

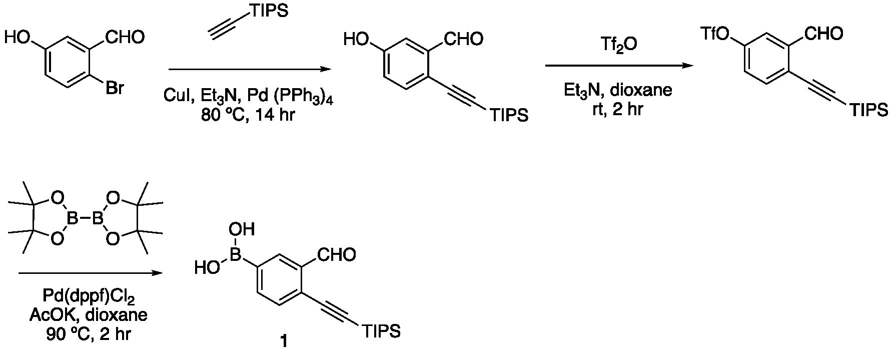

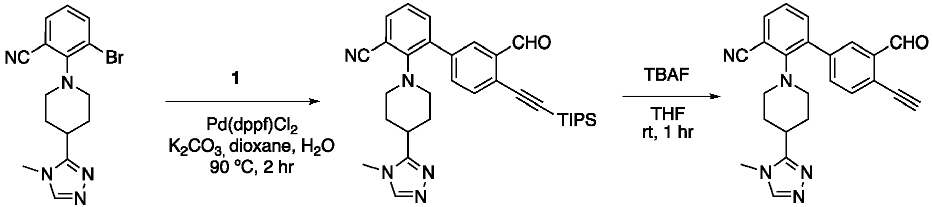

To a stirred mixture of 2-bromo-5-hydroxybenzaldehyde (2 g, 9.95 mmol) and ethynyltriisopropylsilane (2 g, 10.9 mmol) in TEA (100 mL) were added tetrakis(triphenylphosphine)palladium(0) (1.15 g, 0.99 mmol) and CuI (0.38 g, 1.99 mmol) in portions at room temperature. The resulting mixture was stirred overnight at 80 °C under nitrogen atmosphere. The residue was purified by reversed-phase flash chromatography with the following conditions: column, C18 silica gel; mobile phase, MeCN in water (10 mmol/L NH₄HCO₃), 20% to 60% gradient in 30 min; detector, UV 220 nm to obtain 5-hydroxy-2-[2-(triisopropylsilyl)ethynyl]benzaldehyde (1.5 g, 50%) as a brown solid. LCMS (ESI) m/z: 303 [M+H]⁺.

To a stirred mixture of 5-hydroxy-2-[2-(triisopropylsilyl)ethynyl]benzaldehyde (200 mg, 0.66 mmol) and Tf₂O (205.2 mg, 0.73 mmol) in 1,4-dioxane (5 mL) was added TEA (133.8 mg, 1.32 mmol) dropwise at 0 °C under nitrogen atmosphere. The resulting mixture was stirred for 2h at room temperature. The reaction was quenched by the addition of water (150 mL) at room temperature and extracted with EtOAc (3 x 150 mL). The combined organic layers were washed with brine (2 x 20 mL), dried over anhydrous Na₂SO₄. After filtration, the filtrate was concentrated under reduced pressure. The residue was purified by silica gel column chromatography, eluted with CH₂Cl₂ / MeOH (0%-10%) to afford 3-formyl-4-[2-(triisopropylsilyl)ethynyl]phenyl trifluoromethanesulfonate (120 mg, 42%) as a yellow solid. LCMS (ESI) m/z: 435 [M+H]⁺.

To a stirred mixture of 3-formyl-4-[2-(triisopropylsilyl)ethynyl]phenyl trifluoromethanesulfonate (100 mg, 0.23 mmol) and 4,4,5,5-tetramethyl-2-(4,4,5,5-tetramethyl-1,3,2-dioxaborolan-2-yl)-1,3,2-dioxaborolane (87.7 mg, 0.34 mmol) in dioxane (5 mL) were added KOAc (67.7 mg, 0.69 mmol) and Pd(dppf)Cl₂ (16.8 mg, 0.023 mmol) in portions at room temperature. The resulting mixture was stirred for 2 h at 90 °C under nitrogen atmosphere to form 3-formyl-4-[2-(triisopropylsilyl)ethynyl]phenylboronic acid as a brown liquid. which was used in the next step directly without further purification. LCMS (ESI) m/z: 331 [M+H]⁺.

To a stirred mixture of 3-formyl-4-[2-(triisopropylsilyl)ethynyl]phenylboronic acid (75 mg, 0.23 mmol) and Intermediate 1 (78.6 mg, 0.23 mmol) in dioxane (4 mL) and H₂O (0.4 mL) were added K₂CO₃ (94.1 mg, 0.68 mmol) and Pd(dppf)Cl₂ (16.6 mg, 0.023 mmol) in portions at room temperature. The resulting mixture was stirred for 2 h at 90 °C under nitrogen atmosphere. The residue was purified by Prep-TLC (10% MeOH / CH₂Cl₂) to afford 3'-formyl-2-[4-(4-methyl-1,2,4-triazol-3-yl)piperidin-1-yl]-4'-[2-(triisopropylsilyl)ethynyl]-[1,1'-biphenyl]-3-carbonitrile (75 mg, 59%) as a light brown solid. LCMS (ESI) m/z: 552 [M+H]⁺.

A mixture of 3'-formyl-2-[4-(4-methyl-1,2,4-triazol-3-yl)piperidin-1-yl]-4'-[2-(triisopropylsilyl)ethynyl]-[1,1'-biphenyl]-3-carbonitrile (60 mg, 0.11 mmol) and TBAF (5.3 mg, 0.16 mmol) in THF (5 mL) was stirred for 1 h at room temperature. The reaction was quenched by the addition of water (40 mL) at room temperature and was extracted with EtOAc (3 x 60 mL). The combined organic layers were washed with brine (7 x 20 mL), dried over anhydrous Na₂SO₄. After filtration, the filtrate was concentrated under reduced pressure. The crude product was purified by Prep-HPLC with the following conditions (Column: XBridge Shield RP18 OBD Column 30*150 mm, 5m; Mobile Phase A: water (10 mmol/L NH₄HCO₃), Mobile Phase B: ACN; Flow rate: 60 mL/min mL/min; Gradient: 26% B to 42% B in 12 min; Wave Length: 254 nm/220 nm; RT1(min): 12.87) to afford Example 128 (4.7 mg, 11%). LCMS (ESI) m/z: 396.10 [M+H]⁺.

### Example 129: 6-cyclopropyl-3-(6-fluoropyridin-3-yl)-2-(4-(4-methyl-4H-1,2,4-triazol-3-yl)piperazin-1-yl)benzonitrile

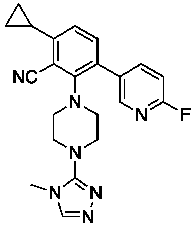

The title compound was prepared using the following procedure:

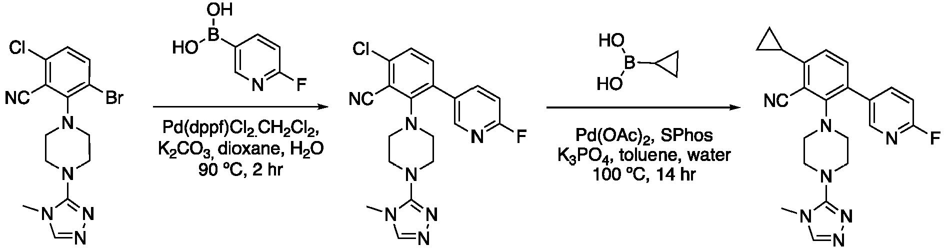

To a stirred mixture of Intermediate 24 (100 mg, 0.26 mmol) and 6-fluoropyridin-3-ylboronic acid (29.5 mg, 0.21 mmol) in H₂O (0.8 mL) and 1,4-dioxane (8 mL) were added K₂CO₃ (72.4 mg, 0.52 mmol) and Pd(dppf)Cl₂CH₂Cl₂ (21.3 mg, 0.026 mmol) in portions at room temperature. The resulting mixture was stirred for 2 h at 90 °C under nitrogen atmosphere. The residue was purified by silica gel column chromatography, eluted with CH₂Cl₂ / MeOH (0%-10%) to afford 6-chloro-3-(6-fluoropyridin-3-yl)-2-[4-(4-methyl-1,2,4-triazol-3-yl)piperazin-1-yl]benzonitrile (70 mg, 84%) as a light brown solid.

To a stirred mixture of 6-chloro-3-(6-fluoropyridin-3-yl)-2-[4-(4-methyl-1,2,4-triazol-3-yl)piperazin-1-yl]benzonitrile (60 mg, 0.15 mmol) and cyclopropylboronic acid (19.4 mg, 0.23 mmol) in toluene (2 mL) and H₂O (0.2 mL) were added K₃PO₄ (96 mg, 0.45 mmol) and SPhos (12.4 mg, 0.03 mmol) and Pd(OAc)₂ (3.4 mg, 0.015 mmol) in portions at room temperature . The resulting mixture was stirred overnight at 100 °C under nitrogen atmosphere. The reaction was quenched by the addition of water (50 mL) at room temperature and was extracted with EtOAc (3 x 60 mL). The combined organic layers were washed with brine (2 x10 mL), dried over anhydrous Na₂SO₄. After filtration, the filtrate was concentrated under reduced pressure. The crude product was purified by Prep-HPLC with the following conditions (Column: Xselect CSH C18 OBD Column 30*150mm 5µm, n; Mobile Phase A: water (0.1% FA), Mobile Phase B: ACN; Flow rate: 60 mL/min mL/min; Gradient: 18% B to 38% B in 8 min; Wave Length: 254 nm/220 nm; RT1(min): 9.07) to afford Example 129 (9 mg, 14%). LCMS (ESI) m/z: 404.10 [M+H]⁺.

### Example 130: 5-fluoro-3-(6-fluoropyridin-3-yl)-2-(4-(4-methyl-4H-1,2,4-triazol-3-yl)piperidin-1-yl)benzonitrile

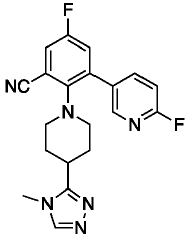

The title compound was prepared using the following procedure:

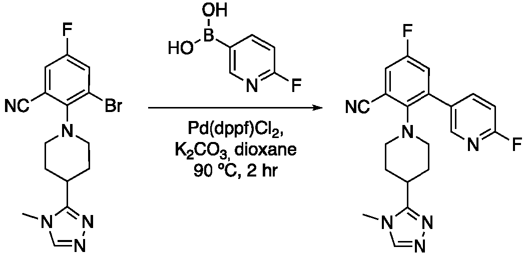

A solution of Intermediate 25 (140 mg, 0.38 mmol), 6-fluoropyridin-3-ylboronic acid (65 mg, 0.46 mmol), K₂CO₃ (265.6 mg, 1.92 mmol) and Pd(dppf)Cl₂ (28.1 mg, 0.038 mmol) in 1,4-dioxane (15 mL) was stirred for 2 h at 90 °C under nitrogen atmosphere. The resulting mixture was diluted with water (50 mL). The resulting mixture was extracted with EtOAc (3 x 50 mL). The combined organic layers were washed with brine (2 x 50 mL), dried over anhydrous Na₂SO₄. After filtration, the filtrate was concentrated under reduced pressure. The crude product was purified by Prep-HPLC with the following conditions (Column: XBridge Prep Phenyl OBD Column 19*250 mm, 5m; Mobile Phase A: water (0.1% FA), Mobile Phase B: ACN; Flow rate: 60 mL/min mL/min; Gradient: 26% B to 42% B in 10 min; Wave Length: 254 nm/220 nm; RT1(min): 7.15) to afford Example 130 (13.3 mg, 9%). LCMS (ESI) m/z: 381.20 [M+H]⁺.

### Example 131: 4-fluoro-3-(6-fluoropyridin-3-yl)-6-(1-methyl-1H-pyrazol-3-yl)-2-(4-(4-methyl-4H-1,2,4-triazol-3-yl)piperidin-1-yl)benzonitrile

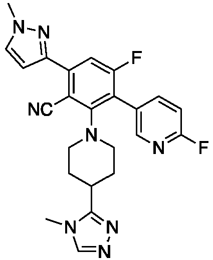

The title compound was prepared using the following procedure:

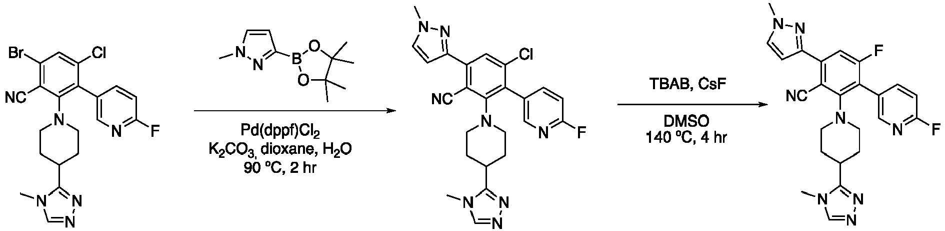

A solution of Intermediate 26 (200 mg, 0.42 mmol), 1-methyl-3-(4,4,5,5-tetramethyl-1,3,2-dioxaborolan-2-yl)pyrazole (87.5 mg, 0.42 mmol), K₂CO₃ (174.3 mg, 1.26 mmol) and Pd(dppf)Cl₂ (30.8 mg, 0.042 mmol) in 1,4-dioxane (15 mL), H₂O (2 mL) was stirred for 2 h at 90 °C under nitrogen atmosphere. The resulting mixture was concentrated under reduced pressure. The residue was purified by silica gel column chromatography, eluted with CH₂Cl₂ / MeOH (9:1) to afford 4-chloro-3-(6-fluoropyridin-3-yl)-2-[4-(4-methyl-1,2,4-triazol-3-yl)piperidin-1-yl]-6-(1-methylpyrazol-3-yl)benzonitrile (80 mg, 30%) as a light yellow solid. LCMS (ESI) m/z: 478 [M+H]⁺.

A solution of 4-chloro-3-(6-fluoropyridin-3-yl)-2-[4-(4-methyl-1,2,4-triazol-3-yl)piperidin-1-yl]-6-(1-methylpyrazol-3-yl)benzonitrile (80 mg, 0.17 mmol) and TBAB (5.4 mg, 0.017 mmol) in DMSO (10 mL) was stirred for 4 h at 140 °C. The resulting mixture was extracted with EtOAc (3 x 50 mL). The combined organic layers were washed with brine (2 x 50 mL), dried over anhydrous Na₂SO₄. After filtration, the filtrate was concentrated under reduced pressure. The crude product was purified by Prep-HPLC with the following conditions (Column: XBridge Prep Phenyl OBD Column 19*250 mm, 5m; Mobile Phase A: water (10 mmol/L NH₄HCO₃), Mobile Phase B: ACN; Flow rate: 60 mL/min mL/min; Wave Length: 254 nm/220 nm; RT1(min): 8.31) to afford Example 131 (15.5 mg, 20%). LCMS (ESI) m/z: 461.15 [M+H]⁺.

### Example 132: 6-fluoro-3'-(4-(4-methyl-4H-1,2,4-triazol-3-yl)piperidin-1-yl)-[3,4'-bipyridine]-2'-carbonitrile

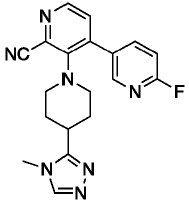

The title compound was prepared using the following procedure:

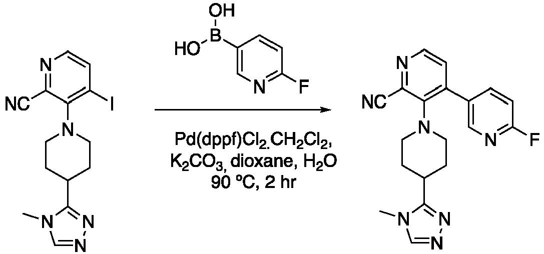

To a stirred solution of Intermediate 27 (200 mg, 0.51 mmol) and 6-fluoropyridin-3-ylboronic acid (143 mg, 1 mmol) in dioxane (10 mL), H₂O (1 mL) were added Pd(dppf)Cl₂CH₂Cl₂ (41.3 mg, 0.051 mmol) and K₂CO₃ (210 mg, 1.52 mmol) in portions at room temperature under nitrogen atmosphere. The resulting mixture was stirred for 2 h at 90 ° C under nitrogen atmosphere. The reaction was quenched by the addition of water (1 mL) at room temperature. The resulting mixture was extracted with EtOAc (2 x 20 mL). The combined organic layers were washed with water (10 mL), dried over anhydrous Na₂SO₄. After filtration, the filtrate was concentrated under reduced pressure. The crude product was purified by Prep-HPLC with the following conditions (Column: XBridge Shield RP18 OBD Column, 19*150 mm, 5µm; Mobile Phase A: water (10 mmol/L NH₄HCO₃), Mobile Phase B: ACN; Flow rate: 25 mL/min; Gradient: 30% B to 35% B in 7 min, 35% B; Wave Length: 220 nm; RT1(min): 6.09; to afford Example 132 (19.3 mg, 10%). LCMS (ESI) m/z: 364.25 [M+H]⁺.

### Example 133: 6'-fluoro-3-(4-(4-methyl-4H-1,2,4-triazol-3-yl)piperidin-1-yl)-[2,3'-bipyridine]-4-carbonitrile

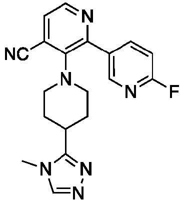

The title compound was prepared using the following procedure:

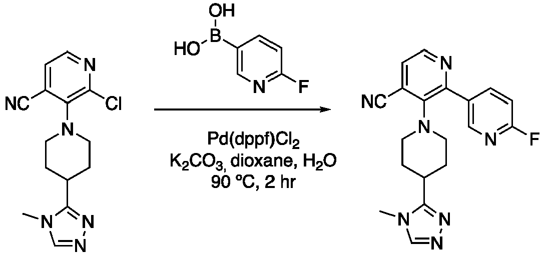

To a stirred mixture of Intermediate 28 (200 mg, 0.66 mmol) and 6-fluoropyridin-3-ylboronic acid (279.2 mg, 1.98 mmol) in dioxane (10 mL) and H₂O (1 mL) were added K₂CO₃ (273.9 mg, 1.98 mmol) and Pd(dppf)Cl₂ (48.3 mg, 0.07 mmol) in portions at room temperature under nitrogen atmosphere. The resulting mixture was stirred for 2 h at 90 ° C under nitrogen atmosphere. The reaction was quenched with water at room temperature. The resulting mixture was extracted with EtOAc (2 x 100 mL). The combined organic layers were washed with brine (50 mL), dried over anhydrous Na₂SO₄. After filtration, the filtrate was concentrated under reduced pressure. The crude product was purified by Prep-HPLC with the following conditions (Column: XBridge Prep Phenyl OBD Column 19*250 mm, 5 *µ* m; Mobile Phase A: water (0.1% FA), Mobile Phase B: ACN; Flow rate: 60 mL/min mL/min; Gradient: 25% B to 41% B in 8 min; Wave Length: 254 nm/220 nm; RT1(min): 7.21) to afford Example 133 (47 mg, 19%). LCMS (ESI) m/z: 364.10 [M+H]⁺.

### Example 134: 6'-fluoro-3-(4-(1-methyl-1H-imidazol-5-yl)piperidin-1-yl)-[2,3'-bipyridine]-4-carbonitrile

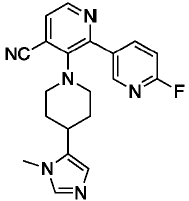

The title compound was prepared using the following procedure:

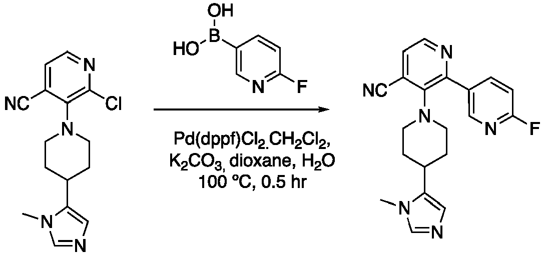

Intermediate 29 (46 mg, 0.15mmol) was dissolved in 1,4-dioxane (0.4 mL) and water (0.1 mL) and added 6-fluoropyridin-3-ylboronic acid (21.5 mg, 0.15 mmol), K₂CO₃ (42.1 mg, 0.35 mmol), Pd(dppf)Cl₂CH₂Cl₂ (6.2 mg, 0.007 mmol). The resulting mixture was stirred for 0.5 h at 100 ° C under nitrogen atmosphere. The reaction was quenched with saturated sodium carbonate aqueous solution and extracted with EtOAc. The organic layers were combined, died over sodium sulfate, and the solvent was removed in vacuo. The residue was purified by prep-HPLC to afford Example 134 (4.1 mg, 7%). LCMS (ESI) m/z: 363.00 [M+H]⁺.

### Example 136: 6'-fluoro-3-(4-(4-methyl-4H-1,2,4-triazol-3-yl)piperazin-1-yl)-[2,3'-bipyridine]-4-carbonitrile

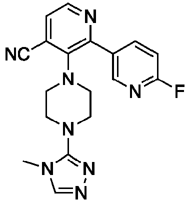

The title compound was prepared using the following procedure:

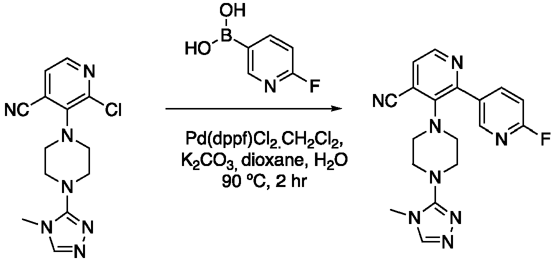

To a stirred mixture of Intermediate 30 (200 mg, 0.66 mmol) and 6-fluoropyridin-3-ylboronic acid (111.3 mg, 0.79 mmol) in 1,4-dioxane (8 mL) and H₂O (0.8 mL) were added K₂CO₃ (182 mg, 1.32 mmol) and Pd(dppf)Cl₂.CH₂Cl₂ (53.6 mg, 0.07 mmol) in portions at room temperature. The resulting mixture was stirred for 2 h at 90 °C under nitrogen atmosphere. The residue was purified by Prep-TLC (10% MeOH/CH₂Cl₂) and then by Prep-HPLC with the following conditions (Column: XBridge Shield RP18 OBD Column 50*250 mm, 10 um; Mobile Phase A: water (10 mmol/L NH₄HCO₃), Mobile Phase B: ACN; Flow rate: 100 mL/min mL/min; Gradient: 5% B to 35% B in 30 min; Wave Length: 254 nm/200 nm; RT1(min): 26.07) to afford Example 136 (122 mg, 50%). LCMS (ESI) m/z: 365.00 [M+H]⁺.

### Example 137: 6'-fluoro-5-methoxy-3-(4-(4-methyl-4H-1,2,4-triazol-3-yl)piperidin-1-yl)-[2,3'-bipyridine]-4-carbonitrile

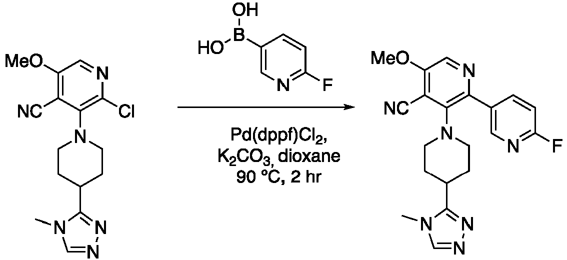

A solution of Intermediate 31 (50 mg, 0.15 mmol), 6-fluoropyridin-3-ylboronic acid (25.4 mg, 0.18 mmol), K₂CO₃ (62.3 mg, 0.45 mmol) and Pd(dppf)Cl₂ (11 mg, 0.015 mmol) in 1,4-dioxane (15 mL) was stirred for 2 h at 90 °C under nitrogen atmosphere. The resulting mixture was diluted with water (30 mL) and extracted with EtOAc (3 x 30 mL). The combined organic layers were washed with brine (2 x 30 mL), dried over anhydrous Na₂SO₄. After filtration, the filtrate was concentrated under reduced pressure. The crude product was purified by Prep-HPLC with the following conditions (Column: YMC-Actus Triart C18 ExRS 30*150 mm, 5 µm; Mobile Phase A: water (10 mmol/L NH₄HCO₃), Mobile Phase B: ACN; Flow rate: 60 mL/min mL/min; Gradient: 16% B to 33% B in 10 min; Wave Length: 254 nm/220 nm; RT1(min): 12.42) to afford Example 137 (25.1 mg, 42%). LCMS (ESI) m/z: 394.15 [M+H]⁺.

### Example 138: 6'-fluoro-5-(1-methyl-1H-pyrazol-3-yl)-3-(4-(4-methyl-4H-1,2,4-triazol-3-yl)piperidin-1-yl)-[2,3'-bipyridine]-4-carbonitrile

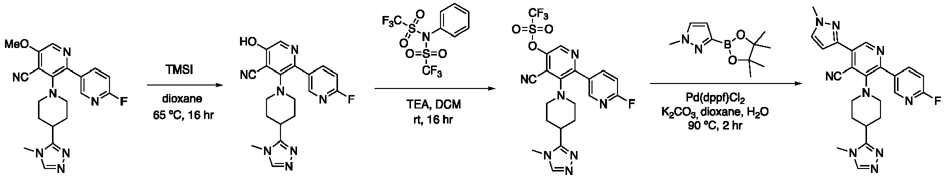

A solution of Example 137 (160 mg, 0.41 mmol) and trimethylsilyl iodide (100 mg, 0.5 mmol) in 1,4-dioxane (10 mL) was stirred for 16 h at 65 °C. The resulting mixture was concentrated under reduced pressure. The residue was purified by silica gel column chromatography, eluted with CH₂Cl₂ / MeOH (5:1) to afford 6'-fluoro-5-hydroxy-3-[4-(4-methyl-1,2,4-triazol-3-yl)piperidin-1-yl]-[2,3'-bipyridine]-4-carbonitrile (100 mg, 44%) as a light yellow oil. LCMS (ESI) m/z: 380 [M+H]⁺.

A solution of 6'-fluoro-5-hydroxy-3-[4-(4-methyl-1,2,4-triazol-3-yl)piperidin-1-yl]-[2,3'-bipyridine]-4-carbonitrile (60 mg, 0.16 mmol), (ditrifluoromethanesulfonylmethyl)benzene (67.6 mg, 0.19 mmol) and TEA (32 mg, 0.32 mmol) in DCM (15 mL) was stirred for 16 h at room temperature. The resulting mixture was concentrated under reduced pressure. The residue was purified by silica gel column chromatography, eluted with CH₂Cl₂ / MeOH (5:1) to afford 4-cyano-6'-fluoro-3-[4-(4-methyl-1,2,4-triazol-3-yl)piperidin-1-yl]-[2,3'-bipyridin]-5-yl trifluoromethanesulfonate (24 mg, 30%) as a light yellow oil. LCMS (ESI) m/z: 512 [M+H]⁺.

A solution of 4-cyano-6'-fluoro-3-[4-(4-methyl-1,2,4-triazol-3-yl)piperidin-1-yl]-[2,3'-bipyridin]-5-yl trifluoromethanesulfonate (24 mg, 0.047 mmol), 1-methyl-3-(4,4,5,5-tetramethyl-1,3,2-dioxaborolan-2-yl)pyrazole (11.7 mg, 0.056 mmol), K₂CO₃ (19.5 mg, 0.14 mmol) and Pd(dppf)Cl₂ (3.4 mg, 0.005 mmol) in 1,4-dioxane (12 mL), H₂O (1.5 mL) was stirred for 2 h at 90 °C under nitrogen atmosphere. The resulting mixture was diluted with water (15 mL) and extracted with EtOAc (3 x 30 mL). The combined organic layers were washed with brine (2 x 30 mL), dried over anhydrous Na₂SO₄. After filtration, the filtrate was concentrated under reduced pressure. The crude product was purified by Prep-HPLC with the following conditions (Column: Sunfire prep C18 column, 30*150 mm, 5m; Mobile Phase A: water (0.1% FA), Mobile Phase B: ACN; Flow rate: 60 mL/min mL/min; Gradient: isocratic 8% B to 28% B in 10 min; Wave Length: 254 nm/220 nm; RT1(min): 11.28) to afford Example 138 (1 mg, 5%). LCMS (ESI) m/z: 444.10 [M+H]⁺.

The following compounds in Table 3 were prepared using procedures similar to those described for Example 35 using appropriate starting materials.

**TABLE 3**

| **Example No.** | **Name** | **[M+H]⁺** |
|---|---|---|
| **42** | 3-(6-fluoropyridin-3-yl)-2-(4-(4-methyl-4H-1,2,4-triazol-3-yl)piperidin-1-yl)-6-(1-(piperidin-4-yl)-1H-pyrazol-4-yl)benzonitrile | 512.15 |
| **44** | 6-(2-aminopyridin-4-yl)-3-(6-fluoropyridin-3-yl)-2-(4-(4-methyl-4H-1,2,4-triazol-3-yl)piperidin-1-yl)benzonitrile | 455.05 |
| **45** | 3-(6-fluoropyridin-3-yl)-2-(4-(4-methyl-4H-1,2,4-triazol-3-yl)piperidin-1-yl)-6-(1H-pyrazol-3-yl)benzonitrile | 429.05 |
| **48** | 6-(1-methyl-1H-pyrazol-4-yl)-2-(4-(4-methyl-4H-1,2,4-triazol-3-yl)piperidin-1-yl)-3-(6-methylpyridazin-4-yl)benzonitrile | 440.30 |
| **54** | 3-(6-fluoropyridin-3-yl)-6-(1-methyl-1H-pyrazol-4-yl)-2-(4-(4-(methyl-d3)-4H-1,2,4-triazol-3-yl)piperidin-1-yl)benzonitrile | 446.05 |
| **55** | 6-(1-methyl-1H-pyrazol-4-yl)-2-(4-(4-(methyl-d3)-4H-1,2,4-triazol-3-yl)piperidin-1-yl)-3-(pyridazin-4-yl)benzonitrile | 429.05 |
| **56** | 6-chloro-3-(6-fluoropyridin-3-yl)-2-(4-(4-(methyl-d3)-4H-1,2,4-triazol-3-yl)piperidin-1-yl)benzonitrile | 400.05 |
| **60** | 6-(1-(2-(dimethylamino)ethyl)-1H-pyrazol-4-yl)-3-(6-fluoropyridin-3-yl)-2-(4-(4-methyl-4H-1,2,4-triazol-3-yl)piperidin-1-yl)benzonitrile | 500.00 |
| **61** | 6-(1-methyl-1H-pyrazol-3-yl)-2-(4-(4-methyl-4H-1,2,4-triazol-3-yl)piperidin-1-yl)-3-(6-methylpyridazin-4-yl)benzonitrile | 440.20 |
| **63** | 6-(1-cyclopropyl-1H-pyrazol-4-yl)-3-(6-fluoropyridin-3-yl)-2-(4-(4-methyl-4H-1,2,4-triazol-3-yl)piperidin-1-yl)benzonitrile | 469.05 |
| **65** | 2-(4-(4-methyl-4H-1,2,4-triazol-3-yl)piperidin-1-yl)-3-(6-methylpyridazin-4-yl)-6-(1H-pyrazol-3-yl)benzonitrile | 426.10 |
| **67** | 3-(6-fluoropyridin-3-yl)-6-methyl-2-(4-(4-methyl-4H-1,2,4-triazol-3-yl)piperidin-1-yl)benzonitrile | 377.30 |
| **68** | 6-cyclopropyl-3-(6-fluoropyridin-3-yl)-2-(4-(4-methyl-4H-1,2,4-triazol-3-yl)piperidin-1-yl)benzonitrile | 403.30 |
| **98** | 6-(1-cyclopropyl-1H-pyrazol-3-yl)-3-(6-fluoropyridin-3-yl)-2-(4-(4-methyl-4H-1,2,4-triazol-3-yl)piperidin-1-yl)benzonitrile | 469.10 |
| **124** | 3'-formyl-4'-hydroxy-4-(1-methyl-1H-pyrazol-3-yl)-2-(4-(4-methyl-4H-1,2,4-triazol-3-yl)piperidin-1-yl)-[1,1'-biphenyl]-3-carbonitrile | 468.15 |

The following compounds in Table 4 were prepared using procedures similar to those described for Example 1 using Intermediate 1 and appropriate starting materials.

**TABLE 4**

| **Example No.** | **Name** | **[M+H]⁺** |
|---|---|---|
| **46** | 2-(4-(4-methyl-4H-1,2,4-triazol-3-yl)piperidin-1-yl)-3-(6-methylpyridazin-4-yl)benzonitrile | 360.10 |
| **47** | 3-(6-fluoro-5-methylpyridin-3-yl)-2-(4-(4-methyl-4H-1,2,4-triazol-3-yl)piperidin-1-yl)benzonitrile | 377.05 |
| **96** | 4'-fluoro-2-(4-(4-methyl-4H-1,2,4-triazol-3-yl)piperidin-1-yl)-[1,1'-biphenyl]-3-carbonitrile | 362.05 |
| **99** | 3-(2-methyl-2H-1,2,3-triazol-4-yl)-2-(4-(4-methyl-4H-1,2,4-triazol-3-yl)piperidin-1-yl)benzonitrile | 349.10 |
| **102** | 3',4'-difluoro-2-(4-(4-methyl-4H-1,2,4-triazol-3-yl)piperidin-1-yl)-[1,1'-biphenyl]-3-carbonitrile | 380.10 |
| **103** | 2',4'-difluoro-2-(4-(4-methyl-4H-1,2,4-triazol-3-yl)piperidin-1-yl)-[1,1'-biphenyl]-3-carbonitrile | 380.25 |
| **104** | 3-(1-methyl-1H-1,2,3-triazol-5-yl)-2-(4-(4-methyl-4H-1,2,4-triazol-3-yl)piperidin-1-yl)benzonitrile | 349.20 |
| **109** | 3'-formyl-4'-hydroxy-2-(4-(4-methyl-4H-1,2,4-triazol-3-yl)piperidin-1-yl)-[1,1'-biphenyl]-3-carbonitrile | 388.15 |
| **110** | 3'-formyl-2-(4-(4-methyl-4H-1,2,4-triazol-3-yl)piperidin-1-yl)-[1,1'-biphenyl]-3-carbonitrile | 372.15 |
| **111** | (3'-cyano-2'-(4-(4-methyl-4H-1,2,4-triazol-3-yl)piperidin-1-yl)-[1,1'-biphenyl]-4-yl)boronic acid | 388.20 |
| **112** | (3'-cyano-2'-(4-(4-methyl-4H-1,2,4-triazol-3-yl)piperidin-1-yl)-[1,1'-biphenyl]-3-yl)boronic acid | 388.15 |
| **114** | 3-(5-bromopyridin-3-yl)-2-(4-(4-methyl-4H-1,2,4-triazol-3-yl)piperidin-1-yl)benzonitrile | 423.10, 425.10 |
| **115** | 3-(5-chloropyridin-3-yl)-2-(4-(4-methyl-4H-1,2,4-triazol-3-yl)piperidin-1-yl)benzonitrile | 379.10 |
| **116** | 3-(6-hydroxypyridin-3-yl)-2-(4-(4-methyl-4H-1,2,4-triazol-3-yl)piperidin-1-yl)benzonitrile | 361.25 |
| **120** | 3-(5-hydroxypyridin-3-yl)-2-(4-(4-methyl-4H-1,2,4-triazol-3-yl)piperidin-1-yl)benzonitrile | 361.25 |

The following compound in Table 5 was prepared using procedures similar to those described for Example 11 using Intermediate 1 and appropriate starting materials.

**TABLE 5**

| **Example No.** | **Name** | **[M+H]⁺** |
|---|---|---|
| **52** | 3-(6-hydroxypyridazin-4-yl)-2-(4-(4-methyl-4H-1,2,4-triazol-3-yl)piperidin-1-yl)benzonitrile | 362.00 |

The following compound in Table 6 was prepared using procedures similar to those described for Example 38 using Intermediate 9 and appropriate starting materials.

**TABLE 6**

| **Example No.** | **Name** | **[M+H]⁺** |
|---|---|---|
| **53** | 4-fluoro-3-(6-fluoro-5-methylpyridin-3-yl)-2-(4-(4-methyl-4H-1,2,4-triazol-3-yl)piperidin-1-yl)benzonitrile | 395.00 |

The following compound in Table 7 was prepared using procedures similar to those described for Example 58 using Intermediate 6 and appropriate starting materials.

**TABLE 7**

| **Example No.** | **Name** | **[M+H]⁺** |
|---|---|---|
| **62** | 6-(2-(dimethylamino)ethoxy)-2-(4-(4-methyl-4H-1,2,4-triazol-3-yl)piperidin-1-yl)-3-(6-methylpyridazin-4-yl)benzonitrile | 447.30 |

The following compounds in Table 8 was prepared using procedures similar to those described for Example 64 using Intermediate 6 and appropriate starting materials.

**TABLE 8**

| **Example No.** | **Name** | **[M+H]⁺** |
|---|---|---|
| **73** | 3-(6-fluoropyridin-3-yl)-2-(4-(4-methyl-4H-1,2,4-triazol-3-yl)piperidin-1-yl)-6-(1H-pyrazol-4-yl)benzonitrile | 429.05 |
| **74** | 2-(4-(4-methyl-4H-1,2,4-triazol-3-yl)piperidin-1-yl)-3-(6-methylpyridazin-4-yl)-6-(1H-pyrazol-4-yl)benzonitrile | 426.05 |

The following compound in Table 9 was prepared using procedures similar to those described for Example 76 using Intermediate 6 and appropriate starting materials.

**TABLE 9**

| **Example No.** | **Name** | **[M+H]⁺** |
|---|---|---|
| **77** | 6-(cyclopropylmethoxy)-3-(6-fluoropyridin-3-yl)-2-(4-(4-methyl-4H-1,2,4-triazol-3-yl)piperidin-1-yl)benzonitrile | 433.30 |

The following compounds in Table 10 were prepared using procedures similar to those described for Example 82 using Intermediate 6 and appropriate starting materials.

**TABLE 10**

| **Example No.** | **Name** | **[M+H]⁺** |
|---|---|---|
| **83** | 3-(6-fluoropyridin-3-yl)-6-(methyl(2-(methylamino)ethyl)amino)-2-(4-(4-methyl-4H-1,2,4-triazol-3-yl)piperidin-1-yl)benzonitrile | 449.10 |
| **87** | 3-(6-fluoropyridin-3-yl)-2-(4-(4-methyl-4H-1,2,4-triazol-3-yl)piperidin-1-yl)-6-morpholinobenzonitrile | 448.05 |

The following compounds in Table 11 were prepared using procedures similar to those described for Example 85 using Intermediate 6 and appropriate starting materials.

**TABLE 11**

| **Example No.** | **Name** | **[M+H]⁺** |
|---|---|---|
| **86** | 3-(6-fluoropyridin-3-yl)-6-(2-methoxyethoxy)-2-(4-(4-methyl-4H-1,2,4-triazol-3-yl)piperidin-1-yl)benzonitrile | 437.10 |
| **88** | 3-(6-fluoropyridin-3-yl)-2-(4-(4-methyl-4H-1,2,4-triazol-3-yl)piperidin-1-yl)-6-((tetrahydrofuran-3-yl)oxy)benzonitrile (Isomer A) | 449.30 |
| **89** | 3-(6-fluoropyridin-3-yl)-2-(4-(4-methyl-4H-1,2,4-triazol-3-yl)piperidin-1-yl)-6-((tetrahydrofuran-3-yl)oxy)benzonitrile (Isomer B) | 449.10 |

The following compound in Table 12 was prepared using procedures similar to those described for Example 93 using Intermediate 7 and appropriate starting materials.

**TABLE 12**

| **Example No.** | **Name** | **[M+H]⁺** |
|---|---|---|
| **94** | 3-(imidazo[1,2-a]pyrimidin-6-yl)-6-methoxy-2-(4-(4-methyl-4H-1,2,4-triazol-3-yl)piperidin-1-yl)benzonitrile | 415.30 |

The following compound in Table 13 was prepared using procedures similar to those described for Example 118 using appropriate starting materials.

**TABLE 13**

| **Example No.** | **Name** | **[M+H]⁺** |
|---|---|---|
| **119** | 5-(3-cyano-2-(4-(4-methyl-4H-1,2,4-triazol-3-yl)piperidin-1-yl)phenyl)pyridin-2-yl sulfurofluoridate | 443.20 |

The following compound in Table 14 was prepared using procedures similar to those described for Example 121 using appropriate starting materials.

**TABLE 14**

| **Example No.** | **Name** | **[M+H]⁺** |
|---|---|---|
| **123** | 3-(1-hydroxy-1,3-dihydrobenzo[c][1,2]oxaborol-5-yl)-2-(4-(4-methyl-4H-1,2,4-triazol-3-yl)piperidin-1-yl)benzonitrile | 400.20 |

The following compound in Table 15 was prepared using procedures similar to those described for Example 131 using appropriate starting materials.

**TABLE 15**

| **Example No.** | **Name** | **[M+H]⁺** |
|---|---|---|
| **135** | 6-cyclopropyl-4-fluoro-3-(6-fluoropyridin-3-yl)-2-(4-(4-methyl-4H-1,2,4-triazol-3-yl)piperidin-1-yl)benzonitrile | 421.15 |

The following compound in Table 16 was prepared using procedures similar to those described for Example 138 using appropriate starting materials.

**TABLE 16**

| **Example No.** | **Name** | **[M+H]⁺** |
|---|---|---|
| **139** | 6'-fluoro-5-(1-methyl-1H-pyrazol-4-yl)-3-(4-(4-methyl-4H-1,2,4-triazol-3-yl)piperidin-1-yl)-[2,3'-bipyridine]-4-carbonitrile | 444.10 |

### II. Biological Evaluation

### Example 1: QPCTL Recombinant Protein Production

Recombinant truncated human QPCTL enzyme was produced by bacterial expression of amino acid residues 53-382 (UniProt ID Q9NXS2) fused at the amino terminus to a thioredoxin-His-TEV tag and cloned into the pET32a plasmid backbone. Protein expression was induced when the bacterial culture OD600 was approximately 0.7 using 1 mM IPTG and the cells incubated at 20 °C for approximately 16 hours. The QPCTL was purified using nickel column purification, digested by TEV, followed by a second nickel column purification. The protein was further purified by size-exclusion chromatography using a Superdex^{™} 75 column. The final protein was concentrated using ultrafiltration tubes at a molecular weight cutoff of 10 kilodaltons and stored frozen in protein buffer (25 mM Tris pH 8.3, 150 mM NaCl).

### Example 2: QPCTL Enzymatic Activity Assay

Compound IC₅₀ values for the inhibition of QPCTL activity were determined using a biochemical fluorescent-based assay. QPCTL activity was measured in a coupled-enzyme assay using the QPCTL substrate, glutamine-7-amido-4-methylcoumarin (H-Gln-AMC, Bachem), which is converted to pyroglutamyl-AMC by QPCTL. Pyroglutamyl-AMC is then converted to the fluorescent molecule, AMC, by incubation with the human enzyme pyroglutamyl peptidase-1. Test compounds or controls (SEN177 and DMSO) diluted in 100% DMSO were preincubated with 10-12 nM QPCTL in multi-well plates in assay buffer (25 mM HEPES pH 7.0) for 30 minutes at 37°C. Final concentrations of DMSO and test compounds were 2% and 30 - 100 µM, respectively. Following the incubation, H-Gln-AMC substrate was diluted in assay buffer and added to each well for a final concentration of 10 µM. The reaction was incubated for 60 min at 37°C, before stopping it by boiling at 100°C for 5 minutes and cooling the plate at 4°C for 3 minutes. An equal volume of 38 nM recombinant, human PGPEP-1 (rhPGPEP-1, R&D Systems) in 100 mM Tris pH 8.0, 5 mM dithiothreitol was added to the reaction for a final concentration of 19 nM and incubated for 25 minutes at room temperature. Following incubation, the fluorescence was measured with a plate reader using excitation at 380 nm and emission at 460 nm. Percent inhibition of enzymatic activity by test compounds was calculated by normalizing the data to the 2% DMSO and 30 - 100 µM SEN177 control values, which represent 0% and 100% inhibition of enzymatic activity, respectively. Concentration-response-curves and IC₅₀ values (Table 17) were generated using curve fit software.

### Example 3: QPCT Enzymatic Activity Assay

Compound IC₅₀ values for the inhibition of QPCT (rhQPCT, R&D Systems) activity were determined using a biochemical fluorescent-based assay. QPCT activity was measured in a coupled-enzyme assay using the QPCT substrate, glutamine-7-amido-4-methylcoumarin (H-Gln-AMC, Bachem), which is converted to pyroglutamyl-AMC by QPCT. Pyroglutamyl-AMC is then converted to the fluorescent molecule, AMC, by incubation with the human enzyme pyroglutamyl peptidase-1 (PGPEP-1). Test compounds or controls (SEN177 and DMSO) diluted in 100% DMSO were preincubated with 10-12 nM QPCT in multi-well plates in assay buffer (25 mM HEPES pH 7.0) for 30 minutes at 37°C. Final concentrations of DMSO and SEN177 were 2% and 30 - 100 µM, respectively. Following the incubation, H-Gln-AMC substrate was diluted in assay buffer and added to each well for a final concentration of 10 µM. The reaction was incubated for 60 min at 37°C, before stopping it by boiling at 100°C for 5 minutes and cooling the plate at 4°C for 3 minutes. An equal volume of 38 nM recombinant, human PGPEP-1 (rhPGPEP-1, R&D Systems) in 100 mM Tris pH 8.0, 5 mM dithiothreitol was added to the reaction for a final concentration of 19 nM and incubated for 25 minutes at room temperature. Following incubation, the fluorescence was measured with a plate reader using excitation at 380 nm and emission at 460 nm. Percent inhibition of enzymatic activity by test compounds was calculated by normalizing the data to the 2% DMSO and 30 - 100 µM SEN177 control values, which represent 0% and 100% inhibition of enzymatic activity, respectively. Concentration-response-curves and IC₅₀ values (Table 17) were generated using Collaborative Drug Discovery software.

**Table 17. Biological Activity of Representative Compounds. QPCTL and QPCT IC₅₀ values are designated within the following ranges:**

| A: ≤ 0.1 µM | | |
|---|---|---|
| B: > 0.1 µM to ≤ 1.0 µM | | |
| C: > 1.0 µM to ≤ 10 µM | | |
| D: >10 µM | | |
| **Example** | **QPCTL Inhibition IC₅₀ (µM)** | **QPCT Inhibition IC₅₀ (µM)** |
| **SEN177** | A | A |
| **1** | A | A |
| **2** | A | A |
| **3** | A | A |
| **4** | B | B |
| **5** | B | B |
| **6** | A | A |
| **7** | C | C |
| **8** | A | A |
| **9** | A | A |
| **10** | A | A |
| **11** | A | A |
| **12** | A | A |
| **13** | C | B |
| **14** | B | B |
| **15** | B | A |
| **16** | A | A |
| **17** | B | B |
| **18** | D | D |
| **19** | B | A |
| **20** | B | A |
| **21** | B | B |
| **22** | B | B |
| **23** | B | B |
| **24** | A | A |
| **25** | A | A |
| **26** | A | A |
| **27** | A | A |
| **28** | A | A |
| **29** | B | B |
| **30** | A | A |
| **31** | A | A |
| **32** | A | A |
| **33** | A | A |
| **34** | A | A |
| **35** | A | A |
| **36** | A | A |
| **37** | A | A |
| **38** | A | A |
| **39** | A | A |
| **40** | A | B |
| **41** | A | A |
| **42** | A | A |
| **43** | A | A |
| **44** | A | A |
| **45** | A | A |
| **46** | A | A |
| **47** | A | A |
| **48** | A | A |
| **49** | B | B |
| **50** | A | A |
| **51** | A | A |
| **52** | A | A |
| **53** | A | A |
| **54** | A | A |
| **55** | A | A |
| **56** | A | A |
| **57** | A | A |
| **58** | A | A |
| **59** | B | B |
| **60** | A | A |
| **61** | A | A |
| **62** | A | B |
| **63** | A | A |
| **64** | A | A |
| **65** | A | A |
| **66** | A | A |
| **67** | A | A |
| **68** | A | A |
| **69** | A | A |
| **70** | A | A |
| **71** | A | A |
| **72** | B | B |
| **73** | A | A |
| **74** | A | A |
| **75** | B | B |
| **76** | A | A |
| **77** | A | A |
| **78** | A | A |
| **79** | A | A |
| **80** | A | A |
| **81** | A | A |
| **82** | A | A |
| **83** | A | A |
| **84** | A | A |
| **85** | A | A |
| **86** | A | A |
| **87** | A | A |
| **88** | A | A |
| **89** | A | A |
| **90** | A | A |
| **91** | A | A |
| **92** | A | A |
| **93** | A | A |
| **94** | A | A |
| **95** | A | A |
| **96** | A | A |
| **97** | A | A |
| **98** | A | A |
| **99** | B | A |
| **100** | A | A |
| **101** | A | A |
| **102** | B | A |
| **103** | B | A |
| **104** | B | B |
| **105** | A | A |
| **106** | A | A |
| **107** | A | A |
| **108** | A | A |
| **109** | A | A |
| **110** | B | A |
| **111** | B | A |
| **112** | A | A |
| **113** | A | A |
| **114** | A | A |
| **115** | A | A |
| **116** | A | A |
| **117** | A | A |
| **118** | A | A |
| **119** | A | B |
| **120** | A | A |
| **121** | B | B |
| **122** | A | A |
| **123** | A | A |
| **124** | A | A |
| **125** | B | A |
| **126** | A | A |
| **127** | A | A |
| **128** | B | A |
| **129** | A | A |
| **130** | C | C |
| **131** | C | B |
| **132** | A | A |
| **133** | A | A |
| **134** | A | A |
| **135** | B | A |
| **136** | A | A |
| **137** | C | B |
| **138** | D | C |
| **139** | D | B |

### Inhibition of Cellular QPCTL Activity - Fluorescence-Activated Cell Sorting (FACS) Flow Cytometry

Compound IC₅₀ values for the inhibition of QPCTL activity in cells were evaluated using the Ramos human Burkitts Lymphoma cell line (ATCC, cat. number CRL-1596) followed by staining with an anti-CD47 antibody that recognizes only the N-terminal pyroglutamated CD47. Ramos cells were plated in tissue-culture treated 12-well plates at a final concentration of 50,000 cells/mL in complete media (ATCC modified RPMI-1640 with addition of fetal bovine serum to 10%). Test compounds or controls (DMSO or SEN177) were added to each well for final concentrations of 0.1% DMSO and 10 µM SEN177.

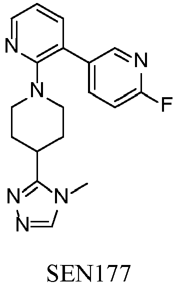

The cells were incubated for 72-96 hours at 37°C and 5% CO₂. At the end of incubation period, cells were transferred to tubes and spun down at 1200 rpm for 5 minutes. The cells were surface stained using an allophycocyanin (APC)-labeled clone CC2C6 anti-CD47 antibody (BioLegend, Cat. Number 323123) at a dilution of 1:100 in Hanks' Balanced Salt Solution containing 0.5% Bovine Serum Albumin (FACS buffer) for 15 minutes at 4°C or on ice, protected from light. After antibody staining, the cells were washed with FACS buffer then stained with SYTOX^{™} Blue dead cell stain at a dilution of 1: 1000. Cells were analyzed on a BD FACS Melody and a positive staining gate on live cells was set using the 0.1% DMSO controls. Percent inhibition of QPCTL activity by test compounds was calculated by normalizing the % CC2C6-positive cells to the 0.1% DMSO (100% QPCTL activity) and 10 µM SEN177 (0% QPCTL activity) control values. Flow cytometry data were analyzed by FlowJo software and concentration-response-curves and IC₅₀ values were generated using Collaborative Drug Discovery software.

**Table 18. Biological Activity of Representative Compounds. IC₅₀ values are designated within the following ranges:**

| A: ≤ 0.1 µM | |
|---|---|
| B: > 0.1 µM to ≤ 1.0 µM | |
| C: > 1.0 µM to ≤ 10 µM | |
| D: >10 µM | |
| **Example** | **FACS Assay IC₅₀ (µM)** |
| **SEN177** | C |
| **1** | A |
| **2** | B |
| **3** | B |
| **4** | C |
| **6** | C |
| **8** | C |
| **9** | C |
| **10** | B |
| **11** | A |
| **12** | C |
| **15** | C |
| **16** | B |
| **19** | C |
| **24** | A |
| **25** | B |
| **26** | A |
| **27** | B |
| **28** | A |
| **30** | A |
| **33** | A |
| **34** | C |
| **35** | A |
| **36** | A |
| **37** | A |
| **38** | A |
| **39** | B |

### Inhibition of Cellular QPCTL Activity - Imaging Assay

Compound IC₅₀ values for the inhibition of QPCTL activity were evaluated by staining treated DLD-1 cells (ATCC, cat. number CCL-221) with clone CC2C6 of an anti-CD47 antibody that recognizes only the N-terminal pyroglutamated form of CD47 followed by high-content imaging. Cells were plated in tissue-culture treated 96-well plates at a final concentration of 20,000 cells/mL in complete media (ATCC modified RPMI-1640 with addition of fetal bovine serum to 10%). Test compounds or controls (DMSO or SEN177) were added to each well for final concentrations of 0.1% DMSO and 30 µM SEN177 and the plate was sealed with Breath-Easy Biofilm (Diversified biotech, cat. number BEM-1). The cells were incubated for 72 hours at 37°C and 5% CO₂. Media was aspirated and the cells were surface stained using clone CC2C6 of an anti-CD47 antibody (BioLegend, Cat. number 323102) at a dilution of 1:500 in Opti-MEM media (Thermo Fisher, Cat. number 11058-021) containing 1% Fetal Bovine Serum (incubation buffer) for 1 hour at room temperature. After primary antibody staining, the cells were fixed with 4% paraformaldehyde in the incubation buffer for 15 minutes at room temperature. The buffer was aspirated, and the cells washed three times with Dulbecco's Phosphate Buffered Saline (DPBS, Thermo Fisher, Cat. number 14040-133). To detect the signal, Tyramide superboost AlexaFluor-488 Kit (Thermo Fisher, Cat. number B40941) was used according to manufacturer's specifications. After the tyramide reaction was stopped, the cells were washed three times in the DPBS buffer and the cell nuclei stained with Hoechst 33342 (Invitrogen, cat. number H3570) at a dilution of 1:10,000 for 15 minutes at room temperature. Cells were analyzed on a ImageXpress Pico Automated Cell Imaging System (Molecular Devices) using 10x objective (3x3 fields). Percent inhibition of QPCTL activity by test compounds was calculated by normalizing the All Cell Average Intensities data (generated by CellReporterXpress software, Molecular Devices) to the 0.1% DMSO (100% QPCTL activity) and 30 µM SEN177 (0% QPCTL activity) control values and concentration-response-curves and IC₅₀ values were generated using CDD Vault software (Collaborative Drug Discovery, Inc., San Diego, CA, USA).

**Table 19. Biological Activity of Representative Compounds. IC₅₀ values are designated within the following ranges:**

| A: ≤ 0.1 µM | |
|---|---|
| B: > 0.1 µM to ≤ 1.0 µM | |
| C: > 1.0 µM to ≤ 10 µM | |
| D: >10 µM | |
| **Example** | **Imaging Assay IC₅₀ (µM)** |
| **1** | A |
| **24** | A |
| **25** | C |
| **34** | B |
| **35** | A |
| **38** | A |
| **42** | B |
| **43** | A |
| **44** | A |
| **45** | A |
| **46** | B |
| **47** | A |
| **48** | A |
| **51** | B |
| **60** | A |
| **62** | D |
| **63** | A |
| **64** | A |
| **65** | D |
| **66** | A |
| **67** | A |
| **68** | A |
| **69** | A |
| **70** | A |
| **71** | A |
| **73** | A |
| **76** | A |
| **77** | A |
| **78** | A |
| **79** | B |
| **80** | A |
| **81** | A |
| **82** | C |
| **83** | C |
| **84** | D |
| **85** | C |
| **86** | A |
| **87** | A |
| **88** | A |
| **89** | A |
| **90** | A |
| **91** | B |

| **Example** | **Imaging Assay IC₅₀ (µM)** |
|---|---|
| **92** | B |
| **93** | B |
| **94** | C |
| **95** | A |
| **96** | C |
| **97** | A |
| **98** | A |
| **100** | A |
| **101** | C |
| **105** | B |
| **106** | A |
| **107** | A |
| **108** | A |
| **109** | A |
| **115** | B |
| **117** | A |
| **120** | A |
| **122** | A |
| **123** | C |
| **124** | B |
| **132** | A |
| **133** | A |

### III. X-ray Crystallography

Compounds of the invention were designed to facilitate a direct and productive interaction between the cyano moiety of the central aryl ring and amino acid residue Glu325 of QPCTL. This interaction is illustrated in a comparison between a QPCTL Reference Compound (SEN177) and Example 1, as shown in the figures discussed below.

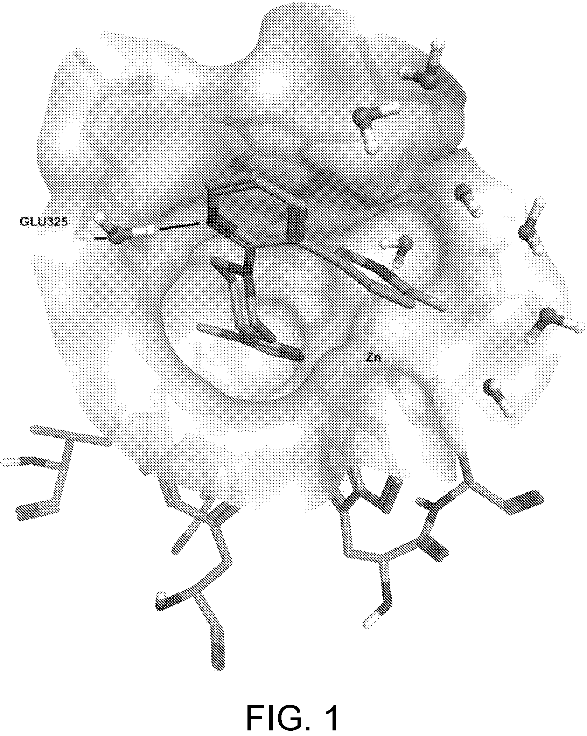

The X-ray crystal structure of QPCTL Reference Compound SEN177 bound in the active site of QPCTL is shown in Figure 1. There is a hydrogen bonding interaction between the pyridyl nitrogen and a bridging water molecule, which also interacts with Glu325 of QPCTL. With consideration to the bonding distance and geometry of the interaction between the pyridyl nitrogen and the bridging water, it was reasoned that replacement of the pyridyl nitrogen with a C-CN moiety would create a direct hydrogen bond between the cyano group of the ligand and Glu325 of QPCTL, by displacement of the bridging water. The entropic gain from the release of bound water resulted in significant enhancement of ligand binding affinity to QPCTL.

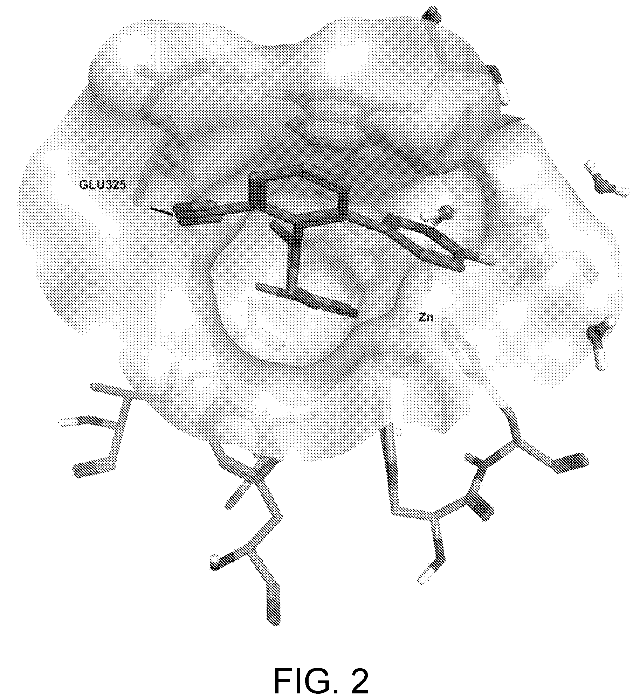

The X-ray structure of Example 1 bound in the active site of QPCTL is shown in Figure 2. In this structure, the bridging water molecule shown in the SEN177 structure (FIG. 1) is no longer present. Instead, the cyano group of Example 1 now occupies the bridging water molecule space and forms a hydrogen bond directly to residue Glu325 of QPCTL.

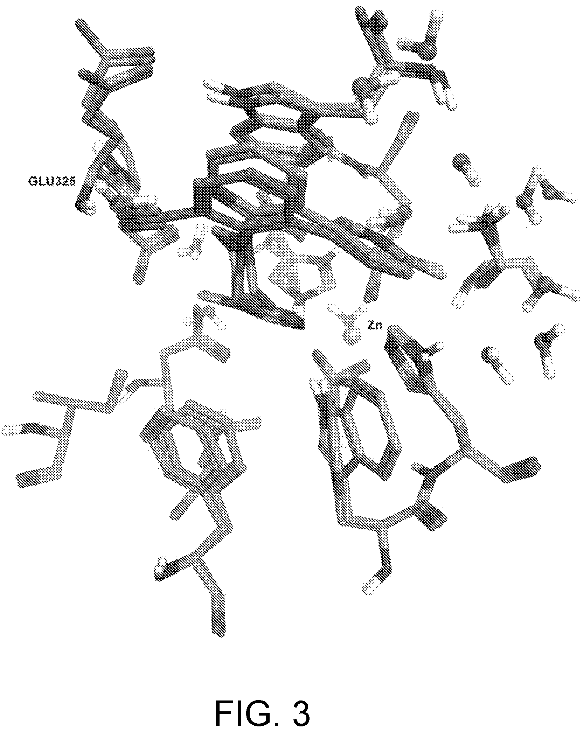

The overlay of ligands SEN177 and Example 1 in the QPCTL active site clearly demonstrates that the two ligands occupy the same binding site in QPCTL (FIG. 3).

The recombinant purified QPCTL truncate protein containing residues 53-382 of QPCTL, which was used in the QPCTL enzymatic assay, was also used for determination of QPCTL-ligand X-ray crystal structures. For the crystal structure of QPCTL with SEN177, QPCTL protein was incubated at 6.5 mg/mL with 1 mM SEN177 in a buffer, containing 25 mM Tris, pH 8.3, 150 mM NaCl. After an incubation period of approximately 1hr, aggregates were removed through centrifugation at 13500g for 3 minutes. The crystals were grown at 295K by the sitting drop vapor-diffusion method and mixing with a precipitant in a 1:1 ratio to give a 0.6ul drop. Crystals were passed quickly through a cryoprotectant buffer (15% w/v PEG 20000, 0.01 M Potassium Tartrate, 20% glycerol).

Diffraction data of the QCPTL ligand complex containing SEN177 were collected at the beamline 23-ID-D, GM/CA-XSD, Advanced Photon Source, equipped with a detector Pilatus3 6M at 100K. Data were reduced and scaled with XDS; CCP4 suite was employed for molecular replacement and refinement, and model building was performed using Coot. The data set was solved and refined to a final resolution of 1.24 Å in space group C2221. PDB entry 3pb7 was used as the search model for QPCTL.

For the crystal structure of QPCTL with Example 1, QPCTL crystals were produced using the hanging drop vapor diffusion method, using 1 uL protein at a concentration of 6.5mg/ml (25 mM Tris, pH 8.3, 150 mM NaCl) and 1uL of well solution consisting of 0.2 M sodium chloride, 0.1 M sodium cacodylate pH 6, 8% w/v PEG 8000. Example 1 (final concentration of 10 mM) was soaked into QPCTL crystals for 0.5~1hr. The crystals were cryoprotected in 30% glycerol.

Diffraction data of the QCPTL ligand complex containing the Example 1 were collected at the beamline P11, the high brilliance 3rd Generation Synchrotron Radiation Source at DESY, equipped with a detector Eiger2 X 16M at 100K. Data were reduced and scaled with XDS; CCP4 suite was employed for molecular replacement and refinement, and model building was performed using Coot. The data set was solved and refined to a final resolution of 2.74 Å in space group P21. PDB entry 3pb7 was used as the search model for QPCTL.

### III. Preparation of Pharmaceutical Dosage Forms

### Example 1: Oral capsule

The active ingredient is a compound described herein, or a pharmaceutically acceptable salt or solvate thereof. A capsule for oral administration is prepared by mixing 1-1000 mg of active ingredient with starch or other suitable powder blend. The mixture is incorporated into an oral dosage unit such as a hard gelatin capsule, which is suitable for oral administration.

### Example 2: Solution for injection

The active ingredient is a compound of Table 1, or a pharmaceutically acceptable salt or solvate thereof, and is formulated as a solution in sesame oil at a concentration of 50 mg-eq/mL.

## Claims

1. A compound of formula (II) or a pharmaceutically acceptable salt and/or solvate thereof: 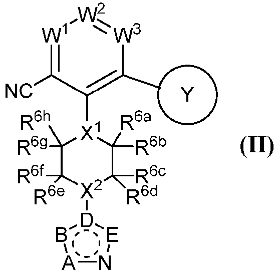 wherein
W¹ is N or CR¹, W² is N or CR², and W³ is N or CR³, wherein no more than one of W¹, W², and W³ is N;
X¹ and X² are independently selected from CR⁴ and N;
Ring Y 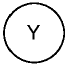 is of formula (a): 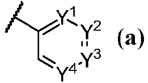 wherein Y¹, Y², Y³, and Y⁴ are independently selected from CR⁵ and N wherein Y¹, Y², Y³, and Y⁴ are not simultaneously N,
or Ring Y 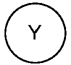 is of formula (b): 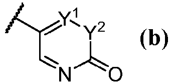 wherein Y¹ is CR⁵ and Y² is NR^{5'};
or Ring Y 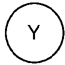 is of formula (c): 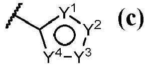 wherein Y¹, Y², Y³, and Y⁴ are independently selected from CR⁵ and N;
optionally, either Y¹ and Y², or Y² and Y³, or Y³ and Y⁴ represent a fused ring selected from a C₅-C₈-cycloalkyl, a C₆-C₁₀-aryl, a 5- to 10-membered heteroaryl (wherein 1-4 heteroaryl members are independently selected from N, O, and S), and a 5- to 8-membered heterocycloalkyl (wherein 1-4 ring members are independently selected from N, B, O, and S), wherein the ring is optionally substituted with 1 to 3 substituents independently selected from the group consisting of C₁-C₆-alkyl, C₃-C₈-cycloalkyl, 3- to 6-membered heterocycloalkyl (wherein 1-4 ring members are independently selected from N, O, and S), C₂-C₆-alkenyl, C₂-C₆-alkynyl, halo, C₁-C₆-haloalkyl, C₂-C₆-haloalkenyl, C₂-C₆-haloalkynyl, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2),
A, B, and E are independently selected from C, N, O, and S, and D is C or N,
wherein 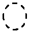 represents the presence of double bonds such that the ring A-B-D-E-N is aromatic and is optionally substituted with one or two substituents independently selected from C₁-C₃-alkyl, C₃-C₅-cycloalkyl, OH, OMe, NH₂, N(H)Me, NMe₂;
wherein no more than two of A, B, D, and E are simultaneously N, O, or S;
R¹, R², and R³ are independently selected from the group consisting of hydrogen, halo, cyano, nitro, -R^{b}-OR^{a}, -R^{b}-O-R^{c}-O-R^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, - R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₁-C₆-haloalkyl, C₂-C₆-alkenyl, C₂-C₆-alkynyl, C₃-C₈-cycloalkyl, C₆-C₁₀-aryl, -(C₁-C₆-alkyl)(C₆-C₁₀-aryl), 5- to 10-membered heteroaryl (wherein 1-4 heteroaryl members are independently selected from N, O, and S) optionally fused to 3- to 6-membered heterocycloalkyl (wherein 1-4 ring members are independently selected from N, O, and S), and 3- to 6-membered heterocycloalkyl (wherein 1-4 ring members are independently selected from N, O, and S),
or R¹ and R², or R² and R³, together with the carbon atoms to which they are bound, form a fused C₅-C₈-cycloalkyl, C₆-C₁₀-aryl, 5- to 10-membered heteroaryl (wherein 1-4 heteroaryl members are independently selected from N, O, and S), and 5- to 8-membered heterocycloalkyl (wherein 1-4 ring members are independently selected from N, O, and S);
wherein any heteroaryl or heterocycloalkyl in R¹, R², and R³ is optionally and independently substituted with 1 to 3 substituents selected from the group consisting of C₁-C₆-alkyl, halo, hydroxy, C₃-C₈-cycloalkyl, heterocycloalkyl (wherein 1-4 ring members are independently selected from N, O, and S), and -R^{b}-N(R^{a})₂;
R⁴ in each instance is independently H, OH, halo, C₁-C₆-alkyl, or C₁-C₆-alkoxy;
R⁵ in each instance is independently selected from the group consisting of hydrogen, halo, cyano, nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, - R^{b}-O-R^{c}-N(R^{a})₂, -B(OR^{a})₂, -R^{b}-N(R^{a})-R^{c}-N(R^{a})₂, -R^{b}-N(R^{a})C(O)OR^{a}, -R^{b}-N(R^{a})C(O)R^{a}, - R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-OS(O)ₜF (where t is 1 or 2), -R^{b}-S(O)ₜN(R^{a})₂ (where t is 1 or 2), C₁-C₆-alkyl, C₂-C₆-alkynyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S);
R^{5'} is selected from the group consisting of hydrogen, -R^{c}-R^{a}, -R^{c}-OR^{a}, -R^{b}-OC(O)-R^{a}, -R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S);
wherein any heteroaryl or heterocycloalkyl in R⁵ and R^{5'} is optionally and independently substituted with 1 to 3 substituents selected from the group consisting of C₁-C₆-alkyl, halo, hydroxy, C₃-C₈-cycloalkyl, and -R^{b}-N(R^{a})₂;
R^{6a}, R^{6b}, R^{6c}, R^{6d}, R^{6e}, R^{6f}, R^{6g}, and R^{6h} are independently selected from the group consisting of H, halo, NO₂, OH, CN, -R^{b}-N(R^{a})₂, -R^{b}-OH, C₁-C₆-alkyl, and C₁-C₆-alkoxy;
and/or, optionally, R^{6a} and R^{6b}, or R^{6c} and R^{6d}, or R^{6e} and R^{6f}, or R^{6g} and R^{6h} independently represent oxo, thioxo, imino, or oximo;
and/or, optionally, R^{6a} and R^{6b}, or R^{6c} and R^{6d}, or R^{6e} and R^{6f}, or R^{6g} and R^{6h}, together with the carbon atoms to which they are bound, independently combine to form a fused ring selected from a C₃-C₆-cycloalkyl and C₃-C₆-heterocycloalkyl (wherein 1-4 ring members are independently selected from N, O, and S);
and/or, optionally, one of R^{6c} and R^{6d} together with one of R^{6e} and R^{6f} represent a bond between the ring carbon members to which they are bound;
R^{a} in each instance is independently selected from hydrogen, C₁-C₆-alkyl, C₃-C₈-cycloalkyl, - (C₁-C₆-alkyl)(C₃-C₈-cycloalkyl), C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S);
R^{b} in each instance is independently selected from a direct bond, a straight or branched C₂-C₆-alkylene, and C₂-C₆-alkenylene chain;wherein any heteroaryl or heterocycloalkyl in R^{a} and R^{b} is optionally and independently substituted with 1 to 3 substituents selected from the group consisting of C₁-C₆-alkyl, halo, hydroxy,
R^{c} in each instance is independently selected from a straight or branched C₂-C₆-alkylene and C₂-C₆-alkenylene chain.

2. The compound or a pharmaceutically acceptable salt and/or solvate thereof according to claim 1, wherein the compound is of formula (I): 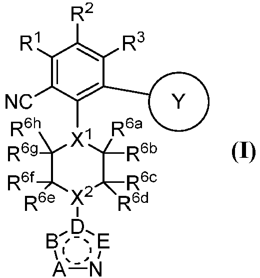 wherein
each of X¹ and X² is N;
Ring Y 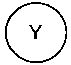 is of formula (a): 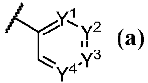 wherein Y¹, Y², Y³, and Y⁴ are independently selected from CR⁵ and N wherein Y¹, Y², Y³, and Y⁴ are not simultaneously N,
or Ring Y 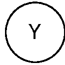 is of formula (b): 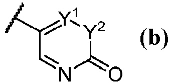 wherein Y¹ is CR⁵ and Y² is NR^{5'};
optionally, either Y¹ and Y², or Y² and Y³, or Y³ and Y⁴ represent a fused ring selected from a C₅-C₈-cycloalkyl, a C₆-C₁₀-aryl, a 5- to 10-membered heteroaryl (wherein 1-4 heteroaryl members are independently selected from N, O, and S), and a 5- to 8-membered heterocycloalkyl (wherein 1-4 ring members are independently selected from N, O, and S), wherein the ring is optionally substituted with 1 to 3 substituents independently selected from the group consisting of C₁-C₆-alkyl, C₃-C₈-cycloalkyl, 3- to 6-membered heterocycloalkyl (wherein 1-4 ring members are independently selected from N, O, and S), C₂-C₆-alkenyl, C₂-C₆-alkynyl, halo, C₁-C₆-haloalkyl, C₂-C₆-haloalkenyl, C₂-C₆-haloalkynyl, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2),
A, B, and E are independently selected from C, N, O, and S, and D is C or N,
wherein 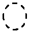 represents the presence of double bonds such that the ring A-B-D-E-N is aromatic and is optionally substituted with one or two substituents independently selected from C₁-C₃-alkyl, C₃-C₅-cycloalkyl, OH, OMe, NH₂, N(H)Me, NMe₂;
wherein no more than two of A, B, D, and E are simultaneously N, O, or S;
R¹, R², and R³ are independently selected from the group consisting of hydrogen, halo, cyano, nitro, -R^{b}-OR^{a}, -R^{b}-O-R^{a}, -R^{b}-OC(O)-R^{a}, -R^{b}-OC(O)-OR^{a}, -R^{b}-OC(O)-N(R^{a})₂, - R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, -(C₁-C₆-alkyl)(C₆-C₁₀-aryl), 5- to 10-membered heteroaryl (wherein 1-4 heteroaryl members are independently selected from N, O, and S), and 3- to 6-membered heterocycloalkyl (wherein 1-4 ring members are independently selected from N, O, and S),
or R¹ and R², or R² and R³, together with the carbon atoms to which they are bound, form a fused C₅-C₈-cycloalkyl, C₆-C₁₀-aryl, 5- to 10-membered heteroaryl (wherein 1-4 heteroaryl members are independently selected from N, O, and S), and 5- to 8-membered heterocycloalkyl (wherein 1-4 ring members are independently selected from N, O, and S);
R⁴ in each instance is independently H, OH, halo, C₁-C₆-alkyl, or C₁-C₆-alkoxy;
R⁵ in each instance is independently selected from the group consisting of hydrogen, halo, cyano, nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, - R^{b}-O-R^{c}-N(R^{a})₂, -W-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, 5-to 10-membered heteroaryl (wherein 1-4 heteroaryl members are independently selected from N, O, and S), and 3- to 6-membered heterocycloalkyl (wherein 1-4 ring members are independently selected from N, O, and S);
R^{5'} is selected from the group consisting of hydrogen, -R^{c}-R^{a}, -R^{c}-OR^{a}, -R^{b}-OC(O)-R^{a}, -R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), -R^{b}-S(O)ₜN(R^{a})₂ (where t is 1 or 2), C₁-C₆-alkyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S);
R^{6a}, R^{6b}, R^{6c}, R^{6d}, R^{6e}, R^{6f}, R^{6g}, and R^{6h} are independently selected from the group consisting of H, halo, NO₂, OH, CN, -R^{b}-N(R^{a})₂, -R^{b}-OH, C₁-C₆-alkyl, and C₁-C₆-alkoxy;
or, optionally, R^{6a} and R^{6b}, or R^{6c} and R^{6d}, or R^{6e} and R^{6f}, or R^{6g} and R^{6h} independently represent oxo, thioxo, imino, or oximo;
or, optionally, R^{6a} and R^{6b}, or R^{6c} and R^{6d}, or R^{6e} and R^{6f}, or R^{6g} and R^{6h}, together with the carbon atoms to which they are bound, independently combine to form a fused ring selected from a C₃-C₆-cycloalkyl and C₃-C₆-heterocycloalkyl (wherein 1-4 ring members are independently selected from N, O, and S);
R^{a} in each instance is independently selected from hydrogen, C₁-C₆-alkyl, C₃-C₈-cycloalkyl, C₆-C₁₀-aryl, 5- to 10-membered heteroaryl (wherein 1-4 heteroaryl members are independently selected from N, O, and S), and 3- to 6-membered heterocycloalkyl (wherein 1-4 ring members are independently selected from N, O, and S);
R^{b} in each instance is independently selected from a direct bond, a straight or branched C₂-C₆-alkylene, and C₂-C₆-alkenylene chain;
R^{c} in each instance is independently selected from a straight or branched C₂-C₆-alkylene and C₂-C₆-alkenylene chain.

3. The compound or pharmaceutically acceptable salt and/or solvate thereof according to claim 1, wherein W¹ is N, W² is CR², and W³ is CR³; or W¹ is CR¹, W² is CR², and W³ is N; or W² is CR², and W³ is CR³.

4. The compound or pharmaceutically acceptable salt and/or solvate thereof according to any of claims 1 to 3, wherein D is C, and wherein A, B, and E are independently selected from C and N, or at least one of A, B, and E is N, or two of A, B, and E are N.

5. The compound or pharmaceutically acceptable salt and/or solvate thereof according to any of claims 1 to 3, wherein the A-B-D-E-N ring is an optionally substituted ring selected from the group consisting of:

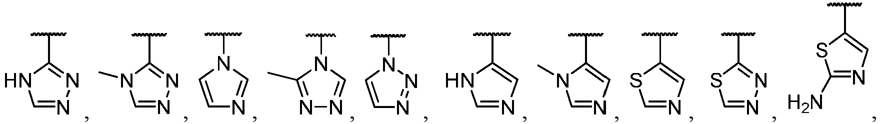

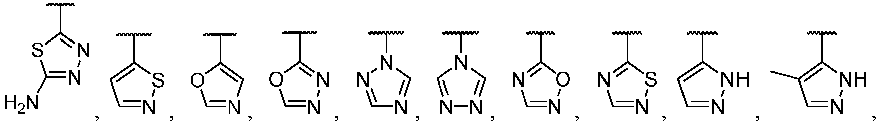

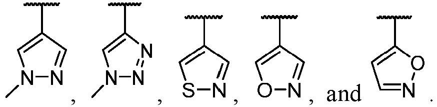

6. The compound or pharmaceutically acceptable salt and/or solvate thereof according to any of claims 1 to 5, wherein Ring Y is of formula (a), and wherein
one of Y¹, Y², Y³, and Y⁴ is N and each of the remaining three is CR⁵, or wherein
two of Y¹, Y², Y³, and Y⁴ are N and each of the remaining two is CR⁵, or wherein
either Y¹ and Y² or Y³ and Y⁴ represent an optionally substituted fused ring.

7. The compound or pharmaceutically acceptable salt and/or solvate thereof according to any of claims 1 to 6, wherein R¹ is selected from the group consisting of H, halo, C₁-C₆-alkyl, -R^{b}-OR^{a} (wherein R^{b} is a direct bond and R^{a} is C₁-C₆-alkyl), C₆-C₁₀-aryl, C₃-C₈-cycloalkyl, 3- to 6-membered heterocycloalkyl (wherein 1-4 ring members are independently selected from N, O, and S), and 5- to 6-membered heteroaryl (wherein 1-4 heteroaryl members are independently selected from N, O, and S), and
each of R² and R³ is independently H, halo, cyano, C₁-C₆-alkyl, C₃-C₈-cycloalkyl, 3- to 6-membered heterocycloalkyl (wherein 1-4 ring members are independently selected from N, O, and S), and 5- to 6-membered heteroaryl (wherein 1-4 heteroaryl ring members are independently selected from N, O, and S), -R^{b}-OR^{a} (wherein R^{b} is a direct bond and R^{a} is C₁-C₆-alkyl), or CF₃.

8. The compound or pharmaceutically acceptable salt and/or solvate thereof according to claim 1, wherein the compound is a compound of formula (IA), (IB), or (ID):

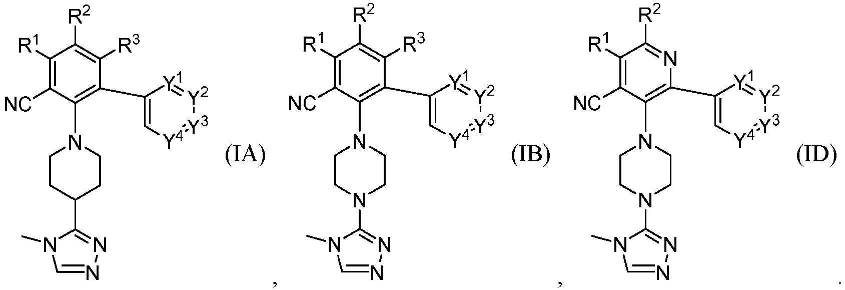

9. The compound, or pharmaceutically acceptable salt and/or solvate thereof, according to claim 8, wherein:
R¹ is selected from the group consisting of H, halo, C₁-C₆-alkoxy, C₆-C₁₀-aryl, C₁-C₆-alkyl, C₃-C₆-cycloalkyl, 5- to 6-membered heteroaryl (wherein 1-4 heteroaryl members are independently selected from N, O, and S), and 3- to 6-membered heterocycloalkyl (wherein 1-4 ring members are independently selected from N, O, and S);
each of R² is H, halo, cyano, -R^{b}-S(O)ₜR^{a} (where t is 1 or 2), C₁-C₆-alkyl, C₃-C₆-cycloalkyl, or CF₃, and
R³, when present, is halo, cyano, -R^{b}-S(O)ₜR^{a} (where t is 1 or 2), C₁-C₆-alkyl, C₃-C₆-cycloalkyl, or CF₃; and wherein
one of Y¹, Y², Y³, and Y⁴ is N and each of the remaining three is CR⁵, or
two of Y¹, Y², Y³, and Y⁴ are N and each of the remaining two is CR⁵.

10. The compound, or pharmaceutically acceptable salt and/or solvate thereof, according to claim 8 or 9, wherein the ring containing Y¹, Y², Y³, and Y⁴ is:

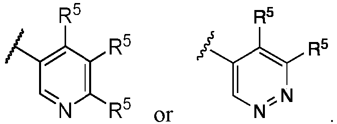

11. The compound, or pharmaceutically acceptable salt and/or solvate thereof, according to any of claims 8 to 10, wherein the ring containing Y¹, Y², Y³, and Y⁴ is:

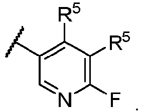

12. A compound or pharmaceutically acceptable salt and/or solvate thereof selected from the following table:
| | |
|---|---|
| **4** | 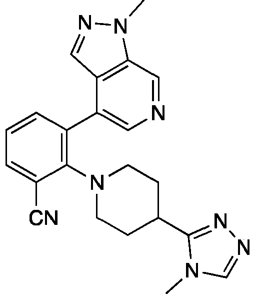 |
| **5** | 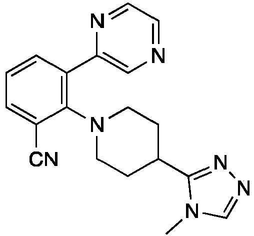 |
| **6** | 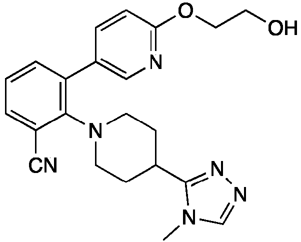 |
| **7** | 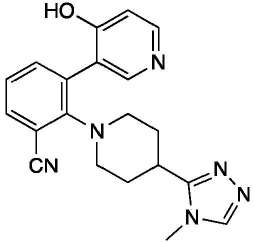 |
| **8** | 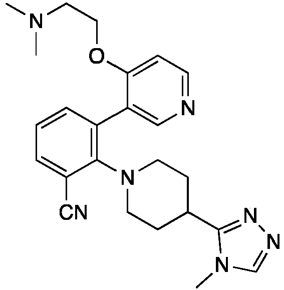 |
| **9** | 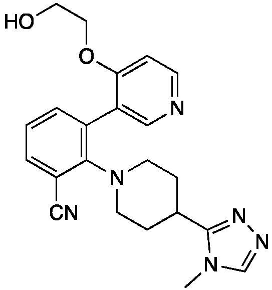 |
| **11** | 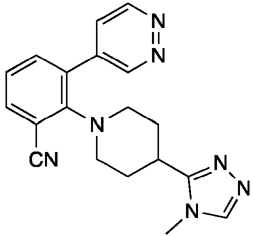 |
| **12** | 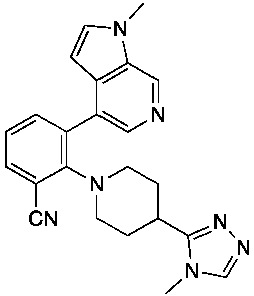 |
| **13** | 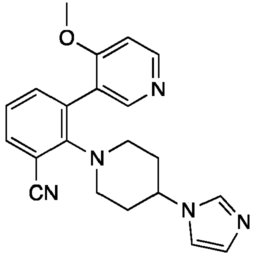 |
| **14** | 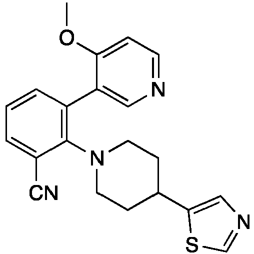 |
| **15** | 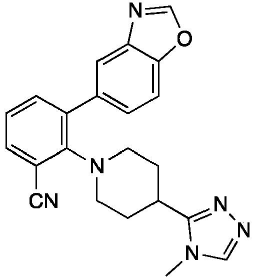 |
| **16** | 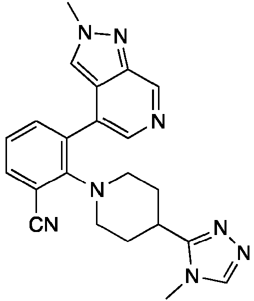 |
| **17** | 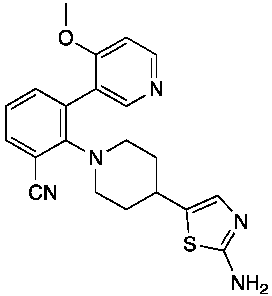 |
| **18** | 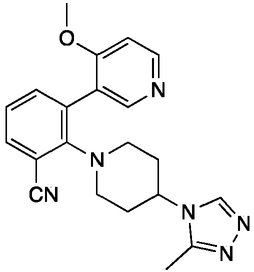 |
| **19** | 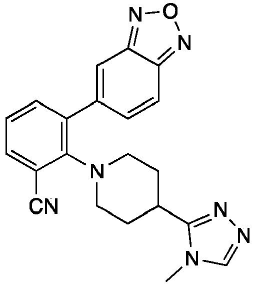 |
| **20** | 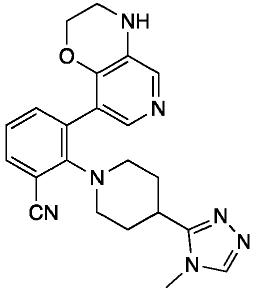 |
| **21** | 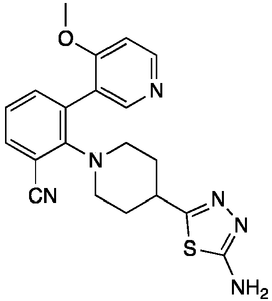 |
| **22** | 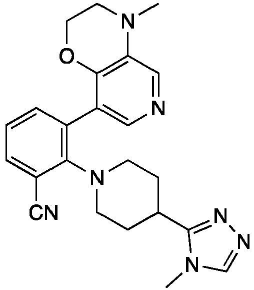 |
| **23** | 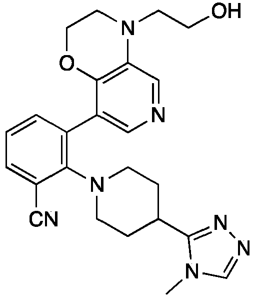 |
| **24** | 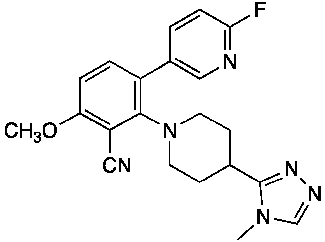 |
| **25** | 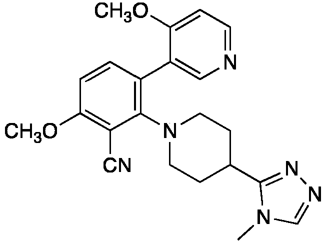 |
| **26** | 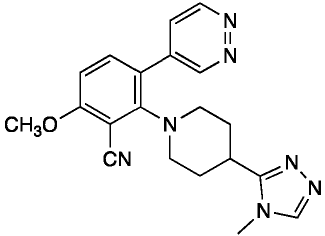 |
| **27** | 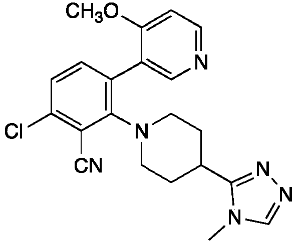 |
| **28** | 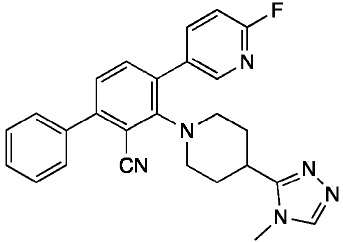 |
| **30** | 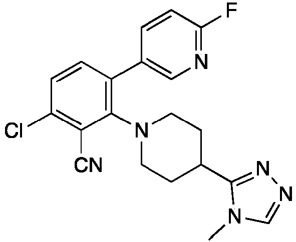 |
| **31** | 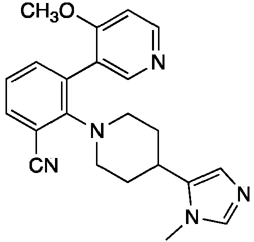 |
| **32** | 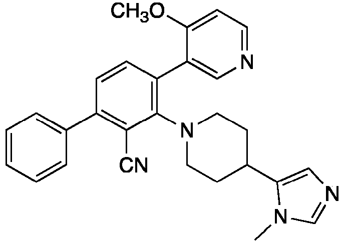 |
| **33** | 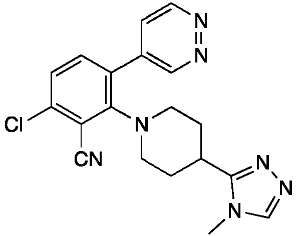 |
| **34** | 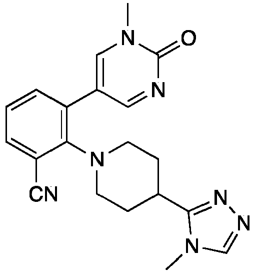 |
| **35** | 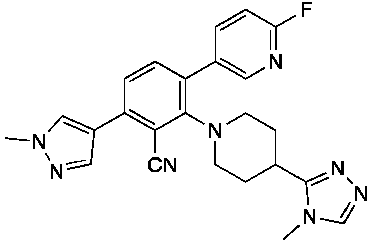 |
| **36** | 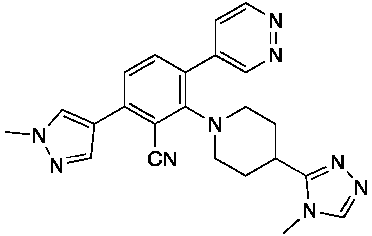 |
| **37** | 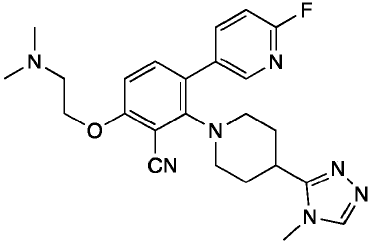 |
| **38** | 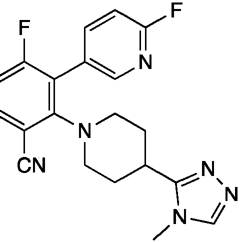 |
| **39** | 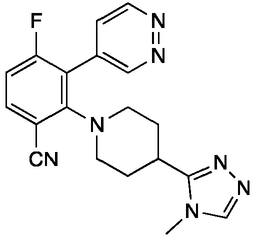 |
| **40** | 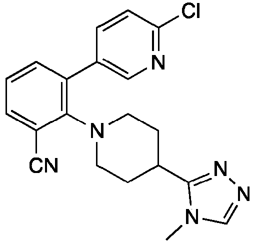 |
| **41** | 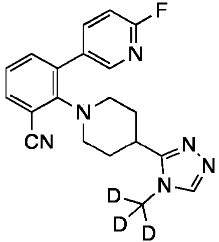 |
| **42** | 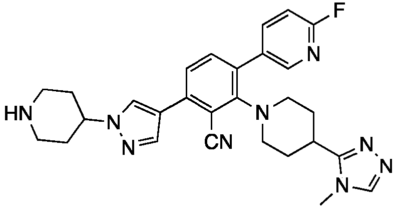 |
| **43** | 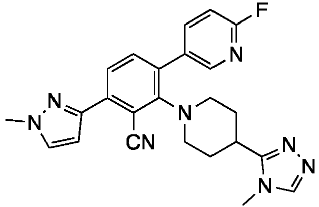 |
| **44** | 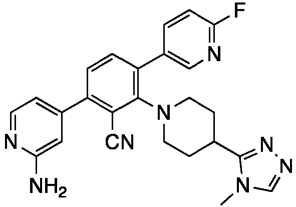 |
| **45** | 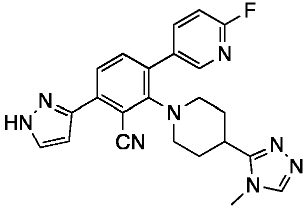 |
| **46** | 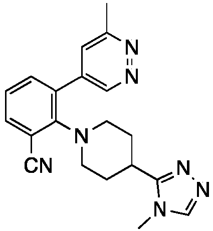 |
| **48** | 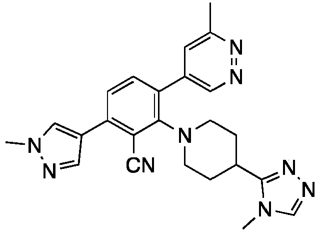 |
| **49** | 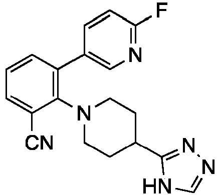 |
| **50** | 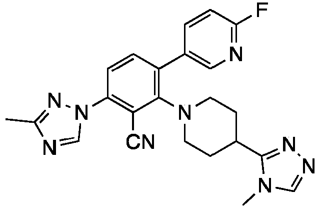 |
| **51** | 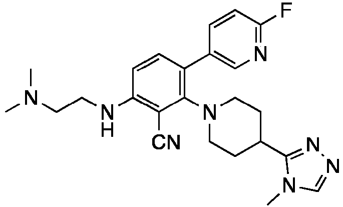 |
| **52** | 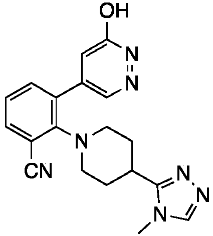 |
| **53** | 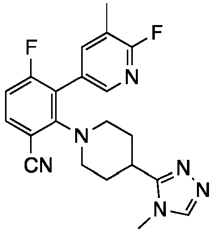 |
| **54** | 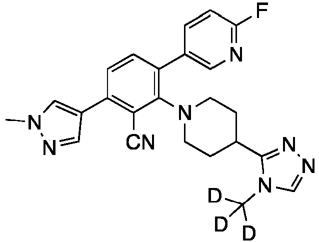 |
| **55** | 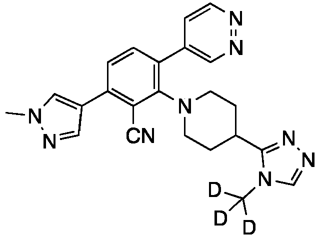 |
| **56** | 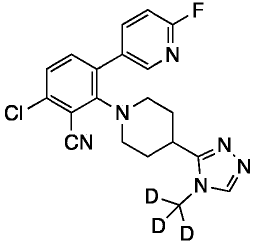 |
| **57** | 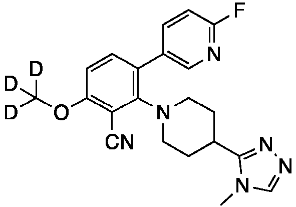 |
| **58** | 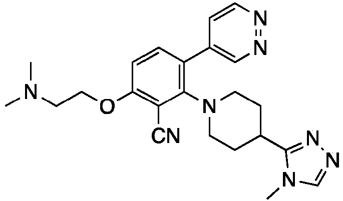 |
| **59** | 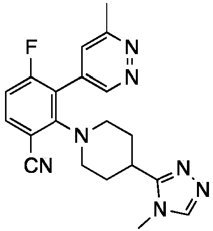 |
| **60** | 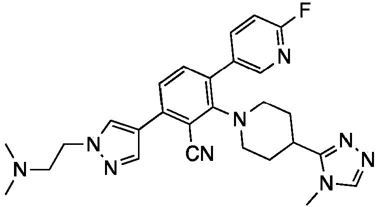 |
| **61** | 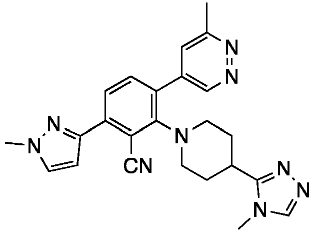 |
| **62** | 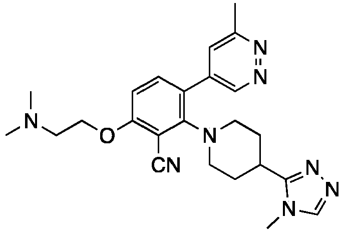 |
| **63** | 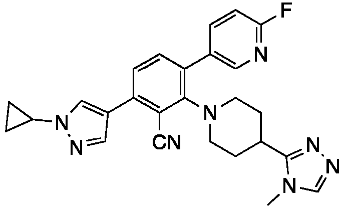 |
| **64** | 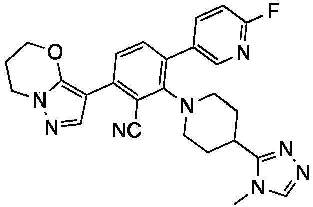 |
| **65** | 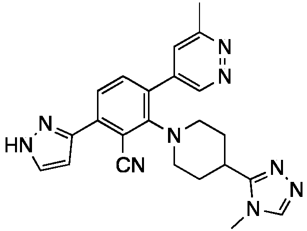 |
| **66** | 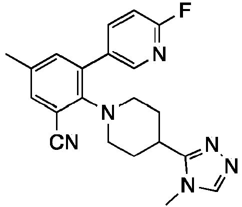 |
| **67** | 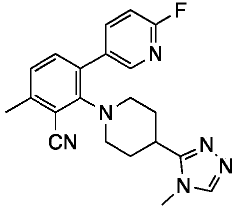 |
| **68** | 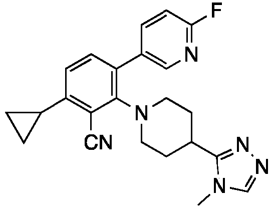 |
| **69** | 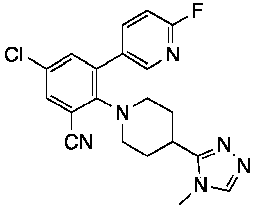 |
| **70** | 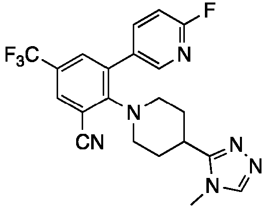 |
| **71** | 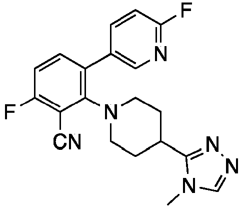 |
| **72** | 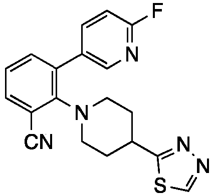 |
| **73** | 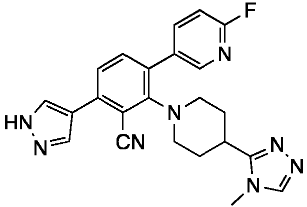 |
| **74** | 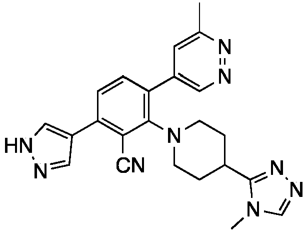 |
| **75** | 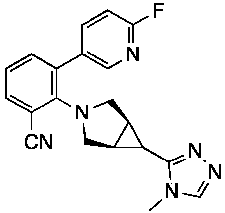 |
| **76** | 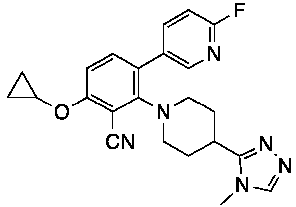 |
| **77** | 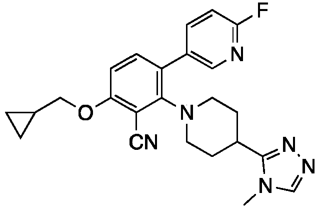 |
| **78** | 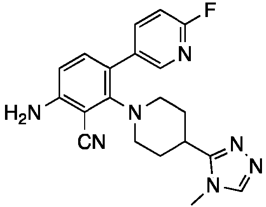 |
| **79** | 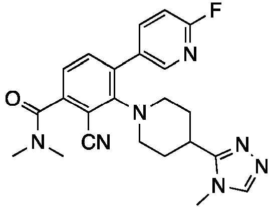 |
| **80** | 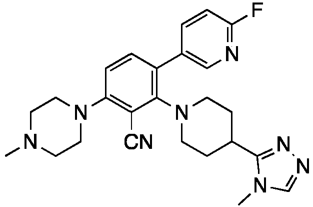 |
| **81** | 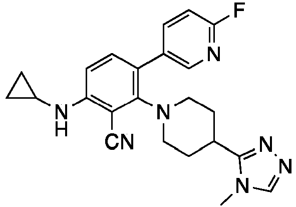 |
| **82** | 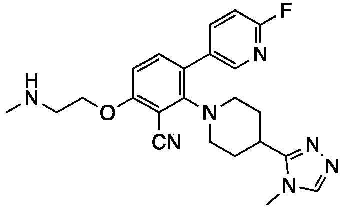 |
| **83** | 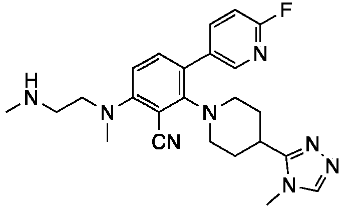 |
| **84** | 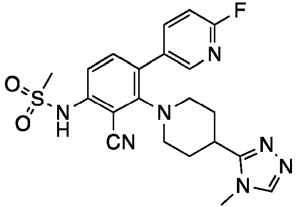 |
| **85** | 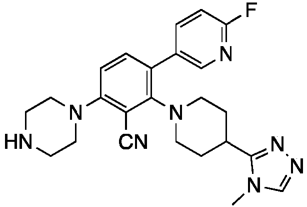 |
| **86** | 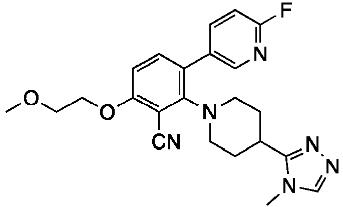 |
| **87** | 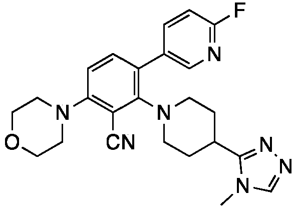 |
| **88** | 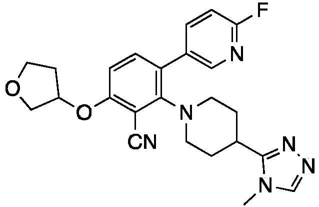 |
| **89** | 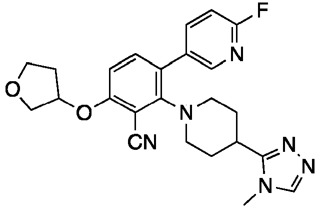 |
| **90** | 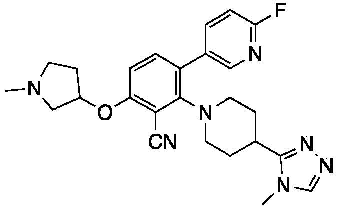 |
| **91** | 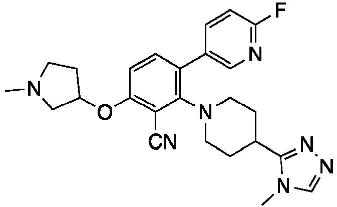 |
| **92** | 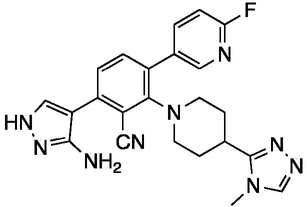 |
| **93** | 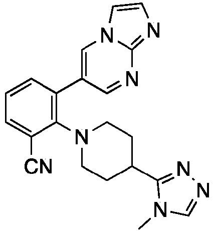 |
| **94** | 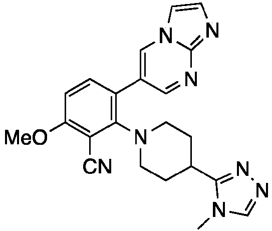 |
| **95** | 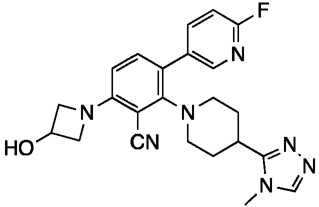 |
| **96** | 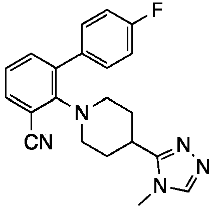 |
| **97** | 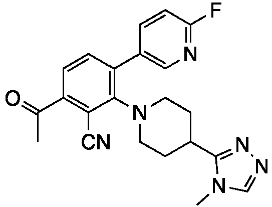 |
| **98** | 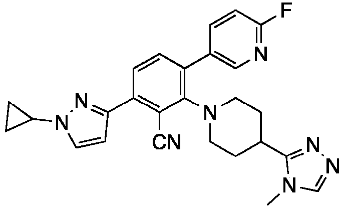 |
| **99** | 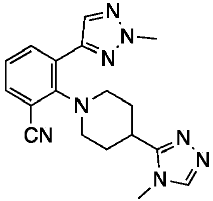 |
| **100** | 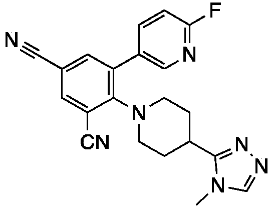 |
| **101** | 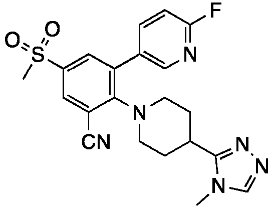 |
| **102** | 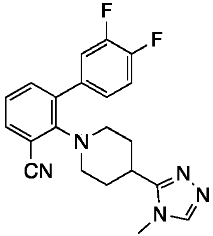 |
| **103** | 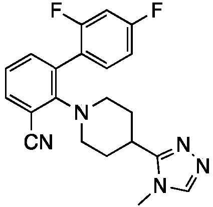 |
| **104** | 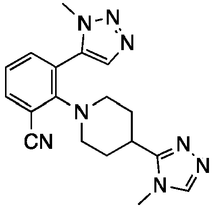 |
| **105** | 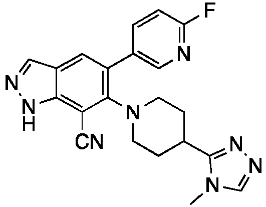 |
| **106** | 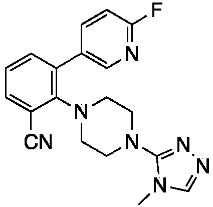 |
| **107** | 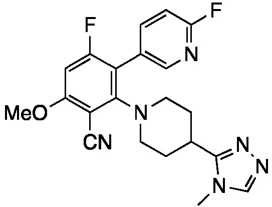 |
| **108** | 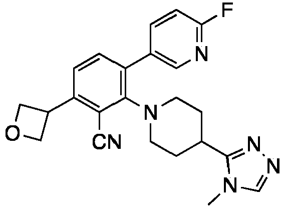 |
| **109** | 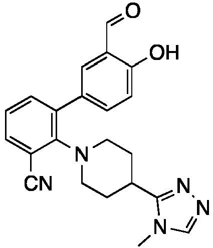 |
| **110** | 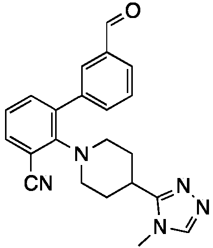 |
| **111** | 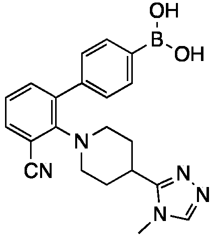 |
| **112** | 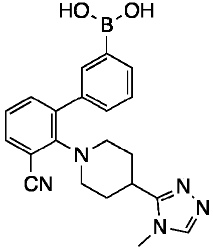 |
| **113** | 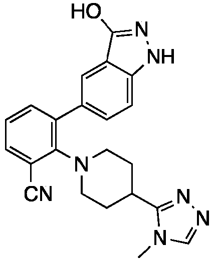 |
| **114** | 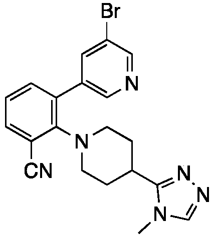 |
| **115** | 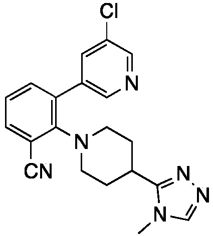 |
| **116** | 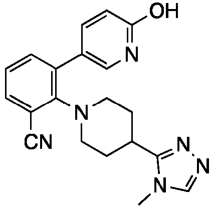 |
| **117** | 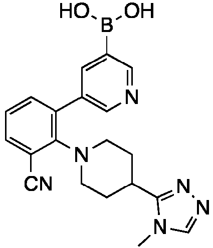 |
| **118** | 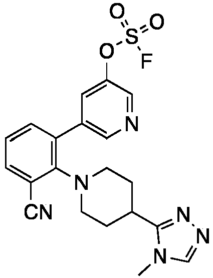 |
| **119** | 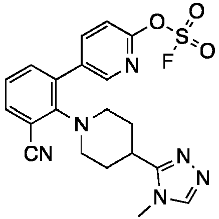 |
| **120** | 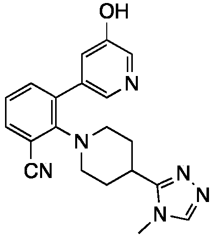 |
| **121** | 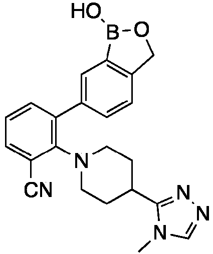 |
| **122** | 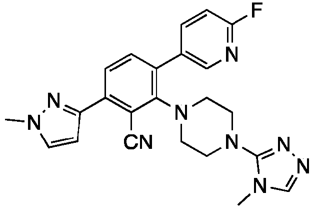 |
| **123** | 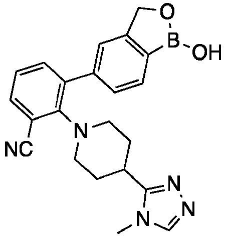 |
| **124** | 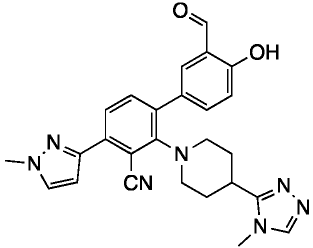 |
| **125** | 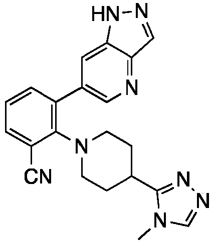 |
| **126** | 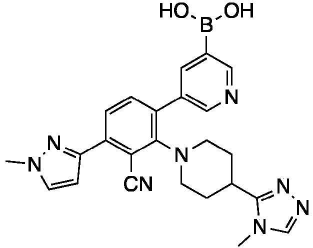 |
| **127** | 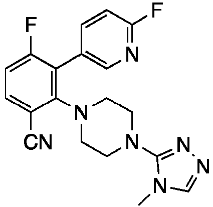 |
| **128** | 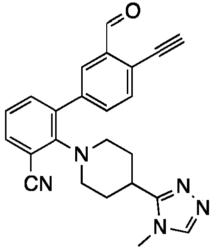 |
| **129** | 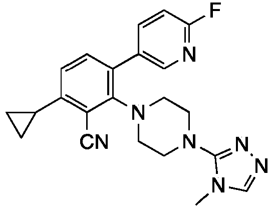 |
| **130** | 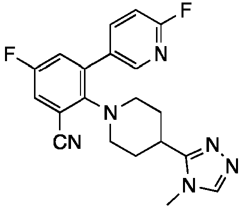 |
| **131** | 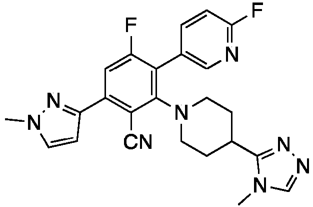 |
| **134** | 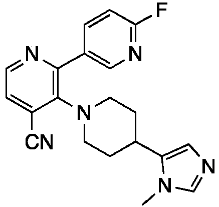 |
| **135** | 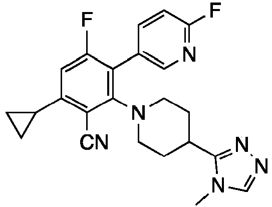 |
| **136** | 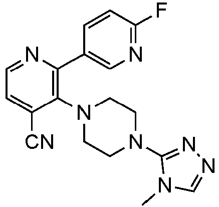 |
| **137** | 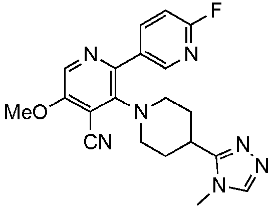 |
| **138** | 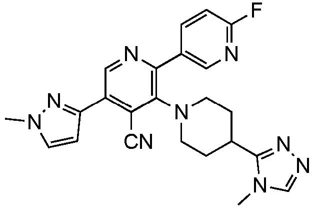 |
| **139** | 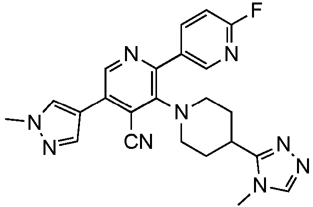 |
or a compound or pharmaceutically acceptable salt and/or solvate thereof selected from the following table:

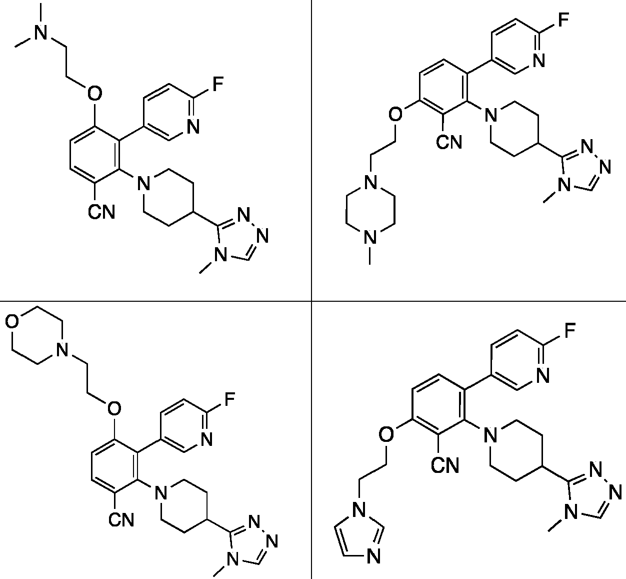

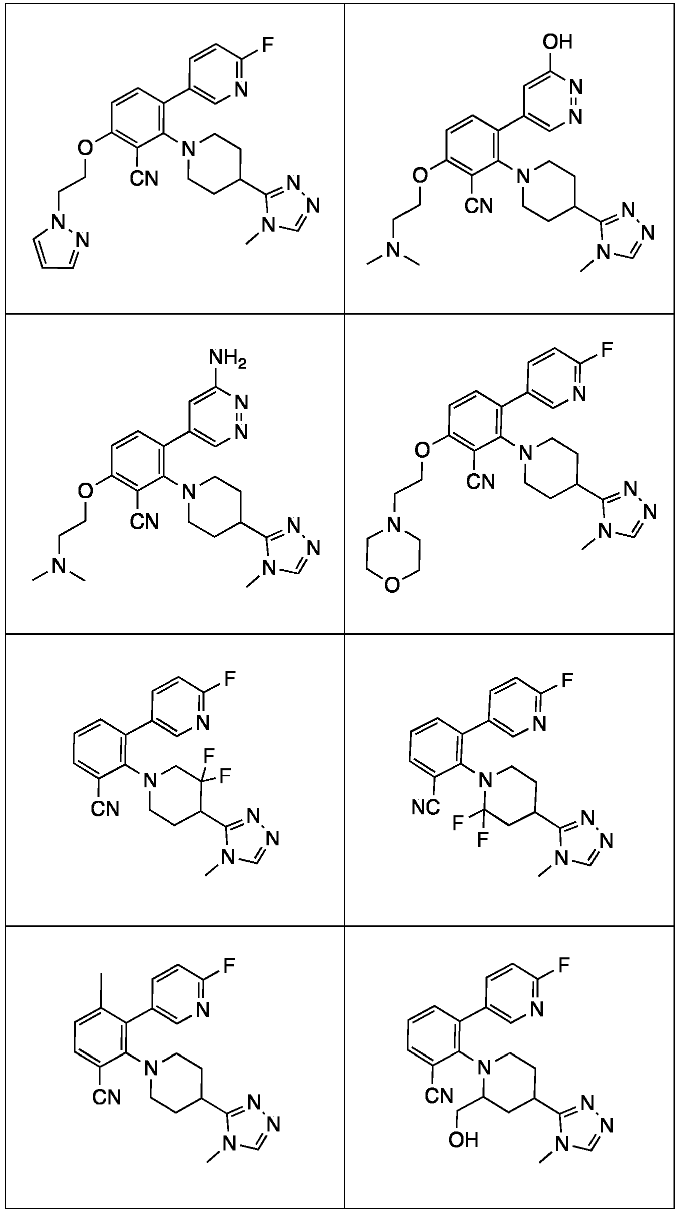

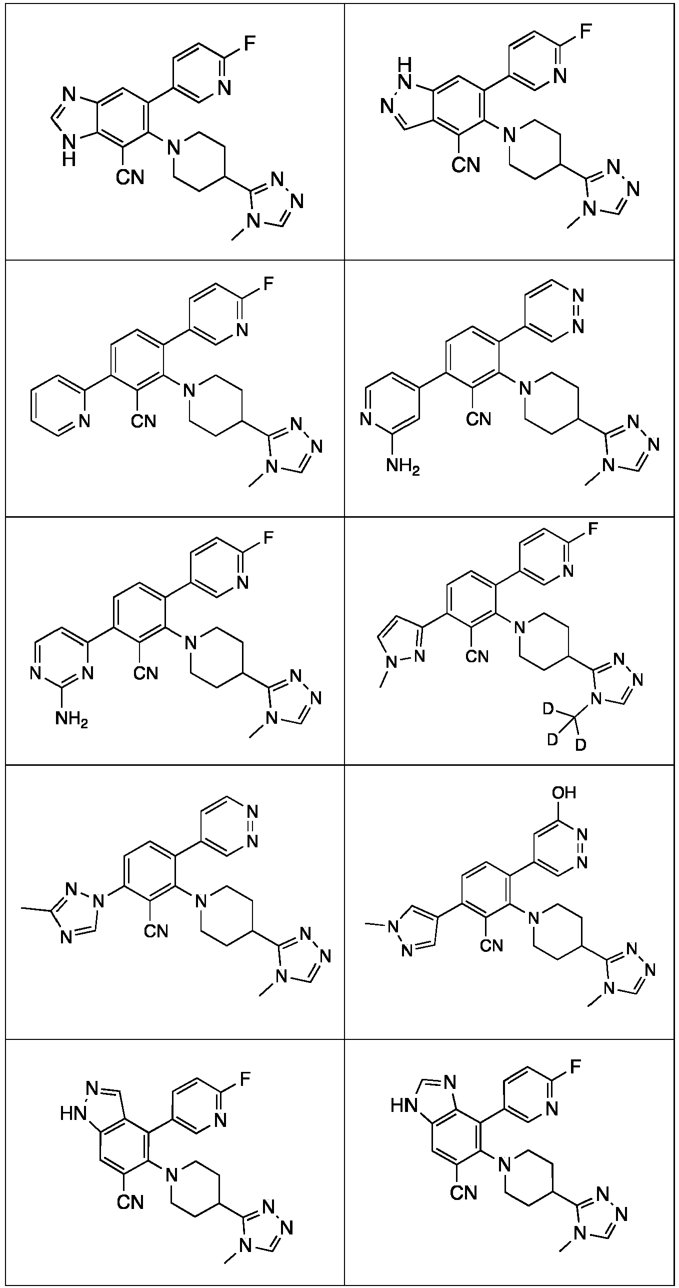

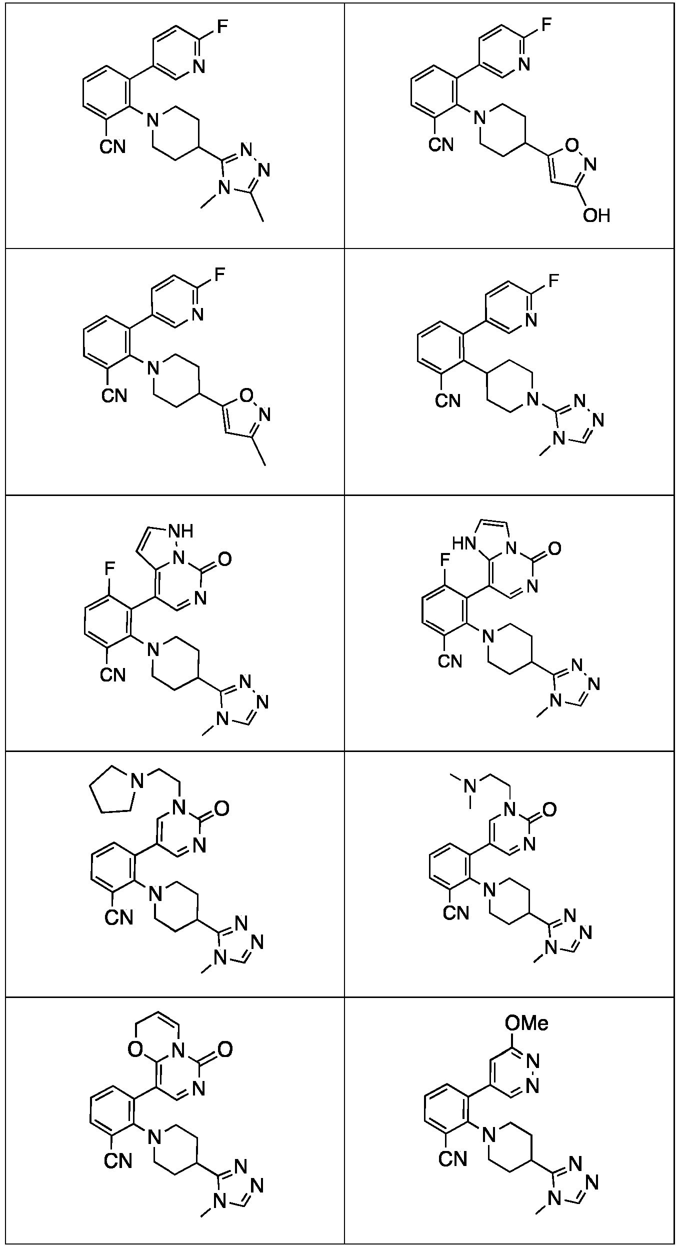

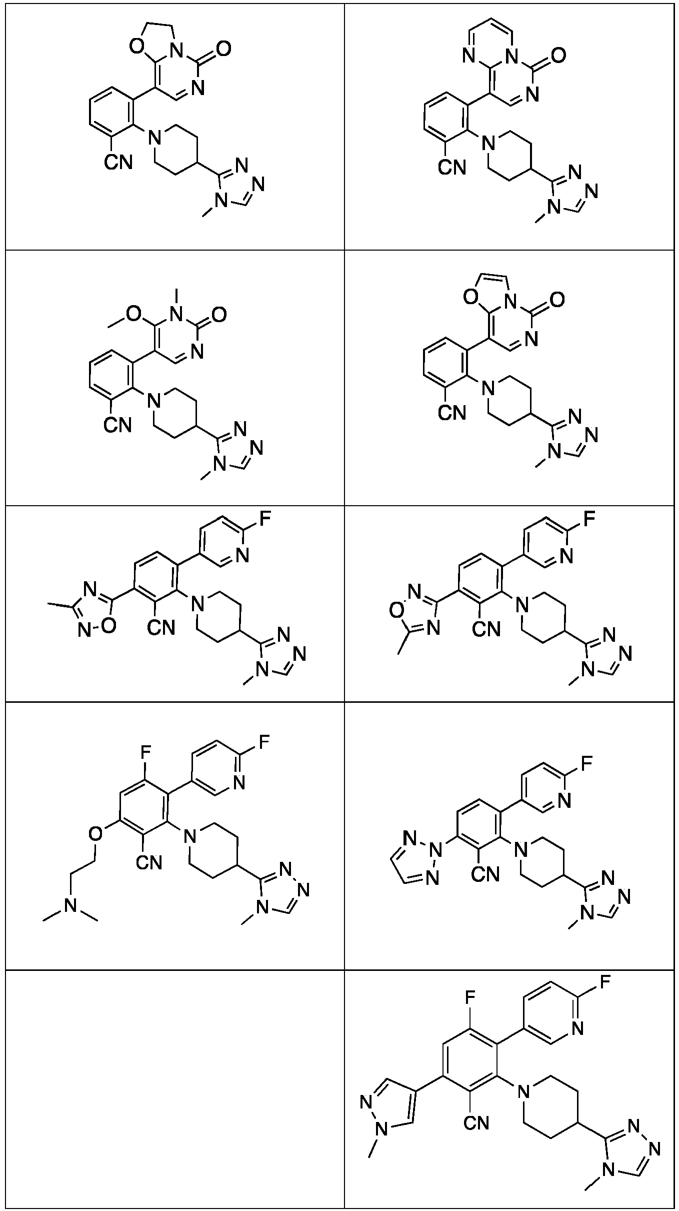

13. A pharmaceutical composition comprising a compound or pharmaceutically acceptable salt and/or solvate thereof according to any of claims 1 to 12 and a pharmaceutically acceptable carrier.

14. A compound according to any of claims 1 - 12 for use in a method for treating a disease in a patient suffering therefrom, wherein the disease is a cancer, a neurodegenerative disease, an inflammatory or autoimmune disease, or cardiovascular disease.

15. The compound for use according to claim 14, wherein the cancer is a leukemia or lymphoma selected from the group consisting of acute myeloid leukemia (AML), chronic myeloid leukemia (CML), acute lymphocytic leukemia (ALL), chronic lymphocytic leukemia (CLL), and non-Hodgkin's lymphoma (NHL), Burkitt lymphoma, hairy cell lymphoma (HCL), Waldenstrom macroglobulinemia, chronic lymphocytic leukemia/small lymphocytic lymphoma (CLL/SLL), diffuse large B cell lymphoma (DLBCL), B cell chronic lymphocytic leukemia (B-CLL), mantle cell lymphoma (MCL), follicular lymphoma (FL), marginal zone lymphoma (MZL), pre-B acute lymphoblastic leukemia (pre-B ALL), multiple myeloma (MM), ovarian cancer, gliomas, colon cancer, breast cancer, bladder cancer, gastric cancer, esophageal cancer, pancreatic cancer, liver cancer, small cell lung cancer (SCLC), non-small cell lung cancer (NSCLC), head and neck squamous cell cancer, mesothelioma, melanoma, glioma, glioblastoma, pancreatic neuroendocrine tumors, basal cell carcinoma, squamous cell carcinoma, renal cell carcinoma, invasive ductal carcinoma, adenocarcinoma, Merkel cell carcinoma, skin cancer, prostate cancer, colorectal cancer, soft tissue sarcoma, osteosarcoma, Ewing's sarcoma, chondrosarcoma, and myeloma;
the neurodegenerative disease is selected from the group consisting of Alzheimer's disease, Parkinson's disease, amyotrophic lateral sclerosis, Friedreich ataxia, Huntington's disease, Lewy body dementia, and spinal muscular atrophy; and
the cardiovascular disease is atherosclerosis.

## Patentansprüche

1. Verbindung der Formel (II) oder pharmazeutisch unbedenkliches Salz und/oder Solvat davon: 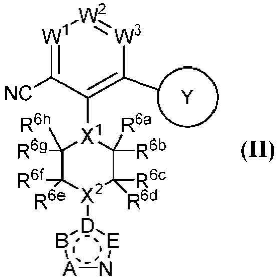 wobei
W¹ N oder CR¹ ist, W² N oder CR² ist und W³ N oder CR³ ist, wobei nicht mehr als eines von W¹, W² und W³ N ist;
X¹ und X² unabhängig ausgewählt sind aus CR⁴ und N;
Ring Y 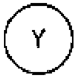 der Formel (a) entspricht: 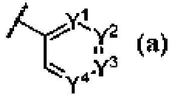
wobei Y¹, Y², Y³ und Y⁴ unabhängig aus CR⁵ und N ausgewählt sind, wobei Y¹, Y², Y³ und Y⁴ nicht gleichzeitig N sind,
oder Ring Y 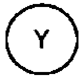 der Formel (b) entspricht: 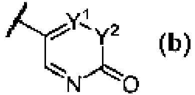
wobei Y¹ CR⁵ ist und Y² NR^{5'} ist;
oder Ring Y 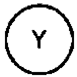 der Formel (c) entspricht: 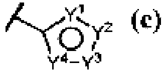
wobei Y¹, Y², Y³ und Y⁴ unabhängig aus CR⁵ und N ausgewählt sind;
optional entweder Y¹ und Y² oder Y² und Y³ oder Y³ und Y⁴ einen kondensierten Ring darstellen, ausgewählt aus einem C₅-C₈-Cycloalkyl, einem C₆-C₁₀-Aryl, einem 5- bis 10-gliedrigen Heteroaryl (wobei 1-4 Heteroarylglieder unabhängig ausgewählt sind aus N, O und S), und einem 5- bis 8-gliedrigen Heterocycloalkyl (wobei 1-4 Ringglieder unabhängig ausgewählt sind aus N, B, O und S), wobei der Ring optional substituiert ist mit 1 bis 3 Substituenten, unabhängig ausgewählt aus der Gruppe, bestehend aus C₁-C₆-Alkyl, C₃-C₈-Cycloalkyl, 3- bis 6-gliedrigem Heterocycloalkyl (wobei 1-4 Ringglieder unabhängig ausgewählt sind aus N, O und S), C₂-C₆-Alkenyl, C₂-C₆-Alkinyl, Halogen, C₁-C₆-Halogenalkyl, C₂-C₆-Halogenalkenyl, C₂-C₆-Halogenalkinyl, Oxo, Thioxo, Cyano, Nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (wobei t 1 oder 2 ist), - R^{b}-S(O)ₜR^{a} (wobei t 1 oder 2 ist), -R^{b}-S(O)ₜOR^{a} (wobei t 1 oder 2 ist) und -R^{b}-S(O)ₜN(R^{a})₂ (wobei t 1 oder 2 ist),
A, B und E unabhängig aus C, N, O und S ausgewählt sind und D C oder N ist,
wobei 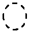 das Vorhandensein von Doppelbindungen darstellt, so dass der Ring A-B-D-E-N aromatisch ist und optional mit einem oder zwei Substituenten substituiert ist, unabhängig ausgewählt aus C₁-C₃-Alkyl, C₃-C₅-Cycloalkyl, OH, OMe, NH₂, N(H)Me, NMe₂;
wobei nicht mehr als zwei von A, B, D und E gleichzeitig N, O oder S sind;
R¹, R² und R³ unabhängig ausgewählt sind aus der Gruppe, bestehend aus Wasserstoff, Halogen, Cyano, Nitro, -R^{b}-OR^{a}, -R^{b}-O-R^{c}-O-R^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (wobei t 1 oder 2 ist), -R^{b}-S(O)ₜR^{a} (wobei t 1 oder 2 ist), -R^{b}-S(O)ₜOR^{a} (wobei t 1 oder 2 ist), -R^{b}-S(O)ₜN(R^{a})₂ (wobei t 1 oder 2 ist), C₁-C₆-Alkyl, C₁-C₆-Halogenalkyl, C₂-C₆-Alkenyl, C₂-C₆-Alkinyl, C₃-C₈-Cycloalkyl, C₆-C₁₀-Aryl, -(C₁-C₆-Alkyl)(C₆-C₁₀-Aryl), 5- bis 10-gliedrigem Heteroaryl (wobei 1-4 Heteroarylglieder unabhängig ausgewählt sind aus N, O und S), optional kondensiert an 3- bis 6-gliedriges Heterocycloalkyl (wobei 1-4 Ringglieder unabhängig ausgewählt sind aus N, O und S) und 3- bis 6-gliedriges Heterocycloalkyl (wobei 1-4 Ringglieder unabhängig ausgewählt sind aus N, O und S),
oder R¹ und R² oder R² und R³ zusammen mit den Kohlenstoffatomen, an die sie gebunden sind, ein kondensiertes C₅-C₈-Cycloalkyl, C₆-C₁₀-Aryl, 5- bis 10-gliedriges Heteroaryl (wobei 1-4 Heteroarylglieder unabhängig ausgewählt sind aus N, O und S) und 5- bis 8-gliedriges Heterocycloalkyl (wobei 1-4 Ringglieder unabhängig ausgewählt sind aus N, O und S) bilden;
wobei ein beliebiges Heteroaryl oder Heterocycloalkyl in R¹, R² und R³ optional und unabhängig substituiert ist mit 1 bis 3 Substituenten, ausgewählt aus der Gruppe, bestehend aus C₁-C₆-Alkyl, Halogen, Hydroxy, C₃-C₈-Cycloalkyl, Heterocycloalkyl (wobei 1-4 Ringelemente unabhängig ausgewählt sind aus N, O und S) und -R^{b}-N(R^{a})₂;
R⁴ in jeder Instanz unabhängig H, OH, Halogen, C₁-C₆-Alkyl oder C₁-C₆-Alkoxy ist;
R⁵ in jeder Instanz unabhängig ausgewählt aus der Gruppe bestehend aus Wasserstoff, Halogen, Cyano, Nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -B(OR^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (wobei t 1 oder 2 ist), -R^{b}-S(O)ₜR^{a} (wobei t 1 oder 2 ist), -R^{b}-S(O)ₜOR^{a} (wobei t 1 oder 2 ist), -R^{b}-OS(O)ₜF (wobei t 1 oder 2 ist), -R^{b}-S(O)ₜN(R^{a})₂ (wobei t 1 oder 2 ist), C₁-C₆-Alkyl, C₂-C₆-Alkinyl, C₃-C₈-Cycloalkyl, C₆-C₁₀-Aryl, 5- bis 10-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S);
R^{5'} ausgewählt ist aus der Gruppe, bestehend aus Wasserstoff, -R^{c}-R^{a}, -R^{c}-OR^{a}, -R^{b}-OC(O)-R^{a}, -R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, - R^{b}-N(R^{a})C(O)R^{a}, -R^{b}-N(R^{a})S(O)ₜR^{a} (wobei t 1 oder 2 ist), -R^{b}-S(O)ₜR^{a} (wobei t 1 oder 2 ist), -R^{b}-S(O)ₜOR^{a} (wobei t 1 oder 2 ist), -R^{b}-S(O)ₜN(R^{a})₂ (wobei t 1 oder 2 ist), C₁-C₆-Alkyl, C₃-C₈-Cycloalkyl, C₆-C₁₀-Aryl, 5- bis 10-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S);
wobei jedes Heteroaryl oder Heterocycloalkyl in R⁵ und R^{5'} optional und unabhängig substituiert ist mit 1 bis 3 Substituenten, ausgewählt aus der Gruppe, bestehend aus C₁-C₆-Alkyl, Halogen, Hydroxy, C₃-C₈-Cycloalkyl und -R^{b}-N(R^{a})₂;
R^{6a}, R^{6b}, R^{6c}, R^{6d}, R^{6e}, R^{6f}, R^{6g} und R^{6h} unabhängig ausgewählt sind aus der Gruppe, bestehend aus H, Halo, NO₂, OH, CN, -R^{b}-N(R^{a})₂, -R^{b}-OH, C₁-C₆-Alkyl und C₁-C₆-Alkoxy;
und/oder optional R^{6a} und R^{6b} oder R^{6c} und R^{6d} oder R^{6e} und R^{6f} oder R^{6g} und R^{6h} unabhängig Oxo, Thioxo, Imino oder Oximo darstellen;
und/oder optional R^{6a} und R^{6b}, oder R^{6c} und R^{6d}, oder R^{6e} und R^{6f}, oder R^{6g} und R^{6h} sich zusammen mit den Kohlenstoffatomen, an die sie gebunden sind, unabhängig kombinieren, um einen kondensierten Ring zu bilden, ausgewählt aus einem C₃-C₆-Cycloalkyl und C₃-C₆-Heterocycloalkyl (wobei 1-4 Ringglieder unabhängig ausgewählt sind aus N, O und S);
und/oder optional eines von R^{6c} und R^{6d} zusammen mit einem von R^{6e} und R^{6f} eine Bindung zwischen den Ringkohlenstoffgliedern darstellen, an die sie gebunden sind;
R^{a} in jeder Instanz unabhängig ausgewählt ist aus Wasserstoff, C₁-C₆-Alkyl, C₃-C₈-Cycloalkyl, -(C₁-C₆-Alkyl)(C₃-C₈-Cycloalkyl), C₆-C₁₀-Aryl, 5- bis 10-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S);
R^{b} in jeder Instanz unabhängig ausgewählt ist aus einer direkten Bindung, einer geraden oder verzweigten C₂-C₆-Alkylen-, und C₂-C₆-Alkenylen-Kette; wobei jedes Heteroaryl oder Heterocycloalkyl in R^{a} und R^{b} optional und unabhängig substituiert ist mit 1 bis 3 Substituenten, ausgewählt aus der Gruppe, bestehend aus C₁-C₆-Alkyl, Halogen, Hydroxy,
R^{c} in jeder Instanz unabhängig ausgewählt ist aus einer geraden oder verzweigten C₂-C₆-Alkylen- und C₂-C₆-Alkenylen-Kette.

2. Verbindung oder pharmazeutisch unbedenkliches Salz und/oder Solvat davon gemäß Anspruch 1, wobei die Verbindung die Formel (I) aufweist: 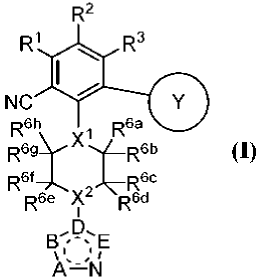 wobei
jedes von X¹ und X² N ist;
Ring Y 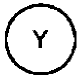 der Formel (a) entspricht: 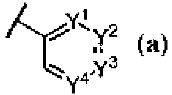
wobei Y¹, Y², Y³ und Y⁴ unabhängig aus CR⁵ und N ausgewählt sind, wobei Y¹, Y², Y³ und Y⁴ nicht gleichzeitig N sind,
oder Ring Y 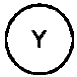 der Formel (b) entspricht: 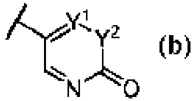
wobei Y¹ CR⁵ ist und Y² NR^{5'} ist;
optional entweder Y¹ und Y² oder Y² und Y³ oder Y³ und Y⁴ einen kondensierten Ring darstellen, ausgewählt aus einem C₅-C₈-Cycloalkyl, einem C₆-C₁₀-Aryl, einem 5- bis 10-gliedrigen Heteroaryl (wobei 1-4 Heteroarylglieder unabhängig ausgewählt sind aus N, O und S), und einem 5- bis 8-gliedrigen Heterocycloalkyl (wobei 1-4 Ringglieder unabhängig ausgewählt sind aus N, O und S), wobei der Ring optional substituiert ist mit 1 bis 3 Substituenten, unabhängig ausgewählt aus der Gruppe, bestehend aus C₁-C₆-Alkyl, C₃-C₈-Cycloalkyl, 3- bis 6-gliedrigem Heterocycloalkyl (wobei 1-4 Ringglieder unabhängig ausgewählt sind aus N, O und S), C₂-C₆-Alkenyl, C₂-C₆-Alkinyl, Halogen, C₁-C₆-Halogenalkyl, C₂-C₆-Halogenalkenyl, C₂-C₆-Halogenalkinyl, Oxo, Thioxo, Cyano, Nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (wobei t 1 oder 2 ist), - R^{b}-S(O)ₜR^{a} (wobei t 1 oder 2 ist), -R^{b}-S(O)ₜOR^{a} (wobei t 1 oder 2 ist) und -R^{b}-S(O)ₜN(R^{a})₂ (wobei t 1 oder 2 ist),
A, B und E unabhängig aus C, N, O und S ausgewählt sind und D C oder N ist,
wobei 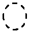 das Vorhandensein von Doppelbindungen darstellt, sodass der Ring A-B-D-E-N aromatisch ist und optional mit einem oder zwei Substituenten substituiert ist, unabhängig ausgewählt aus C₁-C₃-Alkyl, C₃-C₅-Cycloalkyl, OH, OMe, NH₂, N(H)Me, NMe₂;
wobei nicht mehr als zwei von A, B, D und E gleichzeitig N, O oder S sind;
R¹, R² und R³ unabhängig ausgewählt sind aus der Gruppe, bestehend aus Wasserstoff, Halogen, Cyano, Nitro, -R^{b}-OR^{a}, -R^{b}-O-R^{a}, -R^{b}-OC(O)-R^{a}, -R^{b}-OC(O)-OR^{a}, -R^{b}-OC(O)-N(R^{a})₂, - R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (wobei t 1 oder 2 ist), -R^{b}-S(O)ₜR^{a} (wobei t 1 oder 2 ist), -R^{b}-S(O)ₜOR^{a} (wobei t 1 oder 2 ist), -R^{b}-S(O)ₜN(R^{a})₂ (wobei t 1 oder 2 ist), C₁-C₆-Alkyl, C₃-C₈-Cycloalkyl, C₆-C₁₀-Aryl, -(C₁-C₆-Alkyl)(C₆-C₁₀-Aryl), 5- bis 10-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S),
oder R¹ und R² oder R² und R³ zusammen mit den Kohlenstoffatomen, an die sie gebunden sind, ein kondensiertes C₅-C₈-Cycloalkyl, C₆-C₁₀-Aryl, 5- bis 10-gliedriges Heteroaryl (wobei 1-4 Heteroarylglieder unabhängig ausgewählt sind aus N, O und S) und 5- bis 8-gliedriges Heterocycloalkyl (wobei 1-4 Ringglieder unabhängig ausgewählt sind aus N, O und S) bilden;
R⁴ in jeder Instanz unabhängig H, OH, Halogen, C₁-C₆-Alkyl oder C₁-C₆-Alkoxy ist;
R⁵ in jeder Instanz unabhängig ausgewählt ist aus der Gruppe, bestehend aus Wasserstoff, Halogen, Cyano, Nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (wobei t 1 oder 2 ist), -R^{b}-S(O)ₜR^{a} (wobei t 1 oder 2 ist), -R^{b}-S(O)ₜOR^{a} (wobei t 1 oder 2 ist), -R^{b}-S(O)ₜN(R^{a})₂ (wobei t 1 oder 2 ist), C₁-C₆-Alkyl, C₃-C₈-Cycloalkyl, C₆-C₁₀-Aryl, 5- bis 10-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S);
R^{5'} ausgewählt ist aus der Gruppe, bestehend aus Wasserstoff, -R^{c}-R^{a}, -R^{c}-OR^{a}, -R^{b}-OC(O)-R^{a}, -R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, - R^{b}-N(R^{a})C(O)R^{a}, -R^{b}-N(R^{a})S(O)ₜR^{a} (wobei t 1 oder 2 ist), -R^{b}-S(O)ₜR^{a} (wobei t 1 oder 2 ist), -R^{b}-S(O)ₜOR^{a} (wobei t 1 oder 2 ist), -R^{b}-S(O)ₜN(R^{a})₂ (wobei t 1 oder 2 ist), C₁-C₆-Alkyl, C₃-C₈-Cycloalkyl, C₆-C₁₀-Aryl, 5- bis 10-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S);
R^{6a}, R^{6b}, R^{6c}, R^{6d} , R^{6e}, R^{6f}, R^{6g} und R^{6h} unabhängig ausgewählt sind aus der Gruppe, bestehend aus H, Halogen, NO₂, OH, CN, -R^{b}-N(R^{a})₂, -R^{b}-OH, C₁-C₆-Alkyl und C₁-C₆-Alkoxy;
oder optional R^{6a} und R^{6b} oder R^{6c} und R^{6d} oder R^{6e} und R^{6f} oder R^{6g} und R^{6h} unabhängig Oxo, Thioxo, Imino oder Oximo darstellen;
oder optional R^{6a} und R^{6b}, oder R^{6c} und R^{6d}, oder R^{6e} und R^{6f}, oder R^{6g} und R^{6h} sich zusammen mit den Kohlenstoffatomen, an die sie gebunden sind, unabhängig kombinieren, um einen kondensierten Ring zu bilden, ausgewählt aus einem C₃-C₆-Cycloalkyl und C₃-C₆-Heterocycloalkyl (wobei 1-4 Ringglieder unabhängig ausgewählt sind aus N, O und S);
R^{a} in jeder Instanz unabhängig ausgewählt ist aus Wasserstoff, C₁-C₆-Alkyl, C₃-C₈-Cycloalkyl, C₆-C₁₀-Aryl, 5- bis 10-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S);
R^{b} in jeder Instanz unabhängig ausgewählt ist aus einer direkten Bindung, einer geraden oder verzweigten C₂-C₆-Alkylen- und C₂-C₆-Alkenylen-Kette;
R^{c} in jeder Instanz unabhängig ausgewählt ist aus einer geraden oder verzweigten C₂-C₆-Alkylen- und C₂-C₆-Alkenylen-Kette.

3. Verbindung oder pharmazeutisch unbedenkliches Salz und/oder Solvat davon gemäß Anspruch 1, wobei W¹ N ist, W² CR² ist und W³ CR³ ist; oder W¹ CR¹ ist, W² CR² ist und W³ N ist; oder W² CR² ist und W³ CR³ ist.

4. Verbindung oder pharmazeutisch unbedenkliches Salz und/oder Solvat davon gemäß einem der Ansprüche 1 bis 3, wobei D C ist und wobei A, B und E unabhängig ausgewählt sind aus C und N oder zumindest eines von A, B und E N ist oder zwei von A, B und E N sind.

5. Verbindung oder pharmazeutisch unbedenkliches Salz und/oder Solvat davon gemäß einem der Ansprüche 1 bis 3, wobei der A-B-D-E-N-Ring ein optional substituierter Ring ist, ausgewählt aus der Gruppe, bestehend aus:

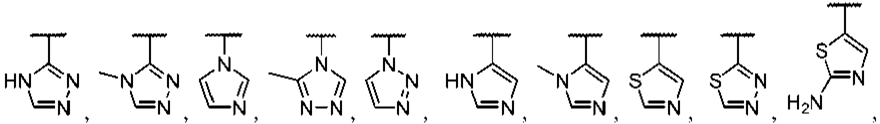

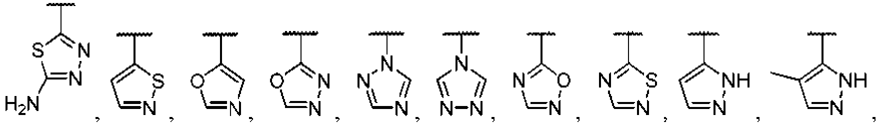

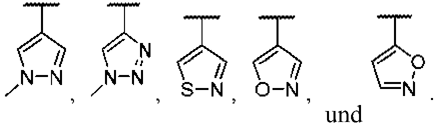

6. Verbindung oder pharmazeutisch unbedenkliches Salz und/oder Solvat davon gemäß einem der Ansprüche 1 bis 5, wobei Ring Y der Formel (a) entspricht, und wobei
eines von Y¹, Y², Y³ und Y⁴ N ist und jedes der verbleibenden drei CR⁵ ist, oder wobei
zwei von Y¹, Y², Y³ und Y⁴ N sind und jedes der verbleibenden zwei CR⁵ ist, oder wobei
entweder Y¹ und Y² oder Y³ und Y⁴ einen optional substituierten kondensierten Ring darstellen.

7. Verbindung oder pharmazeutisch unbedenkliches Salz und/oder Solvat davon gemäß einem der Ansprüche 1 bis 6, wobei R¹ ausgewählt ist aus der Gruppe, bestehend aus H, Halogen, C₁-C₆-Alkyl, -R^{b}-OR^{a} (wobei R^{b} eine direkte Bindung ist und R^{a} C₁-C₆-Alkyl ist), C₆-C₁₀-Aryl, C₃-C₈-Cycloalkyl, 3- bis 6-gliedrigem Heterocycloalkyl (wobei 1-4 Ringglieder unabhängig ausgewählt sind aus N, O und S) und 5- bis 6-gliedrigem Heteroaryl (wobei 1-4 Heteroarylglieder unabhängig ausgewählt sind aus N, O und S), und
jedes von R² und R³ unabhängig H, Halogen, Cyano, C₁-C₆-Alkyl, C₃-C₈-Cycloalkyl, 3-bis 6-gliedriges Heterocycloalkyl (wobei 1-4 Ringglieder unabhängig ausgewählt sind aus N, O und S) und 5- bis 6-gliedriges Heteroaryl (wobei 1-4 Heteroarylringglieder unabhängig ausgewählt sind aus N, O und S), -R^{b}-OR^{a} (wobei R^{b} eine direkte Bindung ist und R^{a} C₁-C₆-Alkyl ist) oder CF₃ ist.

8. Verbindung oder pharmazeutisch unbedenkliches Salz und/oder Solvat davon gemäß Anspruch 1, wobei die Verbindung eine Verbindung der Formel (IA), (IB) oder (ID) ist:

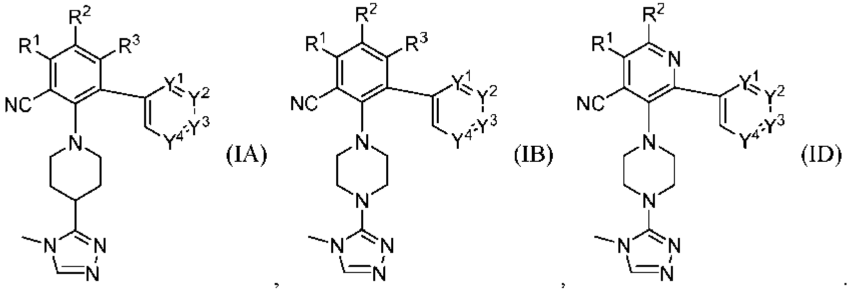

9. Verbindung oder pharmazeutisch unbedenkliches Salz und/oder Solvat davon gemäß Anspruch 8, wobei:
R¹ ausgewählt ist aus der Gruppe, bestehend aus H, Halogen, C₁-C₆-Alkoxy, C₆-C₁₀-Aryl, C₁-C₆-Alkyl, C₃-C₆-Cycloalkyl, 5- bis 6-gliedrigem Heteroaryl (wobei 1-4 Heteroarylglieder unabhängig ausgewählt sind aus N, O und S) und 3- bis 6-gliedrigem Heterocycloalkyl (wobei 1-4 Ringglieder unabhängig ausgewählt sind aus N, O und S);
jedes von R² H, Halogen, Cyano, -R^{b}-S(O)ₜR^{a} (wobei t 1 oder 2 ist), C₁-C₆-Alkyl, C₃-C₆-Cycloalkyl oder CF₃ ist und
R³, falls vorhanden, Halogen, Cyano, -R^{b}-S(O)ₜR^{a} (wobei t 1 oder 2 ist), C₁-C₆-Alkyl, C₃-C₆-Cycloalkyl oder CF₃ ist; und
eines von Y¹, Y², Y³ und Y⁴ N ist und jedes der verbleibenden drei CR⁵ ist, oder
zwei von Y¹, Y², Y³ und Y⁴ N sind und jedes der verbleibenden zwei CR⁵ ist.

10. Verbindung oder pharmazeutisch unbedenkliches Salz und/oder Solvat davon gemäß Anspruch 8 oder 9, wobei der Ring, der Y¹, Y², Y³ und Y⁴ enthält, Folgendes ist:

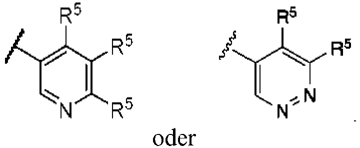

11. Verbindung oder pharmazeutisch unbedenkliches Salz und/oder Solvat davon gemäß einem der Ansprüche 8 bis 10, wobei der Ring, der Y¹, Y², Y³ und Y⁴ enthält, Folgendes ist:

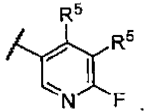

12. Verbindung oder pharmazeutisch unbedenkliches Salz und/oder Solvat davon, ausgewählt aus der folgenden Tabelle:
| | |
|---|---|
| **4** | 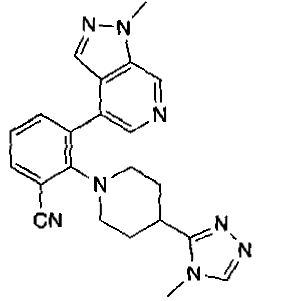 |
| **5** | 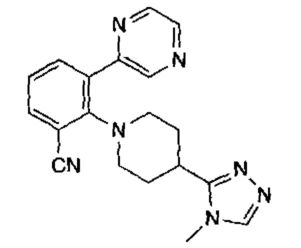 |
| **6** | 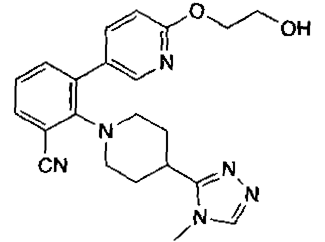 |
| **7** | 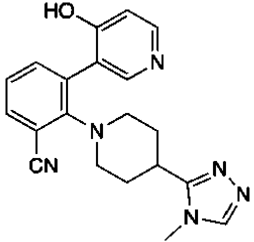 |
| **8** | 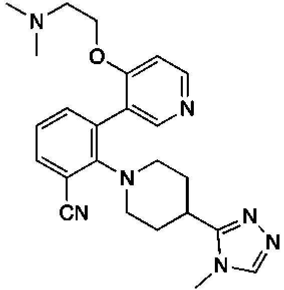 |
| **9** | 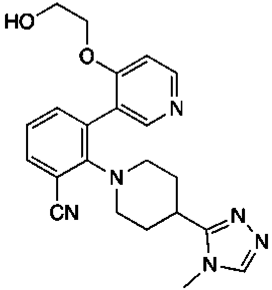 |
| **11** | 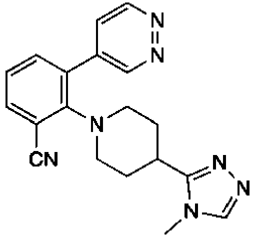 |
| **12** | 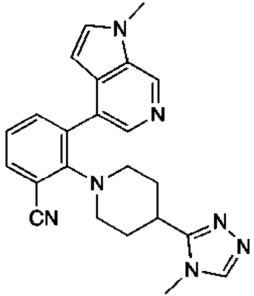 |
| **13** | 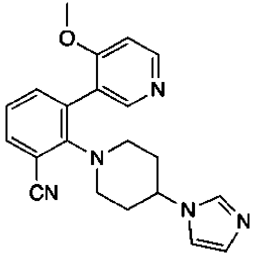 |
| **14** | 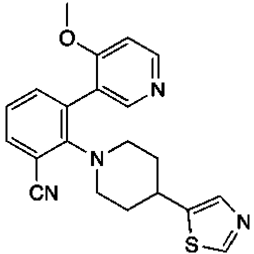 |
| **15** | 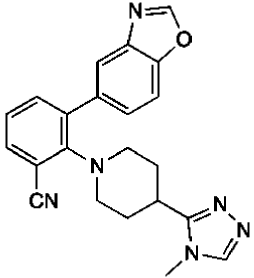 |
| **16** | 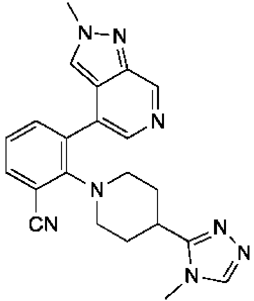 |
| **17** | 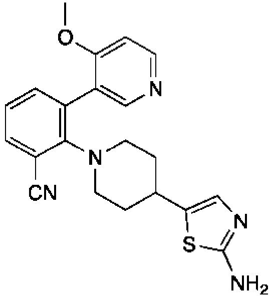 |
| **18** | 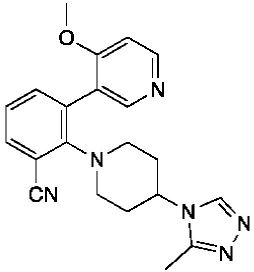 |
| **19** | 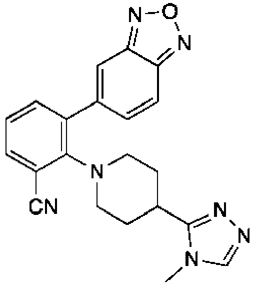 |
| **20** | 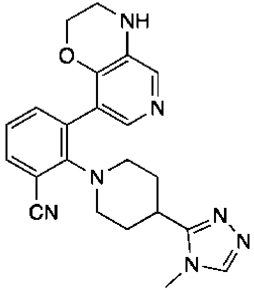 |
| **21** | 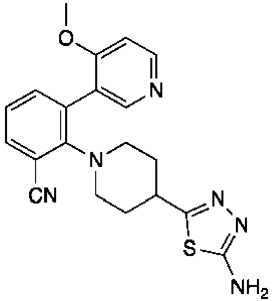 |
| **22** | 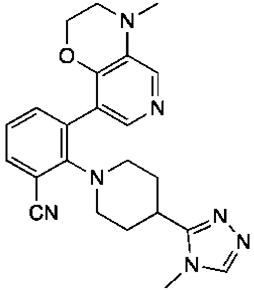 |
| **23** | 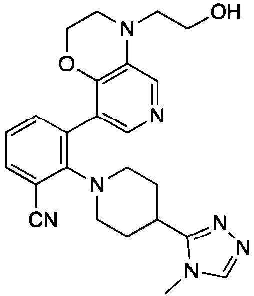 |
| **24** | 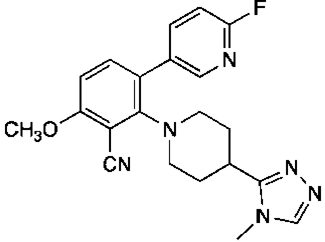 |
| **25** | 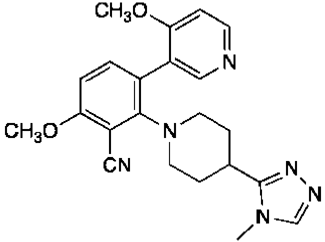 |
| **26** | 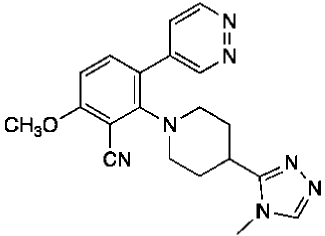 |
| **27** | 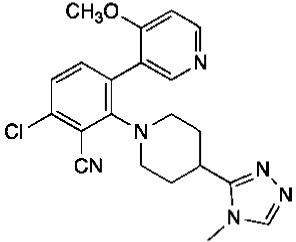 |
| **28** | 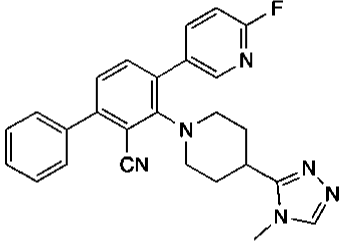 |
| **30** | 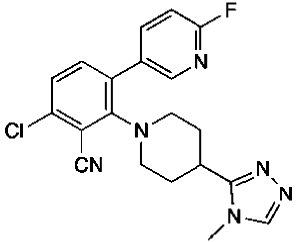 |
| **31** | 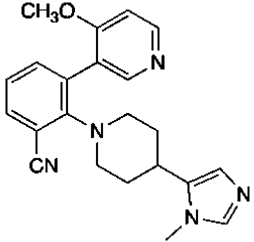 |
| **32** | 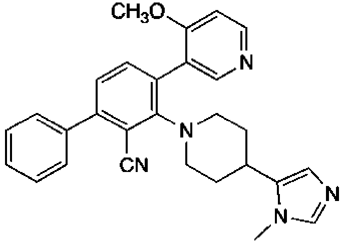 |
| **33** | 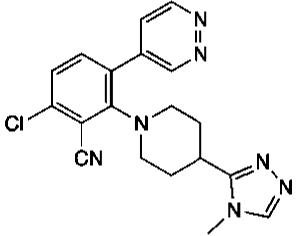 |
| **34** | 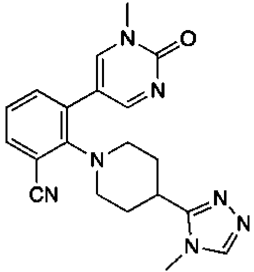 |
| **35** | 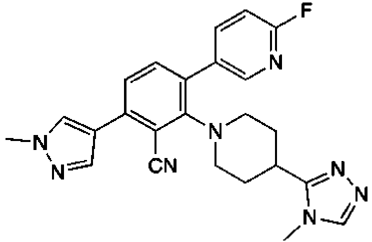 |
| **36** | 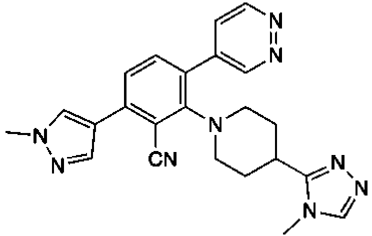 |
| **37** | 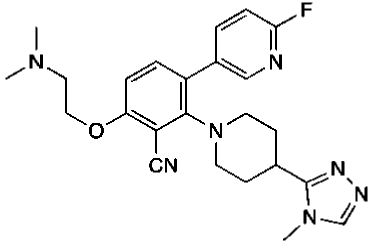 |
| **38** | 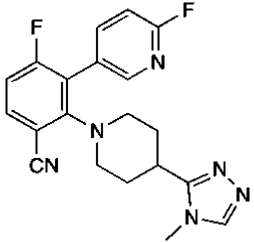 |
| **39** | 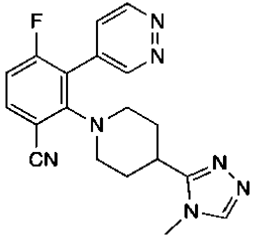 |
| **40** | 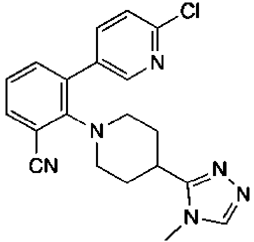 |
| **41** | 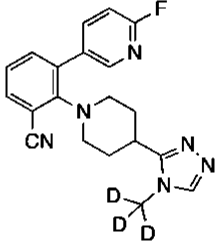 |
| **42** | 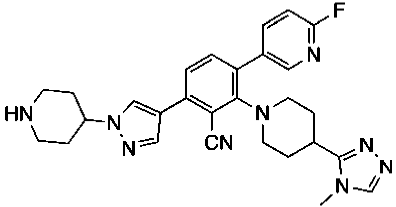 |
| **43** | 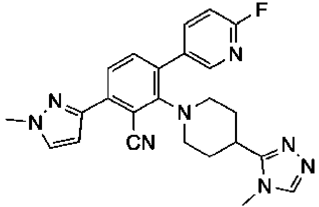 |
| **44** | 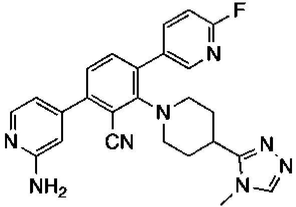 |
| **45** | 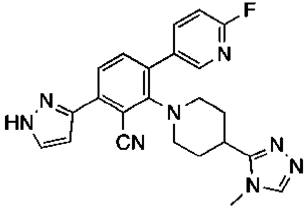 |
| **46** | 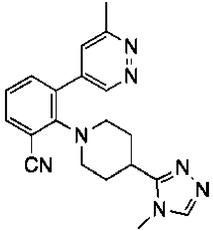 |
| **48** | 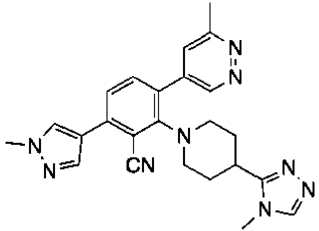 |
| **49** | 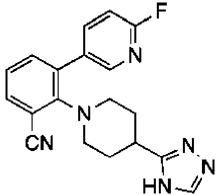 |
| **50** | 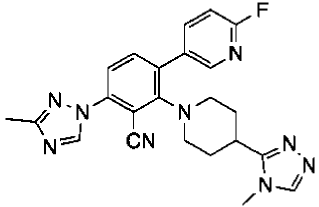 |
| **51** | 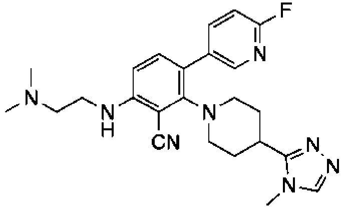 |
| **52** | 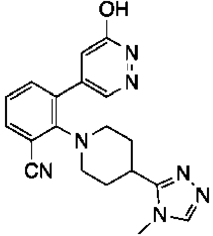 |
| **53** | 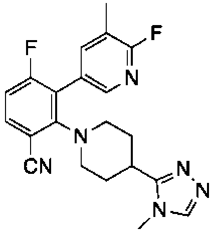 |
| **54** | 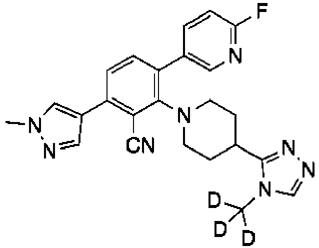 |
| **55** | 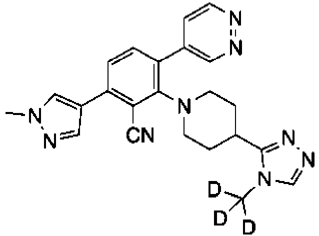 |
| **56** | 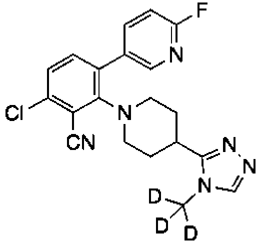 |
| **57** | 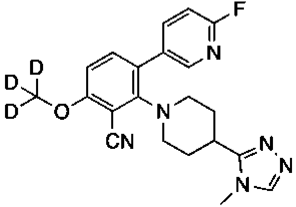 |
| **58** | 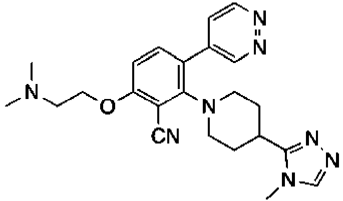 |
| **59** | 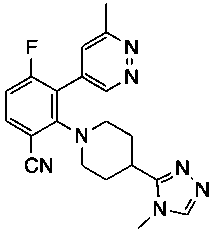 |
| **60** | 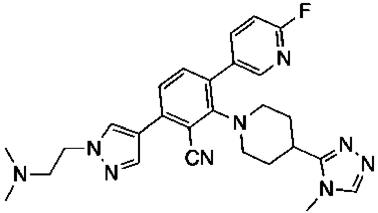 |
| **61** | 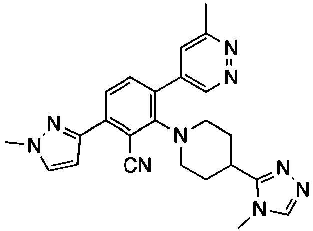 |
| **62** | 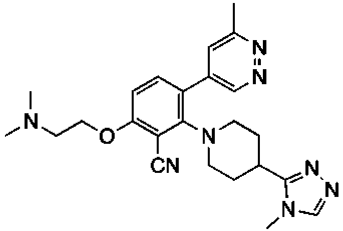 |
| **63** | 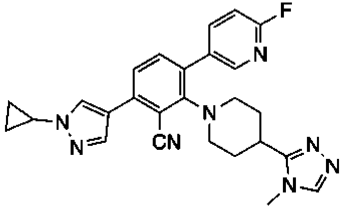 |
| **64** | 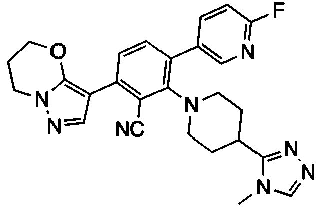 |
| **65** | 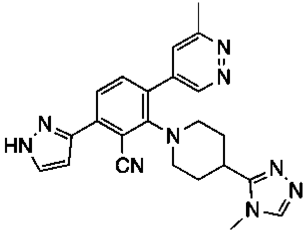 |
| **66** | 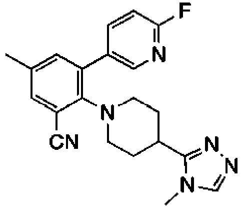 |
| **67** | 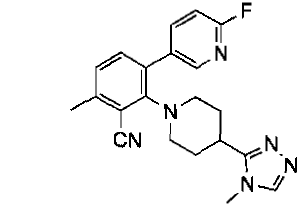 |
| **68** | 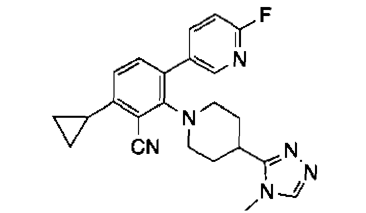 |
| **69** | 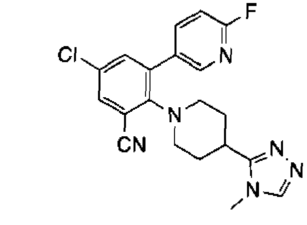 |
| **70** | 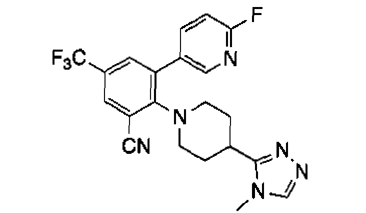 |
| **71** | 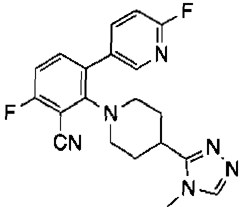 |
| **72** | 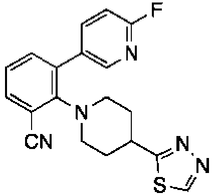 |
| **73** | 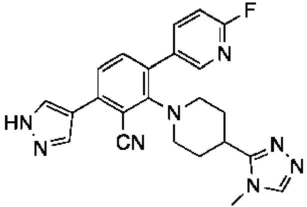 |
| **74** | 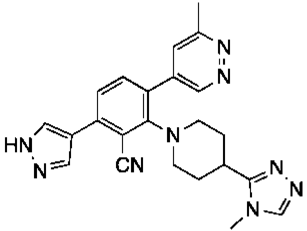 |
| **75** | 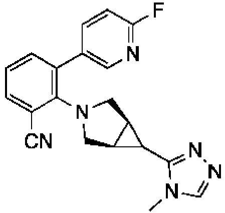 |
| **76** | 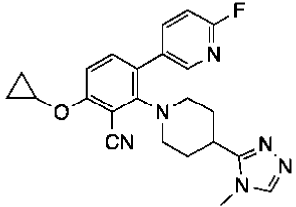 |
| **77** | 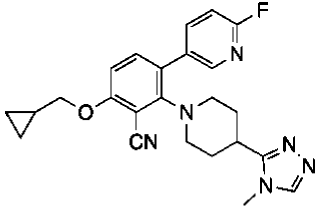 |
| **78** | 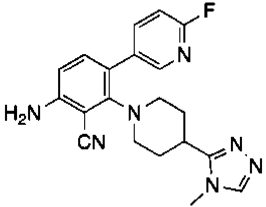 |
| **79** | 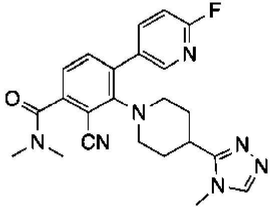 |
| **80** | 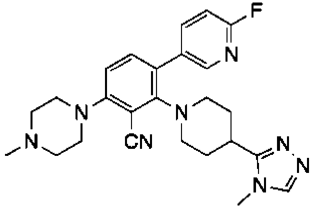 |
| **81** | 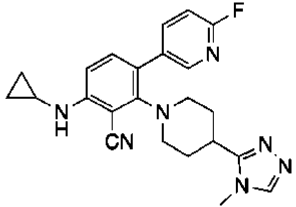 |
| **82** | 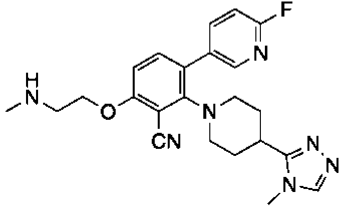 |
| **83** | 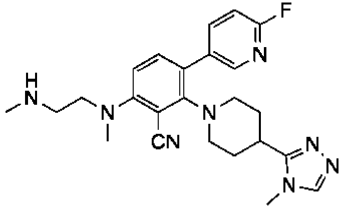 |
| **84** | 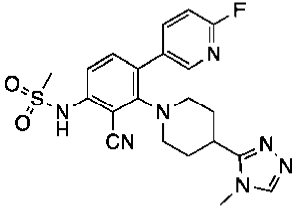 |
| **85** | 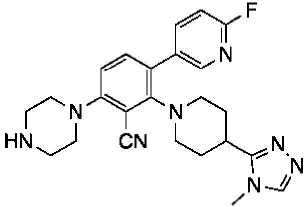 |
| **86** | 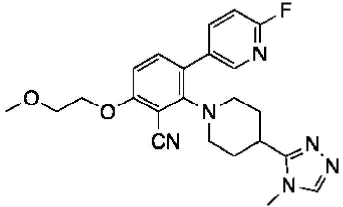 |
| **87** | 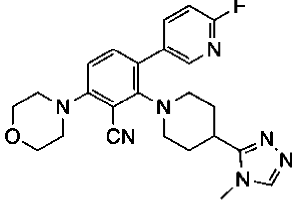 |
| **88** | 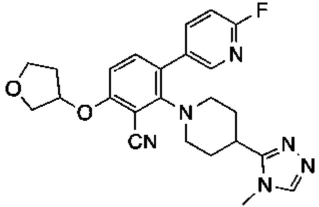 |
| **89** | 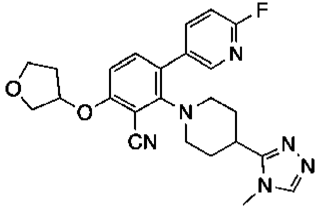 |
| **90** | 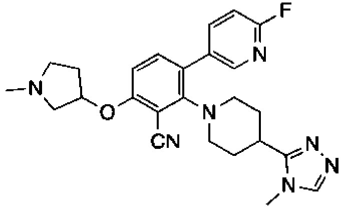 |
| **91** | 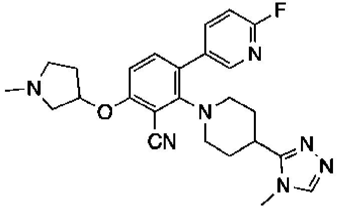 |
| **92** | 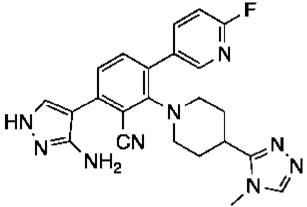 |
| **93** | 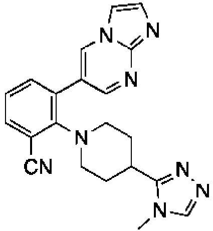 |
| **94** | 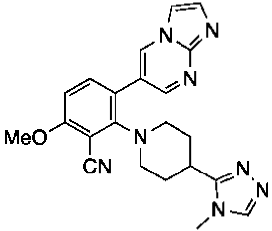 |
| **95** | 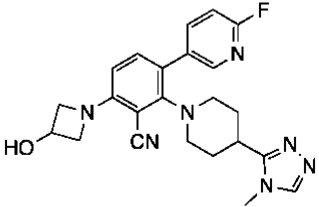 |
| **96** | 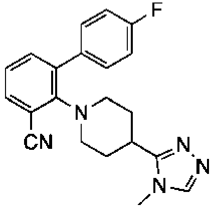 |
| **97** | 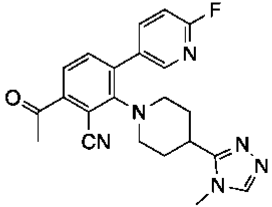 |
| **98** | 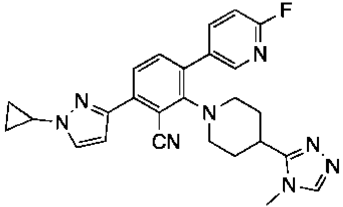 |
| **99** | 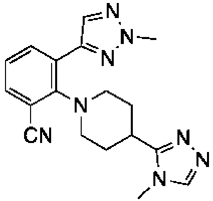 |
| **100** | 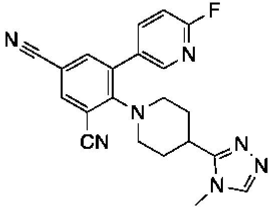 |
| **101** | 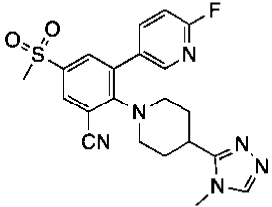 |
| **102** | 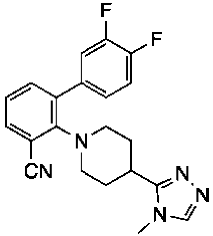 |
| **103** | 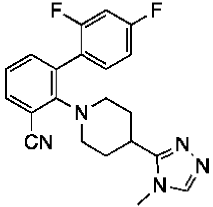 |
| **104** | 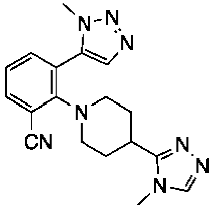 |
| **105** | 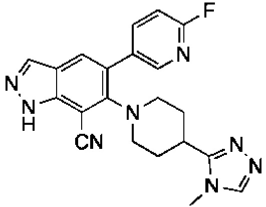 |
| **106** | 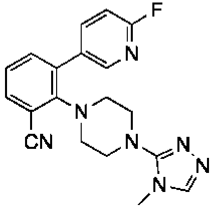 |
| **107** | 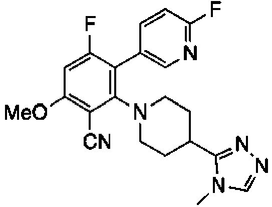 |
| **108** | 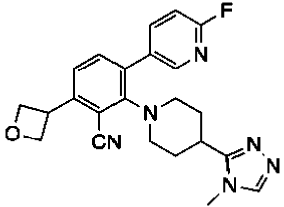 |
| **109** | 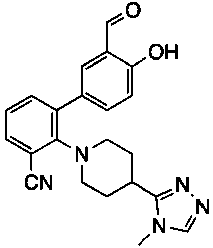 |
| **110** | 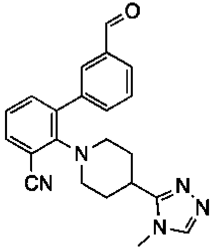 |
| **111** | 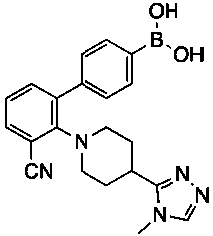 |
| **112** | 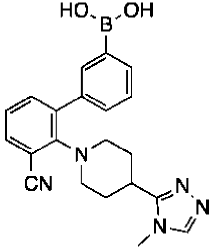 |
| **113** | 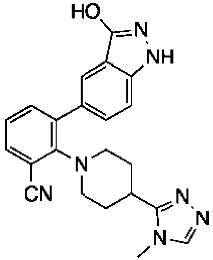 |
| **114** | 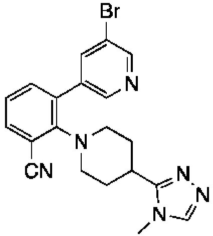 |
| **115** | 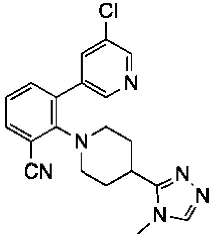 |
| **116** | 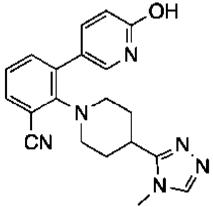 |
| **117** | 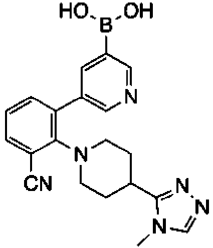 |
| **118** | 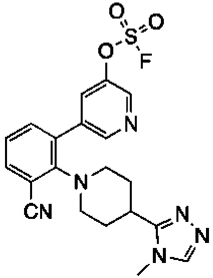 |
| **119** | 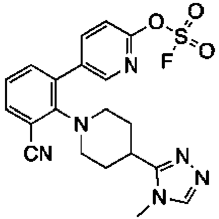 |
| **120** | 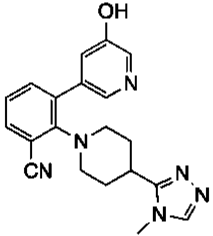 |
| **121** | 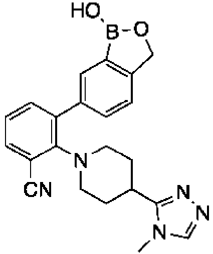 |
| **122** | 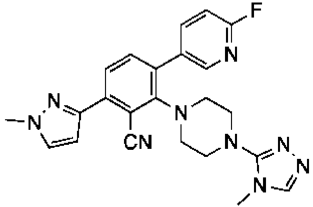 |
| **123** | 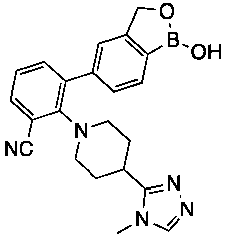 |
| **124** | 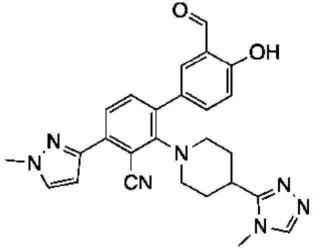 |
| **125** | 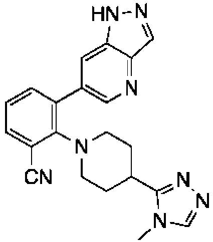 |
| **126** | 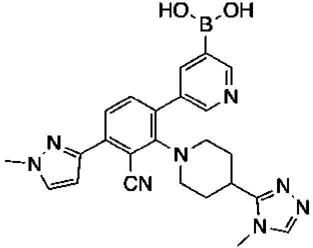 |
| **127** | 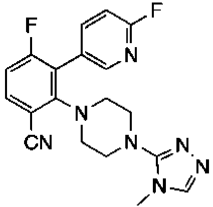 |
| **128** | 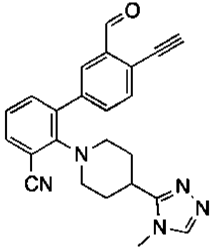 |
| **129** | 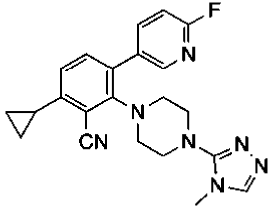 |
| **130** | 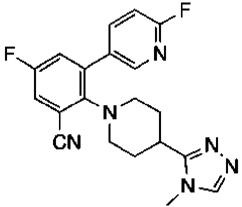 |
| **131** | 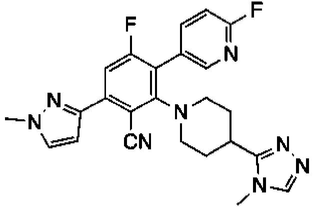 |
| **134** | 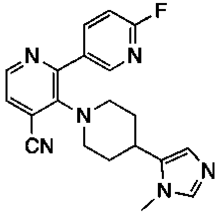 |
| **135** | 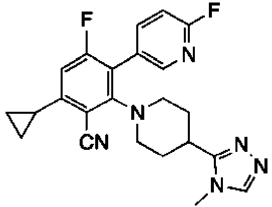 |
| **136** | 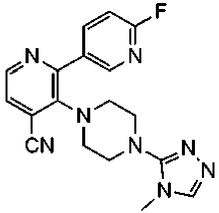 |
| **137** | 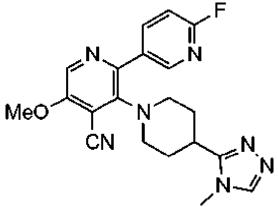 |
| **138** | 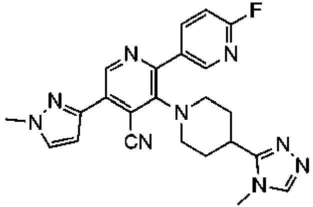 |
| **139** | 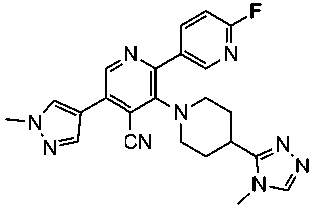 |
oder eine Verbindung oder ein pharmazeutisch unbedenkliches Salz und/oder Solvat davon, ausgewählt aus der folgenden Tabelle:

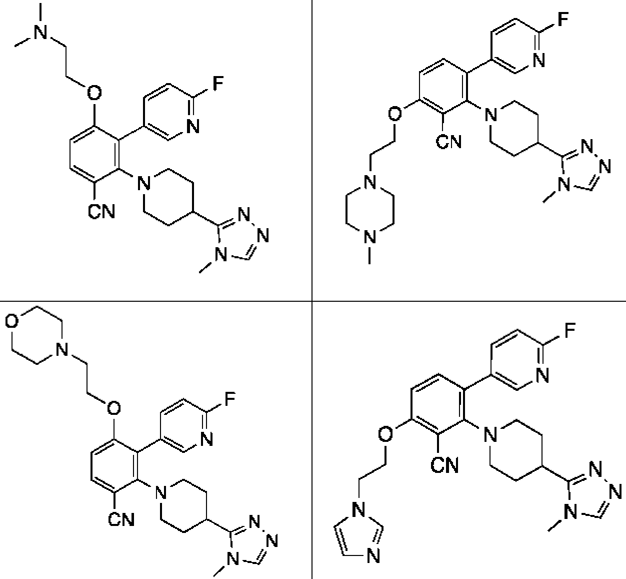

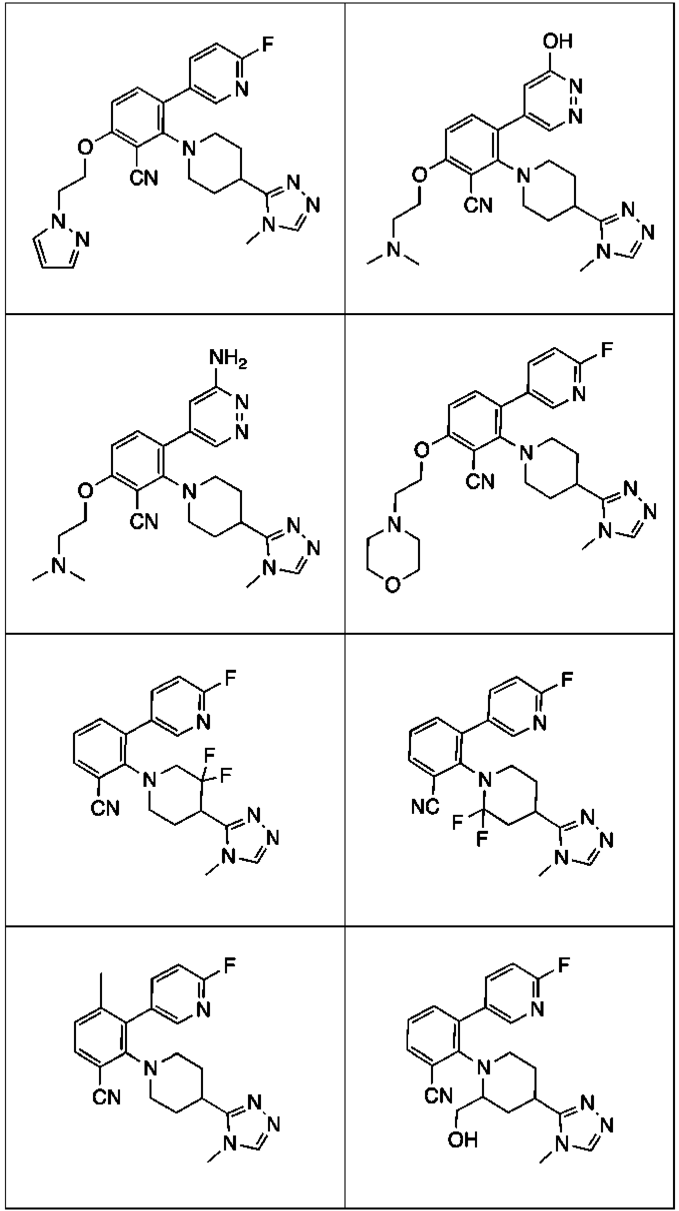

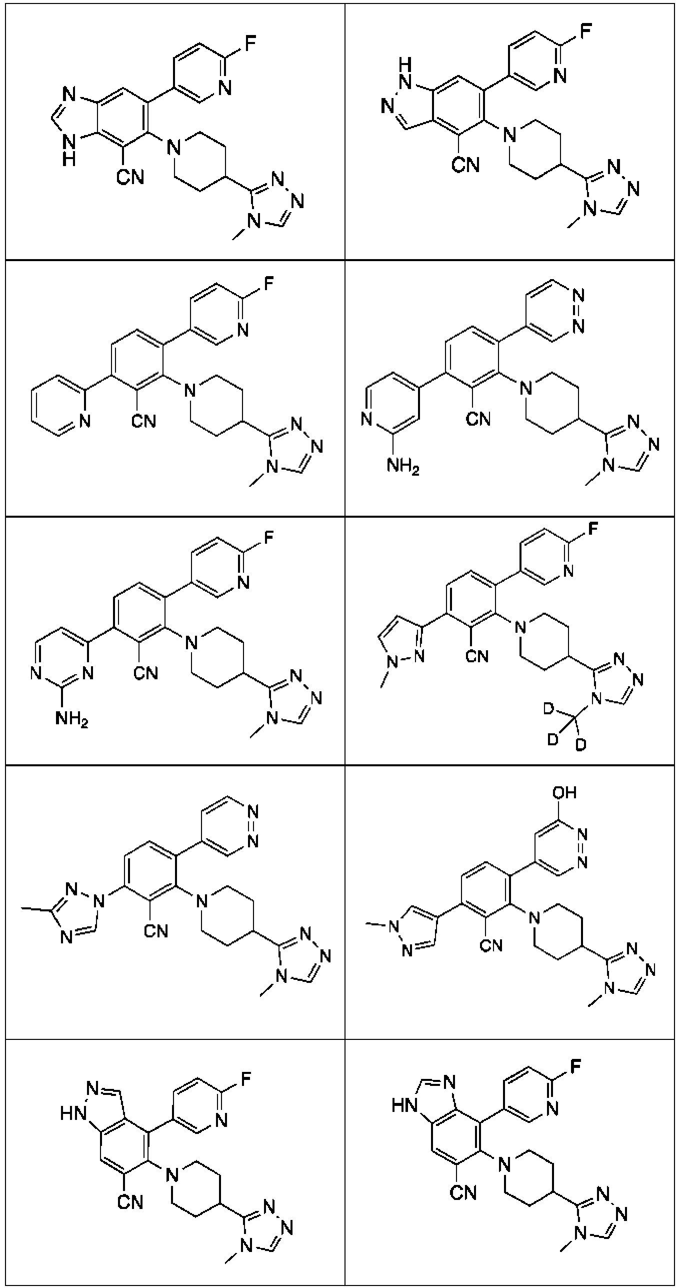

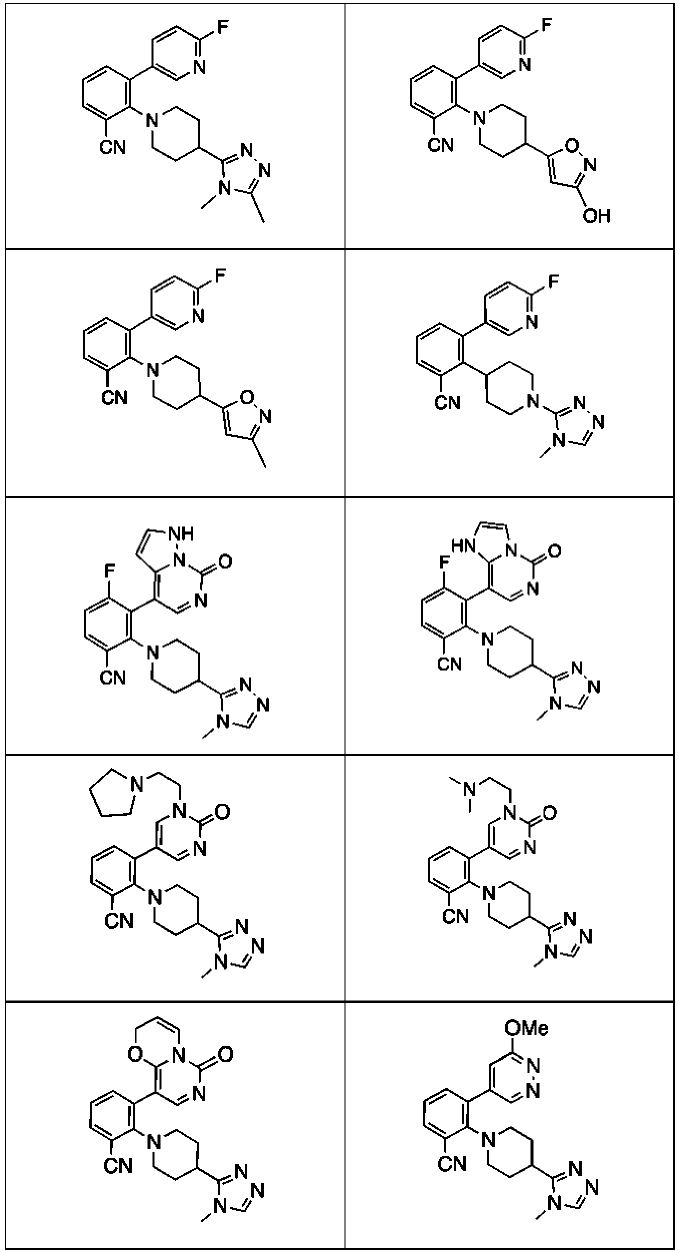

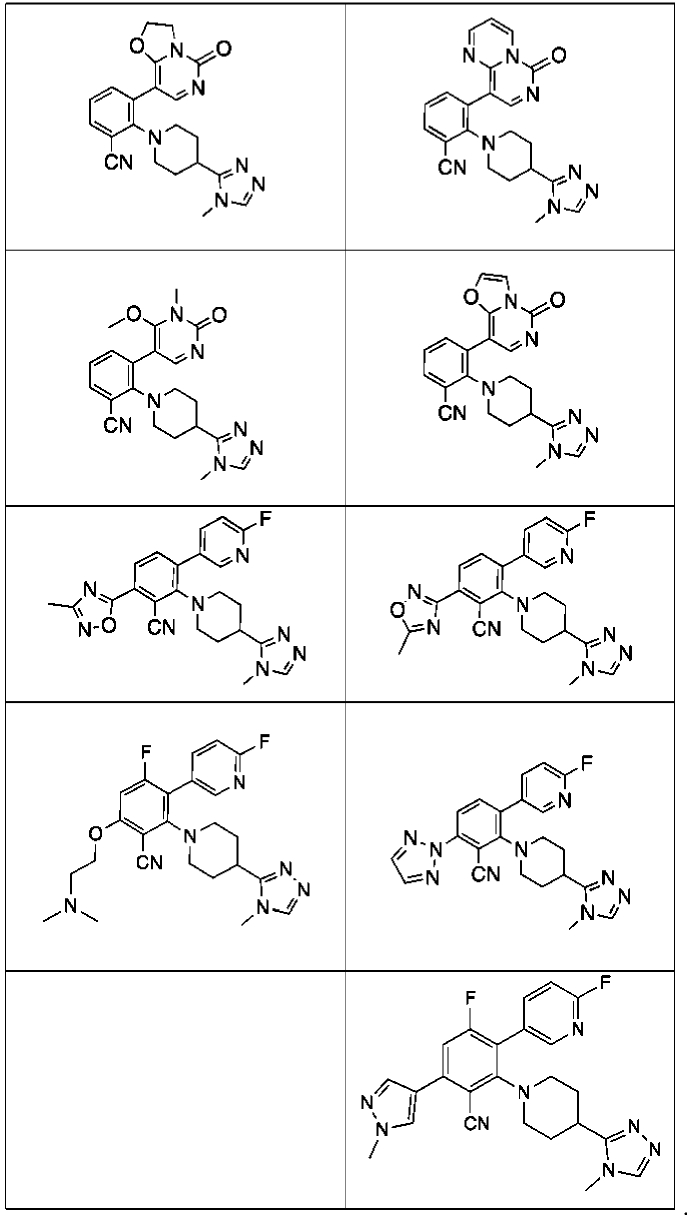

13. Pharmazeutische Zusammensetzung, umfassend eine Verbindung oder ein pharmazeutisch unbedenkliches Salz und/oder Solvat davon gemäß einem der Ansprüche 1 bis 12 und einen pharmazeutisch unbedenklicher Träger.

14. Verbindung gemäß einem der Ansprüche 1 bis 12 zur Verwendung in einem Verfahren zum Behandeln einer Erkrankung bei einem Patienten, der daran leidet, wobei die Erkrankung ein Krebs, eine neurodegenerative Erkrankung, eine entzündliche oder Autoimmunerkrankung oder eine Herz-Kreislauf-Erkrankung ist.

15. Verbindung zur Verwendung gemäß Anspruch 14, wobei der Krebs eine Leukämie oder ein Lymphom ist, ausgewählt aus der Gruppe, bestehend aus akuter myeloischer Leukämie (AML), chronischer myeloischer Leukämie (CML), akuter lymphatischer Leukämie (ALL), chronischer lymphatischer Leukämie (CLL) und Non-Hodgkin-Lymphom (NHL), Burkitt-Lymphom, Haarzelllymphom (HCL), Waldenstrom-Makroglobulinämie, chronischer lymphatischer Leukämie/kleinzelligem lymphatischem Lymphom (CLL/SLL), diffusem großzelligem B-Zell-Lymphom (DLBCL), chronischer lymphatischer B-Zell-Leukämie (B-CLL), Mantelzelllymphom (MCL), follikulärem Lymphom (FL), Marginalzonenlymphom (MZL), akuter lymphoblastischer prä-B-Leukämie (pre-B ALL), multiplem Myelom (MM), Eierstockkrebs, Gliomen, Dickdarmkrebs, Brustkrebs, Blasenkrebs, Magenkrebs, Speiseröhrenkrebs, Bauchspeicheldrüsenkrebs, Leberkrebs, kleinzelligem Lungenkrebs (SCLC), nicht-kleinzelligem Lungenkrebs (NSCLC), Kopf- und Hals-Plattenepitelzellenkrebs, Mesotheliom, Melanom, Gliom, Glioblastom, neuroendokrine Bauchspeicheldrüsentumoren, Basalzellkarzinom, Plattenepithelkarzinom, Nierenzellkarzinom, invasivem duktalem Karzinom, Adenokarzinom, Merkelzellkarzinom, Hautkrebs, Prostatakrebs, Kolorektalkrebs, Weichteilsarkom, Osteosarkom, Ewing-Sarkom, Chondrosarkom und Myelom;
wobei die neurodegenerative Erkrankung ausgewählt ist aus der Gruppe, bestehend aus Alzheimer-Erkrankung, Parkinson-Erkrankung, amyotropher Lateralsklerose, Friedreich-Ataxie, Huntington-Erkrankung, Lewy-Body-Demenz und spinaler Muskelatrophie; und
die kardiovaskuläre Erkrankung Atherosklerose ist.

## Revendications

1. Composé de formule (II), ou sel et/ou solvate pharmaceutiquement acceptable de celui-ci : 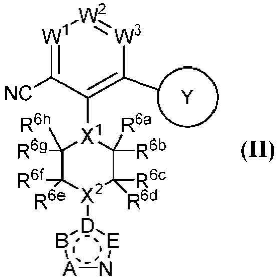 dans lequel
W¹ est N ou CR¹, W² est N ou CR² et W³ est N ou CR³, dans lequel pas plus d'un parmi W¹, W² et W³ est N ;
X¹ et X² sont indépendamment choisis parmi CR⁴ et N ;
Le cycle Y 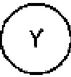 répond à la formule (a) : 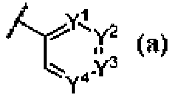
dans laquelle Y¹, Y², Y³ et Y⁴ sont indépendamment choisis parmi CR⁵ et N, dans laquelle Y¹, Y², Y³ et Y⁴ ne sont pas simultanément N,
ou le cycle Y 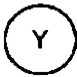 répond à la formule (b) : 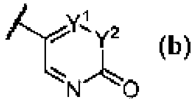
dans laquelle Y¹ est CR⁵ et Y² est NR^{5'} ;
ou le cycle Y 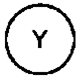 répond à la formule (c) : 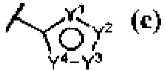
dans laquelle Y¹, Y², Y³ et Y⁴ sont indépendamment choisis parmi CR⁵ et N ;
éventuellement, soit Y¹ et Y², ou Y² et Y³, ou Y³ et Y⁴ représentent un cycle fusionné choisi parmi un cycloalkyle en C₅-C₈, un aryle en C₆-C₁₀, un hétéroaryle à 5 à 10 chaînons (dans lequel 1 à 4 chaînons hétéroaryle sont indépendamment choisis parmi N, O et S) et un hétérocycloalkyle à 5 à 8 chaînons (dans lequel 1 à 4 chaînons cycliques sont indépendamment choisis parmi N, B, O et S), dans lequel le cycle est éventuellement substitué par 1 à 3 substituants indépendamment choisis dans le groupe constitué de alkyle en C₁-C₆, cycloalkyle en C₃-C₈, hétérocycloalkyle à 3 à 6 chaînons (dans lequel 1 à 4 chaînons cycliques sont indépendamment choisis parmi N, O et S), alcényle en C₂-C₆, alcynyle en C₂-C₆, halo, haloalkyle en C₁-C₆, haloalcényle en C₂-C₆, haloalcynyle en C₂-C₆, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, - R^{b}-C(O)N(R^{a})₂, -R^{b}-O-R^{C}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), -R^{b}-S(O)ₜR^{a} (où t est 1 ou 2), -R^{b}-S(O)ₜOR^{a} (où t est 1 ou 2) et -R^{b}-S(O)ₜN(R^{a})₂ (où t est 1 ou 2),
A, B et E sont indépendamment choisis parmi C, N, O et S, et D est C ou N,
dans lequel 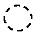 représente la présence de doubles liaisons de sorte que le cycle A-B-D-EN est aromatique et est éventuellement substitué par un ou deux substituants indépendamment choisis parmi alkyle en C₁-C₃, cycloalkyle en C₃-C₅, OH, OMe, NH₂, N(H)Me, NMe₂ ;
dans lequel pas plus de deux parmi A, B, D et E sont simultanément N, O ou S ;
R¹, R² et R³ sont indépendamment choisis dans le groupe constitué de : hydrogène, halo, cyano, nitro, -R^{b}-OR^{a}, -R^{b}-O-R^{c}-O-R^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{C}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), -R^{b}-S(O)ₜR^{a} (où t est 1 ou 2), -R^{b}-S(O)ₜOR^{a} (où t est 1 ou 2), -R^{b}-S(O)ₜN(R^{a})₂ (où t est 1 ou 2), alkyle en C₁-C₆, haloalkyle en C₁-C₆, alcényle en C₂-C₆, alcynyle en C₂-C₆, cycloalkyle en C₃-C₈, aryle en C₆-C₁₀, -(alkyle en C₁-C₆)(aryle en C₆-C₁₀), hétéroaryle à 5 à 10 chaînons (où 1 à 4 chaînons hétéroaryle sont indépendamment choisis parmi N, O et S) éventuellement fusionné à un hétérocycloalkyle à 3 à 6 chaînons (dans lequel 1 à 4 chaînons cycliques sont indépendamment choisis parmi N, O et S), et un hétérocycloalkyle à 3 à 6 chaînons (dans lequel 1 à 4 chaînons cycliques sont indépendamment choisis parmi N, O et S),
ou R¹ et R², ou R² et R³, conjointement avec les atomes de carbone auxquels ils sont liés, forment un cycloalkyle en C₅-C₈ fusionné, un aryle en C₆-C₁₀, un hétéroaryle à 5 à 10 chaînons (dans lequel 1 à 4 chaînons hétéroaryle sont indépendamment choisis parmi N, O et S), et un hétérocycloalkyle à 5 à 8 chaînons (dans lequel 1 à 4 chaînons cycliques sont indépendamment choisis parmi N, O et S) ;
dans lequel tout hétéroaryle ou hétérocycloalkyle dans R¹, R² et R³ est éventuellement et indépendamment substitué par 1 à 3 substituants choisis dans le groupe constitué de alkyle en C₁-C₆, halo, hydroxy, cycloalkyle en C₃-C₈, hétérocycloalkyle (dans lequel 1 à 4 chaînons cycliques sont indépendamment choisis parmi N, O et S), et -R^{b}-N(R^{a})₂ ;
R⁴ dans chaque cas est indépendamment H, OH, halo, alkyle en C₁-C₆ ou alcoxy en C₁-C₆ ;
R⁵ dans chaque cas est indépendamment choisi dans le groupe constitué de : hydrogène, halo, cyano, nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, - R^{b}-N(R^{a})₂, -R^{b}-C(O)R^{a}, -R^{b}-C(O)OR^{a}, -R^{b}-C(O)N(R^{a})₂, -R^{b}-O-R^{C}-C(O)N(R^{a})₂, -R^{b}-O-R^{c}-N(R^{a})₂, -B(OR^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), -R^{b}-S(O)ₜR^{a} (où t est 1 ou 2), -R^{b}-S(O)ₜOR^{a} (où t est 1 ou 2), -R^{b}-OS(O)ₜF (où t est 1 ou 2), -R^{b}-S(O)ₜN(R^{a})₂ (où t est 1 ou 2), alkyle en C₁-C₆, alcynyle en C₂-C₆, cycloalkyle en C₃-C₈, aryle en C₆-C₁₀, hétéroaryle à 5 à 10 chaînons (dans lequel 1 à 4 chaînons hétéroaryle sont indépendamment choisis parmi N, O et S) et hétérocycloalkyle à 3 à 6 chaînons (dans lequel 1 à 4 chaînons cycliques sont indépendamment choisis parmi N, O et S) ;
R^{5'} est choisi dans le groupe constitué de : hydrogène, -R^{c}-R^{a}, -R^{c}-OR^{a}, -R^{b}-OC(O)-R^{a}, - R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), -R^{b}-S(O)ₜR^{a} (où t est 1 ou 2), -R^{b}-S(O)ₜOR^{a} (où t est 1 ou 2), - R^{b}-S(O)ₜN(R^{a})₂ (où t est 1 ou 2), alkyle en C₁-C₆, cycloalkyle en C₃-C₈, aryle en C₆-C₁₀, hétéroaryle à 5 à 10 chaînons (dans lequel 1 à 4 chaînons hétéroaryle sont indépendamment choisis parmi N, O et S) et hétérocycloalkyle à 3 à 6 chaînons (dans lequel 1 à 4 chaînons cycliques sont indépendamment choisis parmi N, O et S) ;
dans lequel tout hétéroaryle ou hétérocycloalkyle dans R⁵ et R^{5'} est éventuellement et indépendamment substitué par 1 à 3 substituants choisis dans le groupe constitué de alkyle en C₁-C₆, halo, hydroxy, cycloalkyle en C₃-C₈ et -R^{b}-N(R^{a})₂ ;
R^{6a}, R^{6b}, R^{6c}, R^{6d}, R^{6c}, R^{6f}, R^{6g} et R^{6h} sont indépendamment choisis dans le groupe constitué de H, halo, NO₂, OH, CN, -R^{b}-N(R^{a})₂, -R^{b}-OH, alkyle en C₁-C₆ et alcoxy en C₁-C₆ ;
et/ou, éventuellement, R^{6a} et R^{6b}, ou R^{6c} et R^{6d}, ou R^{6e} et R^{6f}, ou R^{6g} et R^{6h} indépendamment représentent oxo, thioxo, imino ou oximo ;
et/ou, éventuellement, R^{6a} et R^{6b}, ou R^{6c} et R^{6d}, ou R^{6e} et R^{6f}, ou R^{6g} et R^{6h}, conjointement avec les atomes de carbone auxquels ils sont liés, se combinent indépendamment pour former un cycle fusionné choisi parmi un cycloalkyle en C₃-C₆ et un hétérocycloalkyle en C₃-C₆ (dans lequel 1 à 4 chaînons cycliques sont indépendamment choisis parmi N, O et S) ;
et/ou, éventuellement, l'un de R^{6c} et R^{6d} conjointement avec l'un de R^{6e} et R^{6f} représentent une liaison entre les chaînons de carbone du cycle auxquels ils sont liés ;
R^{a} dans chaque cas est indépendamment choisi parmi hydrogène, alkyle en C₁-C₆, cycloalkyle en C₃-C₈, -(alkyle en C₁-C₆)(cycloalkyle en C₃-C₈), aryle en C₆-C₁₀, hétéroaryle à 5 à 10 chaînons (dans lequel 1 à 4 chaînons hétéroaryle sont indépendamment choisis parmi N, O et S) et hétérocycloalkyle à 3 à 6 chaînons (dans lequel 1 à 4 chaînons cycliques sont indépendamment choisis parmi N, O et S) ;
R^{b} dans chaque cas est indépendamment choisi parmi une liaison directe, une chaîne alkylène en C₂-C₆ et alcénylène en C₂-C₆ droite ou ramifiée; dans lequel tout hétéroaryle ou hétérocycloalkyle dans R^{a} et R^{b} est éventuellement et indépendamment substitué par 1 à 3 substituants choisis dans le groupe constitué de alkyle en C₁-C₆, halo, hydroxy,
R^{c} dans chaque cas est indépendamment choisi parmi une chaîne alkylène en C₂-C₆ et alcénylène en C₂-C₆ droite ou ramifiée.

2. Composé ou sel et/ou solvate pharmaceutiquement acceptable de celui-ci selon la revendication 1, ledit composé répondant à la formule (I) : 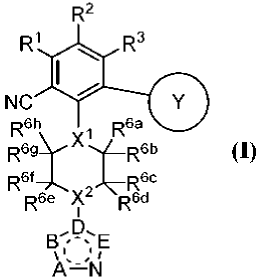 dans laquelle
chacun de X¹ et X² est N ;
Le cycle Y 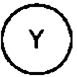 répond à la formule (a) : 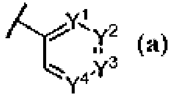
dans laquelle Y¹, Y², Y³ et Y⁴ sont indépendamment choisis parmi CR⁵ et N dans laquelle Y¹, Y², Y³ et Y⁴ ne sont pas simultanément N,
ou le cycle Y 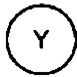 répond à la formule (b) : 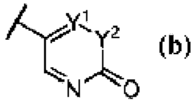
dans laquelle Y¹ est CR⁵ et Y² est NR^{5'} ;
éventuellement, soit Y¹ et Y², ou Y² et Y³, ou Y³ et Y⁴ représentent un cycle fusionné choisi parmi un cycloalkyle en C₅-C₈, un aryle en C₆-C₁₀, un hétéroaryle à 5 à 10 chaînons (dans lequel 1 à 4 chaînons hétéroaryle sont indépendamment choisis parmi N, O et S), et un hétérocycloalkyle à 5 à 8 chaînons (dans lequel 1 à 4 chaînons cycliques sont indépendamment choisis parmi N, O et S), dans lequel le cycle est éventuellement substitué par 1 à 3 substituants indépendamment choisis dans le groupe constitué de alkyle en C₁-C₆, cycloalkyle en C₃-C₈, hétérocycloalkyle à 3 à 6 chaînons (dans lequel 1 à 4 chaînons cycliques sont indépendamment choisis parmi N, O et S), alcényle en C₂-C₆, alcynyle en C₂-C₆, halo, haloalkyle en C₁-C₆, haloalcényle en C₂-C₆, haloalcynyle en C₂-C₆, oxo, thioxo, cyano, nitro, -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-OR^{a}(N(R^{a})₂), -R^{b}-C(O)R^{a}, -R^{b}-C(O)OR^{a}, - R^{b}-C(O)N(R^{a})₂, -R^{b}-O-R^{C}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), -R^{b}-S(O)ₜR^{a} (où t est 1 ou 2), -R^{b}-S(O)ₜOR^{a} (où t est 1 ou 2) et -R^{b}-S(O)ₜN(R^{a})₂ (où t est 1 ou 2),
A, B et E sont indépendamment choisis parmi C, N, O et S, et D est C ou N,
dans lequel 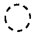 représente la présence de doubles liaisons de sorte que le cycle A-B-D-EN est aromatique et est éventuellement substitué par un ou deux substituants indépendamment choisis parmi alkyle en C₁-C₃, cycloalkyle en C₃-C₅, OH, OMe, NH₂, N(H)Me, NMe₂ ;
dans lequel pas plus de deux parmi A, B, D et E sont simultanément N, O ou S ;
R¹, R² et R³ sont indépendamment choisis dans le groupe constitué de : hydrogène, halo, cyano, nitro, -R^{b}-OR^{a}, -R^{b}-O-R^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{C}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), -R^{b}-S(O)ₜR^{a} (où t est 1 ou 2), -R^{b}-S(O)ₜOR^{a} (où t est 1 ou 2), -R^{b}-S(O)ₜN(R^{a})₂ (où t est 1 ou 2), alkyle en C₁-C₆, cycloalkyle en C₃-C₈, aryle en C₆-C₁₀, -(alkyle en C₁-C₆)(aryle en C₆-C₁₀), hétéroaryle à 5 à 10 chaînons (dans lequel 1 à 4 chaînons hétéroaryle sont indépendamment choisis parmi N, O et S), et hétérocycloalkyle à 3 à 6 chaînons (dans lequel 1 à 4 chaînons cycliques sont indépendamment choisis parmi N, O et S),
ou R¹ et R², ou R² et R³, conjointement avec les atomes de carbone auxquels ils sont liés, forment un cycloalkyle en C₅-C₈ fusionné, un aryle en C₆-C₁₀, un hétéroaryle à 5 à 10 chaînons (dans lequel 1 à 4 chaînons hétéroaryle sont indépendamment choisis parmi N, O et S), et un hétérocycloalkyle à 5 à 8 chaînons (dans lequel 1 à 4 chaînons cycliques sont indépendamment choisis parmi N, O et S) ;
R⁴ dans chaque cas est indépendamment H, OH, halo, alkyle en C₁-C₆ ou alcoxy en C₁-C₆;
R⁵ dans chaque cas est indépendamment choisi dans le groupe constitué de : hydrogène, halo, cyano, nitro, -R^{b}-OR^{a}, -R^{b}-O-R^{c}-OR^{a}, -R^{b}-OC(O)-R^{a}, -R^{b}-OC(O)-OR^{a}, -R^{b}-OC(O)-N(R^{a})₂, - R^{b}-N(R^{a})₂, -R^{b}-C(O)R^{a}, -R^{b}-C(O)OR^{a}, -R^{b}-C(O)N(R^{a})₂, -R^{b}-O-R^{C}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), - R^{b}-S(O)ₜR^{a} (où t est 1 ou 2), -R^{b}-S(O)ₜOR^{a} (où t est 1 ou 2), -R^{b}-S(O)ₜN(R^{a})₂ (où t est 1 ou 2), alkyle en C₁-C₆, cycloalkyle en C₃-C₈, aryle en C₆-C₁₀, hétéroaryle à 5 à 10 chaînons (dans lequel 1 à 4 chaînons hétéroaryle sont indépendamment choisis parmi N, O et S), et hétérocycloalkyle à 3 à 6 chaînons (dans lequel 1 à 4 chaînons cycliques sont indépendamment choisis parmi N, O et S) ;
R^{5'} est choisi dans le groupe constitué de : hydrogène, -R^{c}-R^{a}, -R^{c}-OR^{a}, -R^{b}-OC(O)-R^{a}, - R^{b}-OC(O)-OR^{a}, -R^{b}-OC(O)-N(R^{a})₂, -R^{c}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-O-R^{c}-N(R^{a})₂, -R^{b}-N(R^{a})-R^{c}-N(R^{a})₂, -R^{b}-N(R^{a})C(O)OR^{a}, -R^{b}-N(R^{a})C(O)R^{a}, -R^{b}-N(R^{a})S(O)ₜR^{a} (où t est 1 ou 2), -R^{b}-S(O)ₜR^{a} (où t est 1 ou 2), -R^{b}-S(O)ₜOR^{a} (où t est 1 ou 2), - R^{b}-S(O)ₜN(R^{a})₂ (où t est 1 ou 2), alkyle en C₁-C₆, cycloalkyle en C₃-C₈, aryle en C₆-C₁₀, hétéroaryle à 5 à 10 chaînons (dans lequel 1 à 4 chaînons hétéroaryle sont indépendamment choisis parmi N, O et S), et hétérocycloalkyle à 3 à 6 chaînons (dans lequel 1 à 4 chaînons cycliques sont indépendamment choisis parmi N, O et S) ;
R^{6a}, R^{6b}, R^{6c}, R^{6d}, R^{6c}, R^{6f}, R^{6g} et R^{6h} sont indépendamment choisis dans le groupe constitué de H, halo, NO₂, OH, CN, -R^{b}-N(R^{a})₂, -R^{b}-OH, alkyle en C₁-C₆ et alcoxy en C₁-C₆ ;
ou, éventuellement, R^{6a} et R^{6b}, ou R^{6c} et R^{6d}, ou R^{6e} et R^{6f}, ou R^{6g} et R^{6h} représentent indépendamment oxo, thioxo, imino ou oximo ;
ou, éventuellement, R^{6a} et R^{6b}, ou R^{6c} et R^{6d}, ou R^{6e} et R^{6f}, ou R^{6g} et R^{6h}, conjointement avec les atomes de carbone auxquels ils sont liés, se combinent indépendamment pour former un cycle fusionné choisi parmi un cycloalkyle en C₃-C₆ et un hétérocycloalkyle en C₃-C₆ (dans lequel 1 à 4 chaînons cycliques sont indépendamment choisis parmi N, O et S) ;
R^{a} dans chaque cas est indépendamment choisi parmi hydrogène, alkyle en C₁-C₆, cycloalkyle en C₃-C₈, aryle en C₆-C₁₀, hétéroaryle à 5 à 10 chaînons (dans lequel 1 à 4 chaînons hétéroaryle sont indépendamment choisis parmi N, O et S) et hétérocycloalkyle à 3 à 6 chaînons (dans lequel 1 à 4 chaînons cycliques sont indépendamment choisis parmi N, O et S) ;
R^{b} dans chaque cas est indépendamment choisi parmi une liaison directe, une chaîne alkylène en C₂-C₆ et alcénylène en C₂-C₆ droite ou ramifiée ;
R^{c} dans chaque cas est indépendamment choisi parmi une chaîne alkylène en C₂-C₆ et alcénylène en C₂-C₆ droite ou ramifiée.

3. Composé ou sel et/ou solvate pharmaceutiquement acceptable de celui-ci selon la revendication 1, dans lequel W¹ est N, W² est CR² et W³ est CR³ ; ou W¹ est CR¹, W² est CR² et W³ est N ; ou W² est CR² et W³ est CR³.

4. Composé ou sel et/ou solvate pharmaceutiquement acceptable de celui-ci selon l'une quelconque des revendications 1 à 3, dans lequel D est C, et dans lequel A, B et E sont indépendamment choisis parmi C et N, ou au moins un de A, B et E est N, ou deux de A, B et E sont N.

5. Composé ou sel et/ou solvate pharmaceutiquement acceptable de celui-ci selon l'une quelconque des revendications 1 à 3, dans lequel le cycle A-B-D-E-N est un cycle éventuellement substitué choisi dans le groupe constitué de :

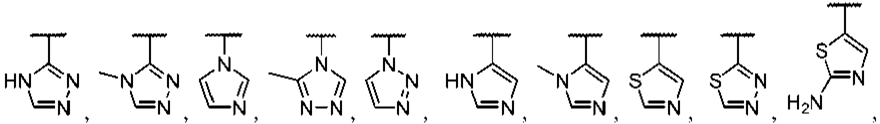

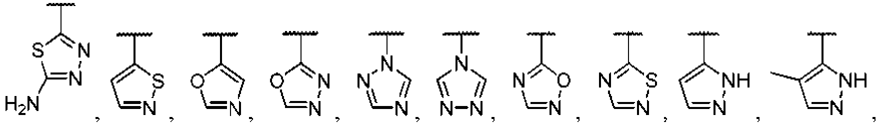

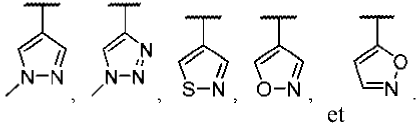

6. Composé ou sel et/ou solvate pharmaceutiquement acceptable de celui-ci selon l'une quelconque des revendications 1 à 5, dans lequel le cycle Y répond à la formule (a), et dans lequel
un de Y¹, Y², Y³ et Y⁴ est N et chacun des trois restants est CR⁵, ou dans lequel
deux de Y¹, Y², Y³ et Y⁴ sont N et chacun des deux restants est CR⁵, ou dans lequel
soit Y¹ et Y² soit Y³ et Y⁴ représentent un cycle fusionné éventuellement substitué.

7. Composé ou sel et/ou solvate pharmaceutiquement acceptable de celui-ci selon l'une quelconque des revendications 1 à 6, dans lequel R¹ est choisi dans le groupe constitué de H, halo, alkyle en C₁-C₆, -R^{b}-OR^{a} (dans lequel R^{b} est une liaison directe et R^{c} est alkyle en C₁-C₆), aryle en C₆-C₁₀, cycloalkyle en C₃-C₈, hétérocycloalkyle à 3 à 6 chaînons (dans lequel 1 à 4 chaînons cycliques sont indépendamment choisis parmi N, O et S) et hétéroaryle à 5 à 6 chaînons (dans lequel 1 à 4 chaînons hétéroaryle sont indépendamment choisis parmi N, O et S), et
chacun de R² et R³ est indépendamment H, halo, cyano, alkyle en C₁-C₆, cycloalkyle en C₃-C₈, hétérocycloalkyle à 3 à 6 chaînons (dans lequel 1 à 4 chaînons cycliques sont indépendamment choisis parmi N, O et S), et hétéroaryle à 5 à 6 chaînons (dans lequel 1 à 4 chaînons hétéroaryle sont indépendamment choisis parmi N, O et S), -R^{b}-OR^{a} (dans lequel R^{b} est une liaison directe et R^{a} est alkyle en C₁-C₆), ou CF₃.

8. Composé ou sel et/ou solvate pharmaceutiquement acceptable de celui-ci selon la revendication 1, ledit composé étant un composé répondant à la formule (IA), (IB) ou (ID) :

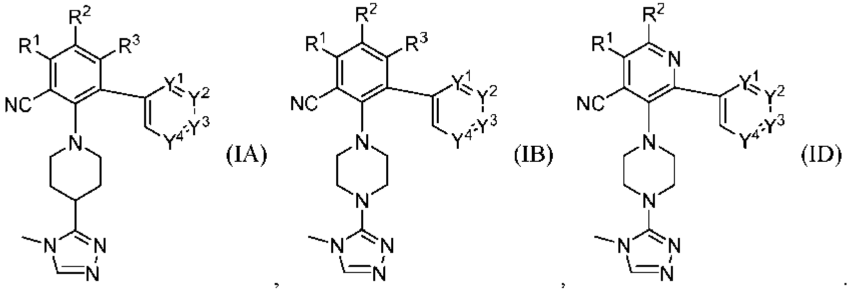

9. Composé ou sel et/ou solvate pharmaceutiquement acceptable de celui-ci selon la revendication 8, dans lequel :
R¹ est choisi dans le groupe constitué de H, halo, alcoxy en C₁-C₆, aryle en C₆-C₁₀, alkyle en C₁-C₆, cycloalkyle en C₃-C₆, hétéroaryle à 5 à 6 chaînons (dans lequel 1 à 4 chaînons hétéroaryle sont indépendamment choisis parmi N, O et S), et hétérocycloalkyle à 3 à 6 chaînons (dans lequel 1 à 4 chaînons cycliques sont indépendamment choisis parmi N, O et S) ;
chacun de R² est H, halo, cyano, -R^{b}-S(O)ₜR^{a} (où t est 1 ou 2), alkyle en C₁-C₆, cycloalkyle en C₃-C₆ ou CF₃, et
R³, lorsqu'il est présent, est halo, cyano, -R^{b}-S(O)ₜR^{a} (où t est 1 ou 2), alkyle en C₁-C₆, cycloalkyle en C₃-C₆ ou CF₃ ; et dans lequel
un de Y¹, Y², Y³ et Y⁴ est N et chacun des trois restants est CR⁵, ou
deux de Y¹, Y², Y³ et Y⁴ sont N et chacun des deux restants est CR⁵.

10. Composé ou sel et/ou solvate pharmaceutiquement acceptable de celui-ci selon la revendication 8 ou 9, dans lequel le cycle contenant Y¹, Y², Y³ et Y⁴ est :

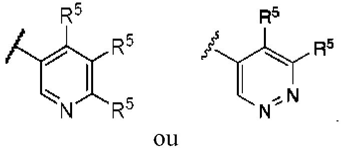

11. Composé ou sel et/ou solvate pharmaceutiquement acceptable de celui-ci selon l'une quelconque des revendications 8 à 10, dans lequel le cycle contenant Y¹, Y², Y³ et Y⁴ est :

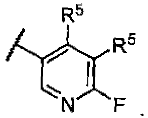

12. Composé ou sel et/ou solvate pharmaceutiquement acceptable de celui-ci choisi dans le tableau suivant :
| | |
|---|---|
| **4** | 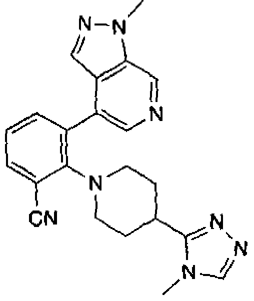 |
| **5** | 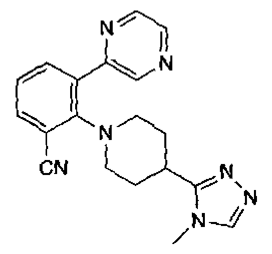 |
| **6** | 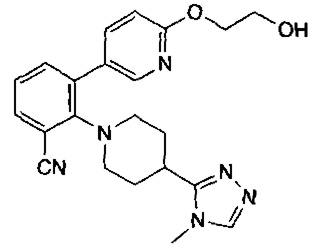 |
| **7** | 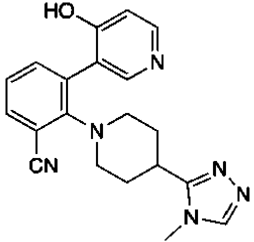 |
| **8** | 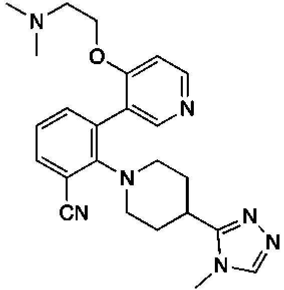 |
| **9** | 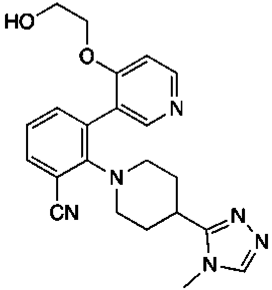 |
| **11** | 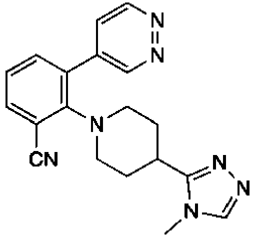 |
| **12** | 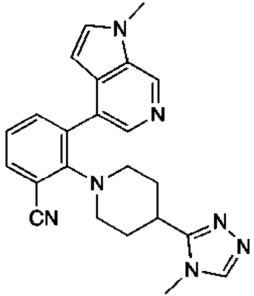 |
| **13** | 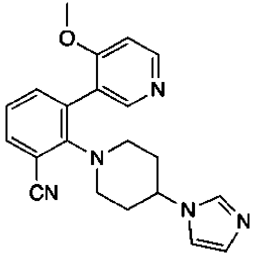 |
| **14** | 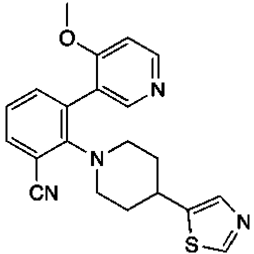 |
| **15** | 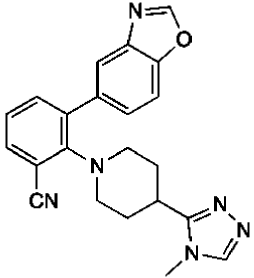 |
| **16** | 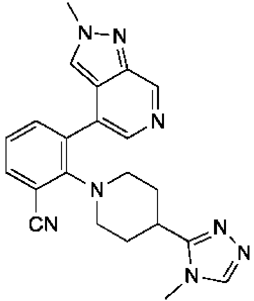 |
| **17** | 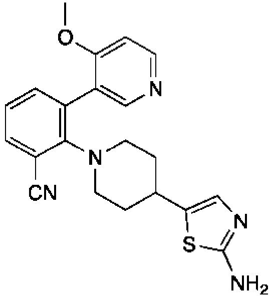 |
| **18** | 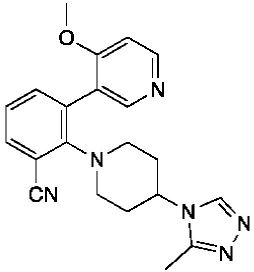 |
| **19** | 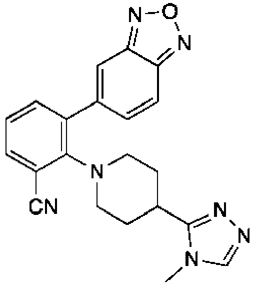 |
| **20** | 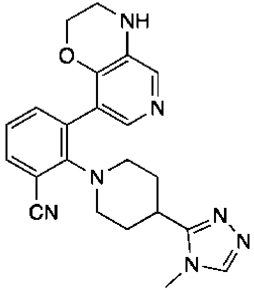 |
| **21** | 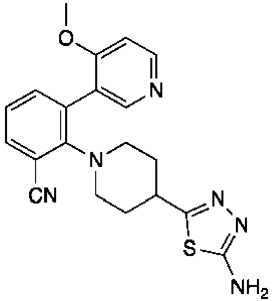 |
| **22** | 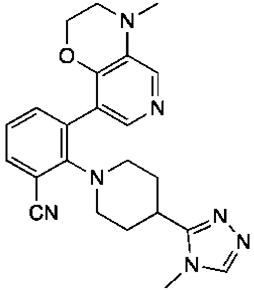 |
| **23** | 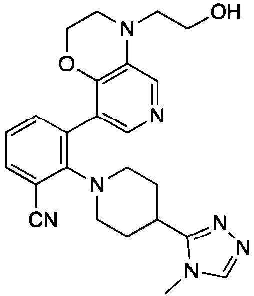 |
| **24** | 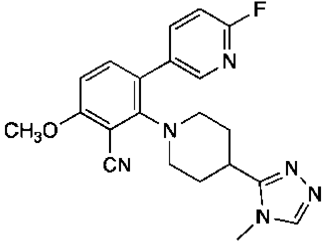 |
| **25** | 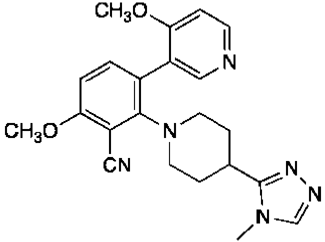 |
| **26** | 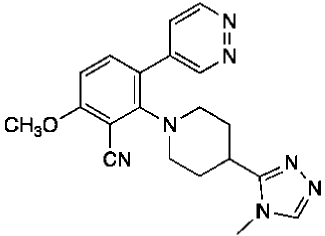 |
| **27** | 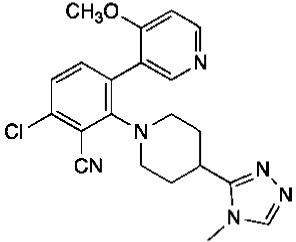 |
| **28** | 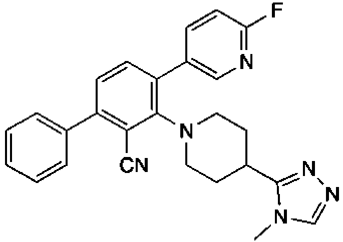 |
| **30** | 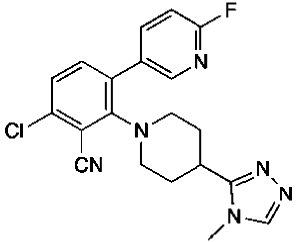 |
| **31** | 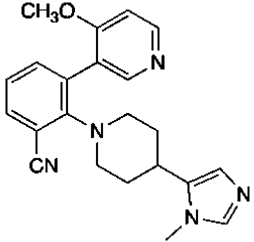 |
| **32** | 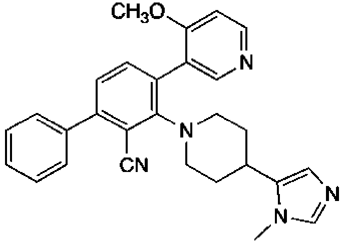 |
| **33** | 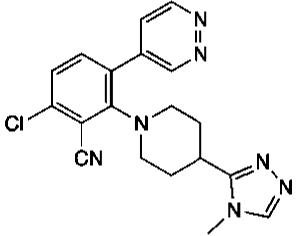 |
| **34** | 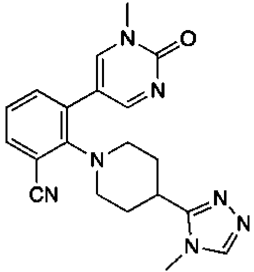 |
| **35** | 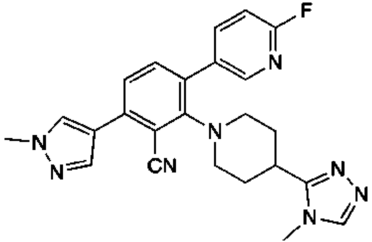 |
| **36** | 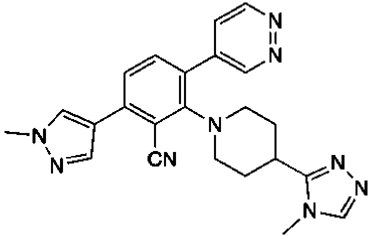 |
| **37** | 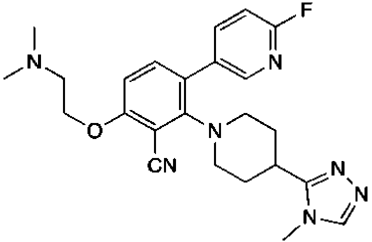 |
| **38** | 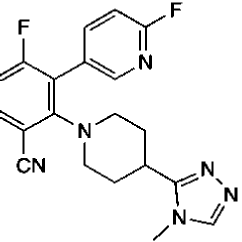 |
| **39** | 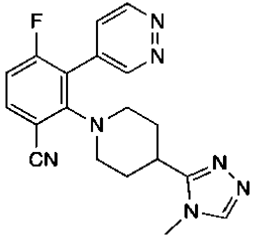 |
| **40** | 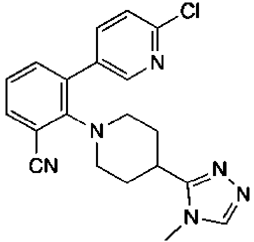 |
| **41** | 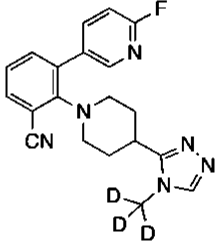 |
| **42** | 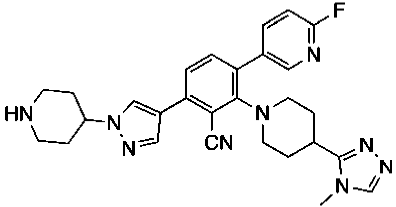 |
| **43** | 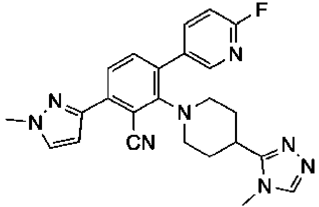 |
| **44** | 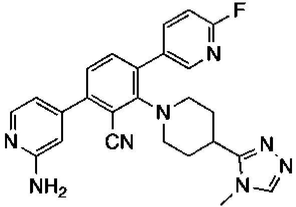 |
| **45** | 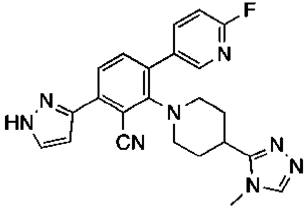 |
| **46** | 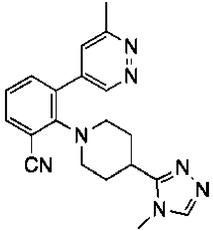 |
| **48** | 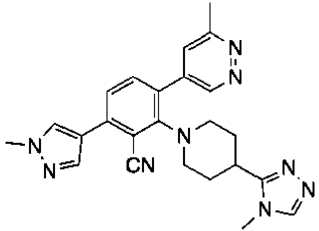 |
| **49** | 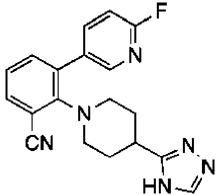 |
| **50** | 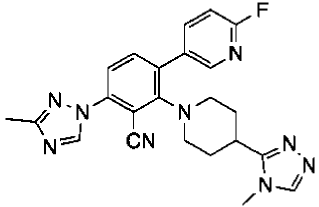 |
| **51** | 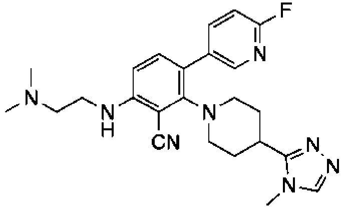 |
| **52** | 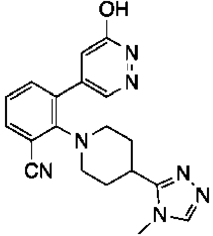 |
| **53** | 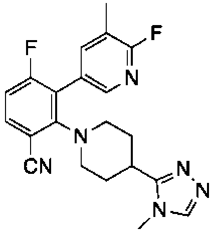 |
| **54** | 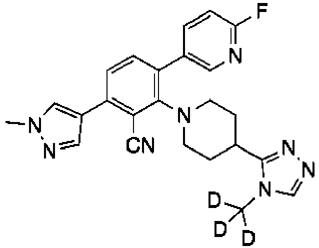 |
| **55** | 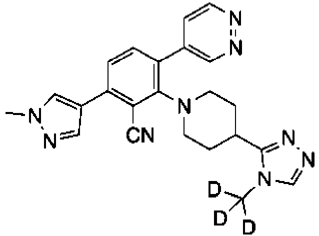 |
| **56** | 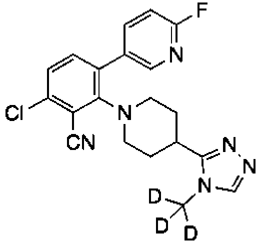 |
| **57** | 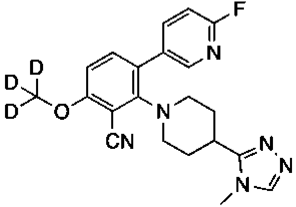 |
| 58 | 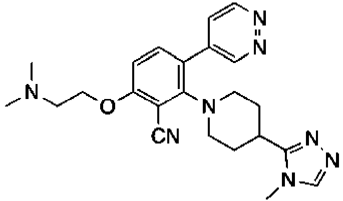 |
| **59** | 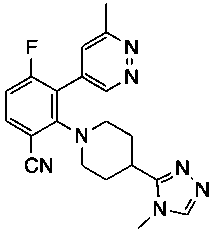 |
| **60** | 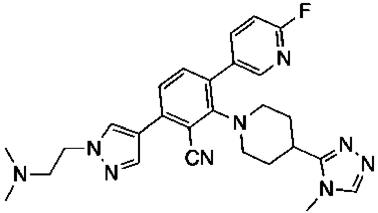 |
| 61 | 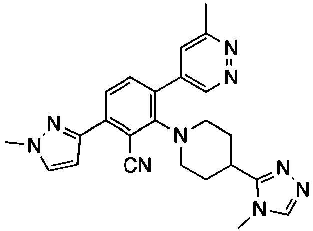 |
| **62** | 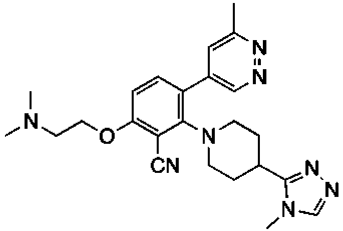 |
| **63** | 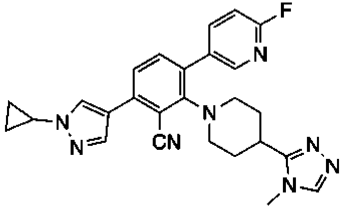 |
| **64** | 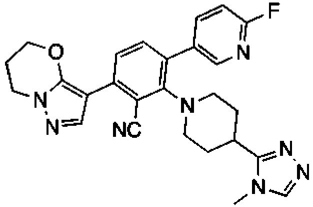 |
| **65** | 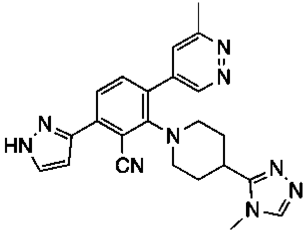 |
| **66** | 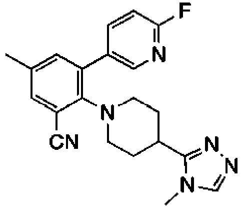 |
| **67** | 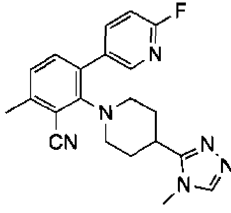 |
| **68** | 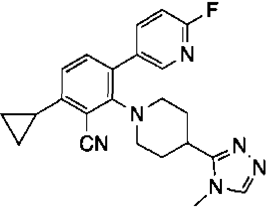 |
| **69** | 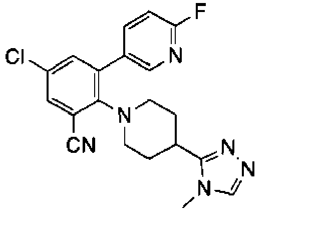 |
| **70** | 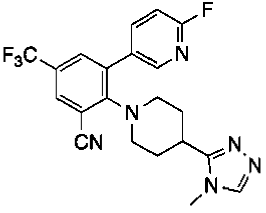 |
| **71** | 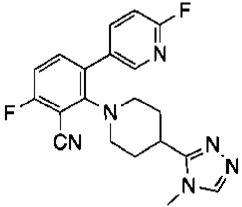 |
| **72** | 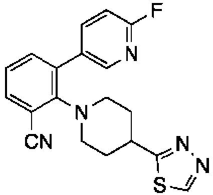 |
| **73** | 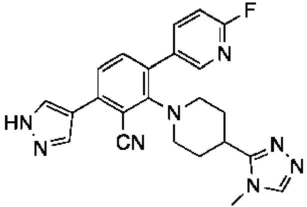 |
| **74** | 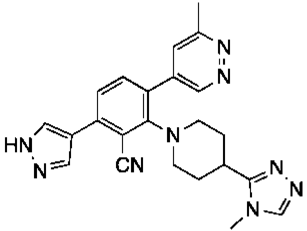 |
| **75** | 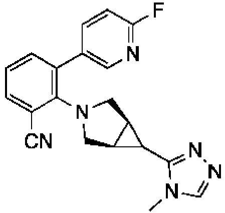 |
| **76** | 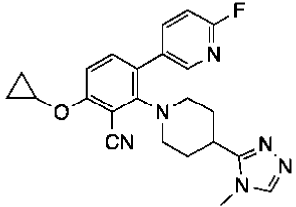 |
| **77** | 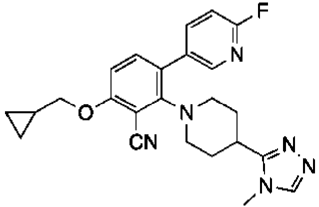 |
| **78** | 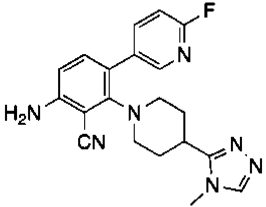 |
| **79** | 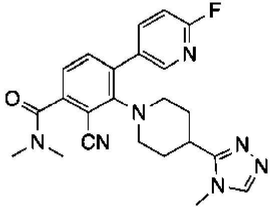 |
| **80** | 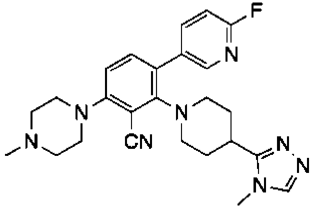 |
| **81** | 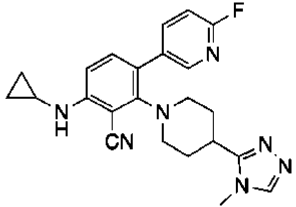 |
| **82** | 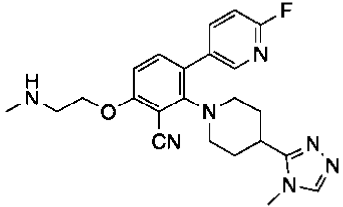 |
| **83** | 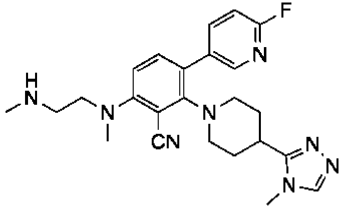 |
| **84** | 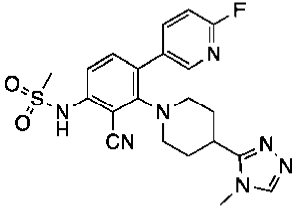 |
| **85** | 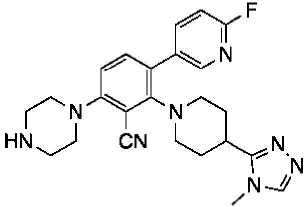 |
| **86** | 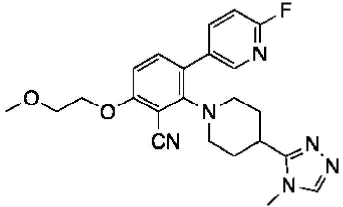 |
| **87** | 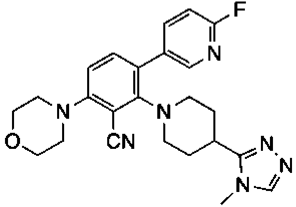 |
| **88** | 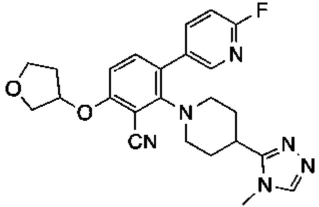 |
| **89** | 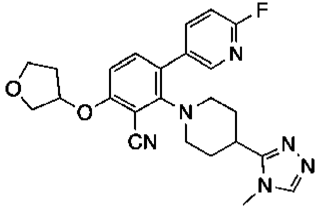 |
| **90** | 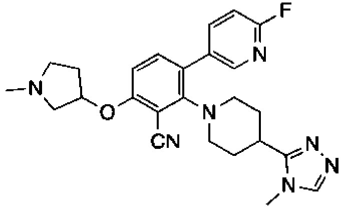 |
| **91** | 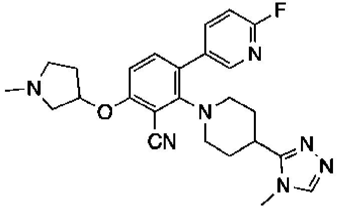 |
| **92** | 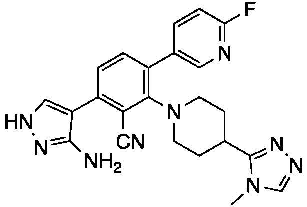 |
| **93** | 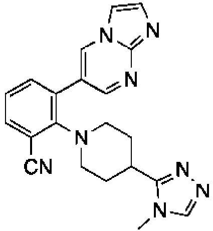 |
| **94** | 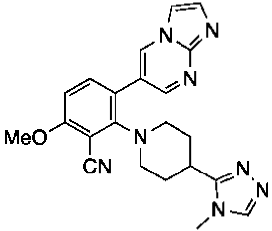 |
| **95** | 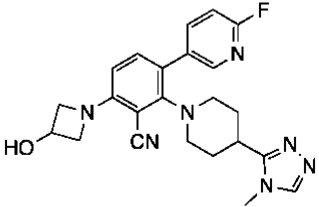 |
| **96** | 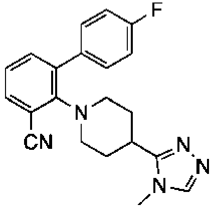 |
| **97** | 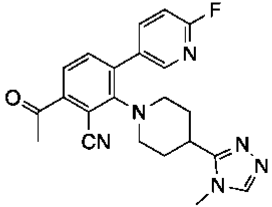 |
| **98** | 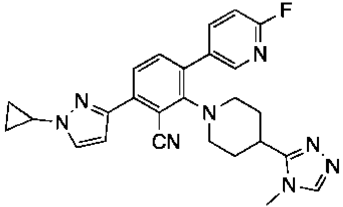 |
| **99** | 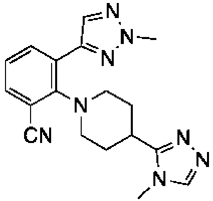 |
| **100** | 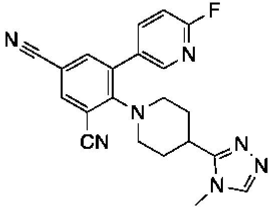 |
| **101** | 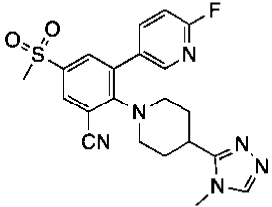 |
| **102** | 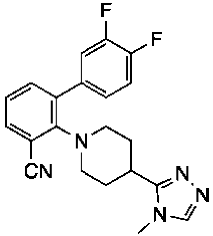 |
| **103** | 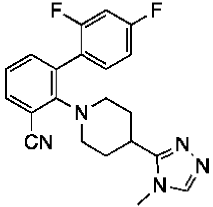 |
| **104** | 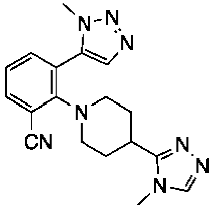 |
| **105** | 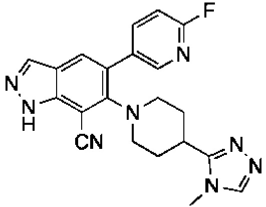 |
| **106** | 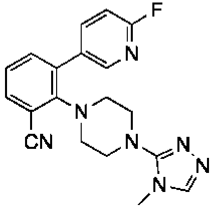 |
| **107** | 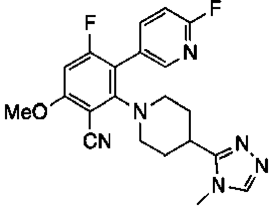 |
| **108** | 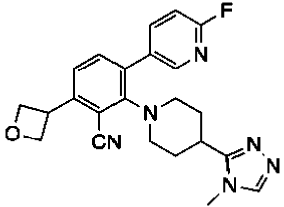 |
| **109** | 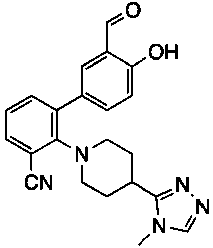 |
| **110** | 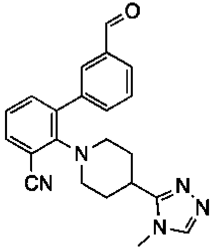 |
| **111** | 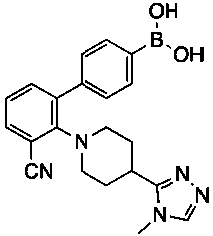 |
| **112** | 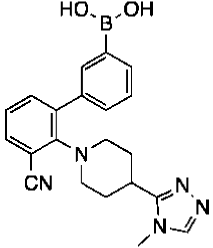 |
| **113** | 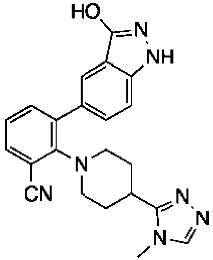 |
| **114** | 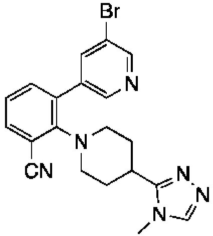 |
| **115** | 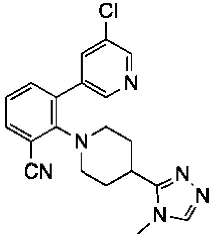 |
| **116** | 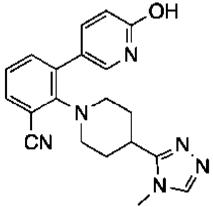 |
| **117** | 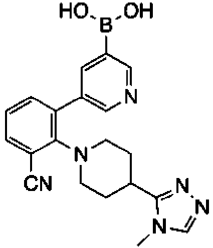 |
| **118** | 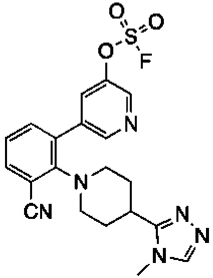 |
| **119** | 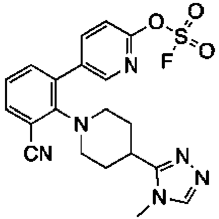 |
| **120** | 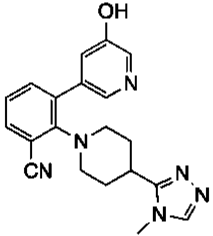 |
| **121** | 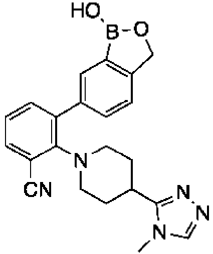 |
| **122** | 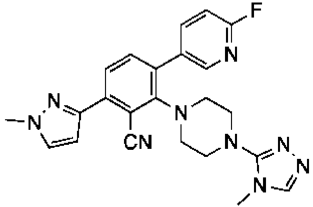 |
| **123** | 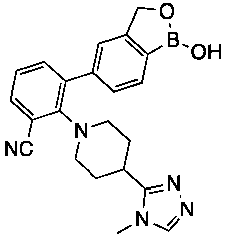 |
| **124** | 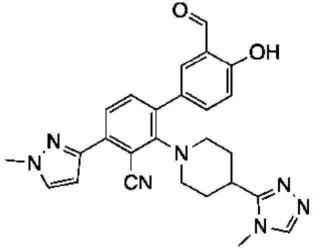 |
| **125** | 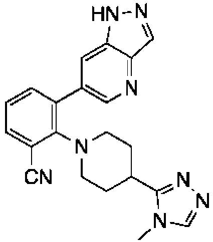 |
| **126** | 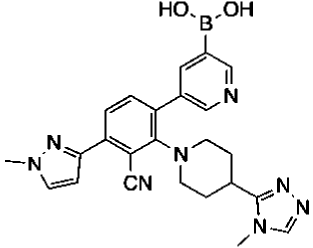 |
| **127** | 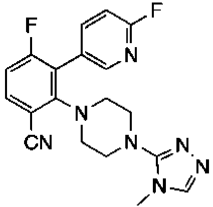 |
| **128** | 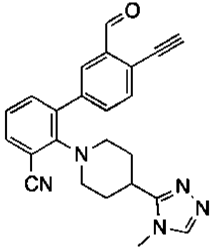 |
| **129** | 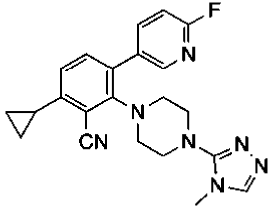 |
| **130** | 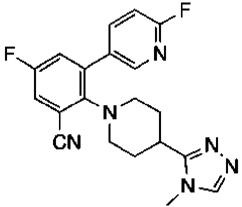 |
| **131** | 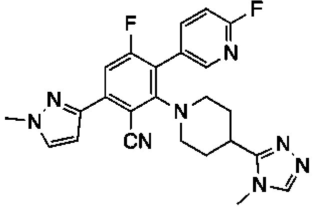 |
| **134** | 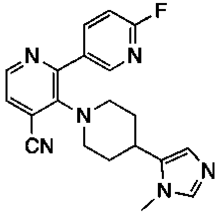 |
| **135** | 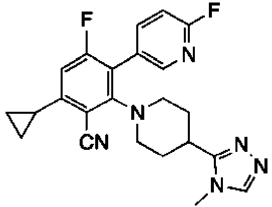 |
| **136** | 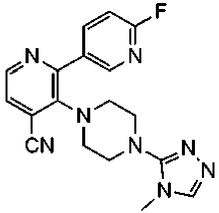 |
| **137** | 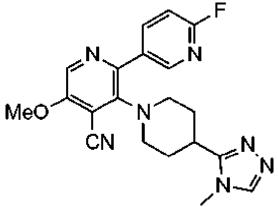 |
| **138** | 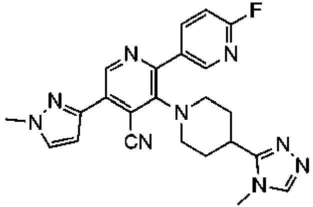 |
| **139** | 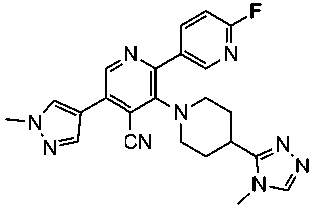 |
ou composé ou sel et/ou solvate pharmaceutiquement acceptable de celui-ci choisi dans le tableau suivant :

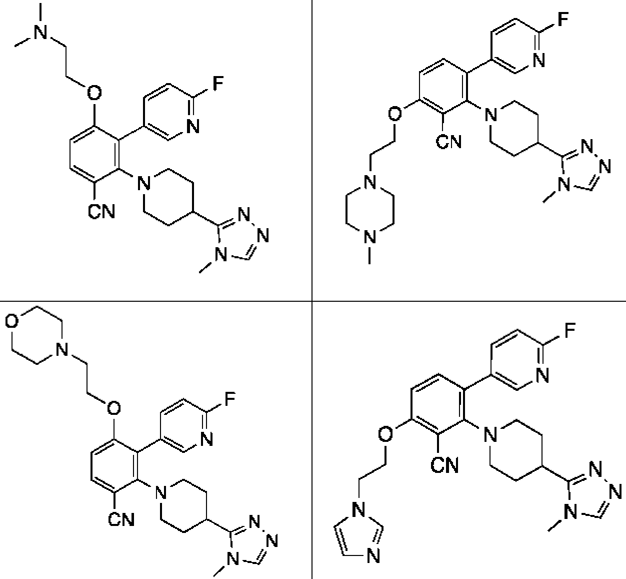

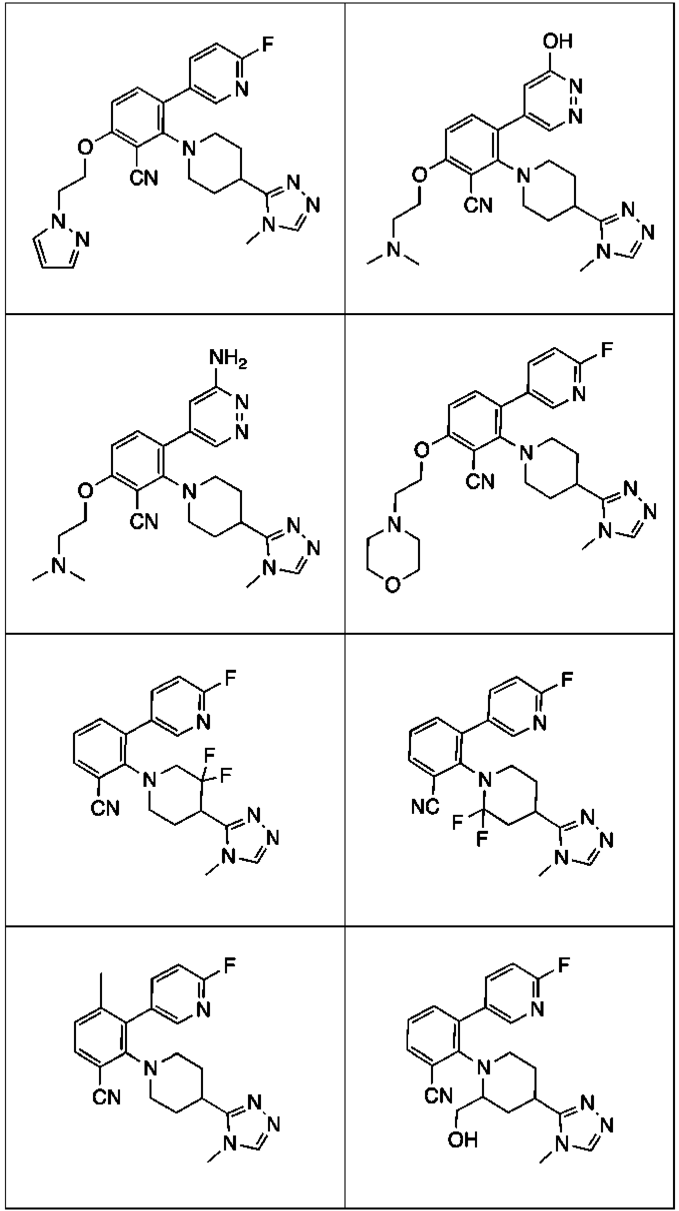

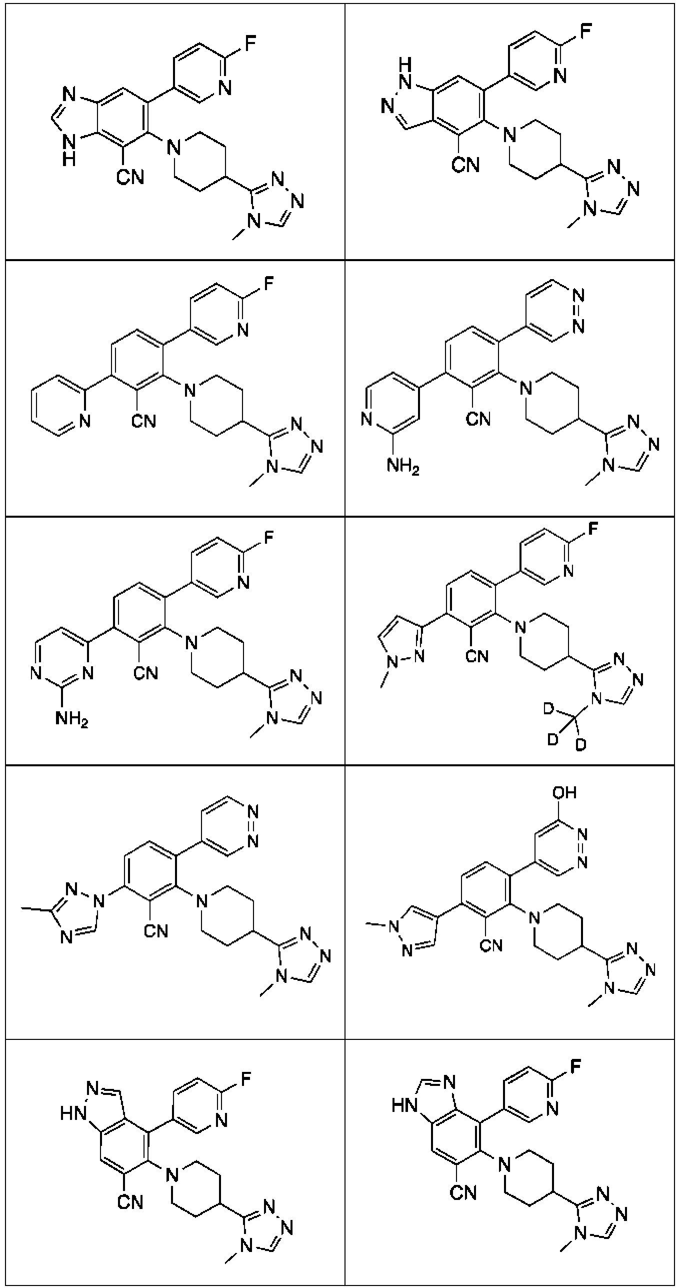

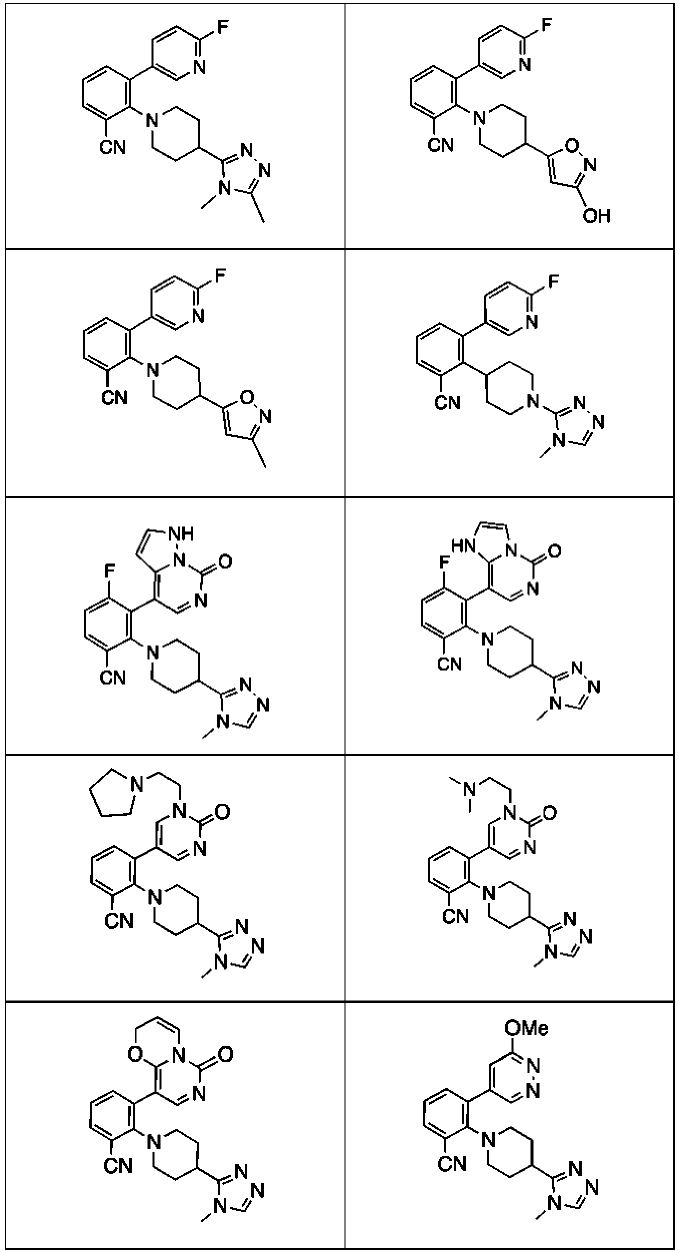

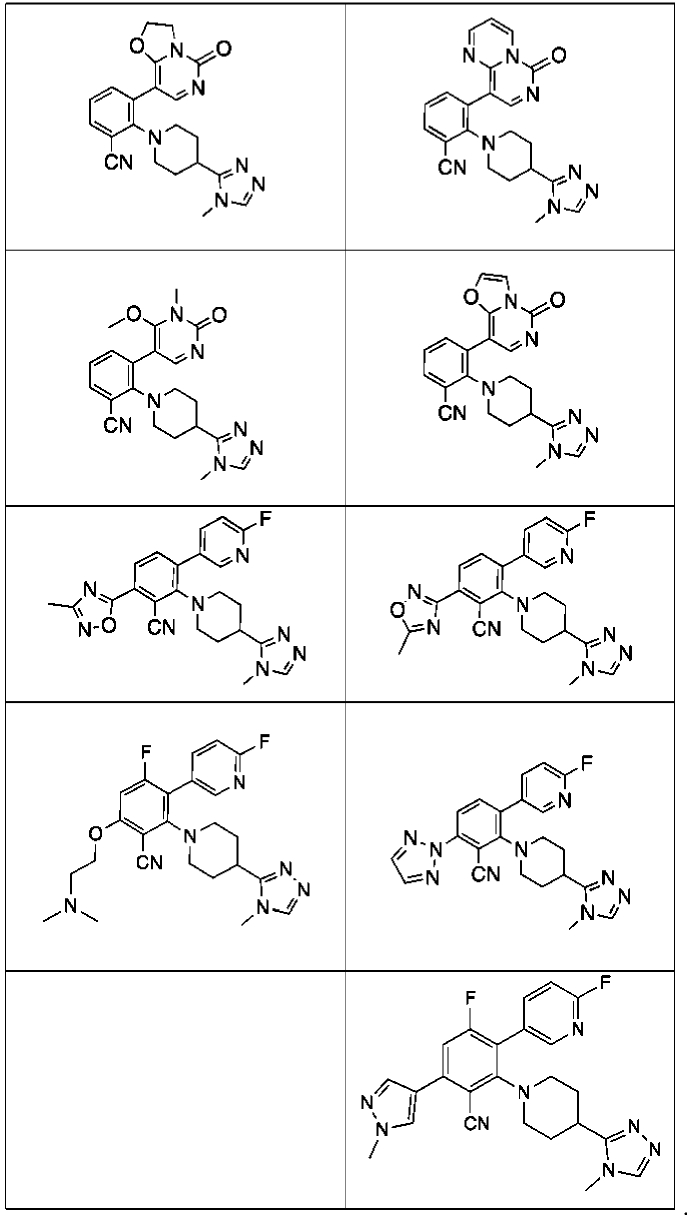

13. Composition pharmaceutique comprenant un composé ou sel et/ou solvate pharmaceutiquement acceptable de celui-ci selon l'une quelconque des revendications 1 à 12 et un support pharmaceutiquement acceptable.

14. Composé selon l'une quelconque des revendications 1 à 12, destiné à être utilisé dans un procédé pour le traitement d'une maladie chez un patient en souffrant, ladite maladie étant un cancer, une maladie neurodégénérative, une maladie inflammatoire ou auto-immune ou une maladie cardiovasculaire.

15. Composé destiné à être utilisé selon la revendication 14, dans lequel le cancer est une leucémie ou un lymphome choisi(e) dans le groupe constitué de la leucémie myéloïde aiguë (LMA), la leucémie myéloïde chronique (LMC), la leucémie lymphoïde aiguë (LAL), la leucémie lymphoïde chronique (LLC) et le lymphome non hodgkinien (LNH), le lymphome de Burkitt, le lymphome à cellules poilues (HCL), la macroglobulinémie de Waldenstrom, la leucémie lymphoïde chronique/le lymphome à petites cellules (LLC/SLL), le lymphome diffus à grandes cellules B (DLBCL), la leucémie lymphoïde chronique à cellules B (B-CLL), le lymphome à cellules du manteau (MCL), le lymphome folliculaire (FL), le lymphome de la zone marginale (MZL), la leucémie lymphoblastique aiguë à précurseur B (LLA préB), le myélome multiple (MM), le cancer de l'ovaire, les gliomes, le cancer du côlon, le cancer de la vessie, le cancer de l'estomac, le cancer de l'œsophage, le cancer du pancréas, le cancer du foie, le cancer du poumon à petites cellules (CPPC), le cancer du poumon non à petites cellules (CPNPC), le cancer squameux de la tête et du cou, le mésothéliome, le mélanome, le gliome, le glioblastome, les tumeurs neuroendocrines du pancréas, le carcinome basocellulaire, le carcinome épidermoïde, le carcinome à cellules rénales, le carcinome canalaire invasif, l'adénocarcinome, le carcinome à cellules de Merkel, le cancer de la peau, le cancer de la prostate, le cancer colorectal, le sarcome des tissus mous, l'ostéosarcome, le sarcome de Ewing, le chondrosarcome et le myélome ;
la maladie neurodégénérative est choisie dans le groupe constitué de la maladie d'Alzheimer, la maladie de Parkinson, la sclérose latérale amyotrophique, l'ataxie de Friedreich, la maladie de Huntington, la démence à corps de Lewy et l'atrophie musculaire spinale ; et
la maladie cardiovasculaire est l'athérosclérose.
